(19) Europäisches Patentamt
European Patent Office
Office européen des brevets

(11) EP 4 269 399 A1

(12) **EUROPEAN PATENT APPLICATION**
published in accordance with Art. 153(4) EPC

(43) Date of publication:
**01.11.2023 Bulletin 2023/44**

(21) Application number: **21909567.6**

(22) Date of filing: **24.12.2021**

(51) International Patent Classification (IPC):
***C07D 401/04*** (2006.01) ***C07D 513/04*** (2006.01)
***C07D 413/14*** (2006.01) ***C07D 495/04*** (2006.01)
***C07D 405/14*** (2006.01) ***A61K 31/497*** (2006.01)
***A61P 3/10*** (2006.01)

(52) Cooperative Patent Classification (CPC):
**A61K 31/497; A61P 3/10; C07D 401/04; C07D 405/14; C07D 413/14; C07D 495/04; C07D 513/04**

(86) International application number:
**PCT/CN2021/141243**

(87) International publication number:
**WO 2022/135572 (30.06.2022 Gazette 2022/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.12.2020 CN 202011557297**
**27.01.2021 CN 202110109531**
**16.04.2021 CN 202110403365**
**02.09.2021 CN 202111000175**

(71) Applicant: **Sichuan Haisco Pharmaceutical Co., Ltd.**
**Sichuan 611130 (CN)**

(72) Inventors:
- **ZHANG, Chen**
**Chengdu, Sichuan 611130 (CN)**
- **LEI, Ming**
**Chengdu, Sichuan 611130 (CN)**
- **ZHAO, Mingliang**
**Chengdu, Sichuan 611130 (CN)**
- **YU, Yan**
**Chengdu, Sichuan 611130 (CN)**
- **TANG, Pingming**
**Chengdu, Sichuan 611130 (CN)**
- **WENG, Guanglin**
**Chengdu, Sichuan 611130 (CN)**
- **MOU, Tao**
**Chengdu, Sichuan 611130 (CN)**
- **LI, Yao**
**Chengdu, Sichuan 611130 (CN)**
- **NI, Jia**
**Chengdu, Sichuan 611130 (CN)**
- **YAN, Pangke**
**Chengdu, Sichuan 611130 (CN)**

(74) Representative: **Hoffmann Eitle**
**Patent- und Rechtsanwälte PartmbB**
**Arabellastraße 30**
**81925 München (DE)**

(54) **FIVE-MEMBERED RING DERIVATIVE AND MEDICAL USE THEREOF**

(57) A compound represented by general formula (I) or a stereoisomer, tautomer, deuterated substance, solvate, prodrug, metabolite, pharmaceutically acceptable salt or co-crystal thereof, an intermediate thereof and a preparation method therefor, as well as an application in preparation of a drug for treating diabetes.

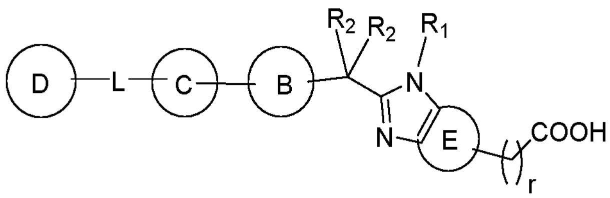

(I)

EP 4 269 399 A1

## Description

### Technical Field

The present invention relates to a compound represented by general formula (I) or a stereoisomer, tautomer, deuterated substance, solvate, prodrug, metabolite, pharmaceutically acceptable salt or co-crystal thereof, an intermediate thereof, and a preparation method therefor, as well as an application in the preparation of a drug for treating diabetes.

### Background Art

Diabetes is a group of metabolic diseases characterized by hyperglycemia. Hyperglycemia is caused by defective insulin secretion or impaired insulin biological action, or both. The long-term high blood sugar in diabetes leads to chronic damage and dysfunction of various tissues, especially eyes, kidneys, heart, blood vessels, and nerves. Diabetes is mainly divided into two types. Type 1 diabetes: destruction of islet B cells leads to absolute insulin deficiency. Type 2 diabetes: insulin resistance is dominant with insulin relative deficiency or impaired insulin secretion is dominant with insulin resistance.

Drugs for type 2 diabetes can be divided into six major classes (insulin, insulin secretagogues, biguanides, glucosidase inhibitors, thiazolidinediones, SGLT2 inhibitors), each of which works through a different primary mechanism. However, with the exception of GLP-1 receptor agonists and SGLT2 inhibitors, these drugs have limited efficacy and cannot address the most important problems, namely decreased cellular function and associated obesity.

GLP-1 is a 30-amino acid long intestinal insulin stimulating hormone secreted by L cells in the intestine. GLP-1 stimulates insulin secretion, reduces glucagon secretion, inhibits gastric emptying, reduces appetite, and stimulates β-cell proliferation in a physiological and glucose-dependent manner. In nonclinical experiments, GLP-1 promotes the sustainability of β cells by stimulating the transcription of genes important for glucose-dependent insulin secretion and by promoting β cell regeneration. In healthy individuals, GLP-1 plays an important role in the regulation of postprandial blood, leading to increased peripheral glucose uptake by stimulating glucose-dependent insulin secretion from the pancreas. GLP-1 also inhibits glucagon secretion, resulting in decreased hepatic glucose output. In addition, GLP-1 delays gastric emptying, slows small bowel motility, and delays food absorption.

GLP-1 receptor agonists, such as GLP-1, liraglutide and exendin-4, are polypeptide drugs and are mostly used for injection. Small molecule GLP-1 receptor agonists have become a hot spot in drug development in recent years due to their high oral bioavailability potential.

### Summary of the Invention

The object of the present invention is to provide a compound capable of stimulating a GLP-1 receptor or a stereoisomer, tautomer, deuterated substance, solvate, prodrug, metabolite, pharmaceutically acceptable salt or co-crystal thereof, and an intermediate and a preparation method thereof, as well as an application in the preparation of a drug for treating diabetes.

The compound of the present invention has good activity of stimulating a GLP-1 receptor, good pharmacokinetic performance and bioavailability, oral performance and good safety.

The present invention provides a compound or a stereoisomer, tautomer, deuterated substance, solvate, prodrug, metabolite, pharmaceutically acceptable salt or co-crystal thereof, and the compound is selected from a compound represented by general formula (I), wherein,

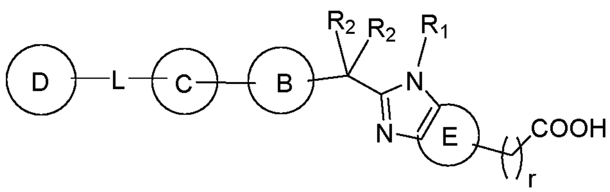

(I)

In some embodiments, ring B is selected from a 4- to 12-membered heterocyclic ring, $C_{5-12}$ carbocyclic ring, $C_{6-10}$ aromatic ring or a 5- to 12-membered heteroaromatic ring. The carbocyclic ring, heterocyclic ring, aromatic ring or heteroaromatic ring is optionally further substituted with 0 to 4 (for example, 0, 1, 2, 3 or 4) $R_B$, the heterocyclyl or heteroaromatic ring contains 1 to 3 (for example 1, 2 or 3) heteroatoms selected from O, S and N.

In some embodiments, ring B is selected from a benzene ring, a 4- to 8-membered heterocyclyl, $C_{5-8}$ carbocyclyl or a 5- to 6-membered heteroaromatic ring. The benzene ring, carbocyclyl, heterocyclyl or heteroaromatic ring is optionally

further substituted with 0 to 4 (for example 0, 1, 2, 3 or 4) $R_B$, the heterocyclic ring contains 1 to 3 (for example, 1, 2 or 3) heteroatoms selected from O, S and N.

In some embodiments, ring B is selected from one of the following substituted or unsubstituted groups: cyclohexyl, cyclohexenyl, azacyclohexenyl, piperidinyl, phenyl, pyrazolyl, pyridyl, pyridazinyl, pyrimidinyl, pyrazinyl or triazinyl, which, when substituted, is optionally further substituted with 0 to 4 (for example, 0, 1, 2, 3 or 4) $R_B$.

In some embodiments, ring B is selected from one of the following substituted or unsubstituted groups: pyrimidin-4(*3H*)-one (that is, the ortho position of the pyrimidine N atom is substituted with a hydroxyl group, and pyrimidin-4(*3H*)-one is formed through tautomerism), pyridazine-3(*2H*)-one (that is, the ortho position of the pyridazine N atom is substituted with a hydroxyl group, and pyridazine-3(*2H*)-one is formed through tautomerism), pyridine-2(*1H*)-one (that is, the ortho position of the pyridine N atom is substituted with a hydroxyl group, and pyridin-2(*1H*)-one is formed through tautomerism).

In some embodiments, ring B is selected from one of the following substituted or unsubstituted groups: piperazinyl, tetrahydropyrrolyl or 1,4-diazepanyl, which, when substituted, is optionally further substituted with 0 to 4 (for example, 0, 1, 2, 3 or 4) $R_B$.

In some embodiments, ring B is selected from one of the following substituted or unsubstituted groups:

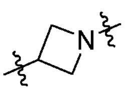, 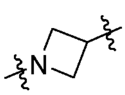, 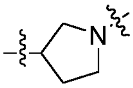, 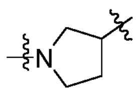, 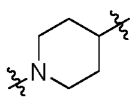,

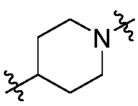, 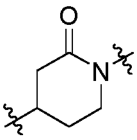, 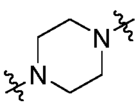, 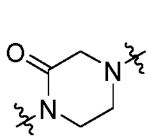, 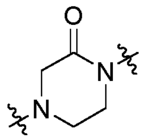, 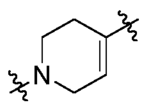, 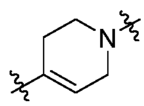,

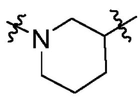, 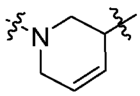, 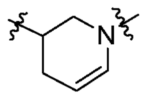, 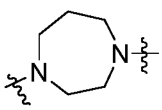,

the left side of which is directly connected to ring C, and which, when substituted, is optionally further substituted with 1, 2 or 3 $R_B$.

In some embodiments, ring B is selected from one of the following substituted or unsubstituted groups:

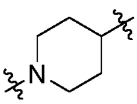, 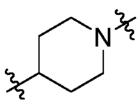, 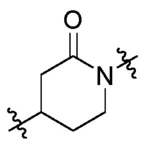, 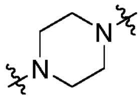, 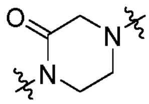,

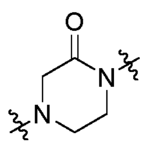 or 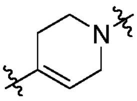

the left side of which is directly connected to ring C, and which, when substituted, is optionally further substituted with 1, 2 or 3 $R_B$.

In some embodiments, ring B is selected from one of the following substituted or unsubstituted groups:

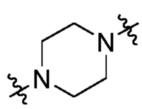, 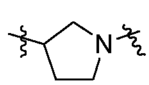 or 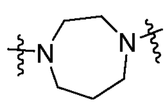,

the left side of which is directly connected to ring C, and which, when substituted, is optionally further substituted with

1, 2 or 3 $R_B$.

In some embodiments, ring B is selected from one of the following substituted or unsubstituted groups:

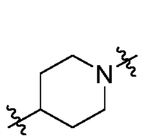, 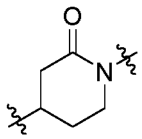, 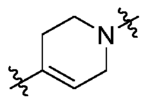, 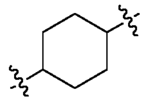, 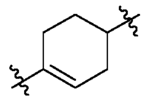,

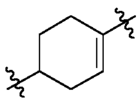 or 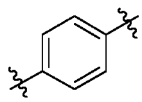,

the left side of which is directly connected to ring C, and which, when substituted, is optionally further substituted with 1, 2 or 3 $R_B$.

In some embodiments, ring B is selected from one of the following substituted or unsubstituted groups:

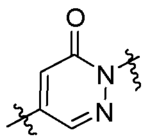 or 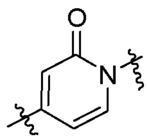,

the left side of which is directly connected to ring C, and which, when substituted, is optionally further substituted with 1, 2 or 3 $R_B$.

In some embodiments, ring B is selected from one of the following substituted or unsubstituted groups:

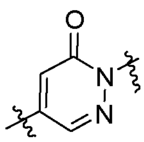, 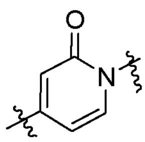, 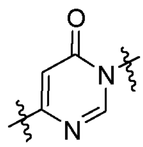, 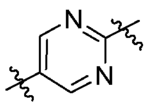, 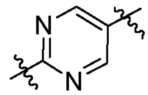,

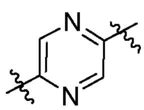, 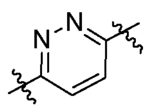, 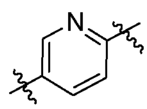 or 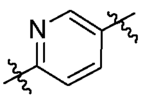,

the left side of which is directly connected to ring C, and which, when substituted, is optionally further substituted with 1, 2 or 3 $R_B$.

In some embodiments,

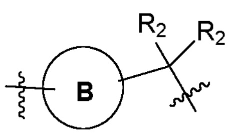

is selected from

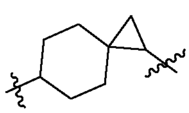

or

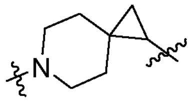,

the left side of which is directly connected to ring C, and which, when the 6-membered ring in the spiro ring is substituted, is optionally further substituted with 1, 2 or 3 $R_B$.

In some embodiments, ring B is selected from one of the following substituted or unsubstituted groups:

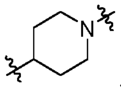, 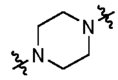, 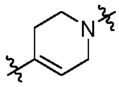, 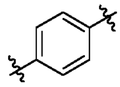 or 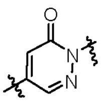,

the left side of which is directly connected to ring C, and which, when substituted, is optionally further substituted with 1, 2 or 3 $R_B$.

In some embodiments, ring C is selected from $C_{6-10}$ carbocyclic ring, a 5- to 10-membered heterocyclic ring, $C_{6-10}$ aromatic ring or a 5- to 10-membered heteroaromatic ring. The carbocyclic ring, heterocyclic ring, aromatic ring or heteroaromatic ring is optionally further substituted with 0 to 4 (for example, 0, 1, 2, 3 or 4) Rc, the heterocyclic ring or heteroaromatic ring contains 1 to 5 (for example, 1, 2, 3, 4 or 5) heteroatoms selected from O, S and N.

In some embodiments, ring C is selected from one of the following substituted or unsubstituted groups: A benzene ring, a pyrrole ring, a pyrazole ring, a pyridine ring, a furan ring, a thiophene ring, an imidazole ring, a thiazole ring, a oxazole ring, an isothiazole ring, an isoxazole ring, a triazole ring, a tetrazole ring, a oxadiazole ring, a thiadiazole ring, a pyridazine ring, a pyrimidine ring, a pyrazine ring or a triazine ring, which, when substituted, is optionally further substituted with 0 to 4 (for example, 0, 1, 2, 3 or 4) Re.

In some embodiments, ring C is selected from a benzene ring, a 5- to 6-memberedmonocyclic heteroaromatic ring, a 5-membered fused 5-membered heteroaromatic ring, a 5-membered fused 6-membered heteroaromatic ring or a 6-membered fused 6-membered heteroaromatic ring. The benzene ring or heteroaromatic ring is optionally further substituted with 0 to 4 (for example, 0, 1, 2, 3 or 4) Rc, the heteroaromatic ring contains 1 to 5 (for example, 1, 2, 3, 4 or 5) heteroatoms selected from O, S and N.

In some embodiments, ring C is selected from one of the following substituted or unsubstituted groups: a benzene ring,

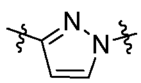, 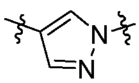, 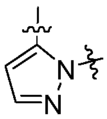, 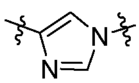,

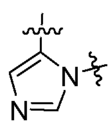, 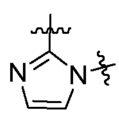, 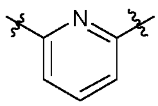, 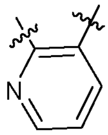, 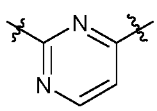, 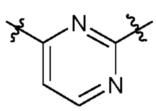, 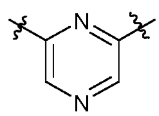,

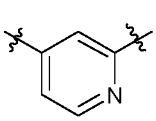, 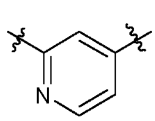 or 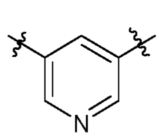,

which, when substituted, is optionally further substituted with 1, 2 or 3 $R_C$, and the left side of which is connected to L.

In some embodiments, ring C is selected from one of the following substituted or unsubstituted groups:

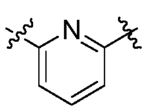 or 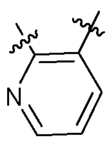,

which, when substituted, is optionally further substituted with 1, 2 or 3 Rc, and the left side of which is connected to L.

In some embodiments,

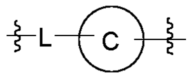

is selected from

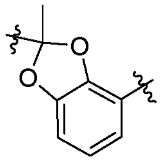

, the right side of which is connected to ring B, and in which the benzene ring is optionally further substituted with 1, 2 or 3 $R_C$.

In some embodiments, L is selected from a bond, O, S, $-NR_L-$, $-C(R_L)_2-$, $-C(R_L)_2-C(R_L)_2-$, $-Y-C(R_L)_2-$, $-C(R_L)_2-Y-$, $-Y-C(R_L)_2-C(R_L)_2-$, $-C(R_L)_2-C(R_L)_2-Y-$ or $-C(R_L)_2-Y-C(R_L)_2-$.

In some embodiments, L is selected from a bond, O, S, $-NR_L-$, $-CHR_L-$, $-CHR_L-CHR_L-$, $-Y-CHR_L-$, $-CHR_L-Y-$, $-Y-CHR_L-CHR_L-$, $-CHR_L-CHR_L-Y-$ or $-CHR_L-Y-CHR_L-$.

In some embodiments, L is selected from $-Y-CHR_L-$, $-CHR_L-Y-$, $-CHR_L-CHR_L-$, $-CHR_L-CHR_L-Y-$ or $-Y-CHR_L-CHR_L-$.

In some embodiments, Y is selected from O, S or $-NR_L-$.

In some embodiments, L is selected from $-CH_2O-$, $-OCH_2-$, $-CH_2NH-$, $-NHCH_2-$, $-CH_2CH_2-$, $-OCH_2CH_2-$, $-CH_2CH_2O-$, $-NHCH_2CH_2-$ or $-CH_2CH_2NH-$, and the right side of L is connected with ring C.

In some embodiments, L is selected from $-CH_2O-$, and the right side of L is connected to ring C.

In some embodiments, ring D is selected from a 6- to 10-membered aromatic ring or a 5- to 12-membered heteroaromatic ring. The aromatic ring or heteroaromatic ring is optionally further substituted with 0 to 5 $R_D$. The heteroaromatic ring contains 1 to 5 (for example, 1, 2, 3, 4 or 5) heteroatoms selected from O, S and N.

In some embodiments, ring D is selected from a benzene ring, a naphthalene ring, a 5- to 6-membered monocyclic heteroaromatic ring, a 5-membered fused 5-membered heteroaromatic ring, a 5-membered fused 6-membered heteroaromatic ring or a 6-membered fused 6-membered heteroaromatic ring. The benzene ring, naphthalene ring or heteroaromatic ring is optionally further substituted with 0 to 4 (for example, 0, 1, 2, 3 or 4) $R_D$, the heteroaromatic ring contains 1 to 5 (for example, 1, 2, 3, 4 or 5) heteroatoms selected from O, S and N.

In some embodiments, ring D is selected from a benzene ring, a naphthalene ring, a pyrrole ring, a pyrazole ring, a pyridine ring, a furan ring, a thiophene ring, an imidazole ring, a thiazole ring, a oxazole ring, an isothiazole ring, an isoxazole ring, a triazole ring, a oxadiazole ring, a thiadiazole ring, a pyridazine ring, a pyrimidine ring, a pyrazine ring or a triazine ring, which, when substituted, is optionally further substituted with 0 to 4 (for example, 0, 1, 2, 3 or 4) $R_D$.

In some embodiments, ring D is selected from a benzene ring or a pyridine ring, and the benzene ring or pyridine ring is optionally further substituted with 1, 2 or 3 $R_D$.

In some embodiments, ring D is selected from a substituted or unsubstituted benzene ring, and when substituted, is optionally further substituted with 1, 2 or 3 $R_D$.

In some embodiments, ring E is selected from a 5-membered heterocyclic ring, and the heterocyclic ring is optionally further substituted with 0 or 1 substituent selected from H, halogen, CN, OH, $NH_2$, $C_{1-6}$ alkyl, $C_{1-6}$ alkoxy or $C_{3-6}$ cycloalkyl. The heterocyclic ring contains 1 to 2 heteroatoms selected from O, S and N.

In some embodiments, r is selected from 0 or 1.

In some embodiments, r=1,

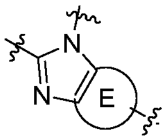

is selected from

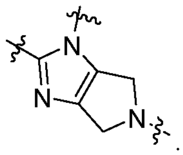

In some embodiments, r=0,

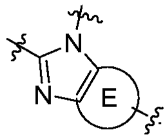

is selected from

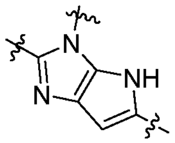,

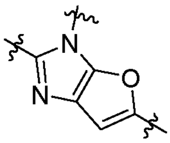, 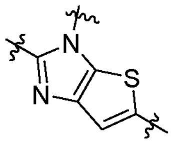, 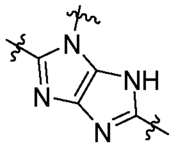, 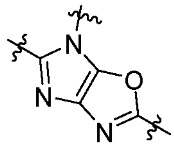, 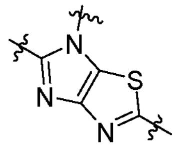, 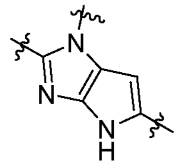,

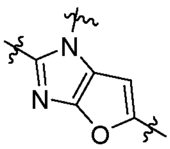, 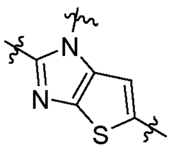, 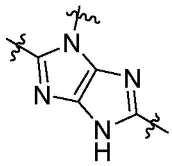, 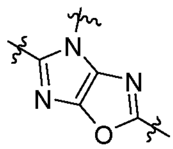, or 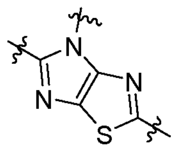.

In some embodiments, r=0,

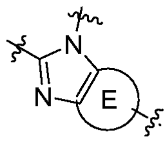

is selected from

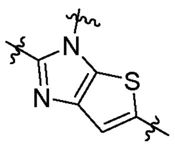,

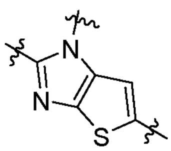, or 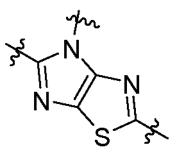.

In some embodiments, ring E is selected from a 5-membered non-aromatic heterocyclic ring or a 5-membered heteroaromatic ring, and the heterocyclic ring or heteroaromatic ring is optionally further substituted with 0 or 1 substituent selected from H, halogen, CN, OH, $NH_2$, $C_{1-4}$ alkyl, $C_{1-4}$ alkoxy or $C_{3-6}$ cycloalkyl, the heterocyclic ring or heteroaromatic ring contains 1 to 2 heteroatoms selected from O, S and N.

In some embodiments, $R_1$ is selected from H, halogen, OH, -SH, $CF_3$, CN, $C_{1-6}$ alkyl, $C_{1-6}$ alkoxy, -$C_{1-3}$ alkylene-Z-$C_{0-3}$ alkylene-$R_{1a}$, -$C_{0-4}$ alkylene-$R_{1a}$. The alkyl, alkoxy, and alkylene are optionally further substituted with 0 to 4 (such as 0, 1 , 2, 3 or 4) substituents selected from H, halogen, =O, CN, OH, -$N(R_{1b})_2$, $C_{1-6}$ alkyl, halogen-substituted $C_{1-6}$ alkyl, hydroxy-substituted $C_{1-6}$ alkyl, cyano-substituted $C_{1-6}$ alkyl, $C_{1-6}$ alkoxy, $C_{3-6}$ cycloalkyl, a 3- to 6 -membered heterocycloalkyl, a 5- to 10-membered heteroaryl. The heterocycloalkyl or heteroaryl contains 1 to 3 (for example, 1, 2 or 3) heteroatoms selected from O, S and N.

In some embodiments, $R_1$ is selected from H, halogen, OH, -SH, $CF_3$, CN, $C_{1-4}$ alkyl, $C_{1-4}$ alkoxy, -$C_{1-2}$ alkylene-Z-$C_{0-2}$ alkylene-$R_{1a}$, -$C_{0-4}$ alkylene-$R_{1a}$. The alkyl, alkoxy, and alkylene are optionally further substituted with 0 to 4 (such as 0, 1 , 2, 3 or 4) substituents selected from H, halogen, =O, CN, OH, -$N(R_{1b})_2$, $C_{1-4}$ alkyl, halogen-substituted $C_{1-4}$ alkyl, hydroxy-substituted $C_{1-4}$ alkyl, cyano-substituted $C_{1-4}$ alkyl, $C_{1-4}$ alkoxy, $C_{3-6}$ cycloalkyl, a 3- to 6 -membered heterocycloalkyl, a 5- to 6-membered heteroaryl. The heterocycloalkyl or heteroaryl contains 1 to 3 (for example, 1, 2 or 3) heteroatoms selected from O, S and N.

In some embodiments, $R_1$ is selected from H, F, Cl, OH, CN, $CF_3$, methyl, ethyl, propyl, methoxy, ethoxy, propoxy, isopropoxy, -ethylene-Z-methylene-$R_{1a}$, - ethylene-Z-ethylene-$R_{1a}$, -ethylene-Z-$R_{1a}$, -$R_{1a}$, -methylene-$R_{1a}$, -ethylene-$R_{1a}$. The methyl, ethyl, propyl, methoxy, ethoxy, methylene, ethylene are optionally further substituted with 0 to 3 (for example 0, 1, 2 or 3) substituents selected from H, F, Cl, =O, CN, OH, -$N(R_{1b})_2$, methyl, ethyl, propyl, $CF_3$, -$CH_2F$, -$CHF_2$, 1 to 3 F-substituted ethyl, hydroxymethyl, hydroxyethyl, cyano-substituted methyl, cyano-substituted ethyl, methoxy, ethoxy, isopropoxy, cyclopropyl, cyclobutyl, cyclopentyl, cyclohexyl, oxiranyl, aziridinyl, oxetanyl, azetidinyl, tetrahydrofuranyl, tetrahydro-2*H*-pyranyl, dioxolanyl, dioxanyl, pyrrolidinyl, piperidinyl, imidazolidinyl, oxazolidinyl, oxazinanyl, morpholinyl, hexahydropyrimidinyl, piperazinyl, pyrrolyl, pyridyl, pyrazolyl, triazolyl, tetrazolyl, imidazolyl, thiazolyl, thienyl, furyl, oxazolyl, isoxazolyl, oxadiazolyl, isothiazolyl, pyrimidinyl, pyrazinyl or pyridazinyl.

In some embodiments, $R_1$ is selected from H, CN, $CF_3$, $CHF_2$, $CH_2F$, -$CH_2OH$, -$CH(OH)CH_3$, methyl, ethyl, methoxy, ethoxy, propoxy, isopropoxy, methoxymethyl, methoxyethyl, ethoxymethyl, isopropoxymethyl, -ethylene-Z-methylene-$R_{1a}$, -ethylene-Z-ethylene-$R_{1a}$, -ethylene-Z-$R_{1a}$, -$R_{1a}$ or -methylene-$R_{1a}$.

In some embodiments, $R_1$ is selected from -$CH_2CH_2OCH_3$,

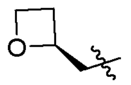,

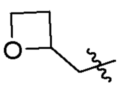, 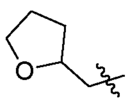, 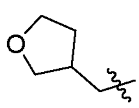, 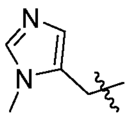, 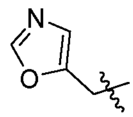, 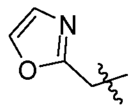, or 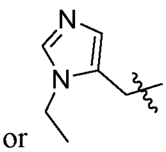.

In some embodiments, $R_1$ is selected from

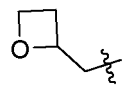, 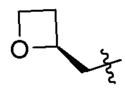, 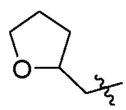

or

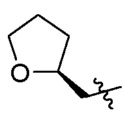.

In some embodiments, Z is selected from a bond, $N(R_{1b})$, O or S.

In some embodiments, Z is selected from NH, $N(CH_3)$ or O.

In some embodiments, $R_{1a}$ is selected from $C_{1-6}$ alkyl, $C_{1-6}$ alkoxy, $C_{3-8}$ cycloalkyl, a 3- to 8-membered heterocycloalkyl, a 6- to 10-membered aryl or a 5- to 12-membered heteroaryl. The cycloalkyl, heterocycloalkyl, aryl or heteroaryl is optionally further substituted with 0 to 4 (for example 0, 1, 2, 3 or 4) substituents selected from H, halogen, =O, OH, CN, $C_{1-6}$ alkyl, $C_{1-6}$ alkoxy or -N($R_{1b}$)$_2$. The alkyl or alkoxy is optionally further substituted with 0 to 4 (such as 0, 1, 2, 3 or 4) substituents selected from H, halogen, =O, CN, OH, -N($R_{1b}$)$_2$, $C_{1-6}$ alkyl, $C_{1-6}$ alkoxy or $C_{3-6}$ cycloalkyl. The heterocycloalkyl or heteroaryl contains 1 to 3 (for example, 1, 2 or 3) heteroatoms selected from O, S and N.

In some embodiments, $R_{1a}$ is selected from $C_{1-4}$ alkyl, $C_{1-4}$ alkoxy, $C_{3-6}$ cycloalkyl, a 3- to 6-membered heterocycloalkyl, phenyl or a 5- to 6-membered heteroaryl. The cycloalkyl, heterocycloalkyl, phenyl or heteroaryl is optionally further substituted with 0 to 4 (for example, 0, 1, 2, 3 or 4) substituents selected from H, halogen, =O, OH, CN, $C_{1-4}$ alkyl, $C_{1-4}$ alkoxy or -N($R_{1b}$)$_2$. The alkyl or alkoxy is optionally further substituted with 0 to 4 (such as 0, 1, 2, 3 or 4) substituents selected from H, halogen, =O, CN, OH, -N($R_{1b}$)$_2$, $C_{1-4}$ alkyl, $C_{1-4}$ alkoxy or $C_{3-6}$ cycloalkyl. The heterocycloalkyl or heteroaryl contains 1 to 3 (for example, 1, 2 or 3) heteroatoms selected from O, S and N.

In some embodiments, $R_{1a}$ is selected from methyl, ethyl, isopropyl, propyl, methoxy, ethoxy, propoxy, isopropoxy, cyclopropyl, cyclobutyl, cyclopentyl, cyclohexyl, oxiranyl, aziridinyl, oxetanyl, azetidinyl, tetrahydrofuranyl, tetrahydro-2*H*-pyranyl, dioxolanyl, dioxanyl, dioxanyl, pyrrolidinyl, piperidinyl, imidazolidinyl, oxazolidinyl, oxazinanyl, morpholinyl, hexahydropyrimidinyl, piperazinyl, pyrrolyl, pyridyl, pyrazolyl, triazolyl, tetrazolyl, imidazolyl, thiazolyl, thienyl, furyl, oxazolyl, isoxazolyl, oxadiazolyl, isothiazolyl, pyrimidinyl, pyrazinyl or phenyl. The $R_{1a}$ is optionally further substituted with 0 to 3 substituents selected from H, F, =O, OH, CN, methyl, ethyl, propyl, $CF_3$, -$CH_2F$, - $CHF_2$, 1 to 3 F-substituted ethyl, hydroxymethyl, hydroxyethyl, cyano-substituted methyl, cyano-substituted ethyl, methoxy, ethoxy or -N($R_{1b}$)$_2$.

In some embodiments, $R_{1a}$ is selected from methyl, ethyl, isopropyl, propyl, methoxy, ethoxy, propoxy, isopropoxy, cyclopropyl, cyclobutyl, cyclopentyl, cyclohexyl, oxiranyl, oxetanyl, azetidinyl, tetrahydrofuranyl, tetrahydro-2*H*-pyranyl, pyrrolidinyl, piperidinyl, pyrazolyl, 1,2,3-triazolyl, 1,2,4-triazolyl, imidazolyl, thiazolyl, thienyl, furyl, oxazolyl, isoxazolyl, 1,2,4-oxadiazolyl, isothiazolyl, pyrimidyl, pyridine or phenyl. The $R_{1a}$ is optionally further substituted with 0 to 3 (such as 0, 1, 2 or 3 ) substituents selected from H, F, CN, methyl, ethyl, propyl, $CF_3$, methoxy or ethoxy.

In some embodiments, each $R_{1b}$ is independently selected from H or $C_{1-6}$ alkyl; the alkyl is optionally further substituted with 0 to 3 (such as 0, 1, 2 or 3) substituents selected from H, halogen, =O, CN, OH, $NH_2$, $C_{1-6}$ alkyl, $C_{1-6}$ alkoxy or $C_{3-6}$ cycloalkyl.

In some embodiments, each $R_{1b}$ is independently selected from H or $C_{1-4}$ alkyl which alkyl is optionally further substituted with 0 to 3 (such as 0, 1, 2 or 3) substituents selected from H, halogen, =O, CN, OH, $NH_2$, $C_{1-4}$ alkyl, $C_{1-4}$ alkoxy or $C_{3-6}$ cycloalkyl.

In some embodiments, each $R_{1b}$ is independently selected from H, methyl or ethyl.

In some embodiments, each $R_L$ is independently selected from H, halogen, - SH, CN, OH, $CF_3$, $NH_2$, $C_{1-6}$ alkyl, $C_{1-6}$ alkoxy, $C_{3-6}$ cycloalkyl. The alkyl, cycloalkyl or alkoxy is optionally further substituted with 0 to 4 (for example, 0, 1, 2, 3 or 4) substituents selected from H, halogen, =O, OH, CN, $NH_2$, $C_{1-6}$ alkyl, $C_{3-6}$ cycloalkyl or $C_{1-6}$ alkoxy.

In some embodiments, each $R_L$ is independently selected from H, halogen, CN, OH, -SH, $CF_3$, $NH_2$, $C_{1-4}$ alkyl, $C_{1-4}$ alkoxy, $C_{3-6}$ cycloalkyl. The alkyl, cycloalkyl or alkoxy is optionally further substituted with 0 to 4 (for example, 0, 1, 2, 3 or 4) substituents selected from H, halogen, =O, OH, CN, $NH_2$, $C_{1-4}$ alkyl, $C_{3-6}$ cycloalkyl or $C_{1-4}$ alkoxy.

In some embodiments, each $R_L$ is independently selected from H, F, Cl, CN, $CF_3$, OH, $NH_2$, methyl, ethyl, propyl, $CF_3$, -$CH_2F$, -$CHF_2$, 1 to 3 F-substituted ethyl, hydroxymethyl, hydroxyethyl, cyano-substituted methyl, cyano-substituted ethyl, methoxy, ethoxy, cyclopropyl, cyclobutyl or cyclopentyl.

In some embodiments, each $R_L$ is independently selected from H, F, methyl, ethyl or propyl.

In some embodiments, each $R_L$ is independently selected from H or methyl.

In some embodiments, $R_2$ is selected from H, halogen or $C_{1-6}$alkyl.

In some embodiments, $R_2$ is selected from H, halogen or $C_{1-4}$alkyl.

In some embodiments, $R_2$ is selected from H.

In some embodiments, $R_2$ is selected from H, F, methyl or ethyl.

In some embodiments, each $R_B$, $R_C$ or $R_D$ is independently selected from H, halogen, =O, CN, $CF_3$, OH, -SH, $NH_2$, $C_{1-6}$ alkyl, $C_{3-6}$ cycloalkyl or $C_{1-6}$ alkoxy. The alkyl, cycloalkyl or alkoxy is optionally further substituted with 0 to 4 (for example, 0, 1, 2, 3 or 4) substituents selected from H, halogen, =O, OH, CN, $NH_2$, $C_{1-6}$ alkyl, $C_{3-6}$ cycloalkyl or $C_{1-6}$ alkoxy.

In some embodiments, each $R_B$, $R_C$ or $R_D$ is independently selected from H, halogen, =O, CN, $CF_3$, OH, -SH, $NH_2$, $C_{1-4}$ alkyl, $C_{3-6}$ cycloalkyl or $C_{1-4}$ alkoxy. The alkyl, cycloalkyl or alkoxy is optionally further substituted with 0 to 4 (for example, 0, 1, 2, 3 or 4) substituents selected from H, halogen, =O, OH, CN, $NH_2$, $C_{1-4}$ alkyl, $C_{3-6}$ cycloalkyl or $C_{1-4}$ alkoxy.

In some embodiments, each $R_B$, $R_C$ or $R_D$ is independently selected from H, halogen, =O, CN, OH, -SH, $NH_2$, methyl, ethyl, propyl, isopropyl, methoxy, ethoxy, propoxy, isopropoxy, tert-butyloxy, butoxy, cyclopropyl or cyclobutyl. The methyl, ethyl, propyl, isopropyl, methoxy, ethoxy, propoxy, isopropoxy, tert-butyloxy, butoxy, cyclopropyl or cyclobutyl is optionally further substituted with 0 to 4 (for example 0, 1, 2, 3 or 4) substituents selected from H, halogen, =O, OH, CN, $NH_2$, $C_{1-4}$ alkyl, $C_{3-6}$ cycloalkyl or $C_{1-4}$ alkoxy substituents.

In some embodiments, each $R_B$, $R_C$ or $R_D$ is independently selected from H, F, Cl, =O, CN, $CF_3$, OH, $NH_2$, methyl, ethyl, propyl, $-CH_2F$, $-CHF_2$, 1 to 3 F-substituted ethyl, hydroxymethyl, hydroxyethyl, cyano-substituted methyl, cyano-substituted ethyl, methoxy, ethoxy, propoxy, isopropoxy, tert-butyloxy, trifluoromethoxy, cyclopropyl, cyclobutyl or cyclopentyl.

In some embodiments, each $R_B$ is independently selected from H, =O, F, OH, CN, $CF_3$, methyl, ethyl, propyl, cyclopropyl, cyclobutyl, cyclopentyl, methoxy or ethoxy.

In some embodiments, each Rc is independently selected from H, F, Cl, OH, CN, $NH_2$, $CF_3$, methyl, ethyl, propyl, cyclopropyl, cyclobutyl, cyclopentyl, methoxy or ethoxy.

In some embodiments, each $R_B$ is independently selected from H, =O, F, CN, $CF_3$, methyl, ethyl or cyclopropyl.

In some embodiments, each $R_B$ is independently selected from H, F, =O or methyl.

In some embodiments, each Rc is independently selected from H, F, $CF_3$ or methyl.

In some embodiments, each Rc is independently selected from H, F, Cl, CN, $CF_3$, methyl, ethyl, methoxy or ethoxy.

In some embodiments, each $R_D$ is independently selected from H, F, Cl, CN, $CF_3$, $CHF_2$, methyl, ethyl, methoxy, ethoxy, tert-butyloxy or trifluoromethoxy.

In some embodiments, each $R_B$ is independently selected from H, =O, F, CN, $CF_3$, methyl, ethyl or cyclopropyl.

In some embodiments, each Rc is independently selected from H, F, Cl, CN, $CF_3$, methyl, ethyl, methoxy or ethoxy.

In some embodiments, each $R_D$ is independently selected from H, F, Cl, CN, $CF_3$, $CHF_2$, methyl or ethyl.

In some embodiments, two $R_B$, two Rc, two $R_D$, $R_C$ and $R_L$, or $R_D$ and $R_L$ and the atoms attached thereto together form a $C_{3-10}$carbocyclic ring or a 3- to 10-membered heterocyclic ring. The carbocyclic ring or heterocyclic ring is optionally further substituted with 0 to 4 (for example, 0, 1, 2, 3 or 4) substituents selected from H, halogen, =O, OH, CN, $NH_2$, $C_{1-6}$ alkyl, $C_{3-6}$ cycloalkyl or $C_{1-6}$ alkoxy. The alkyl, cycloalkyl or alkoxy is optionally further substituted with 0 to 4 (for example 0, 1, 2, 3 or 4) substituents selected from H, halogen, =O, OH, CN, $NH_2$, $C_{1-6}$ alkyl, $C_{3-6}$ cycloalkyl or $C_{1-6}$ alkoxy. The heterocyclic ring contains 1 to 3 (for example, 1, 2 or 3) heteroatoms selected from O, S and N.

In some embodiments, two $R_B$, two Rc, two $R_D$, $R_C$ and $R_L$, or $R_B$ and $R_2$ and the atoms attached thereto together form a $C_{3-7}$carbocyclic ring or a 4- to 7-membered heterocyclic ring. The carbocyclic ring or heterocyclic ring is optionally further substituted with 0 to 4 (for example, 0, 1, 2, 3 or 4) substituents selected from H, halogen, =O, OH, CN, $NH_2$, $C_{1-4}$ alkyl, $C_{3-6}$ cycloalkyl or $C_{1-4}$ alkoxy. The alkyl, cycloalkyl or alkoxy is optionally further substituted with 0 to 4 (for example 0, 1, 2, 3 or 4) substituents selected from H, halogen, =O, OH, CN, $NH_2$, $C_{1-4}$ alkyl, $C_{3-6}$ cycloalkyl or $C_{1-4}$ alkoxy. The heterocyclic ring contains 1 to 3 (for example, 1, 2 or 3) heteroatoms selected from O, S and N.

In some embodiments, $R_C$ and $R_L$ together with the atoms attached thereto form a $C_{3-6}$ carbocyclic ring or a 4- to 7-membered heterocyclic ring. The carbocyclic ring or heterocyclic ring is further substituted with 0 to 4 (for example, 0, 1, 2, 3 or 4) substituents selected from H, halogen, =O, OH, CN, $NH_2$, $C_{1-4}$ alkyl, $C_{3-6}$ cycloalkyl or $C_{1-4}$ alkoxy. The alkyl, cycloalkyl or alkoxy is optionally further substituted with 0 to 4 (for example 0, 1, 2, 3 or 4) substituents selected from H, halogen, =O, OH, CN, $NH_2$, $C_{1-4}$ alkyl, $C_{3-6}$ cycloalkyl or $C_{1-4}$ alkoxy. The heterocyclic ring contains 1 to 3 (for example, 1, 2 or 3) heteroatoms selected from O, S and N.

In some embodiments, two $R_B$, or $R_L$ and $R_C$ are directly connected (between two $R_B$, or $R_L$ and $R_C$) to form $C_{4-6}$carbocyclic ring or a 4- to 7-membered heterocyclic ring. The carbocyclic ring or heterocyclic ring is optionally further substituted with 0 to 4 (for example, 0, 1, 2, 3 or 4) substituents selected from H, halogen, =O, OH, CN, $NH_2$, $C_{1-4}$ alkyl, $C_{3-6}$ cycloalkyl or $C_{1-4}$ alkoxy. The alkyl, cycloalkyl or alkoxy is optionally further substituted with 0 to 4 (for example 0, 1, 2, 3 or 4) substituents selected from H, halogen, =O, OH, CN, $NH_2$, $C_{1-4}$ alkyl, $C_{3-6}$ cycloalkyl or $C_{1-4}$ alkoxy. The heterocyclic ring contains 1 to 3 (for example, 1, 2 or 3) heteroatoms selected from O, S and N.

In some embodiments, $R_L$ and $R_C$ are directly connected to form a 4, 5, 6 or 7-membered heterocyclic ring. The heterocyclic ring is optionally further substituted with 0 to 4 (for example 0, 1, 2, 3 or 4) substituents selected from H, F, =O, OH, CN, $NH_2$, methyl, ethyl, isopropyl, cyclopropyl, cyclobutyl, cyclopentyl or methoxy. The heterocyclic ring contains 1 to 2 heteroatoms selected from O, S and N.

In some embodiments, $R_C$ and $R_L$ together with the atoms attached thereto form a 4- to 7-membered heterocyclic ring. The heterocyclic ring is further substituted with 0, 1, 2, 3 or 4 substituents selected from H, F, methyl or ethyl. The heterocyclic ring contains 1 to 2 heteroatoms selected from O, S and N.

In some embodiments, $R_B$ and $R_2$ together with the atoms attached thereto form a $C_{3-6}$ carbocyclic ring or a 4- to 7-membered heterocyclic ring. The carbocyclic ring or heterocyclic ring is further substituted with 0 to 4 (for example, 0, 1, 2, 3 or 4) substituents selected from H, halogen, =O, OH, CN, $NH_2$, $C_{1-4}$ alkyl, $C_{3-6}$ cycloalkyl or $C_{1-4}$ alkoxy. The alkyl, cycloalkyl or alkoxy is optionally further substituted with 0 to 4 (for example 0, 1, 2, 3 or 4) substituents selected from H, halogen, =O, OH, CN, $NH_2$, $C_{1-4}$ alkyl, $C_{3-6}$ cycloalkyl or $C_{1-4}$ alkoxy. The heterocyclic ring contains 1 to 3 (for example, 1, 2 or 3) heteroatoms selected from O, S and N.

As the first embodiment of the present invention, the compound represented by the above general formula (I) or a stereoisomer, tautomer, deuterated substance, solvate, prodrug, metabolite, pharmaceutically acceptable salt or

co-crystal, wherein,
Ring B is selected from a 4- to 12-membered heterocyclic ring, $C_{5-12}$ carbocyclic ring, $C_{6-10}$ aromatic ring or a 5- to 12-membered heteroaromatic ring. The carbocyclic ring, heterocyclic ring, aromatic ring or heteroaromatic ring is optionally further substituted with 0 to 4 (for example, 0, 1, 2, 3 or 4) $R_B$. The heterocyclyl or heteroaromatic ring contains 1 to 3 (for example 1, 2 or 3) heteroatoms selected from O, S and N.

Ring C is selected from $C_{6-10}$ carbocyclic ring, a 5- to 10-membered heterocyclic ring, $C_{6-10}$ aromatic ring or a 5- to 10-membered heteroaromatic ring. The carbocyclic ring, heterocyclic ring, aromatic ring or heteroaromatic ring is optionally further substituted with 0 to 4 (for example, 0, 1, 2, 3 or 4) Re. The heterocyclic ring or heteroaromatic ring contains 1 to 5 (for example, 1, 2, 3, 4 or 5) heteroatoms selected from O, S and N.

L is selected from a bond, O, S, $-NR_L-$, $-C(R_L)_2-$, $-C(R_L)_2-C(R_L)_2-$, $-Y-C(R_L)_2-$, $-C(R_L)_2-Y-$, $-Y-C(R_L)_2-C(R_L)_2-$, $-C(R_L)_2-C(R_L)_2-Y-$ or $-C(R_L)_2-Y-C(R_L)_2-$.

Y is selected from O, S or $-NR_L-$.

Ring D is selected from a 6- to 10-membered aromatic ring or a 5- to 12-membered heteroaromatic ring. The aromatic ring or heteroaromatic ring is optionally further substituted with 0 to 5 $R_D$. The heteroaromatic ring contains 1 to 5 (for example, 1, 2, 3, 4 or 5) heteroatoms selected from O, S and N.

Ring E is selected from a 5-membered heterocyclic ring, and the heterocyclic ring is optionally further substituted with 0 or 1 substituent selected from H, halogen, CN, OH, $NH_2$, $C_{1-6}$ alkyl, $C_{1-6}$ alkoxy or $C_{3-6}$ cycloalkyl. The heterocyclic ring contains 1 to 2 heteroatoms selected from O, S and N.

$R_1$ is selected from H, halogen, OH, -SH, $CF_3$, CN, $C_{1-6}$ alkyl, $C_{1-6}$ alkoxy, - $C_{1-3}$ alkylene-Z-$C_{0-3}$ alkylene-$R_{1a}$, -$C_{0-4}$ alkylene-$R_{1a}$ or $-N(R_{1b})_2$. The alkyl, alkoxy, and alkylene are optionally further substituted with 0 to 4 (such as 0, 1 , 2, 3 or 4) substituents selected from H, halogen, =O, CN, OH, $-N(R_{1b})_2$, $C_{1-6}$ alkyl, halogen-substituted $C_{1-6}$ alkyl, hydroxy-substituted $C_{1-6}$ alkyl, cyano-substituted $C_{1-6}$ alkyl, $C_{1-6}$ alkoxy, $C_{3-6}$ cycloalkyl, a 3- to 6 -membered heterocycloalkyl, a 5- to 10-membered heteroaryl. The heterocycloalkyl or heteroaryl contains 1 to 3 (for example, 1, 2 or 3) heteroatoms selected from O, S and N.

Z is selected from a bond, $N(R_{1b})$, O or S.

$R_{1a}$ is selected from $C_{1-6}$ alkyl, $C_{1-6}$ alkoxy, $C_{3-8}$ cycloalkyl, a 3- to 8-membered heterocycloalkyl, a 6- to 10-membered aryl or a 5- to 12-membered heteroaryl. The cycloalkyl, heterocycloalkyl, aryl or heteroaryl is optionally further substituted with 0 to 4 (for example 0, 1, 2, 3 or 4) substituents selected from H, halogen, =O, OH, CN, $C_{1-6}$ alkyl, $C_{1-6}$ alkoxy or $-N(R_{1b})_2$. The alkyl or alkoxy is optionally further substituted with 0 to 4 (such as 0, 1, 2, 3 or 4) substituents selected from H, halogen, =O, CN, OH, $-N(R_{1b})_2$, $C_{1-6}$ alkyl, $C_{1-6}$ alkoxy or $C_{3-6}$ cycloalkyl. The heterocycloalkyl or heteroaryl contains 1 to 3 (for example, 1, 2 or 3) heteroatoms selected from O, S and N.

Each $R_{1b}$ is independently selected from H or $C_{1-6}$ alkyl; the alkyl is optionally further substituted with 0 to 3 substituents selected from H, halogen, =O, CN, OH, $NH_2$, $C_{1-6}$ alkyl, $C_{1-6}$ alkoxy or $C_{3-6}$ cycloalkyl.

$R_2$ is selected from H, halogen or $C_{1-6}$alkyl.

Each $R_L$ is independently selected from H, halogen, -SH, CN, OH, $CF_3$, $NH_2$, $C_{1-6}$ alkyl, $C_{1-6}$ alkoxy, $C_{3-6}$ cycloalkyl. The alkyl, cycloalkyl or alkoxy is optionally further substituted with 0 to 4 (for example, 0, 1, 2, 3 or 4) substituents selected from H, halogen, =O, OH, CN, $NH_2$, $C_{1-6}$ alkyl, $C_{3-6}$ cycloalkyl or $C_{1-6}$ alkoxy.

Each $R_B$, $R_C$ or $R_D$ is independently selected from H, halogen, =O, CN, $CF_3$, OH, -SH, $NH_2$, $C_{1-6}$ alkyl, $C_{3-6}$ cycloalkyl or $C_{1-6}$ alkoxy. The alkyl, cycloalkyl or alkoxy is optionally further substituted with 0 to 4 (for example, 0, 1, 2, 3 or 4) substituents selected from H, halogen, =O, OH, CN, $NH_2$, $C_{1-6}$ alkyl, $C_{3-6}$ cycloalkyl or $C_{1-6}$ alkoxy.

Alternatively, two $R_B$, two Re, two $R_D$, $R_C$ and $R_L$, $R_D$ and $R_L$, or $R_B$ and $R_2$ and the atoms attached thereto together form a $C_{3-10}$carbocyclic ring or a 3- to 10-membered heterocyclic ring. The carbocyclic ring or heterocyclic ring is optionally further substituted with 0 to 4 (for example, 0, 1, 2, 3 or 4) substituents selected from H, halogen, =O, OH, CN, $NH_2$, $C_{1-6}$ alkyl, $C_{3-6}$ cycloalkyl or $C_{1-6}$ alkoxy. The alkyl, cycloalkyl or alkoxy is optionally further substituted with 0 to 4 (for example 0, 1, 2, 3 or 4) substituents selected from H, halogen, =O, OH, CN, $NH_2$, $C_{1-6}$ alkyl, $C_{3-6}$ cycloalkyl or $C_{1-6}$ alkoxy. The said heterocyclic ring contains 1 to 3 (for example, 1, 2 or 3) heteroatoms selected from O, S and N.
r is selected from 0 or 1.

As the second embodiment of the present invention, the compound represented by the above general formula (I) or a stereoisomer, tautomer, deuterated substance, solvate, prodrug, metabolite, pharmaceutically acceptable salt or co-crystal, wherein,

$R_1$ is selected from H, halogen, OH, -SH, $CF_3$, CN, $C_{1-4}$ alkyl, $C_{1-4}$ alkoxy, - $C_{1-2}$ alkylene-Z-$C_{0-2}$ alkylene-$R_{1a}$, -$C_{0-4}$ alkylene-$R_{1a}$. The alkyl, alkoxy, and alkylene are optionally further substituted with 0 to 4 (such as 0, 1 , 2, 3 or 4) substituents selected from H, halogen, =O, CN, OH, $-N(R_{1b})_2$, $C_{1-4}$ alkyl, halogen-substituted $C_{1-4}$ alkyl, hydroxy-substituted $C_{1-4}$ alkyl, cyano-substituted $C_{1-4}$ alkyl, $C_{1-4}$ alkoxy, $C_{3-6}$ cycloalkyl, a 3- to 6 - membered heterocycloalkyl, a 5- to 6-membered heteroaryl. The heterocycloalkyl or heteroaryl contains 1 to 3 (for example, 1, 2 or 3) heteroatoms selected from O, S and N.

$R_{1a}$ is selected from $C_{1-4}$ alkyl, $C_{1-4}$ alkoxy, $C_{3-6}$ cycloalkyl, a 3- to 6-membered heterocycloalkyl, phenyl or a 5- to 6-membered heteroaryl. The cycloalkyl, heterocycloalkyl, phenyl or heteroaryl is optionally further substituted with

0 to 4 (for example, 0, 1, 2, 3 or 4) substituents selected from H, halogen, =O, OH, CN, $C_{1-4}$ alkyl, $C_{1-4}$ alkoxy or $-N(R_{1b})_2$. The alkyl or alkoxy is optionally further substituted with 0 to 4 (such as 0, 1, 2, 3 or 4) substituents selected from H, halogen, =O, CN, OH, $-N(R_{1b})_2$, $C_{1-4}$ alkyl, $C_{1-4}$ alkoxy or $C_{3-6}$ cycloalkyl. The heterocycloalkyl or heteroaryl contains 1 to 3 (for example, 1, 2 or 3) heteroatoms selected from O, S and N.

Each $R_{1b}$ is independently selected from H or $C_{1-4}$ alkyl; the alkyl is optionally further substituted with 0 to 3 substituents selected from H, halogen, =O, CN, OH, $NH_2$, $C_{1-4}$ alkyl, $C_{1-4}$ alkoxy or $C_{3-6}$ cycloalkyl.

Each $R_L$ is independently selected from H, halogen, CN, OH, -SH, $CF_3$, $NH_2$, $C_{1-4}$ alkyl, $C_{1-4}$ alkoxy, $C_{3-6}$ cycloalkyl. The alkyl, cycloalkyl or alkoxy is optionally further substituted with 0 to 4 (for example, 0, 1, 2, 3 or 4) substituents selected from H, halogen, =O, OH, CN, $NH_2$, $C_{1-4}$ alkyl, $C_{3-6}$ cycloalkyl or $C_{1-4}$ alkoxy.

Each $R_B$, $R_C$ or $R_D$ is independently selected from H, halogen, =O, CN, $CF_3$, OH, -SH, $NH_2$, $C_{1-4}$ alkyl, $C_{3-6}$ cycloalkyl or $C_{1-4}$ alkoxy. The alkyl, cycloalkyl or alkoxy is optionally further substituted with 0 to 4 (for example, 0, 1, 2, 3 or 4) substituents selected from H, halogen, =O, OH, CN, $NH_2$, $C_{1-4}$ alkyl, $C_{3-6}$ cycloalkyl or $C_{1-4}$ alkoxy.

Alternatively, two $R_B$, two Re, two $R_D$, $R_C$ and $R_L$ or $R_B$ and $R_2$ and the atoms attached thereto together form a $C_{3-7}$carbocyclic ring or a 4- to 7-membered heterocyclic ring. The carbocyclic ring or heterocyclic ring is optionally further substituted with 0 to 4 (for example, 0, 1, 2, 3 or 4) substituents selected from H, halogen, =O, OH, CN, $NH_2$, $C_{1-4}$ alkyl, $C_{3-6}$ cycloalkyl or $C_{1-4}$ alkoxy. The alkyl, cycloalkyl or alkoxy is optionally further substituted with 0 to 4 (for example 0, 1, 2, 3 or 4) substituents selected from H, halogen, =O, OH, CN, $NH_2$, $C_{1-4}$ alkyl, $C_{3-6}$ cycloalkyl or $C_{1-4}$ alkoxy. The heterocyclic ring contains 1 to 3 (for example, 1, 2 or 3) heteroatoms selected from O, S and N.

The definitions of other groups are the same as those in the first embodiment.

As the third embodiment of the present invention, the compound represented by the above general formula (I) or a stereoisomer, tautomer, deuterated substance, solvate, prodrug, metabolite, pharmaceutically acceptable salt or co-crystal, wherein,

Ring B is selected from a benzene ring, a 4- to 8-membered heterocyclyl, $C_{5-8}$ carbocyclyl or a 5- to 6-membered heteroaromatic ring. The benzene ring, carbocyclyl, heterocyclyl or heteroaromatic ring is optionally further substituted with 0 to 4 (for example 0, 1, 2, 3 or 4) $R_B$, the heterocyclic ring contains 1 to 3 (for example, 1, 2 or 3) heteroatoms selected from O, S and N.

Ring C is selected from a benzene ring, a 5- to 6-membered monocyclic heteroaromatic ring, a 5-membered fused 5-membered heteroaromatic ring, a 5-membered fused 6-membered heteroaromatic ring or a 6-membered fused 6-membered heteroaromatic ring. The benzene ring or heteroaromatic ring is optionally further substituted with 0 to 4 (for example, 0, 1, 2, 3 or 4) Re, the heteroaromatic ring contains 1 to 5 (for example, 1, 2, 3, 4 or 5) heteroatoms selected from O, S and N.

Ring D is selected from a benzene ring, a naphthalene ring, a 5- to 6-membered monocyclic heteroaromatic ring, a 5-membered fused 5-membered heteroaromatic ring, a 5-membered fused 6-membered heteroaromatic ring or a 6-membered fused 6-membered heteroaromatic ring. The benzene ring, naphthalene ring or heteroaromatic ring is optionally further substituted with 0 to 4 (for example, 0, 1, 2, 3 or 4) $R_D$, the heteroaromatic ring contains 1 to 5 (for example, 1, 2, 3, 4 or 5) heteroatoms selected from O, S and N.

Ring E is selected from a 5-membered non-aromatic heterocyclic ring or a 5-membered heteroaromatic ring, and the heterocyclic ring or heteroaromatic ring is optionally further substituted with 0 or 1 substituent selected from H, halogen, CN, OH, $NH_2$, $C_{1-4}$ alkyl, $C_{1-4}$ alkoxy or $C_{3-6}$ cycloalkyl, the heterocyclic ring or heteroaromatic ring contains 1 to 2 heteroatoms selected from O, S and N.

$R_1$ is selected from H, F, Cl, OH, CN, $CF_3$, methyl, ethyl, propyl, methoxy, ethoxy, propoxy, isopropoxy, -ethylene-Z-methylene-$R_{1a}$, -ethylene-Z-ethylene-$R_{1a}$, - ethylene-Z-$R_{1a}$, $-R_{1a}$, -methylene-$R_{1a}$, -ethylene-$R_{1a}$. The methyl, ethyl, propyl, methoxy, ethoxy, methylene, ethylene are optionally further substituted with 0 to 3 substituents selected from H, F, Cl, =O, CN, OH, $-N(R_{1b})_2$, methyl, ethyl, propyl, $CF_3$, $-CH_2F$, $-CHF_2$, 1 to 3 F-substituted ethyl, hydroxymethyl, hydroxyethyl, cyano-substituted methyl, cyano-substituted ethyl, methoxy, ethoxy, isopropoxy, cyclopropyl, cyclobutyl, cyclopentyl, cyclohexyl, oxiranyl, aziridinyl, oxetanyl, azetidinyl, tetrahydrofuranyl, tetrahydro-2*H*-pyranyl, dioxolanyl, dioxanyl, pyrrolidinyl, piperidinyl, imidazolidinyl, oxazolidinyl, oxazinanyl, morpholinyl, hexahydropyrimidinyl, piperazinyl, pyrrolyl, pyridyl, pyrazolyl, triazolyl, tetrazolyl, imidazolyl, thiazolyl, thienyl, furyl, oxazolyl, isoxazolyl, oxadiazolyl, isothiazolyl, pyrimidinyl, pyrazinyl or pyridazinyl.

Z is selected from O, S or $N(R_{1b})$.

$R_{1a}$ is selected from methyl, ethyl, isopropyl, propyl, methoxy, ethoxy, propoxy, isopropoxy, cyclopropyl, cyclobutyl, cyclopentyl, cyclohexyl, oxiranyl, aziridinyl, oxetanyl, azetidinyl, tetrahydrofuranyl, tetrahydro-2*H*-pyranyl, dioxolanyl, dioxanyl, dioxanyl, pyrrolidinyl, piperidinyl, imidazolidinyl, oxazolidinyl, oxazinanyl, morpholinyl, hexahydropyrimidinyl, piperazinyl, pyrrolyl, pyridyl, pyrazolyl, triazolyl, tetrazolyl, imidazolyl, thiazolyl, thienyl, furyl, oxazolyl, isoxazolyl, oxadiazolyl, isothiazolyl, pyrimidinyl, pyrazinyl or phenyl. The $R_{1a}$ is optionally further substituted with 0 to 3 substituents selected from H, F, =O, OH, CN, methyl, ethyl, propyl, $CF_3$, $-CH_2F$, - $CHF_2$, 1 to 3 F-substituted ethyl, hydroxymethyl, hydroxyethyl, cyano-substituted methyl, cyano-substituted ethyl, methoxy, ethoxy or $-N(R_{1b})_2$.

Each $R_{1b}$ is independently selected from H, methyl or ethyl.

$R_2$ is selected from H, F, methyl or ethyl.

Each $R_L$ is independently selected from H, F, Cl, CN, $CF_3$, OH, $NH_2$, methyl, ethyl, propyl, $CF_3$, $-CH_2F$, $-CHF_2$, 1 to 3 F-substituted ethyl, hydroxymethyl, hydroxyethyl, cyano-substituted methyl, cyano-substituted ethyl, methoxy, ethoxy, cyclopropyl, cyclobutyl or cyclopentyl.

Each $R_B$, $R_C$ or $R_D$ is independently selected from H, F, Cl, =O, CN, $CF_3$, OH, $NH_2$, methyl, ethyl, propyl, $-CH_2F$, $-CHF_2$, 1 to 3 F-substituted ethyl, hydroxymethyl, hydroxyethyl, cyano-substituted methyl, cyano-substituted ethyl, methoxy, ethoxy, propoxy, isopropoxy, tert-butyloxy, trifluoromethoxy, cyclopropyl, cyclobutyl or cyclopentyl.

Alternatively, $R_B$ and $R_2$ together with the atoms attached thereto form a $C_{3-6}$ carbocyclic ring (for example, $C_3$, $C_4$, $C_5$ or $C_6$ carbocyclic ring) or a 4- to 7-membered (for example, 4, 5, 6 or 7-membered) heterocyclic ring. The carbocyclic ring or heterocyclic ring is further substituted with 0 to 4 (for example, 0, 1, 2, 3 or 4) substituents selected from H, halogen, =O, OH, CN, $NH_2$, $C_{1-4}$ alkyl, $C_{3-6}$ cycloalkyl or $C_{1-4}$ alkoxy. The alkyl, cycloalkyl or alkoxy is optionally further substituted with 0 to 4 (for example 0, 1, 2, 3 or 4) substituents selected from H, halogen, =O, OH, CN, $NH_2$, $C_{1-4}$ alkyl, $C_{3-6}$ cycloalkyl or $C_{1-4}$ alkoxy. The heterocyclic ring contains 1 to 3 (for example, 1, 2 or 3) heteroatoms selected from O, S and N.

Alternatively, $R_C$ and $R_L$ together with the atoms attached thereto form a $C_{3-6}$ carbocyclic ring (for example, $C_3$, $C_4$, $C_5$ or $C_6$ carbocyclic ring) or a 4- to 7-membered (for example, 4, 5, 6 or 7-membered) heterocyclic ring. The carbocyclic ring or heterocyclic ring is further substituted with 0 to 4 (for example, 0, 1, 2, 3 or 4) substituents selected from H, halogen, =O, OH, CN, $NH_2$, $C_{1-4}$ alkyl, $C_{3-6}$ cycloalkyl or $C_{1-4}$ alkoxy. The alkyl, cycloalkyl or alkoxy is optionally further substituted with 0 to 4 (for example 0, 1, 2, 3 or 4) substituents selected from H, halogen, =O, OH, CN, $NH_2$, $C_{1-4}$ alkyl, $C_{3-6}$ cycloalkyl or $C_{1-4}$ alkoxy. The heterocyclic ring contains 1 to 3 (for example, 1, 2 or 3) heteroatoms selected from O, S and N.

The definitions of other groups are the same as those in any one of the first and second embodiments.

As the fourth embodiment of the present invention, the compound represented by the above general formula (I) or a stereoisomer, tautomer, deuterated substance, solvate, prodrug, metabolite, pharmaceutically acceptable salt or co-crystal, wherein,

L is selected from a bond, O, S, $-NR_L-$, $-CHR_L-$, $-CHR_L-CHR_L-$, $-Y-CHR_L-$, $-CHR_L-Y-$, $-Y-CHR_L-CHR_L-$, $-CHR_L-CHR_L-Y-$ or $-CHR_L-Y-CHR_L-$.

Each $R_L$ is independently selected from H, F, methyl, ethyl or propyl.

Y is selected from O, S or $-NR_L-$.

Ring B is selected from one of the following substituted or unsubstituted groups: cyclohexyl, cyclohexenyl, azacyclohexenyl, piperidinyl, phenyl, pyrazolyl, pyridyl, pyridazinyl, pyrimidinyl, pyrazinyl or triazinyl, which, when substituted, is optionally further substituted with 0 to 4 (for example, 0, 1, 2, 3 or 4) $R_B$.

Or ring B is selected from one of the following substituted or unsubstituted groups: piperazinyl, tetrahydropyrrolyl or 1,4-diazepanyl, which, when substituted, is optionally further substituted with 0 to 4 (for example, 0, 1, 2, 3 or 4) $R_B$.

Ring C is selected from one of the following substituted or unsubstituted groups: A benzene ring, a pyrrole ring, a pyrazole ring, a pyridine ring, a furan ring, a thiophene ring, an imidazole ring, a thiazole ring, a oxazole ring, an isothiazole ring, an isoxazole ring, a triazole ring, a tetrazole ring, a oxadiazole ring, a thiadiazole ring, a pyridazine ring, a pyrimidine ring, a pyrazine ring or a triazine ring, which, when substituted, is optionally further substituted with 0 to 4 (for example, 0, 1, 2, 3 or 4) Re.

Ring D is selected from one of the following substituted or unsubstituted groups: A benzene ring, a naphthalene ring, a pyrrole ring, a pyrazole ring, a pyridine ring, a furan ring, a thiophene ring, an imidazole ring, a thiazole ring, a oxazole ring, an isothiazole ring, an isoxazole ring, a triazole ring, a oxadiazole ring, a thiadiazole ring, a pyridazine ring, a pyrimidine ring, a pyrazine ring or a triazine ring, which, when substituted, is optionally further substituted with 0 to 4 (for example, 0, 1, 2, 3 or 4) $R_D$.

When r=1,

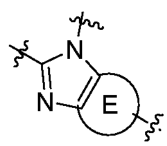

is selected from

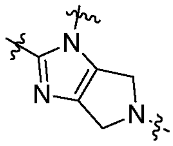.

When r=0,

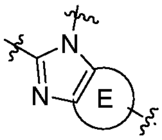

is selected from

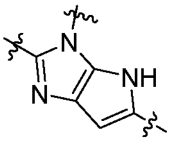, 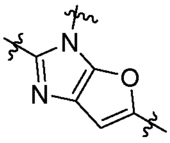,

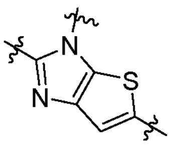, 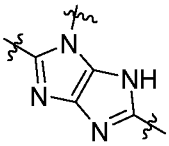, 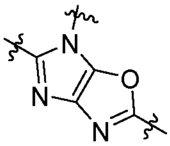, 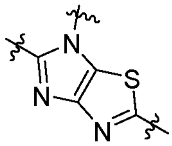, 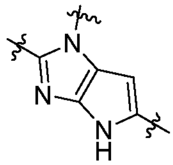, 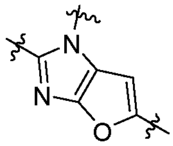,

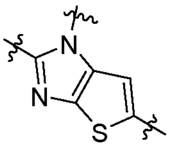, 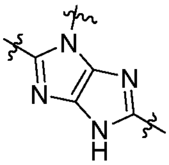, 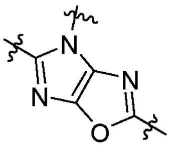 or 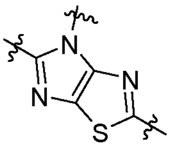.

The definitions of other groups are the same as those in any one of the first, second and third embodiments.

As the fifth embodiment of the present invention, the compound represented by the above general formula (I) or a stereoisomer, tautomer, deuterated substance, solvate, prodrug, metabolite, pharmaceutically acceptable salt or co-crystal, wherein,

L is selected from -Y-$CHR_L$-, -$CHR_L$-Y-, -$CHR_L$-$CHR_L$-, -$CHR_L$-$CHR_L$-Y- or -Y-$CHR_L$-$CHR_L$-.

Y is selected from O, S or -$NR_L$-.

Each $R_L$ is independently selected from H or methyl.

Ring B is selected from one of the following substituted or unsubstituted groups:

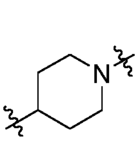, 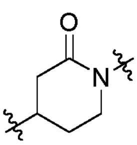, 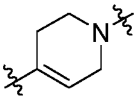, 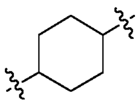, 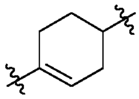, 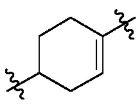 or

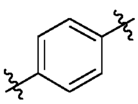,

the left side of which is directly connected to ring C, and which, when substituted, is optionally further substituted with 1, 2 or 3 $R_B$.

Or ring B is selected from one of the following substituted or unsubstituted groups

: 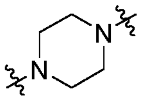, 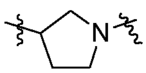 or 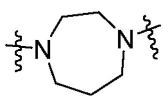,

the left side of which is directly connected to ring C, and which, when substituted, is optionally further substituted with 1, 2 or 3 $R_B$.

Or ring B is selected from one of the following substituted or unsubstituted groups:

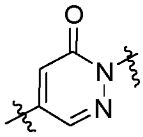, 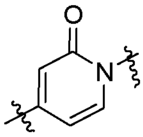, 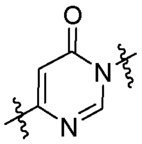, 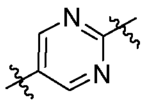, 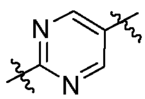, 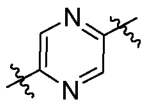,

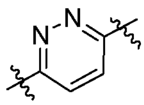, 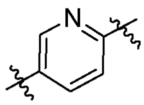 or 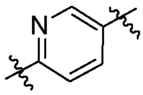,

the left side of which is directly connected to ring C, and which, when substituted, is optionally further substituted with 1, 2 or 3 $R_B$.

Or

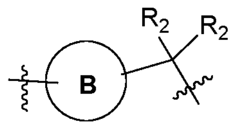

is selected from

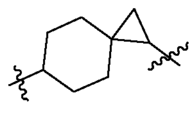

or

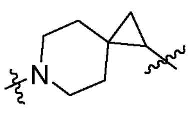,

the left side of which is directly connected to ring C, and which, when the 6-membered ring in the spiro ring is substituted, is optionally further substituted with 1, 2 or 3 $R_B$.

Ring C is selected from one of the following substituted or unsubstituted groups: a benzene ring,

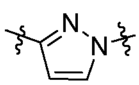, 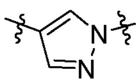, 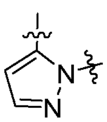, 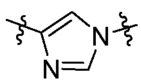, 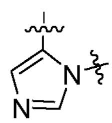, 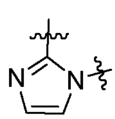,

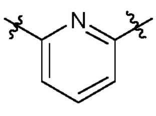, 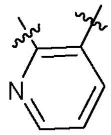, 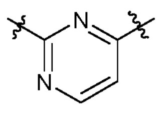, 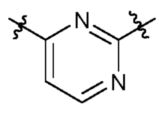, 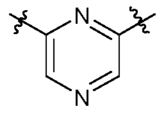, 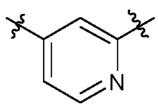, 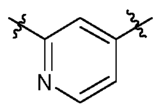

or

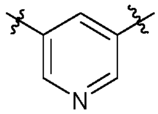

, which, when substituted, is optionally further substituted with 1, 2 or 3 Rc, and the left side of which is connected to L.

Ring D is selected from a benzene ring or a pyridine ring, and the benzene ring or pyridine ring is optionally further substituted with 1, 2 or 3 $R_D$.

Each $R_B$ is independently selected from H, =O, F, OH, CN, $CF_3$, methyl, ethyl, propyl, cyclopropyl, cyclobutyl, cyclopentyl, methoxy or ethoxy.

Each $R_C$ is independently selected from H, F, Cl, OH, CN, $NH_2$, $CF_3$, methyl, ethyl, propyl, cyclopropyl, cyclobutyl, cyclopentyl, methoxy or ethoxy.

Each $R_D$ is independently selected from H, F, Cl, CN, $CF_3$, $CHF_2$, methyl, ethyl, methoxy, ethoxy, tert-butyloxy or trifluoromethoxy.

Alternatively, $R_C$ and $R_L$ together with the atoms attached thereto form a 4- to 7-membered heterocyclic ring. The heterocyclic ring is further substituted with 0, 1, 2, 3 or 4 substituents selected from H, F, methyl or ethyl. The heterocyclic ring contains 1 to 2 heteroatoms selected from O, S and N.

The definitions of other groups are the same as those in any one of the first, second, third and fourth embodiments.

As the sixth embodiment of the present invention, the compound represented by the above general formula (I) or a stereoisomer, tautomer, deuterated substance, solvate, prodrug, metabolite, pharmaceutically acceptable salt or co-crystal, wherein,

L is selected from $-CH_2O-$, and the right side of L is connected to ring C.

$R_1$ is selected from H, CN, $CF_3$, $CHF_2$, $CH_2F$, $-CH_2OH$, $-CH(OH)CH_3$, methyl, ethyl, methoxy, ethoxy, propoxy, isopropoxy, methoxymethyl, methoxyethyl, ethoxymethyl, isopropoxymethyl, -ethylene-Z-methylene-$R_{1a}$, -ethylene-Z-ethylene-$R_{1a}$, - ethylene-Z-$R_{1a}$, -$R_{1a}$ or -methylene-$R_{1a}$.

Z is selected from NH, $N(CH_3)$ or O.

$R_{1a}$ is selected from methyl, ethyl, isopropyl, propyl, methoxy, ethoxy, propoxy, isopropoxy, cyclopropyl, cyclobutyl, cyclopentyl, cyclohexyl, oxiranyl, oxetanyl, azetidinyl, tetrahydrofuranyl, tetrahydro-2*H*-pyranyl, pyrrolidinyl, piperidinyl, pyrazolyl, 1,2,3-triazolyl, 1,2,4-triazolyl, imidazolyl, thiazolyl, thienyl, furyl, oxazolyl, isoxazolyl, 1,2,4-oxadiazolyl, isothiazolyl, pyrimidyl, pyridine or phenyl. The $R_{1a}$ is optionally further substituted with 0, 1, 2 or 3 substituents selected from H, F, CN, methyl, ethyl, propyl, $CF_3$, methoxy or ethoxy.

Ring C is selected from one of the following substituted or unsubstituted groups:

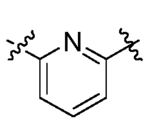 or 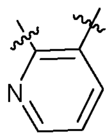,

which, when substituted, is optionally further substituted with 1, 2 or 3 Rc, and the left side of which is connected to L.

Ring D is selected from a benzene ring or pyridine, and the benzene ring or pyridine is optionally further substituted with 1, 2 or 3 $R_D$.

Or

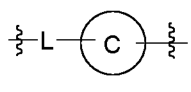

is selected from

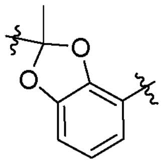,

the right side of which is connected to ring B, and in which the benzene ring is optionally further substituted with 1, 2 or 3 Rc.

Each $R_B$ is independently selected from H, =O, F, CN, $CF_3$, methyl, ethyl or cyclopropyl.

Each $R_C$ is independently selected from H, F, Cl, CN, $CF_3$, methyl, ethyl, methoxy or ethoxy.

Each $R_D$ is independently selected from H, F, Cl, CN, $CF_3$, $CHF_2$, methyl or ethyl.

The definitions of other groups are the same as those in any one of the first, second, third, fourth and fifth embodiments.

As the seventh embodiment of the present invention, the compound represented by the above general formula (I) or a stereoisomer, tautomer, deuterated substance, solvate, prodrug, metabolite, pharmaceutically acceptable salt or co-crystal, wherein,

$R_1$ is selected from

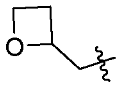, 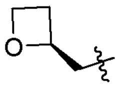, 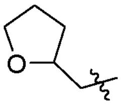 or 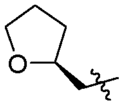.

$R_2$ is selected from H;

Ring B is selected from one of the following substituted or unsubstituted groups:

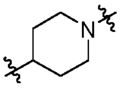, 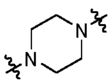, 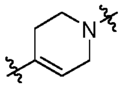, 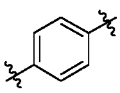 or 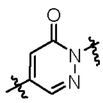,

the left side of which is directly connected to ring C, and which, when substituted, is optionally further substituted with 1, 2 or 3 $R_B$;

or

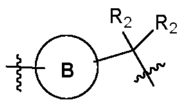

is selected from

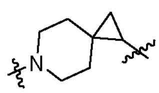,

the left side of which is directly connected to ring C, and which, when the 6-membered ring in the spiro ring is substituted, is optionally further substituted with 1, 2 or 3 $R_B$;

r=0,

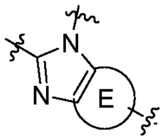

is selected from

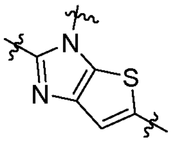

,

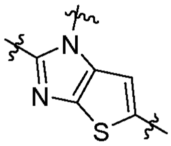

or

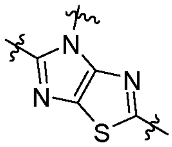

.

The definitions of other groups are the same as those in any one of the first, second, third, fourth, fifth and sixth embodiments.

The present invention provides the following compound or a stereoisomer, tautomer, deuterated substance, solvate, prodrug, metabolite, pharmaceutically acceptable salt or co-crystal thereof,

| | |
|---|---|
| 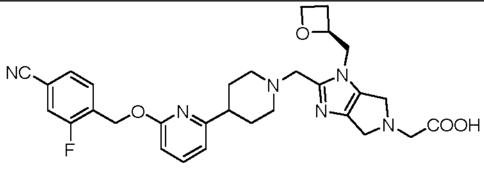 | 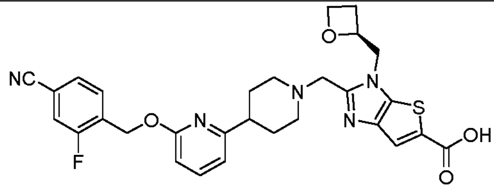 |
| 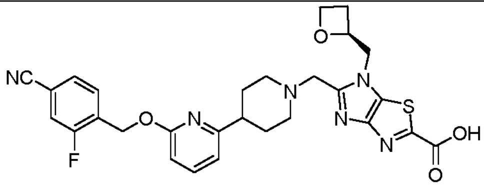 | 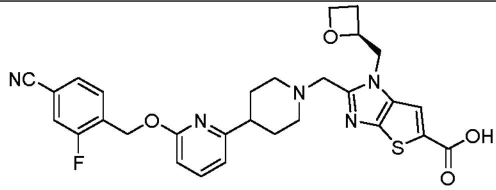 |
| 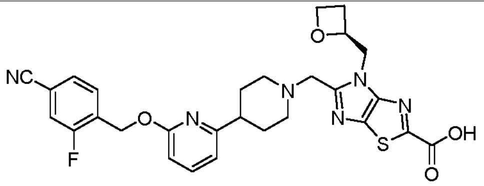 | 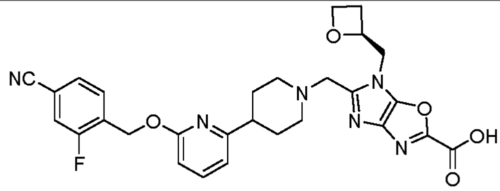 |
| 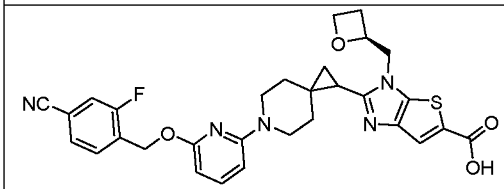 | 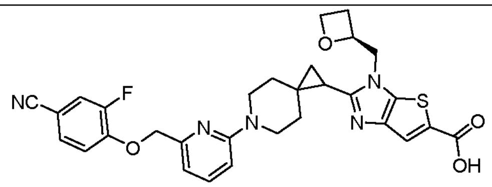 |
| 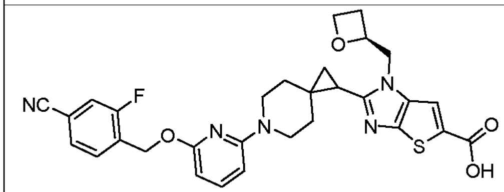 | 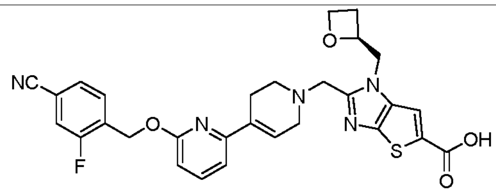 |
| 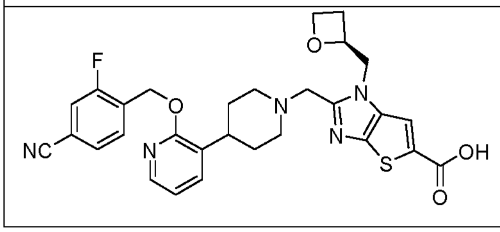 | 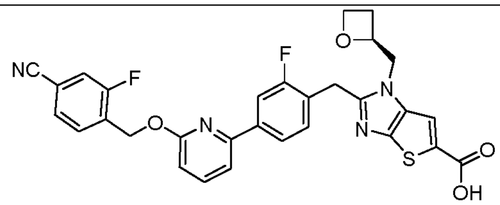 |

| | |
|---|---|
| 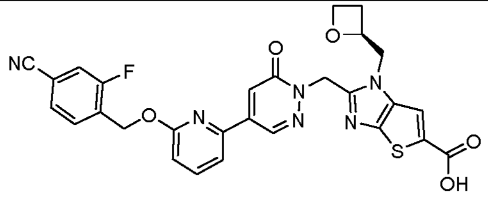 | 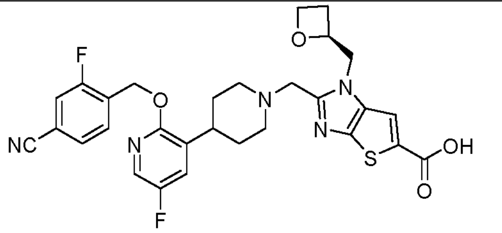 |
| 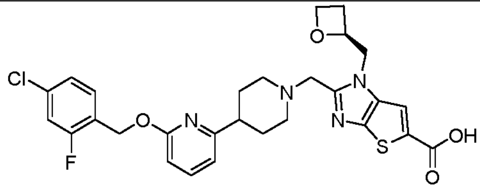 | 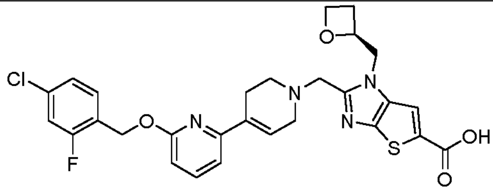 |
| 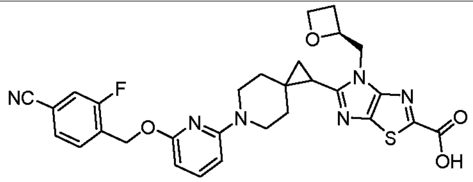 | 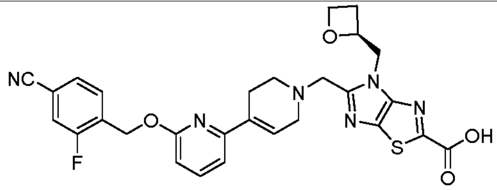 |
| 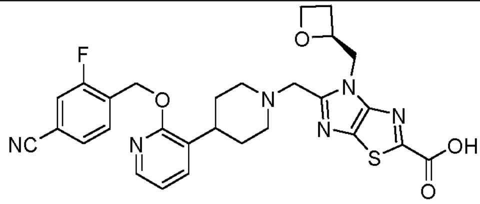 | 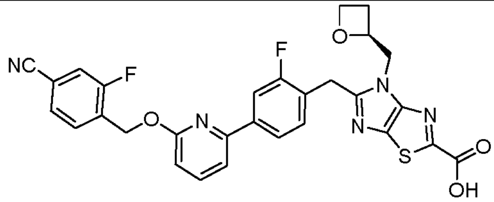 |
| 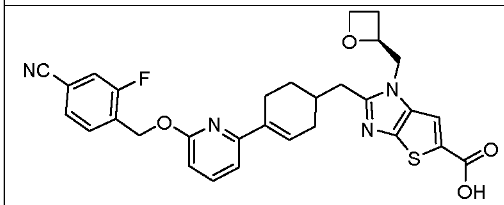 | 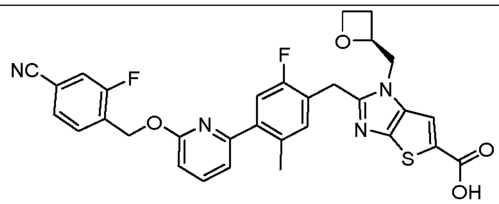 |
| 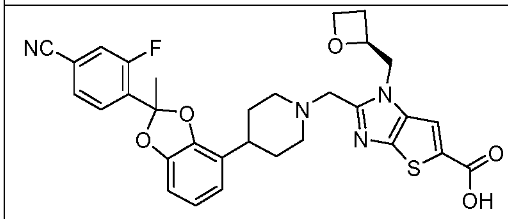 | 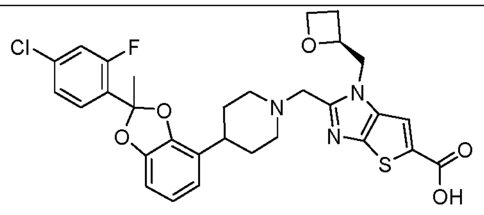 |
| 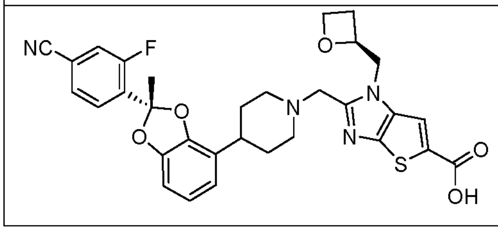 | 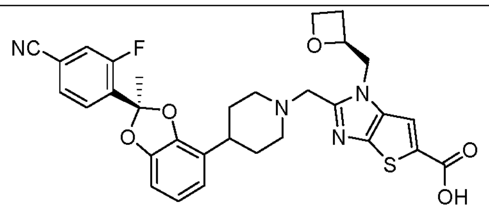 |

| | |
|---|---|
| 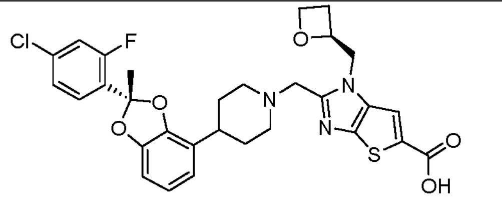 | 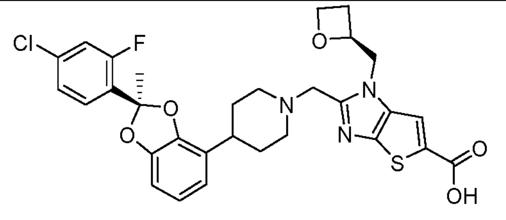 |
| 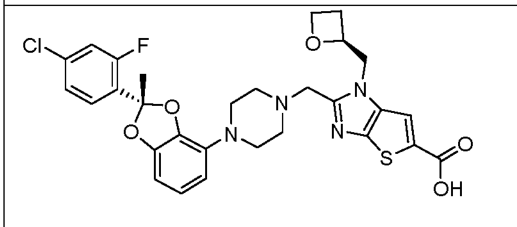 | 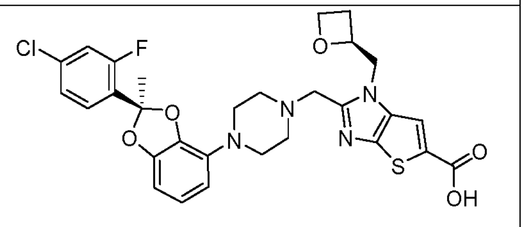 |
| 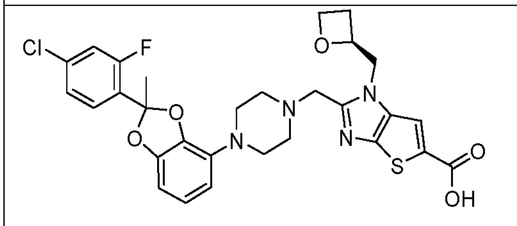 | 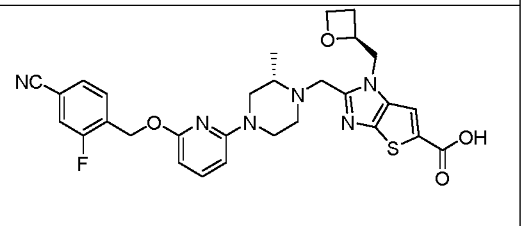 |
| 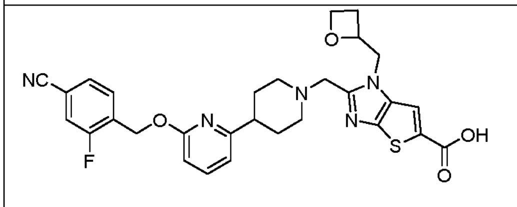 | 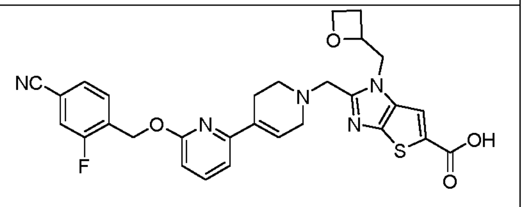 |
| 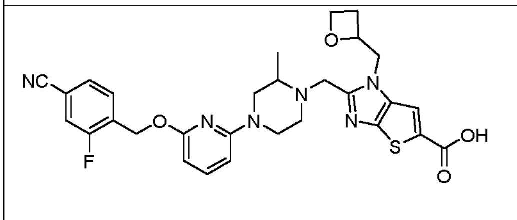 | 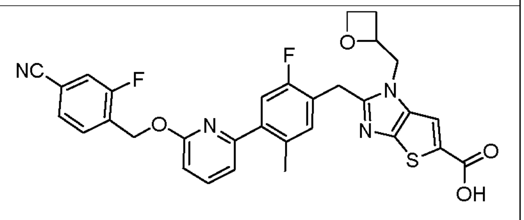 |
| 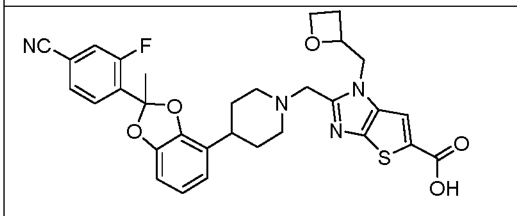 | 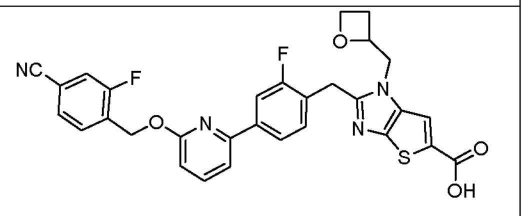 |
| 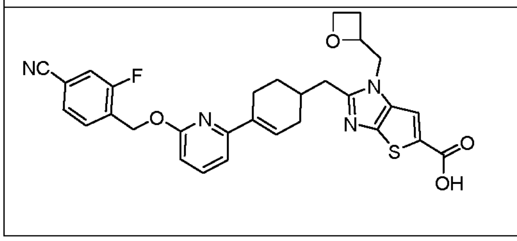 | 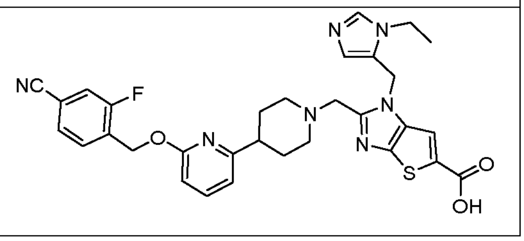 |

| | |
|---|---|
| 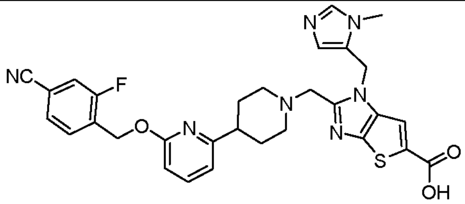 | 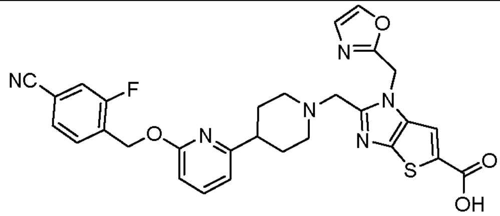 |
| 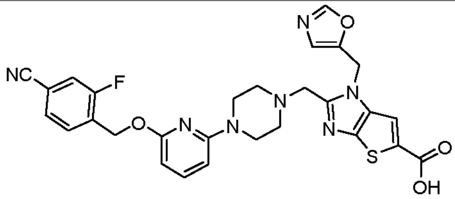 | 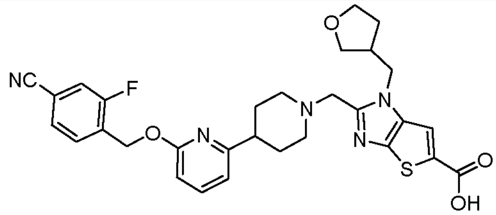 |
| 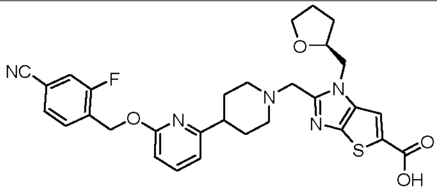 | 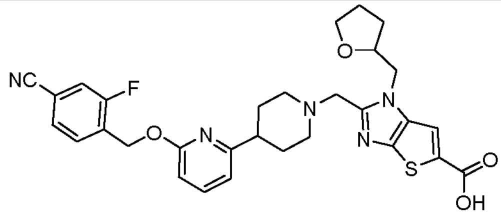 |
| 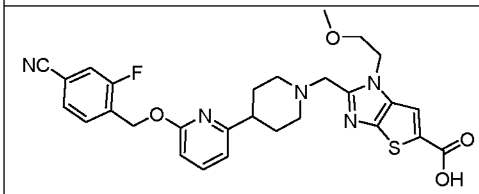 | 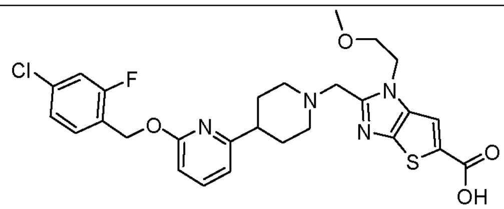 |
| 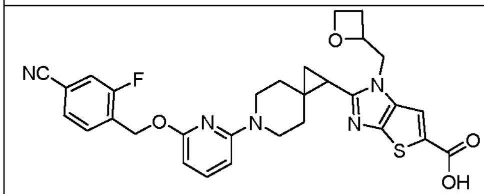 | 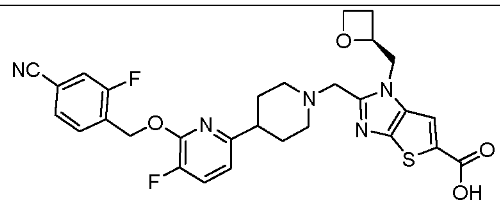 |
| 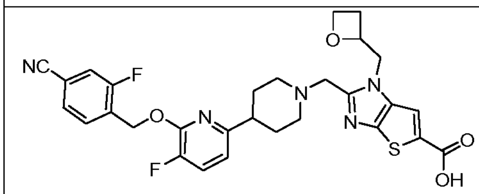 | 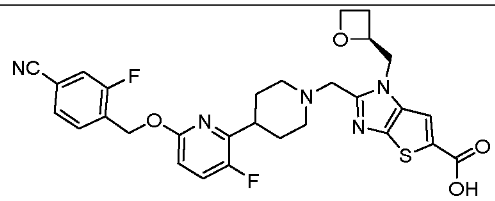 |
| 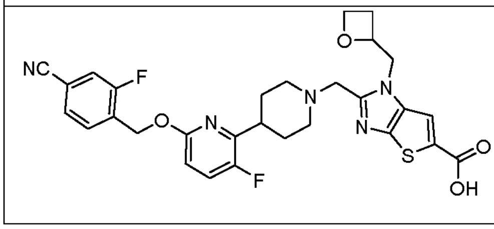 | 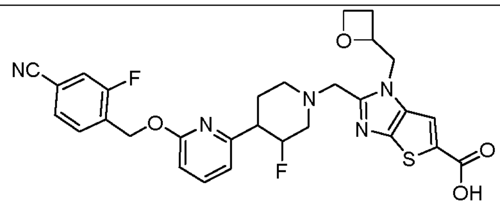 |

| | |
|---|---|
| 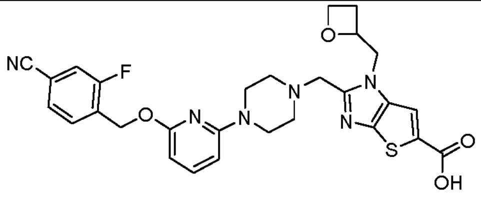 | 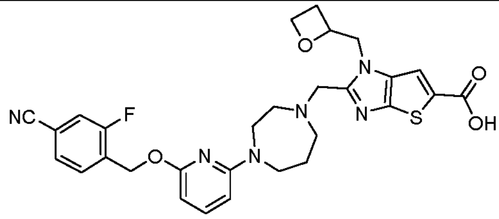 |
| 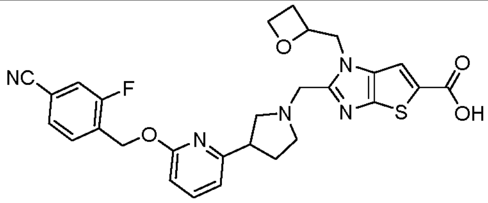 | 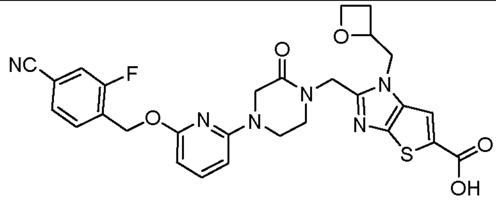 |
| 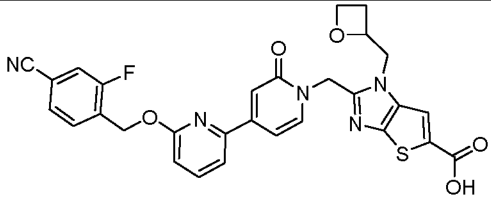 | 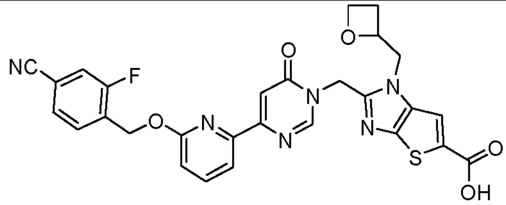 |
| 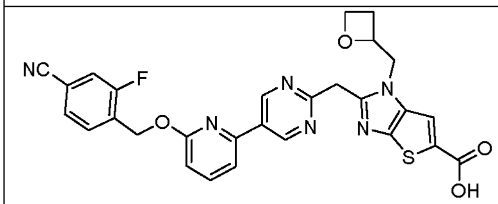 | 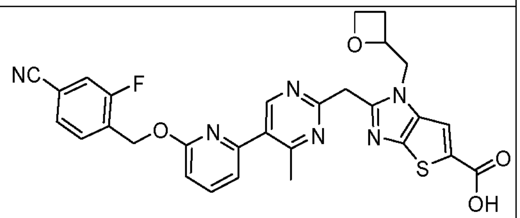 |
| 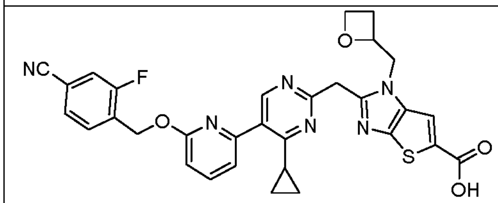 | 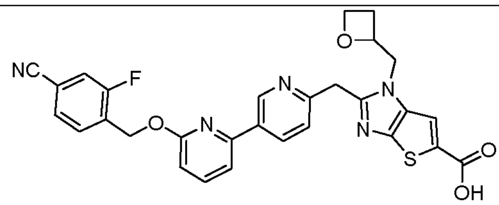 |
| 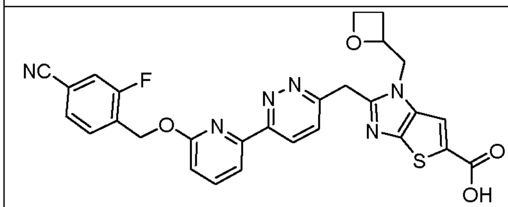 | 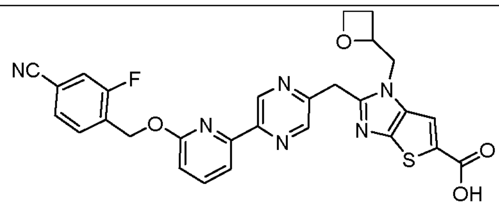 |
| 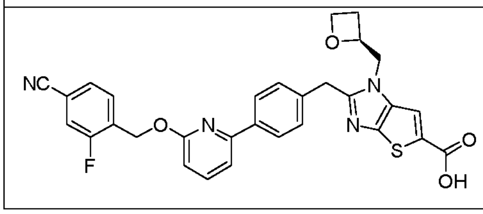 | 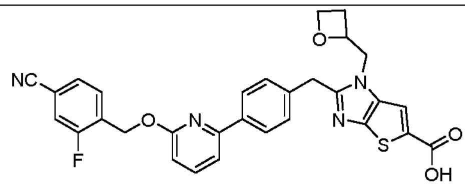 |

| | |
|---|---|
| 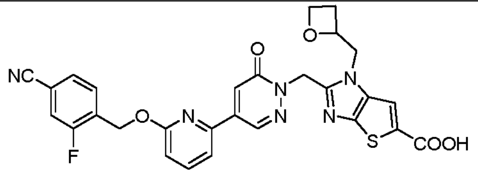 | 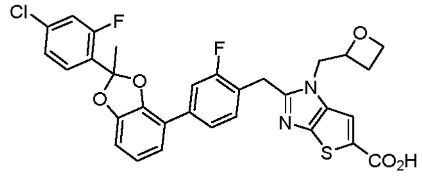 |
| 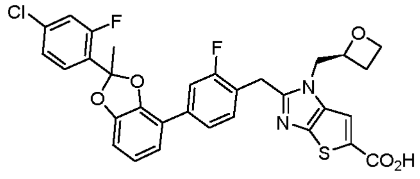 | 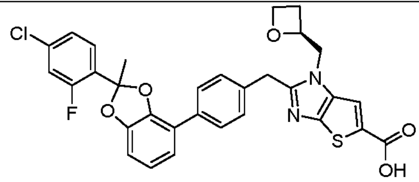 |
| 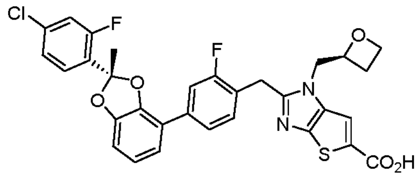 | 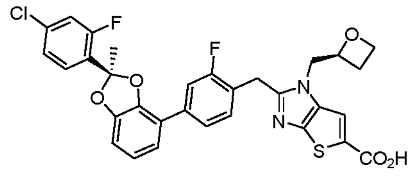 |
| 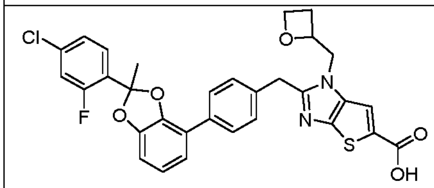 | 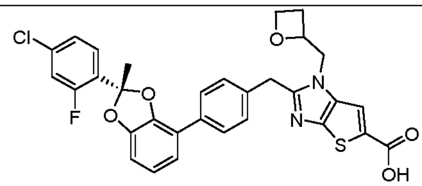 |
| 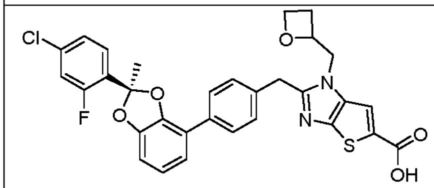 | 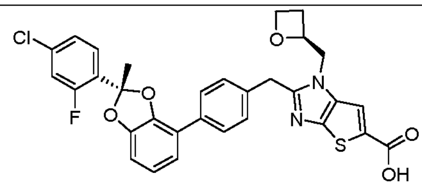 |
| 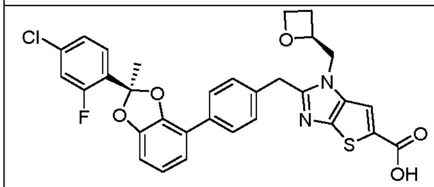 | 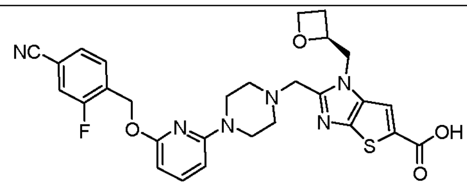 |
| 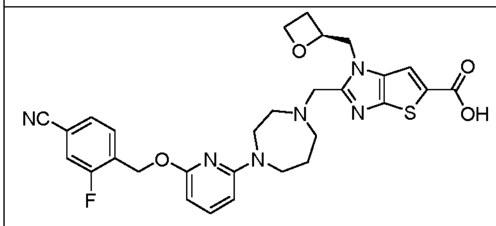 | 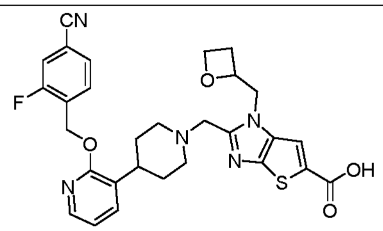 |
| 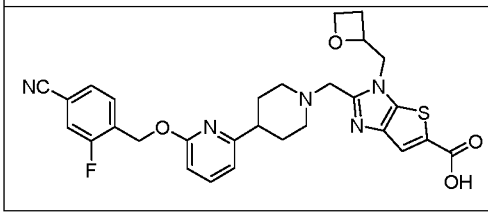 | 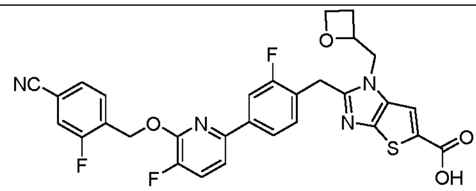 |

| | |
|---|---|
| 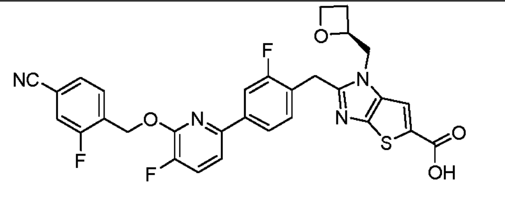 | 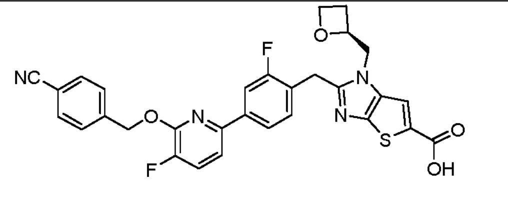 |
| 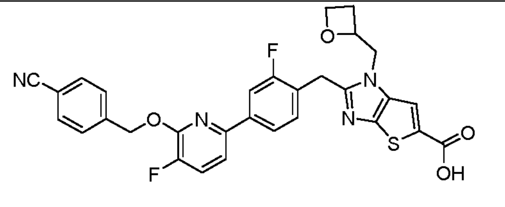 | 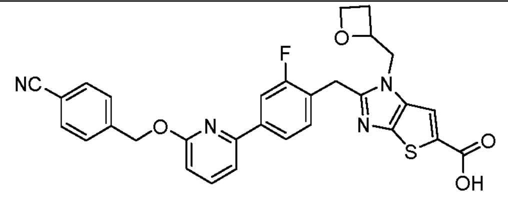 |
| 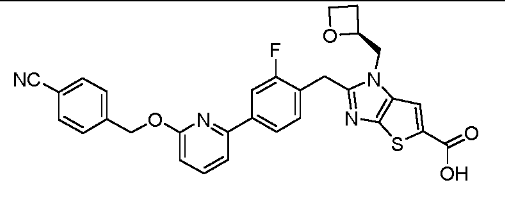 | 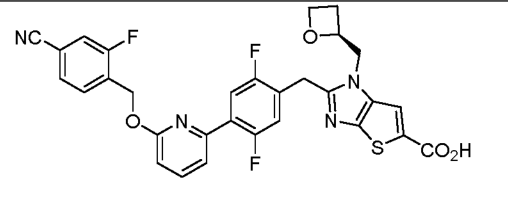 |
| 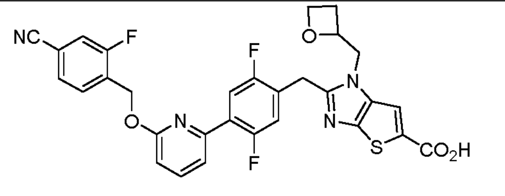 | 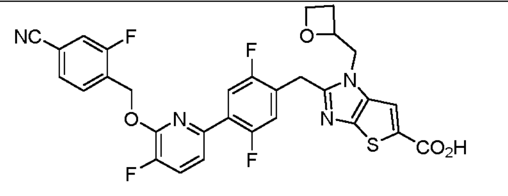 |
| 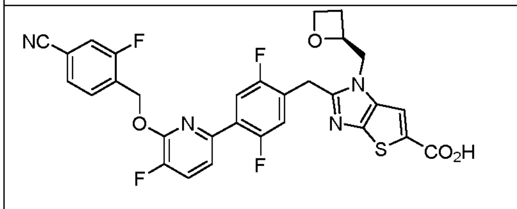 | 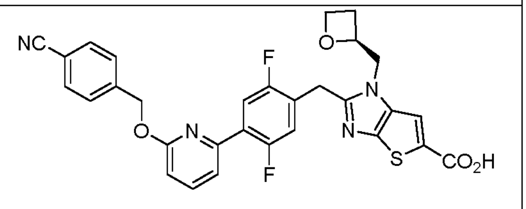 |
| 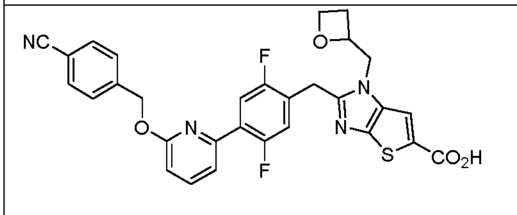 | 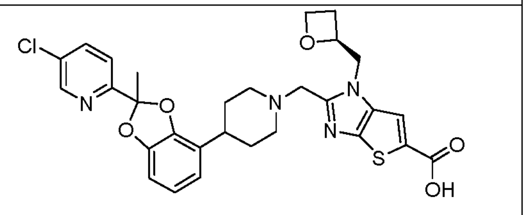 |
| 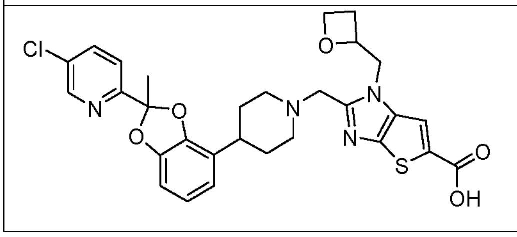 | 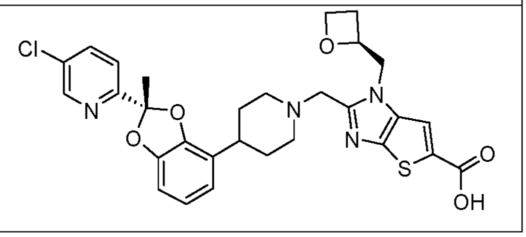 |

| | |
|---|---|
| 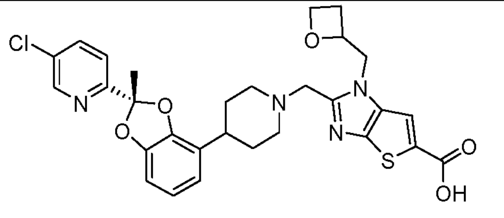 | 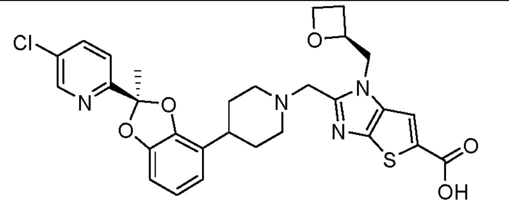 |
| 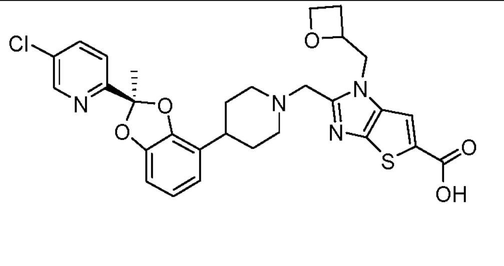 | 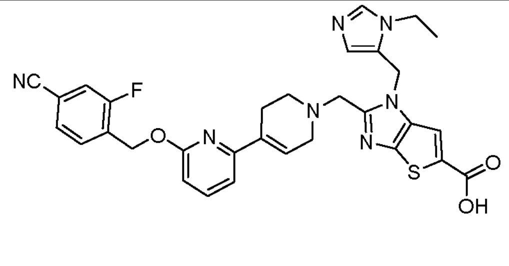 |

The present invention relates to a pharmaceutical composition comprising the above-mentioned compound or a stereoisomer, tautomer, deuterated substance, solvate, prodrug, metabolite, pharmaceutically acceptable salt or co-crystal thereof, and a pharmaceutically acceptable carrier.

The present invention relates to the above-mentioned compound or a stereoisomer, tautomer, deuterated substance, solvate, prodrug, metabolite, pharmaceutically acceptable salt or co-crystal thereof, or an application of the above-mentioned pharmaceutical composition in the preparation of a drug for treating diabetes.

A compound has a structure as shown below,

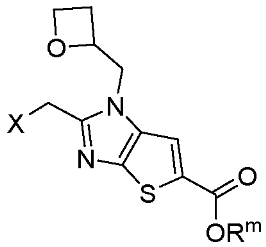

X is selected from H or Br;

$R^m$ is selected from H or $C_{1-4}$alkyl; the alkyl is optionally further substituted with 0 to 4 substituents selected from halogen, $C_{1-4}$ alkyl, $C_{1-4}$ alkoxy or $C_{6-10}$ carbocyclic ring.

Synthetic method I:

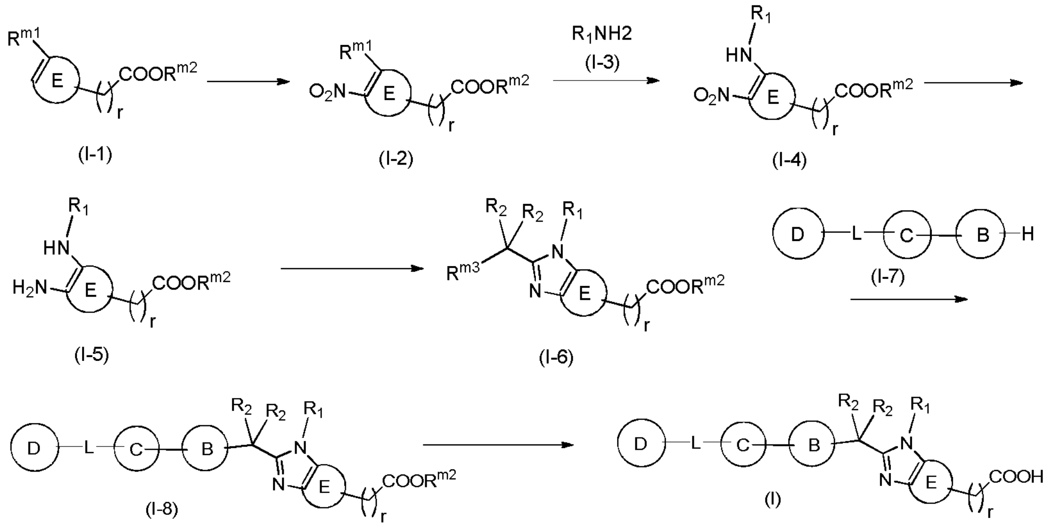

(I-1) (I-2) (I-4) (I-5) (I-6) (I-7) (I-8) (I)

The compound of general formula (I-1) is nitrated to obtain the corresponding compound of general formula (I-2).

The compound of general formula (I-2) and the compound of general formula (I-3) are subjected to a substitution reaction to obtain the corresponding compound of general formula (I-4).

The compound of general formula (I-4) is reduced to obtain the corresponding compound of general formula (I-5).

The compound of general formula (I-5) is subjected to a ring-closing reaction to obtain the corresponding compound of general formula (I-6).

The compound of general formula (I-6) and the compound of general formula (I-7) are subjected to a substitution reaction to obtain the corresponding compound of general formula (I-8).

The compound of the general formula (I-8) is subjected to a decarboxylation protecting group reaction to obtain the corresponding compound of the general formula (I).

$R^{m1}$ and $R^{m3}$ are selected from F, Cl, Br, I, OTf, *etc.*

$R^{m2}$ is selected from carboxyl protecting groups, preferably $C_{1-6}$alkyl or benzyl.

The definitions of other groups are the same as those in any embodiment of the above general formula (I).

Unless stated to the contrary, the terms used in the description and claims have the following meanings.

The carbon, hydrogen, oxygen, sulfur, nitrogen or F, Cl, Br, I involved in the groups and compounds of the present invention all comprise their isotopes, and the carbon, hydrogen, oxygen, sulfur or nitrogen involved in the groups and compounds of the present invention is optionally further substituted with one or more of their corresponding isotopes, wherein the isotopes of carbon comprise $^{12}C$, $^{13}C$ and $^{14}C$, the isotopes of hydrogen comprise protium (H), deuterium (D, also known as heavy hydrogen), tritium (T, also known as superheavy hydrogen), the isotopes of oxygen comprise $^{16}O$, $^{17}O$ and $^{18}O$, the isotopes of sulfur comprise $^{32}S$, $^{33}S$, $^{34}S$ and $^{36}S$, the isotopes of nitrogen comprise $^{14}N$ and $^{15}N$, the isotopes of fluorine comprise $^{17}F$ and $^{19}F$, the isotopes of chlorine comprise $^{35}Cl$ and $^{37}Cl$, and the isotopes of bromine comprise $^{79}Br$ and $^{81}Br$.

"Halogen" means F, Cl, Br or I.

"Halogen-substituted" refers to F, Cl, Br or I substitution, including but not limited to a substitution with 1 to 10 substituents selected from F, Cl, Br or I, a substitution with 1 to 6 substituents selected from F, Cl, Br or I, a substitution with 1 to 4 substituents selected from F, Cl, Br or I. "Halogen-substituted" is referred to simply as "halo".

"Alkyl" means a substituted or unsubstituted linear or branched saturated aliphatic hydrocarbon group, including but not limited to an alkyl group of 1 to 20 carbon atoms, an alkyl group of 1 to 8 carbon atoms, an alkyl group of 1 to 6 carbon atoms, or an alkyl group of 1 to 4 carbon atoms. Non-limiting examples include methyl, ethyl, n-propyl, isopropyl, n-butyl, sec-butyl, neobutyl, tert-butyl, n-pentyl, isoamyl, neopentyl, n-hexyl and various branched isomers thereof; The definition of the alkyl described herein is consistent with this definition. Alkyl group can be monovalent, divalent, trivalent or tetravalent.

"Heteroalkyl" refers to a substituted or unsubstituted alkyl group in which one or more (including but not limited to 2, 3, 4, 5 or 6) carbon atoms are replaced by heteroatoms (including but not limited to N, O or S). Non-limiting examples include $-X(CH_2)v-X(CH_2)v-X(CH_2)v-H$ (v is an integer from 1 to 5, X is independently selected from bonds or heteroatoms, heteroatoms include but not limited to N, O or S, and at least one X is selected from heteroatoms, and N or S in heteroatoms can be oxidized to various oxidation states). Heteroalkyl group can be monovalent, divalent, trivalent or tetravalent.

An "alkylene" means a substituted or unsubstituted straight or branched chain divalent saturated hydrocarbon group, including $-(CH_2)_v-$ (v is an integer from 1 to 10), and examples of alkylene include, but are not limited to, methylene,

ethylene, propylene, butylene, *etc.*

"Heteroalkylene" means a substituted or unsubstituted alkylene group in which one or more (including but not limited to 2, 3, 4, 5 or 6) carbon atoms are replaced by heteroatoms (including but not limited to N, O or S). Non-limiting examples include - $X(CH_2)v-X(CH_2)v-X(CH_2)v-$, wherein v is an integer from 1 to 5, each X is independently selected from a bond, N, O or S, and at least one X is selected from N, O or S.

"Cycloalkyl" means a substituted or unsubstituted saturated carbocyclic hydrocarbon group, usually having from 3 to 10 carbon atoms, and non-limiting examples include cyclopropyl, cyclobutyl, cyclopentyl, cyclohexyl, cycloheptyl, etc. The "cycloalkyl" herein is as defined above. Cycloalkyl group can be monovalent, divalent, trivalent or tetravalent.

"Heterocycloalkyl" means a substituted or unsubstituted saturated heteroatom-containing cyclic hydrocarbon group including but not limited to 3 to 10 atoms, 3 to 8 atoms, and 1 to 3 atoms selected from N, O or S. N and S selectively substituted in heterocycloalkyl ring can be oxidized to various oxidation states. The heterocycloalkyl group can be connected to a heteroatom or a carbon atom, the heterocycloalkyl group can be connected to an aromatic ring or a non-aromatic ring, and the heterocycloalkyl group can be connected to a bridged ring or a spiro ring. Non-limiting examples include oxiranyl, aziridinyl, oxetanyl, azetidinyl, tetrahydrofuranyl, tetrahydro-2*H*-pyranyl, dioxolanyl, dioxanyl, pyrrolidinyl, piperidinyl, imidazolidinyl, oxazolidinyl, oxazinanyl, morpholinyl, hexahydropyrimidinyl or piperazinyl. Heterocycloalkyl group can be monovalent, divalent, trivalent or tetravalent.

"Alkenyl" means a substituted or unsubstituted straight and branched unsaturated hydrocarbon group having at least 1, usually 1, 2 or 3 carbon-carbon double bonds, with a main chain including but not limited to 2 to 10, 2 to 6, or 2 to 4 carbon atoms. Examples of alkenyl include, but are not limited to vinyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, *etc.* The definition of the alkenyl described herein is consistent with this definition. Alkenyl group can be monovalent, divalent, trivalent or tetravalent.

"Alkynyl" means a substituted or unsubstituted straight and branched monovalent unsaturated hydrocarbon group having at least 1, usually 1, 2 or 3 carbon-carbon triple bonds, with a main chain including but not limited to 2 to 10 carbon atoms, 2 to 6 carbon atoms or 2 to 4 carbon atoms. Examples of alkynyl include but are not limited to ethynyl, propargyl, 1-propynyl, 2-propynyl, 1-butynyl, 2-butynyl, 3-butynyl, 1-pentynyl, 2-pentynyl, 3-pentynyl, 4-pentynyl, 1-methyl-1-butynyl, 2-methyl-1-butynyl, 2-methyl-3-butynyl, 1-hexynyl, 2-hexynyl, 3-hexynyl, 4-hexynyl, 5- hexynyl, 1-methyl-1-pentynyl, 2-methyl-1-pentynyl, 1-heptynyl, 2-heptynyl, 3-heptynyl, 4-heptynyl, 1-octynyl, 3-octynyl, 1-nonynyl, 3-nonynyl, 1-decynyl, 4-decynyl, *etc.* Alkynyl group can be monovalent, divalent, trivalent or tetravalent.

"Alkoxy" means a substituted or unsubstituted -O-alkyl group. Non-limiting examples include methoxy, ethoxy, n-propoxy, isopropoxy, n-butoxy, sec-butoxy, tert-butoxy, n-pentoxy, n-hexyloxy, cyclopropoxy and cyclobutoxy.

"Carbocyclyl" or "carbocyclic ring" means a substituted or unsubstituted saturated or unsaturated aromatic ring or non-aromatic ring, and the aromatic ring or non-aromatic ring can be 3- to 8-membered monocyclic ring, 4- to 12-membered bicyclic ring or 10- to 15-membered tricyclic ring system. The carbocyclyl can be connected to an aromatic ring or a nonaromatic ring, and the aromatic ring or nonaromatic ring is optionally a monocyclic ring, a bridged ring or a spirocyclic ring. Non-limiting examples include cyclopropane, cyclobutane, cyclopentane, cyclohexane, cycloheptane, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, benzene ring, naphthalene ring,

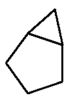 , 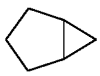 ,

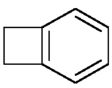 , 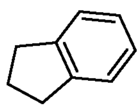 or 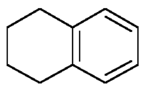 .

A "carbocyclyl" or "carbocyclic ring" can be monovalent, divalent, trivalent or tetravalent.

"Heterocyclyl" or "heterocyclic ring" refers to a substituted or unsubstituted saturated or unsaturated aromatic ring or non-aromatic ring, and the aromatic ring or non-aromatic ring can be 3- to 8-membered monocyclic ring, 4- to 12-membered bicyclic ring or 10- to 15-membered tricyclic ring system, and contains one or more (including but not limited to 2, 3, 4 or 5) heteroatoms selected from N, O or S, and the optionally substituted N or S in the heterocyclyl ring can be oxidized into various oxidation states. Heterocyclyl can be connected to other groups through its heteroatoms or carbon atoms, heterocyclyl can be connected to other groups through its aromatic ring or nonaromatic ring, and

heterocyclyl can be connected to a bridged ring or a spiro ring (when the ring of the heterocyclyl is a bridged ring or a spiro ring, the site where the heterocyclyl is connected to other groups is on the bridged ring or spiro ring). Non-limiting examples include oxiranyl, azacyclopropyl, oxetanyl, azetidinyl, 1,3-dioxolane, 1,4-dioxolane, 1,3-dioxane, azacycloheptyl, pyridyl, furanyl, thienyl, pyranyl, N-alkylpyrrolyl, pyrimidinyl, pyrazinyl, pyridazinyl, imidazolyl, piperidyl, morpholinyl, thiomorpholinyl, 1,3-dithianyl, dihydrofuranyl, dihydropyranyl, dithiolanyl, tetrahydrofuranyl, tetrahydropyrrolyl, tetrahydroimidazolyl, tetrahydrothiazolyl, tetrahydropyranyl, benzimidazolyl, benzopyridyl, pyrrolopyridyl, benzodihydrofuranyl, pyrrolyl, pyrazolyl, thiazolyl, oxazolyl, pyrazinyl, indazolyl, benzothiophenyl, benzofuranyl, benzopyrrolyl, benzimidazolyl, benzothiazolyl, benzoxazolyl, benzopyridyl, benzopyrimidinyl, benzopyrazinyl, piperazinyl, azabicyclo[3.2.1]octyl, azabicyclo[5.2.0]nonalkyl, oxatricyclo[5.3.1.1]dodecyl, azaadamantyl, oxaspiro[3.3]heptyl,

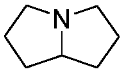, 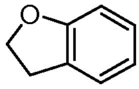,

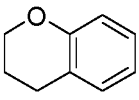, 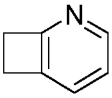, 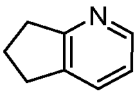, 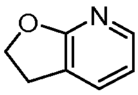, 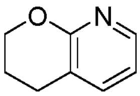, 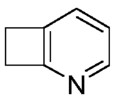, 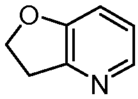,

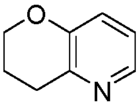, 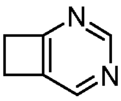, 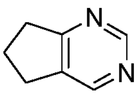, 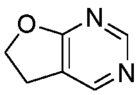 or 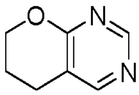.

A "heterocyclyl" or "heterocyclic ring" can be monovalent, divalent, trivalent or tetravalent.

"Spiro ring" or "spiro ring group" means a polycyclic group that shares one atom (called spiro atom) between substituted or unsubstituted monocyclic rings. The number of ring atoms in the spiro ring system includes but is not limited to 5 to 20, 6 to 14, 6 to 12, or 6 to 10, wherein one or more rings may contain 0 or more (including but not limited to 1, 2, 3 or 4) double bonds, and can optionally contain 0 to 5 heteroatoms selected from N, O or $S(=O)_n$.

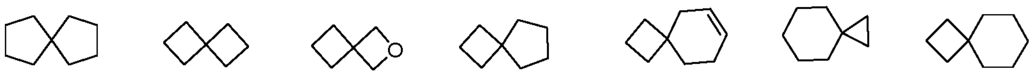

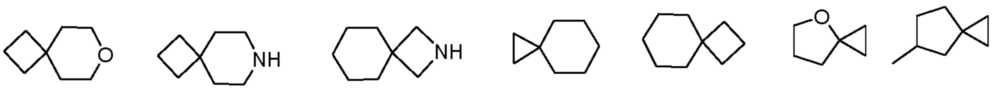

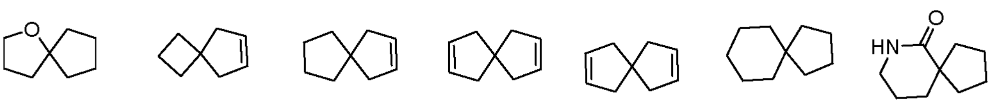

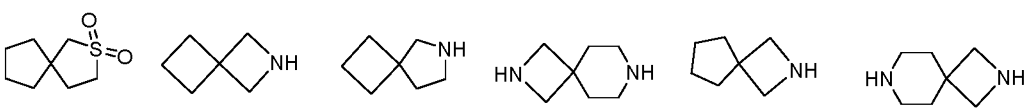.

"Spiro ring" or "spiro ring group" can be monovalent, divalent, trivalent or tetravalent.

"Fused ring" or "fused ring group" refers to a polycyclic group in which each ring in the system shares an adjacent pair of atoms with other rings in the system, wherein one or more rings may contain 0 or more (including but not limited to 1, 2, 3 or 4) double bonds, and may be substituted or unsubstituted, and each ring in the fused ring system may contain 0 to 5 heteroatoms or groups containing heteroatoms (including but not limited to N, $S(=O)_n$ or O, n is 0, 1 or 2). The number of the ring atoms in the fused ring system includes but is not limited to 5 to 20, 5 to 14, 5 to 12, or 5 to 10. Non-limiting examples include:

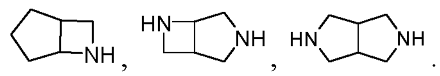

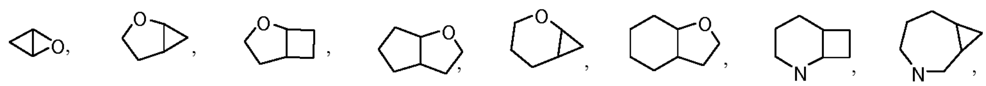

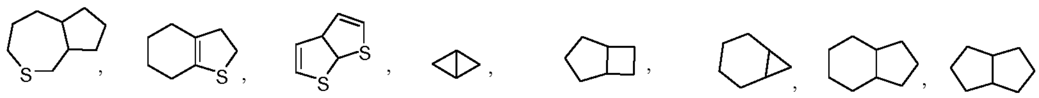

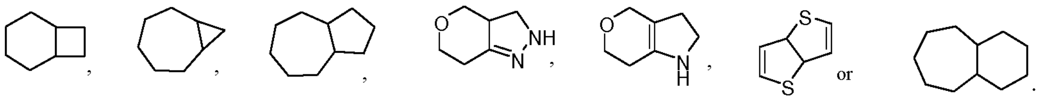 or

"Fused ring" or "fused ring group" may be monovalent, divalent, trivalent or tetravalent.

"Bridged ring" or "bridged ring group" means a substituted or unsubstituted polycyclic group containing any two atoms that are not directly connected, and may contain 0 or more double bonds. Any ring in the bridged ring system may contain 0 to 5 groups selected from heteroatoms or groups containing heteroatoms (including but not limited to N, S(=O)n or O, wherein n is 0, 1 or 2). The number of ring atoms includes, but is not limited to, 5 to 20, 5 to 14, 5 to 12 or 5 to 10. Non-limiting examples include

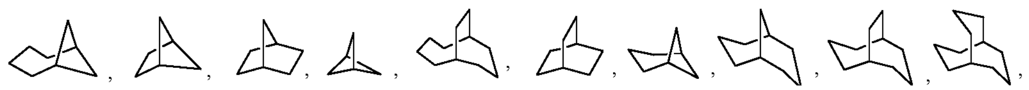

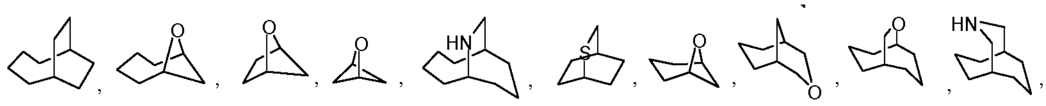

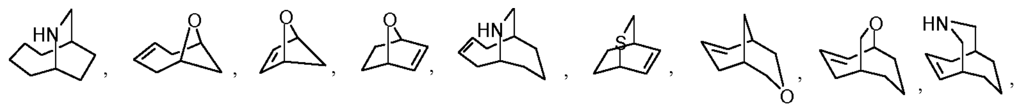

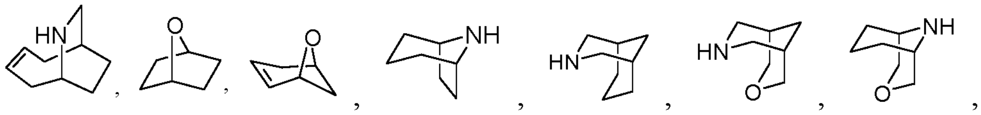

cubane or adamantane. "Bridged ring" or "bridged ring group" may be monovalent, divalent, trivalent or tetravalent.

"Carbospiro ring", "spiro ring carbocyclyl", "spirocarbocyclyl" or "carbospiro ring group" means a "spiro ring" with a ring system consisting only of carbon atoms. The definitions of "carbospiro ring", "spiro ring carbocyclyl", "spirocarbocyclyl" or "carbospiro ring group" used herein is consistent with that of spiro ring.

"Carbo-fused ring", "fused ring carbocyclyl", "fused carbocyclyl" or "carbo-fused ring group" means a "fused ring" with a ring system consisting only of carbon atoms. The definition of "carbo-fused ring", "fused ring carbocyclyl", "fused carbocyclyl" or "carbo-fused ring group" used herein is consistent with that of fused ring.

"Carbo-bridged ring", "bridged ring carbocyclyl", "bridged carbocyclyl" or "carbo-bridged ring group" means a "bridged ring" with a ring system consisting only of carbon atoms. The definitions of "carbo-bridged ring", "bridged ring carbocyclyl", "bridged carbocyclyl" or "carbo-bridged ring group" used herein is consistent with that of bridged ring.

"Heteromonocyclic ring", "monocyclic heterocyclyl" or "heteromonocyclyl" means "heterocyclyl" or "heterocyclic ring" with a monocyclic system. The definition of the heterocyclyl, "monocyclic heterocyclyl" or "heteromonocyclyl" used herein is consistent with that of heterocyclic ring.

"Hetero-fused ring", "hetero-fused ring group", "fused ring heterocyclyl" or "hetero-fused ring group" means a

"fused ring" containing a heteroatom. The definition of hetero-fused ring, "hetero-fused ring group", "fused ring heterocyclyl" or "hetero-fused ring group" used herein is consistent with that of fused ring.

"Hetero-spiro ring", "hetero-spiro ring group", "spiro ring heterocyclyl" or "hetero-spiro ring group" means a "spiro ring" containing a heteroatom. The definition of hetero-spiro ring, "hetero-spiro ring group", "spiro ring heterocyclyl" or "hetero-spiro ring group" used herein is consistent with that of spiro ring.

"Hetero-bridged ring", "hetero-bridged ring group", "bridged ring heterocyclyl" or "hetero-bridged ring group" means a "bridged ring" containing a heteroatom. The definition of hetero-bridged ring, "hetero-bridged ring group", "bridged ring heterocyclyl" or "hetero-bridged ring group" used herein is consistent with that of bridged ring.

"Aryl" or "aromatic ring" means a substituted or unsubstituted aromatic hydrocarbon group with a single ring or a fused ring, and the number of ring atoms in the aromatic ring includes but not limited to 6 to 18, 6 to 12 or 6 to 10 carbon atoms. The aryl ring may be fused to a saturated or unsaturated carbocyclic ring or heterocyclic ring, wherein the ring connected to the parent structure is an aryl ring. Non-limiting examples include a benzene ring, a naphthalene ring or

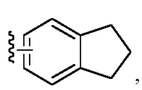, 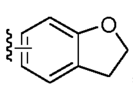, 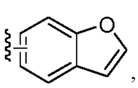, 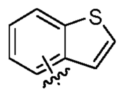, 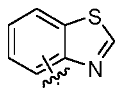, .

"Aryl" or "aromatic ring" may be monovalent, divalent, trivalent or tetravalent. When divalent, trivalent or tetravalent, the point of connection is on the aryl ring.

"Heteroaryl" or "heteroaromatic ring" means a substituted or unsubstituted aromatic hydrocarbon group containing 1 to 5 heteroatoms or groups containing heteroatoms (including but not limited to N, O or S(=O )n, n is 0, 1 or 2), and the number of ring atoms in the heteroaromatic ring includes but not limited to 5-15, 5-10 or 5-6. Non-limiting examples of heteroaryl include, but are not limited to pyridyl, furanyl, thienyl, pyridinyl, pyranyl, N-alkylpyrrolyl, pyrimidinyl, pyrazinyl, pyridazinyl, imidazolyl, benzopyrazole, benzimidazolyl, benzopyridine, pyrrolopyridine and the like. The heteroaryl ring may be fused to a saturated or unsaturated carbocyclic ring or heterocyclic ring, wherein the ring connected to the parent structure is an heteroaryl ring. Non-limiting examples include

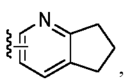, 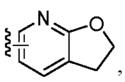, 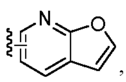, 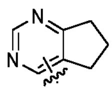, 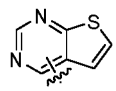, 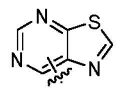, 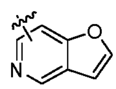

and

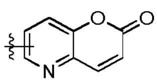.

The definition of the heteroaryl used herein is consistent with this definition. Heteroaryl group can be monovalent, divalent, trivalent or tetravalent. When divalent, trivalent or tetravalent, the point of connection is on the heteroaryl ring.

"5-membered ring fused 5-membered heteroaromatic ring" means a 5 fused 5-membered fused heteroaromatic ring, wherein at least one of the two fused rings contains more than one heteroatom (including but not limited to O, S or N), and the entire group is aromatic. Non-limiting examples include pyrrolopyrrole ring, pyrazolopyrrole ring, pyrazolopyrazole ring, pyrrolofuran ring, pyrazolofuran ring, pyrrolothiophene ring and pyrazolothiophene ring.

"5 fused 6-membered heteroaromatic ring" means a 5 fused 6-membered fused heteroaromatic ring, at least one of the two fused rings contains more than one heteroatom (including but not limited to O, S or N), and the entire group is aromatic. Non-limiting examples include benzo 5-membered heteroaryl and 6-membered heteroaromatic ring fused 5-membered heteroaromatic ring.

"Substitution" or "substituted" means a substitution with 1 or more (including but not limited to 2, 3, 4 or 5) substituents including but not limited to H, F, Cl, Br, I, alkyl, cycloalkyl, alkoxy, haloalkyl, mercaptan, hydroxyl, nitro, sulfhydryl, amino, cyano, isocyano, aryl, heteroaryl, heterocyclyl, bridged ring group, spiro ring group, fused ring group, hydroxyalkyl, =O, carbonyl, aldehyde, carboxylic acid, formate, $-(CH_2)_m-C(=O)-R^a$, $-O-(CH_2)_m-C(=O)-R^a$, $-(CH_2)_m-C(=O)-NR^bR^c$, $-(CH_2)_mS(=O)_nR^a$, $-(CH_2)_m$-alkenyl-$R^a$, $OR^d$ or $-(CH_2)_m$-alkynyl-$R^a$ (wherein m and n are 0, 1 or 2), arylthio, thiocarbonyl, silyl or - $NR^bR^c$ and the like, wherein $R^b$ and $R^c$ are independently selected from H, hydroxyl, amino, carbonyl, alkyl, alkoxy, cycloalkyl, heterocyclyl, aryl, heteroaryl, sulfonyl, trifluoromethylsulfonyl. Alternatively, $R^b$ and $R^c$ may form a five- or six-membered cycloalkyl or heterocyclyl. $R^a$ and $R^d$ are each independently selected from

aryl, heteroaryl, alkyl, alkoxy, cycloalkyl, heterocyclyl, carbonyl, ester group, bridged ring group, spiro ring group or bicyclic ring group.

"Containing 1 to 5 heteroatoms selected from O, S or N" means containing 1, 2, 3, 4 or 5 heteroatoms selected from O, S or N.

"Substituted with 0 to X substituents" refers to substituted with 0, 1, 2, 3 ... X substituents, wherein X is selected from any integer between 1 and 10. For example, "substituted with 0 to 4 substituents" refers to substituted with 0, 1, 2, 3 or 4 substituents. For example, "substituted with 0 to 5 substituents" refers to substituted with 0, 1, 2, 3, 4 or 5 substituents. For example, "bridged-heterocyclic ring is optionally further substituted with 0 to 4 substituents selected from H or F" means that the bridged-heterocyclic ring is optionally further substituted with 0, 1, 2, 3 or 4 substituents selected from H or F.

An X- to Y-membered ring (X is selected from an integer less than Y and greater than or equal to 3, and Y is selected from any integer between 4 and 12) includes X-, X+1-, X+2-, X+3-, X+4-, ..., Y-membered rings. Rings include a heterocyclic ring, a carbocyclic ring, an aromatic ring, aryl, heteroaryl, cycloalkyl, a mono-heterocyclic ring, a fused heterocyclic ring, a spiro-heterocyclic ring or a bridged-heterocyclic ring. For example, "a 4- to 7-membered mono-heterocyclic ring" refers to a 4-membered, 5-membered, 6-membered or 7-membered mono-heterocyclic ring, and "a 5- to 10-membered fused heterocyclic ring" refers to a 5-membered, 6-membered, 7-membered, 8-membered, 9-membered or 10-membered fused heterocyclic ring.

"Pharmaceutically acceptable salt" or "pharmaceutically acceptable salt thereof' refers to a salt of the compound of the present invention maintaining the biological effectiveness and characteristics of the free acid or free base, and obtained by reacting the free acid with a non- toxic inorganic base or organic base, reacting the free base with a non-toxic inorganic acid or organic acid.

"Pharmaceutical composition" refers to a mixture of one or more compounds of the present invention, or stereoisomers, tautomers, deuterated substances, solvates, prodrugs, metabolites, pharmaceutically acceptable salts or co-crystals thereof and other chemical components, wherein "other chemical components" refer to pharmaceutically acceptable carriers, excipients and/or one or more other therapeutic agents.

"Carrier" refers to a material that does not cause significant irritation to the organism and does not eliminate the biological activity and characteristics of the administered compound.

"Excipient" refers to an inert substance added to a pharmaceutical composition to facilitate the administration of a compound. Non-limiting examples include calcium carbonate, calcium phosphate, sugar, starch, cellulose derivatives (including microcrystalline cellulose), gelatin, vegetable oils, polyethylene glycols, diluents, granulating agents, lubricants, adhesives and disintegrants.

"Prodrug" refers to a substance that can be converted into a biologically active compound of the present invention through *in vivo* metabolism. The prodrug of the present invention is prepared by modifying the amino or carboxyl group in the compound of the present invention, and the modification can be removed by conventional operations or *in vivo* to obtain the parent compound. When the prodrug of the present invention is administered to a mammalian individual, the prodrug is split to form free amino or carboxyl group.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

"Animal" is meant to include mammals, such as humans, companion animals, zoo animals, and domestic animals, preferably humans, horses, or dogs.

The term "stereoisomer" refers to an isomer produced as a result of different spatial arrangement of atoms in molecules, including cis-trans isomers, enantiomers and conformational isomers.

"Tautomer" refers to a functional group isomer produced by the rapid movement of an atom in two positions in a molecule, such as keto-enol isomerization, amide-imino alcohol isomerization, *etc.*

"Optional" or "optionally" or "selective" or "selectively" refers to that the events or conditions subsequently described may but not necessarily occur, and the description includes the case where the events or conditions occur and do not occur. For example, "heterocyclyl optionally substituted with alkyl" refers to that the alkyl may but not necessarily exist, and the description includes the case where the heterocyclyl is substituted with an alkyl group and the case where the heterocyclyl is not substituted with alkyl.

"$IC_{50}$" refers to the concentration of a drug or inhibitor required to inhibit half of a given biological process (or a component of the process such as an enzyme, a receptor and a cell).

## Detailed Description of Embodiments

The technical solutions of the present invention will be described in detail by the following examples, but the

scope of protection of the present invention includes but is not limited thereto.

The compounds used in the reactions described herein are prepared according to organic synthesis techniques known to those skilled in the art, and starting from commercially available chemicals and(or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific and the like.

The structures of the compounds are determined by nuclear magnetic resonance (NMR) or (and) mass spectrometry (MS). The NMR shift (δ) is given in the unit of $10^{-6}$ (ppm). NMR is measured with (Bruker Avance III 400 and Bruker Avance 300) NMR instrument, and the solvent for determination is deuterated dimethyl sulfoxide (DMSO-$d_6$), deuterated chloroform ($CDCl_3$), deuterated methanol ($CD_3OD$), and the internal standard is tetramethylsilane (TMS);

MS is measured with (Agilent 6120B(ESI) and Agilent 6120B(APCI));

HPLC is determined with Agilent 1260DAD high pressure liquid chromatograph (Zorbax SB-C18 100 × 4.6 mm, 3.5 μM);

Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm.

For the column chromatography, Yantai Huanghai silica gel of 200-300 mesh silica gel is generally used as a carrier.

**Example 1** (S)-2-((4-(6-((4-cyano-2-fluorobenzyl)oxy)pyridin-2-yl)piperidin-1-yl)methyl)-1-(oxetan-2-ylmethyl)-1H-thieno[2,3-d]imidazole-5-carboxylic acid (Compound 1)

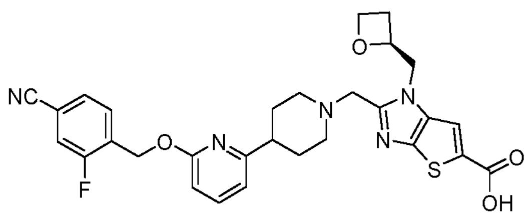

**Compound 1**

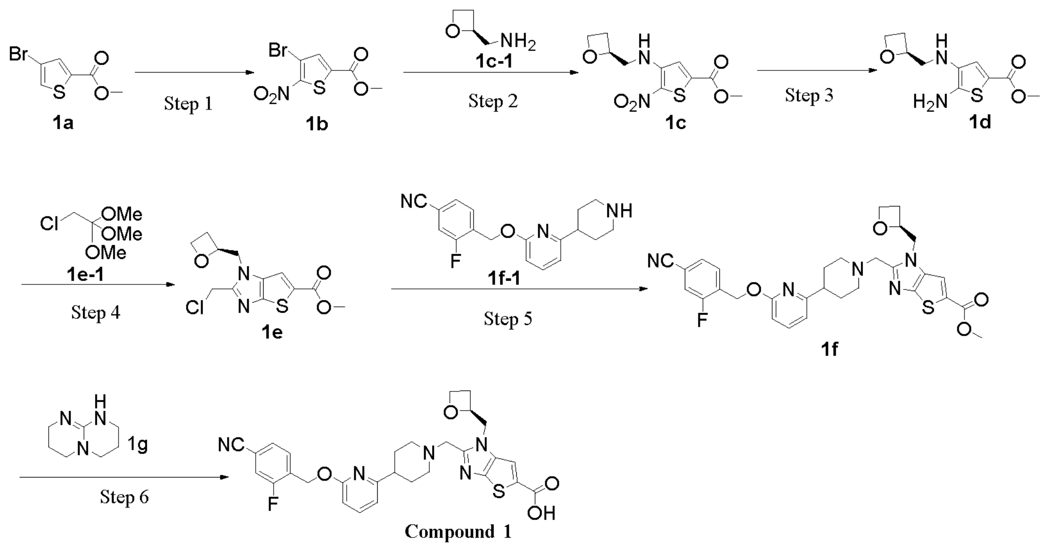

Step 1: methyl 4-bromo-5-nitrothiophene-2-carboxylate (1c)

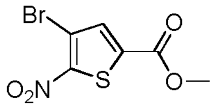

**1b**

Under ice-salt bath conditions, 1a (2.2 g, 10.0 mmol) was added to concentrated sulfuric acid (10 mL), and fuming nitric acid (945.0 mg, 15.0 mmol) was dissolved in concentrated sulfuric acid (5 mL) and slowly added dropwise into the reaction liquid; after the dropwise addition, the reaction was continued for 30 minutes in the ice-salt bath. The reaction liquid was slowly poured into an ice-water solution, and a large amount of white solids precipitated out. The filter cake was collected by filtration, washed with water (10 mL × 2), and dried to obtain 1b (2.6 g, yield 99.3%).
LCMS m/z = 265.9 $[M+1]^+$.
$^1H$ NMR (400 MHz, $CDCl_3$) δ 7.71 (s, 1H), 3.96 (s, 3H).

Step 2: methyl (S)-5-nitro-4-((oxetan-2-ylmethyl)amino)thiophene-2-carboxylate (1c)

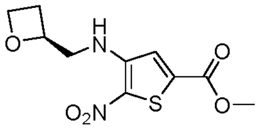

**1c**

At room temperature, 1b (1.3 g, 5.0 mmol) was dissolved in acetonitrile (30 mL), then 1c-1 (436.0 mg, 5.0 mmol) and potassium carbonate (2.1 g, 15.0 mmol) were added (100 mL) successively, heated to 60°C, and continued to react for 18 h. The reaction liquid was cooled to room temperature, filtered, and the filtrate was collected and concentrated. The obtained crude product was separated and purified by silica gel column chromatography (petroleum ether/ethyl acetate (v/v) = 2:1), and concentrated to obtain 1c (854.0 mg, yield 62.7%).
LCMS m/z = 273.1 $[M+1]^+$.
$^1H$ NMR (400 MHz, $CDCl_3$) δ 8.12 - 8.03 (m, 1H), 7.35 (s, 1H), 5.10 - 5.00 (m, 1H), 4.74 - 4.66 (m, 1H), 4.58 - 4.50 (m, 1H), 3.92 (s, 3H), 3.71 - 3.57 (m, 2H), 2.80 - 2.68 (m, 1H), 2.60 - 2.48 (m, 1H).

Step 3: methyl (S)-5-amino-4-((oxetan-2-ylmethyl)amino)thiophene-2-carboxylate (1d)

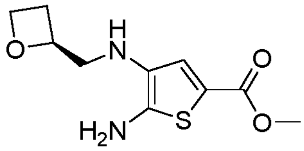

**1d**

At room temperature, 1c (854.0 mg, 3.1 mmol) was dissolved in methanol (30 mL), then palladium on carbon (170.0 mg, 1.6 mmol) was added, and the reaction was continued for 18 h under hydrogen atmosphere. The reaction liquid was filtered to remove palladium on carbon, the filtrate was collected and concentrated to obtain a crude product, and the obtained crude product was separated and purified by silica gel column chromatography (petroleum ether/ethyl acetate (v/v)=1:1), and concentrated to obtain 1d (410.0 mg, yield 53.9%).
LCMS m/z = 243.1 $[M+1]^+$.
$^1H$ NMR (400 MHz, DMSO - $d_6$) δ 7.15 (s, 1H), 5.89 (s, 2H), 4.83 - 4.74 (m, 1H), 4.55 - 4.47 (m, 1H), 4.47 - 4.39 (m, 1H), 4.23 - 4.15 (m, 1H), 3.66 (s, 3H), 3.25 - 3.11 (m, 2H), 2.64 - 2.55 (m, 1H), 2.46 - 2.36 (m, 1H).

Step 4: methyl (S)-2-(chloromethyl)-1-(oxetan-2-ylmethyl)-1H-thieno[2,3-d]imidazole-5-carboxylate (1e)

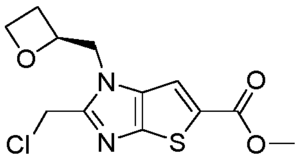

**1e**

At room temperature, 1d (410.0 mg, 1.7 mmol) was dissolved in acetonitrile (10 mL), then 1e-1 (391.0 mg, 2.5 mmol) and p-toluenesulfonic acid (15.0 mg, 0.1 mmol) were added successively, and finally the reaction liquid was heated to 60°C for 5 h under the protection of nitrogen. The reaction liquid was cooled to room temperature, saturated sodium bicarbonate solution was added to quench the reaction, extracted with dichloromethane (25 mL × 3), and the organic phase was combined, dried over anhydrous sodium sulfate, filtered and concentrated to obtain a crude product, which was directly used in the next reaction without further purification.

LCMS m/z = 301.0 $[M+1]^+$.

Step 5: methyl (S)-2-((4-(6-((4-cyano-2-fluorobenzyl)oxy)pyridin-2-yl)piperidin-1-yl)methyl)-1-(oxetan-2-ylmethyl)-1H-thieno[2,3-d]imidazole-5-carboxylate (1f)

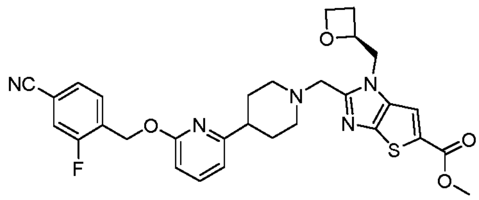

**1f**

At room temperature, 1f-1 (527.0 mg, 1.7 mmol) was dissolved in acetonitrile (15 mL) solution, and then crude product 1e (508.0 mg, 1.7 mmol) and potassium carbonate (702.0 mg, 5.1 mmol) were added to the reaction system successively, and heated to 60°C for 18 h. The reaction liquid was cooled to room temperature, concentrated to remove the solvent. The crude product was dissolved in water (20 mL), and then extracted with ethyl acetate (20 mL × 4). The organic phase was washed with aqueous saturated NaCl solution, dried over anhydrous sodium sulfate, and concentrated by filtration. The obtained crude product was separated and purified by silica gel column chromatography (petroleum ether/ethyl acetate (v/v)=1:3), and concentrated to obtain 1f (135.0 mg, yield 13.8%).

LCMS m/z = 576.2 $[M+1]^+$.

Step 6: (S)-2-((4-(6-((4-cyano-2-fluorobenzyl)oxy)pyridin-2-yl)piperidin-1 -yl)methyl)-1 - (oxetan-2-ylmethyl)-1H-thieno[2,3-d]imidazole-5-carboxylic acid **(Compound 1)**

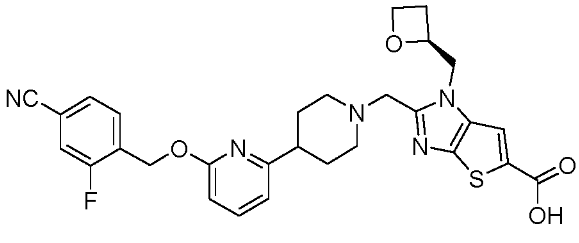

**Compound 1**

At room temperature, 1f (137.0 mg, 0.24 mmol) was dissolved in (6 mL) and water (3 mL) solution, then 1 g (CAS: 5807-14-7) (166.0 mg, 1.20 mmol) was added, and the reaction was continued for 24 h. 1*N* hydrochloric acid was used to adjust pH to 6, then dichloromethane:methanol = 10:1 (20 mL × 3) was used for extraction. The organic phase was combined, dried over anhydrous sodium sulfate, filtered and concentrated. The obtained crude product was subjected to preparative liquid chromatography to obtain (S)-2-((4-(6-((4-cyano-2-fluorobenzyl)oxy)pyridin-2-yl)piperidin-1-yl)methyl)-1-(oxetan-2-ylmethyl)-1H-thieno[2,3-d]imidazole-5-carboxylic acid (compound 1) trifluoroacetic acid salt (60 mg, yield 44.9%). Compound 1 was obtained by preparative thin layer chromatography (dichloromethane/methanol (v/v) = 10: 1).

Conditions for preparative liquid chromatography:

Instrument and preparative column: waters 2767 (preparative liquid phase chromatographic instrument) was used; the preparative column model was SunFire@Prep C18 (19 mm × 250 mm).
Preparation method: The crude product was dissolved in DMF and filtered with a 0.45 μm filter membrane to prepare into a sample liquid.
Mobile phase system: acetonitrile/water (containing 1% TFA). Gradient elution: acetonitrile content 20-65%, elution flow rate: 12 mL/min, elution time 18 min.

Compound 1:

LCMS m/z = 562.2 $[M+1]^+$.
$^1H$ NMR (400 MHz, $CD_3OD$) δ 7.79 (s, 1H), 7.70-7.61 (m, 2H), 7.60-7.53 (m, 2H), 6.90 (d, 1H), 6.75 (d, 1H), 5.53 (s, 2H), 5.26-5.17 (m, 1H), 4.74-4.65 (m, 2H), 4.62-4.55 (m, 1H), 4.52-4.40 (m, 3H), 3.67-3.55 (m, 2H), 3.15-3.03 (m, 2H), 2.98-2.87 (m, 1H), 2.85 - 2.75 (m, 1H), 2.56-2.45 (m, 1H), 2.11 - 1.99 (m, 4H).

**Example 2** (S)-2-((6-((4-cyano-2-fluorobenzyl)oxy)-3',6'-dihydro-[2,4'-bipyridin]-1'(2'H)-yl)methyl)-1-(oxetan-2-ylmethyl)-1H-thieno[2,3-d]imidazole-5-carboxylic acid (Compound 2)

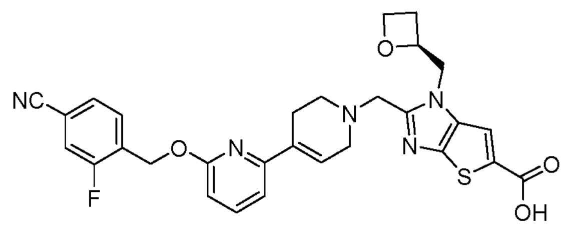

**Compound 2**

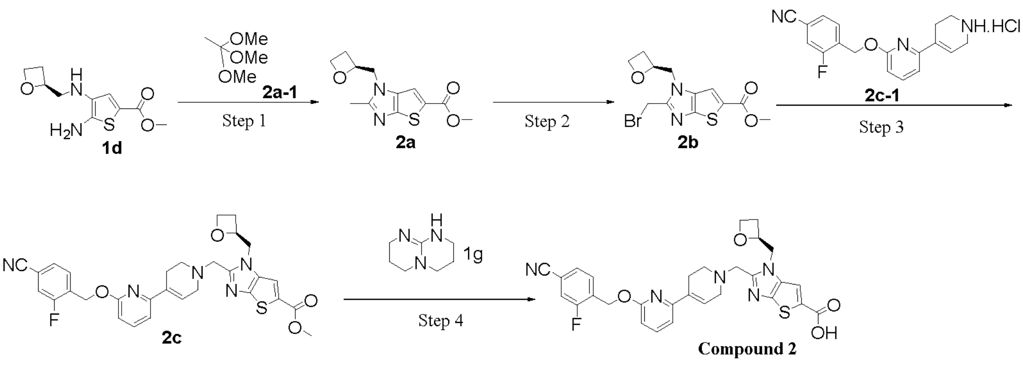


Step 1: methyl (S)-2-methyl-1-(oxetan-2-ylmethyl)-1H-thieno[2,3-d]imidazole-5-carboxylate (2a)

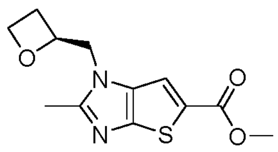

**2a**

At room temperature, 1d (1.2 g, 5.1 mmol) was added to glacial acetic acid (50 mL), 2a-1 (926.0 mg, 7.7 mmol) was added to the reaction liquid, and the temperature was raised to 70°C for 1 hour. The glacial acetic acid was distilled off under reduced pressure, and the obtained crude product was separated and purified by silica gel column chromatography (petroleum ether/ethyl acetate (v/v)=1:4), and concentrated to obtain 2a (680.0 mg, yield 50.1%).

LCMS m/z = 267.1 $[M+1]^+$.

$^1H$ NMR (400 MHz, $CDCl_3$) δ 7.68 (s, 1H), 5.19 - 5.11 (m, 1H), 4.66 - 4.58 (m, 1H), 4.36 - 4.29 (m, 1H), 4.26 (d, 2H), 3.89 (s, 3H), 2.79 - 2.68 (m, 1H), 2.63 (s, 3H), 2.44 - 2.34 (m, 1H).

Step 2: methyl (S)-2-(bromomethyl)-1-(oxetan-2-ylmethyl)-1H-thieno[2,3-d]imidazole-5-carboxylate (2b)

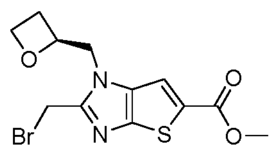

**2b**

At room temperature, 2a (680.0 mg, 2.6 mmol) was dissolved in 1,2-dichloroethane (50 mL) solution, then AIBN (84.0 mg, 0.5 mmol) was added, and NBS(1.1 g, 6.4 mmol) was added in portions in the process of slowly raising the temperature to 50°C. After the addition, the reaction liquid was heated to 70°C and continued to react for 8 h. The reaction liquid was cooled to room temperature, saturated sodium thiosulfate solution (30 mL) was added to quench the reaction and extracted with dichloromethane (30 mL × 3). The organic phase was combined, dried over anhydrous sodium sulfate, filtered and concentrated to obtain a crude product. The crude product was separated and purified by silica gel column chromatography (petroleum ether/ethyl acetate (v/v)=1:1), and concentrated to obtain 2b (440.0 mg, yield 51.5%).

LCMS m/z = 344.9 $[M+1]^+$.

Step 3: methyl (S)-2-((6-((4-cyano-2-fluorobenzyl)oxy)-3',6'-dihydro-[2,4'-bipyridin]-1'(2'H)-yl)methyl)-1-(oxetan-2-ylmethyl)-1H-thieno[2,3-d]imidazole-5-carboxylate (2c)

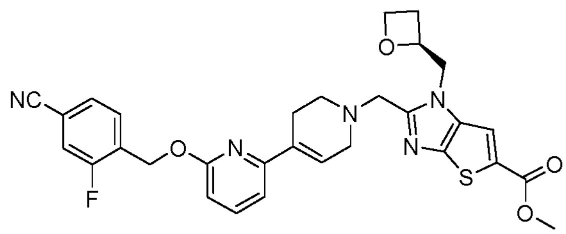

**2c**

At room temperature, 2c-1 (69.0 mg, 0.2 mmol) was dissolved in acetonitrile (5 mL) solution, then potassium carbonate (83.0 mg, 0.6 mmol) and 2b (69.0 mg, 0.2 mmol) were added, and the temperature was raised to 40°C for 4 h. The reaction liquid was filtered to remove the inorganic base, the filtrate was collected and concentrated to obtain a crude product, and the obtained crude product was separated and purified by silica gel column chromatography (dichloromethane/methanol (v/v) = 20:1), and concentrated to obtain 2c (75.0 mg, yield 66.0%).

LCMS m/z = 574.2 $[M+1]^+$.

Step 4: (S)-2-((6-((4-cyano-2-fluorobenzyl)oxy)-3',6'-dihydro-[2,4'-bipyridin]-1'(2'H)-yl)methyl)-1-(oxetan-2-ylmethyl)-1H-thieno[2,3-d]imidazole-5-carboxylic acid (Compound 2)

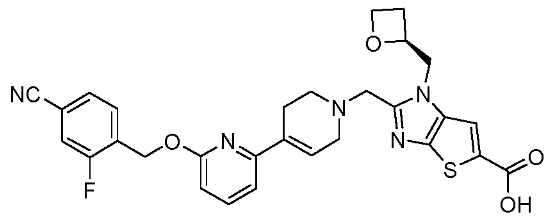

**Compound 2**

At room temperature, 2c (75.0 mg, 0.13 mmol) was dissolved in acetonitrile (5 mL) and water (1 mL), then 1 g (CAS: 5807-14-7) (91.0 mg, 0.66 mmol) was added, and the reaction was continued for 24 h. 1*N* hydrochloric acid was used to adjust pH to 6, then dichloromethane:methanol = 10:1 (20 mL × 3) was used for extraction. The organic phase was combined, dried over anhydrous sodium sulfate, filtered and concentrated to obtain a crude product. The obtained crude product was subjected to prep-HPLC to obtain (S)-2-((4-(6-((4-cyano-2-fluorobenzyl)oxy)pyridin-2-yl)piperidin-1-yl)methyl)-1-(oxetan-2-ylmethyl)-1H-thieno[2,3-d]imidazole-5-carboxylic acid (compound 2) trifluoroacetic acid salt and then preparative thin-layer chromatography (dichloromethane: methanol (v/v)=10: 1) to obtain the compound 2 (16.0 mg, yield 21.9%).

Preparation conditions:

instrument and preparative column: waters 2767 (preparative liquid phase chromatographic instrument) was used; the preparative column model was SunFire@Prep C18 (19 mm × 250 mm).

Preparation method: The crude product was dissolved in DMF and filtered with a 0.45 μm filter membrane to prepare into a sample liquid.

Mobile phase system: acetonitrile/water (containing 1% TFA). Gradient elution: acetonitrile content 20-65%, elution flow rate: 12 mL/min, elution time 18 min.

Compound 2:

LCMS m/z = 560.2 $[M+1]^+$.

$^1H$ NMR (400 MHz, $CD_3OD$) δ 7.73 (s, 1H), 7.69 - 7.60 (m, 2H), 7.59 - 7.51 (m, 2H), 7.07 (d, 1H), 6.73 (d, 1H), 6.70 - 6.65 (m, 1H), 5.53 (s, 2H), 5.22 - 5.14 (m, 1H), 4.72 - 4.53 (m, 3H), 4.45 - 4.37 (m, 1H), 4.10 - 3.99 (m, 2H), 3.37-3.33 (m, 2H), 2.95 - 2.87 (m, 2H), 2.77 - 2.69 (m, 1H), 2.66 - 2.57 (m, 2H),2.49 - 2.42 (m, 1H).

**Example** 3 2-(((S)-4-(6-((4-cyano-2-fluorobenzyl)oxy)pyridin-2-yl)-2-methylpiperazin-1-yl)methyl)-1-(((S)-oxetan-2-yl)methyl)-1H-thieno[2,3-d]imidazole-5-carboxylic acid (Compound 3)

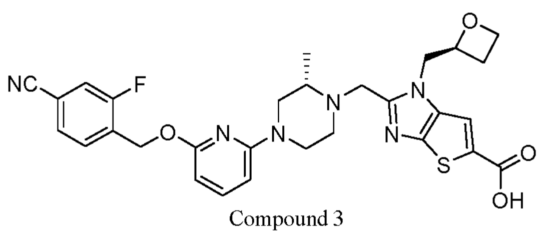

Compound 3

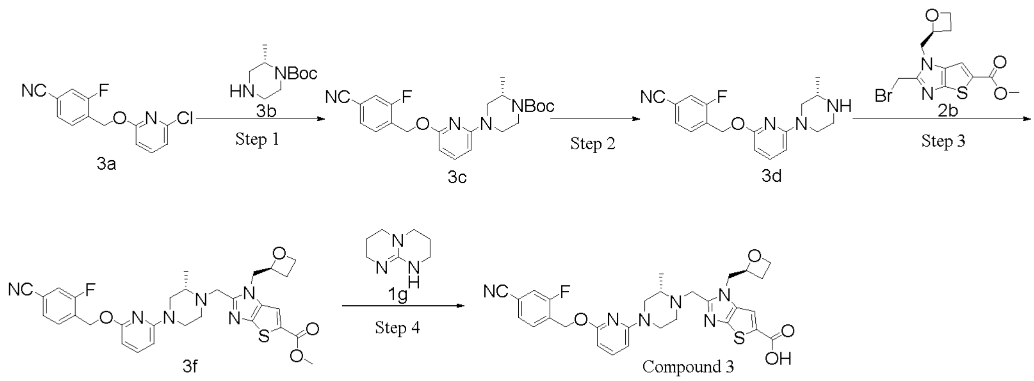


Step 1: tert-butyl (S)-4-(6-((4-cyano-2-fluorobenzyl)oxy)pyridin-2-yl)-2-methylpiperazine-1-carboxylate (3c)

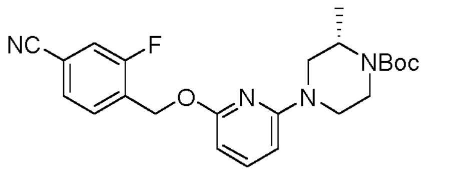

3c

3a (524 mg, 2.0 mmol), 3b (400 mg, 2.0 mmol), $Pd_2(dba)_3$ (192 mg, 0.2 mmol), RuPhos (2-bicyclohexylphosphine-2',6'-diisopropoxybiphenyl) (184 mg, 0.4 mmol) and cesium carbonate (1.3 g, 4.0 mmol) were added to 1,4-dioxane (20 mL). The reaction flask was placed at 90°C and stirred for 6 hours after nitrogen replacement for protection. Then the reaction liquid was cooled to room temperature and quenched by pouring into aqueous saturated ammonium chloride solution (100 mL), then extracted with ethyl acetate (30 mL × 3), and the organic phase was dried over anhydrous sodium sulfate, filtered and spin-dried. The crude product was separated and purified by silica gel column chromatography (petroleum ether/ethyl acetate (v/v) = 5:1), and concentrated to obtain 3c (700 mg, yield 82%).

LCMS m/z = 427.3$[M+1]^+$.

Step 2: (S)-3-fluoro-4-(((6-(3-methylpiperazin-1-yl)pyridin-2-yl)oxy)methyl) benzonitrile (3d)

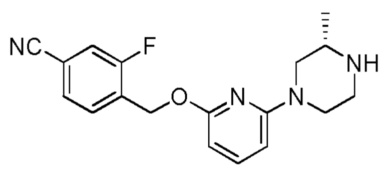

3d

3c (213 mg, 0.5 mmol) was added into a mixed solvent of dichloromethane (6 mL) and trifluoroacetic acid (1 mL), and stirred at 15°C for 1 hour. The reaction liquid was directly concentrated under reduced pressure to obtain 3d trifluoroacetic acid salt (230 mg, crude product), which was directly used in the next reaction without further purification.

LCMS m/z = 327.1 $[M+1]^+$.

Step 3: methyl 2-(((S)-4-(6-((4-cyano-2-fluorobenzyl)oxy)pyridin-2-yl)-2-methylpiperazin-1-yl)methyl)-1-(((S)-oxetan-2-yl)methyl)-1H-thieno[2,3-d]imidazole-5-carboxylate (3f)

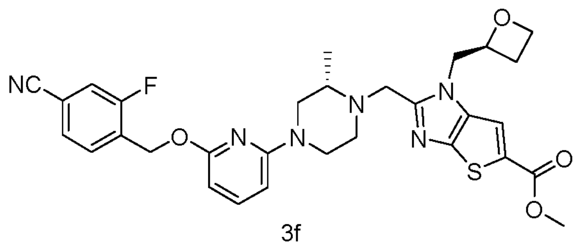

3f

3d (40 mg, 0.11 mmol) and 2b (38 mg, 0.11 mmol) and potassium carbonate (47 mg, 0.33 mmol) were added into acetonitrile (3 mL), and stirred at 12°C for 7 hours. The reaction system was quenched by adding into an aqueous saturated ammonium chloride solution (20 mL), then extracted with ethyl acetate (30 mL × 3). The organic phase was combined, dried over anhydrous sodium sulfate and filtered and the filtrate was spin-dried. Separation and purification by silica gel column chromatography (dichloromethane/methanol (v/v) = 20:1) was performed to give 3f (40 mg, yield 60%).
LCMS m/z = 591.2[M+1]$^+$.

Step 4: 2-(((S)-4-(6-((4-cyano-2-fluorobenzyl)oxy)pyridin-2-yl)-2-methylpiperazin-1 - yl)methyl)-1-(((S)-oxetan-2-yl)methyl)-1H-thieno[2,3-d]imidazole-5-carboxylic acid (Compound 3)

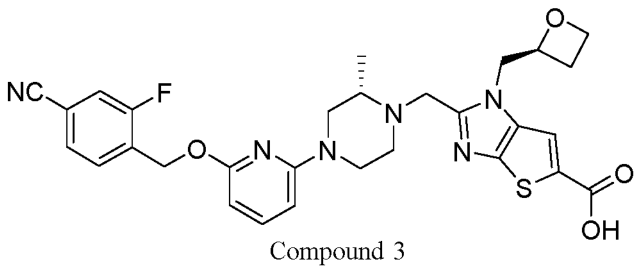

Compound 3

3f (40 mg, 0.067 mmol) and 1 g (47 mg, 0.033 mmol) were added to a mixed solution of acetonitrile (3 mL) and water (0.6 mL), and stirred at 12°C for 10 hours. The reaction liquid was then adjusted to pH = 6 with 17V hydrochloric acid, then extracted with ethyl acetate (10 mL × 3). The organic phase was combined and dried over anhydrous sodium sulfate, filtered and spin-dried, and prep-HPLC was performed to give 2-((S)-4-(6-((4-cyano-2-fluorobenzyl)oxy)pyridin-2-yl)-2-methylpiperazin-1-yl)methyl)-1-((S)-oxetan-2-yl)methyl)-1H-thieno[2,3-d]imidazole-5-carboxylic acid (compound 3) trifluoroacetate salt. After separation by silica gel column chromatography (dichloromethane/methanol (v/v) = 20:1), compound 3 (10 mg, yield 25.6%) was obtained.
Preparation conditions:
instrument and preparative column: waters 2767 (preparative liquid phase chromatographic instrument) was used; the preparative column model was SunFire@Prep C18 (19 mm × 250 mm).
Preparation method: The crude product was dissolved in DMF and filtered with a 0.45 μm filter membrane to prepare into a sample liquid.
Mobile phase system: acetonitrile/water (containing 1% TFA). Gradient elution: acetonitrile content 20-65%, elution flow rate: 12 mL/min, elution time 18 min.
LCMS m/z = 577.2 [M+1]$^+$.
$^1$H NMR (400 MHz, $CD_3OD$) δ 7.80 (s, 1H), 7.61 (t, 1H), 7.53 (t, 2H), 7.44 (t, 1H), 6.27 (d, 1H), 6.13 (d, 1H), 5.43 (s, 2H), 5.27 - 5.21 (m, 1H), 4.75 - 4.68 (m, 1H), 4.65 - 4.57 (m, 2H), 4.43 - 4.32 (m, 2H), 3.85 - 3.79 (m, 1H), 3.76 - 3.70 (m, 1H), 3.61 - 3.56 (m, 1H), 3.12 - 3.03 (m, 1H), 2.93 - 2.85 (m, 1H), 2.80- 2.71 (m, 2H), 2.63 - 2.57 (m, 1H), 2.51 - 2.43 (m, 1H), 2.40-2.32 (m, 1H), 1.18 (d, 3H).

**Example 4** 2-((4-(2-(4-chloro-2-fluorophenyl)-2-methylbenzo[d][1,3]dioxol-4-yl)piperidin-1-yl)methyl)-1-(((S)-oxetan-2-yl)methyl)-1H-thieno[2,3-d]imidazole-5-carboxylic acid (Compound 4)

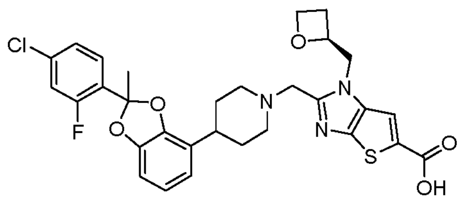

Compound 4

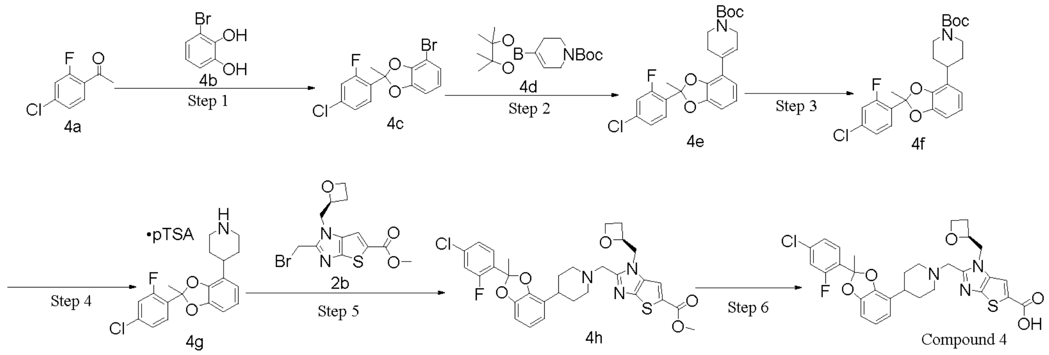


Step 1: 4-Bromo-2-(4-chloro-2-fluorophenyl)-2-methylbenzo[d][1,3]dioxole (4c)

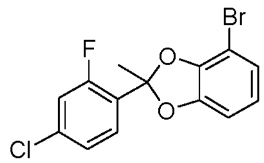

4c

Substrates 4a (41.0 g, 0.2 mol) and 4b (45.0 g, 0.237 mol), and p-toluenesulfonic acid monohydrate (900 mg, 4.75 mmol) were added to toluene (200 mL), subjected to nitrogen replacement for protection, and placed at 140°C for reflux and water separation for 60 hours. The reaction liquid was cooled to room temperature, spin-dried, and the residue was separated and purified by silica gel column chromatography to obtain 4c (18.0 g, yield 22%).

Step 2: tert-butyl 4-(2-(4-chloro-2-fluorophenyl)-2-methylbenzo[d][1,3]dioxol-4-yl)-3,6-dihydropyridine-1(2H)-carboxylate (4e)

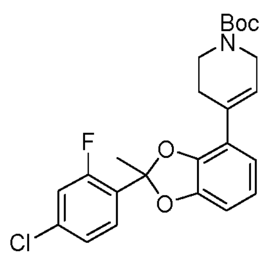

4e

4b (2.0 g, 5.82 mmol) and 4c (1.62 g, 5.24 mmol), Pd(dppf)$Cl_2$ (470 mg, 0.58 mmol) and sodium carbonate (3.08 g, 29.1 mmol) were added to a mixed solvent of 1,4-dioxane (30 mL) and water (8 mL), subjected to nitrogen replacement for protection, stirred at 90°C for 20 hours, cooled to room temperature, and extracted with ethyl acetate (80 mL × 3). The organic phase was combined, dried over anhydrous sodium sulfate, filtered, and spin-dried, and the

residue was separated and purified by silica gel column chromatography to obtain 4e (1.6 g, yield 62%).

LCMS m/z = 390.0[M-55]$^+$.

Step 3: tert-butyl 4-(2-(4-chloro-2-fluorophenyl)-2-methylbenzo[d][1,3]dioxol-4-yl)piperidine-1-carboxylate (4f)

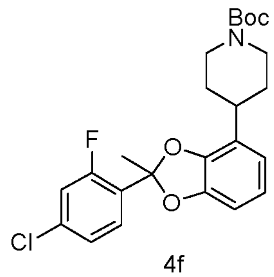

4f

4e (1.6 g, 3.59 mmol) and Raney nickel (160 mg) were added to a mixed solvent of methanol (15 mL) and tetrahydrofuran (10 mL), subjected to hydrogen replacement for protection, and stirred at room temperature for 8 hours. The reaction liquid was filtered and spin-dried, and the residue was separated and purified by silica gel column chromatography to obtain 4f (1.4 g, yield 87%).

LCMS m/z = 392.2[M-55]$^+$.

Step 4: 4-(2-(4-chloro-2-fluorophenyl)-2-methylbenzo[d][1,3]dioxol-4-yl)piperidine P-toluenesulfonate (4g)

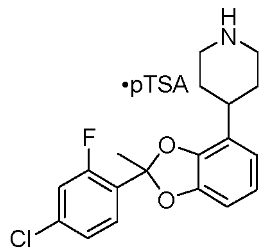

4g

4f (0.8 g, 1.79 mmol) and p-toluenesulfonic acid monohydrate (410 mg, 2.15 mmol) were added to ethyl acetate (20 mL), subjected to nitrogen replacement for protection, and stirred at 45°C for 12 hours. The reaction liquid was cooled to room temperature, filtered, and washed with ethyl acetate to obtain the p-toluenesulfonate 4g (620 mg, yield 99%) as a solid.

LCMS m/z = 348.0 [M+1]$^+$.

Step 5: methyl 2-((4-(2-(4-chloro-2-fluorophenyl)-2-methylbenzo[d][1,3]dioxol-4-yl)piperidin-1-yl) methyl)-1-(((S)-oxetan-2-yl)methyl)-1H-thieno[2,3-d]imidazole-5-carboxylate (4f)

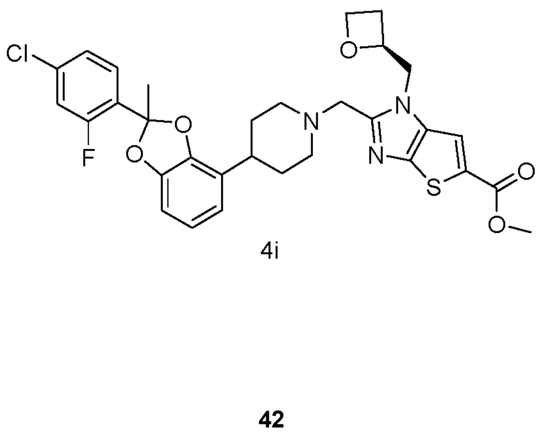

4i

4g (150 mg, 0.3 mmol) and 2b (103 mg, 0.3 mmol), and potassium carbonate (210 mg, 1.5 mmol) were added into acetonitrile (5 mL) and stirred at room temperature for 5 hours. The reaction liquid was directly mixed with silica gel, and separated and purified by silica gel column chromatography to obtain 4i (200 mg, yield 76%).
LCMS m/z = 612.3 $[M+1]^+$.

Step 6: 2-((4-(2-(4-chloro-2-fluorophenyl)-2-methylbenzo[d][1,3]dioxol-4-yl)piperidin-1-yl)methyl)-1-(((S)-oxetan-2-yl)methyl)-1H-thieno[2,3-d]imidazole-5-carboxylic acid (Compound 4)

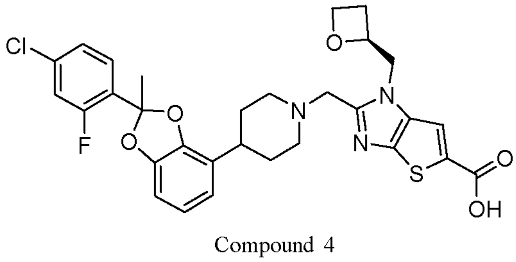

Compound 4

4i (200 mg, 0.33 mmol) and lithium hydroxide monohydrate (140 mg, 3.3 mmol) were added to a mixed solvent of acetonitrile (4 mL) and water (1 mL), and stirred at 50°C for 8 hours. The reaction liquid was cooled to room temperature, adjusted with 1N hydrochloric acid to pH=6, and extracted with ethyl acetate. The organic phase was dried with anhydrous sodium sulfate, filtered and spin-dried. The residue was separated and purified by silica gel column chromatography to obtain the product (2-((4-(2-(4-chloro-2-fluorophenyl)-2-methylbenzo[d][1,3]dioxol-4-yl)piperidine-1-yl)methyl)-1-((S)-oxetan-2-yl)methyl)-1H-thieno[2,3-d]imidazole-5-carboxylic acid (compound 4) (50 mg, yield 25%).
LCMS m/z = 598.1 $[M+1]^+$.
$^1$H NMR (400 MHz, DMSO-$d_6$) $\delta$ 7.73 (s, 1H),7.63 - 7.51 (m, 2H), 7.34 (d, 1H), 6.83- 6.70 (m, 3H), 5.15 - 5.04 (m, 1H), 4.67 - 4.58 (m, 1H), 4.55 - 4.43 (m, 2H), 4.41 - 4.33(m, 1H), 3.80 (d, 1H), 3.69 (d, 1H), 2.97 (d, 1H), 2.87 (d, 1H), 2.73- 2.59 (m, 2H), 2.46 - 2.37 (m, 1H), 2.23 - 2.09 (m, 2H), 2.02 (s, 3H), 1.83 - 1.61 (m, 4H).

**Example 5** 2-((4-(2-(4-cyano-2-fluorophenyl)-2-methylbenzo[d][1,3]dioxol-4-yl)piperidin-1-yl)methyl)-1-(((S)-oxetan-2-yl)methyl)-1H-thieno[2,3-d]imidazole-5-carboxylic acid (Compound 5)

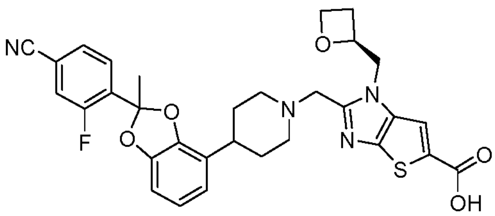

Compound 5

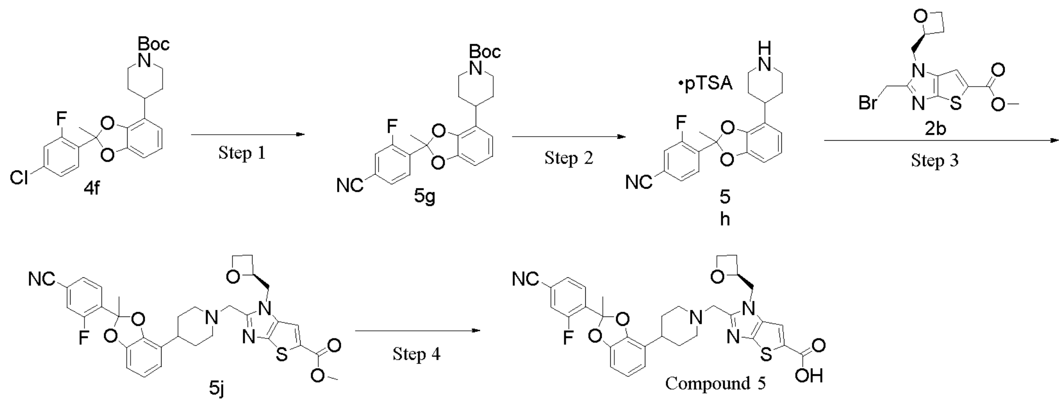


Step 1: tert-butyl 4-(2-(4-cyano-2-fluorophenyl)-2-methylbenzo[d][1,3]dioxol-4-yl)piperidine-1-carboxylate (5g)

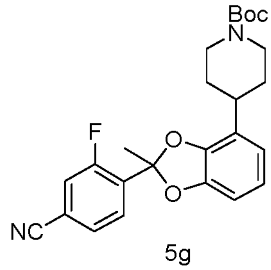

5g

4f(600 mg, 1.34 mmol) and $Pd_2(dba)_3$ (65 mg, 0.07 mmol), dppf (84 mg, 0.15 mmol), $Zn(CN)_2$ (264 mg, 2.25 mmol), and zinc powder (24 mg, 0.37 mmol) were added to DMA (15 mL), subjected to nitrogen replacement for protection, and stirred at 120°C for 20 hours. The reaction liquid was cooled to room temperature, filtered and spin-dried, and the residue was separated and purified by silica gel column chromatography to obtain 5 g (150 mg, yield 23%).

LCMS m/z = 383.0$[M-55]^+$.

Step 2: 4-(2-(4-cyano-2-fluorophenyl)-2-methylbenzo[d][1,3]dioxol-4-yl)piperidine P-toluenesulfonate (5h)

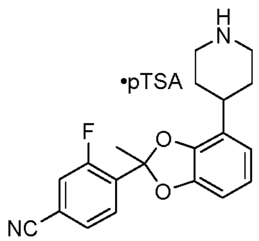

5h

5g (200 mg, 0.46 mmol) and p-toluenesulfonic acid monohydrate (130 mg, 0.69 mmol) were added to ethyl acetate (8 mL), subjected to nitrogen replacement for protection, and stirred at 45°C for 12 hours. The reaction liquid was cooled to room temperature, filtered, and washed with ethyl acetate to obtain the p-toluenesulfonate 5h (250 mg, yield 90%) as a solid.

LCMS m/z = 339.2 $[M+1]^+$.

Step 3: methyl 2-((4-(2-(4-cyano-2-fluorophenyl)-2-methylbenzo[d][1,3]dioxol-4-yl)piperidin-1-yl) methyl)-1-(((S)-oxetan-2-yl)methyl)-1H-thieno[2,3-d]imidazole-5-carboxylate (5j)

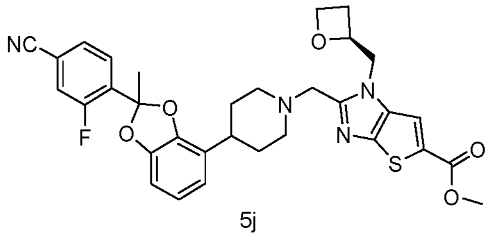

5j

5h (120 mg, 0.2 mmol) and 2b (82mg, 0.22mmol), and potassium carbonate (160 mg, 1.1 mmol) were added into acetonitrile (5 mL) and stirred at room temperature for 5 hours. The reaction liquid was directly mixed with silica gel, and separated and purified by silica gel column chromatography to obtain 5j (100 mg, yield 83%).
LCMS m/z = 603.2 $[M+1]^+$.

Step 4: 2-((4-(2-(4-cyano-2-fluorophenyl)-2-methylbenzo[d][1,3]dioxol-4-yl)piperidin-1-yl)methyl)-1-(((S)-oxetan-2-yl)methyl)-1H-thieno[2,3-d]imidazole-5-carboxylic acid (Compound 5)

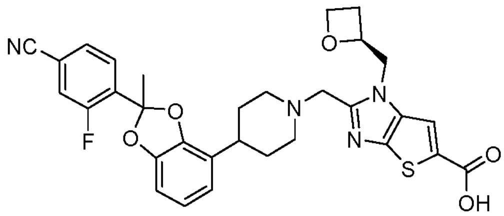

Compound 5

5j (100mg, 0.0.17 mmol) and lithium hydroxide monohydrate (115 mg, 2.73 mmol) were added to a mixed solvent of acetonitrile (4 mL) and water (1 mL), and stirred at 50°C for 8 hours. The reaction liquid was cooled to room temperature, adjusted with 1N hydrochloric acid to pH=6, and extracted with ethyl acetate. The organic phase was dried with anhydrous sodium sulfate, filtered and spin-dried. The residue was separated and purified by silica gel column chromatography to obtain (2-((4-(2-(4-cyano-2-fluorophenyl)-2-methylbenzo[d][1,3]dioxol-4-yl)piperidine-1-yl)methyl)-1-((S)-oxoalkane-2-yl)methyl)-1H-thieno[2,3-d]imidazole-5-carboxylic acid (compound 5) (50 mg, 48% yield).
LCMS m/z = 589.2 $[M+1]^+$.
$^1$H NMR (400 MHz, DMSO-$d_6$) δ 12.94 (br, 1H), 7.97 (d, 1H), 7.81 (s, 1H), 7.77 - 7.69 (m, 2H), 6.85- 6.70 (m, 3H), 5.14 - 5.05 (m, 1H), 4.69 - 4.61 (m, 1H), 4.57-4.44 (m, 2H), 4.40 - 4.32(m, 1H), 3.82 (d, 1H), 3.72 (d, 1H), 2.98 (d, 1H), 2.88 (d, 1H), 2.73- 2.60 (m, 2H), 2.45- 2.36 (m, 1H), 2.24 - 2.10 (m, 2H), 2.05 (s, 3H), 1.79 - 1.63 (m, 4H).

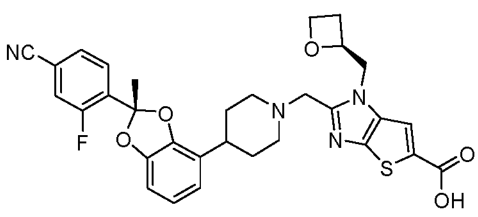

**Compound 5-a**

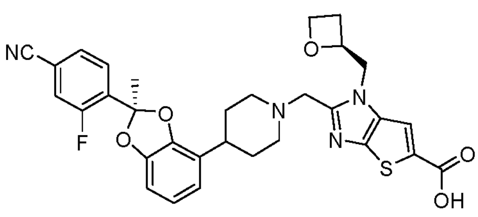

**Compound 5-b**

2g compound 5 was prepared by SFC and dried to obtain **compound 5-1** (600 mg, yield 30%) as a white solid with a retention time of 6.441 minutes. **compound 5-2** (500 mg, yield 25%) as a white solid with a retention time of 7.104 minutes.
Preparation conditions:
instrument and preparative column: Waters 150 MGM chiral liquid phase chromatographic instrument was used, and the preparative column model was DAICEL CHIRALPAK AD, I.D., 5 μm, inner diameter × length = 250 × 30 mm.
Preparation method: The crude product was dissolved in ethanol and filtered with a 0.45 μm filter membrane to prepare into a sample liquid.

Mobile phase system: carbon dioxide/ethanol (0.1% $NH_3$ $H_2O$). Gradient elution: the ethanol content was 25%, and the elution time was 11 minutes.

**Compound 5-1:**

LCMS m/z = 589.2 [M+1]$^+$.

$^1$H NMR (400 MHz, DMSO-$d_6$) δ 8.01-7.93 (m, 1H), 7.79(s, 1H),7.77-7.70 (m, 2H), 6.84-6.69 (m, 3H), 5.15-5.06 (m, 1H), 4.69-4.60 (m, 1H), 4.57-4.44 (m, 2H), 4.42-4.34(m, 1H), 3.85-3.78 (m, 1H), 3.76-3.68 (m, 1H), 3.02-2.93 (m, 1H), 2.92-2.84 (m, 1H), 2.74-2.58 (m, 2H), 2.46-2.36 (m, 1H), 2.25-2.10(m, 2H), 2.05 (s, 3H), 1.81-1.64 (m, 4H).

The structure of compound 5-1 is one of the above formulas 5-a and 5-b;

**Compound 5-2:**

LCMS m/z = 589.2 [M+1]$^+$.

$^1$H NMR (400 MHz, DMSO-$d_6$) δ 8.00-7.93 (m, 1H), 7.77-7.67 (m, 3H), 6.84-6.72 (m, 3H), 5.13-5.04 (m, 1H), 4.67-4.58 (m, 1H), 4.56-4.44 (m, 2H), 4.41-4.33(m, 1H), 3.83-3.77 (m, 1H), 3.74-3.67 (m, 1H), 3.01-2.93 (m, 1H), 2.91-2.83 (m, 1H), 2.72-2.59 (m, 2H), 2.46-2.35 (m, 1H), 2.23-2.10 (m, 2H), 2.05 (s, 3H), 1.79-1.61 (m, 4H).

The structure of compound 5-2 is one of the above formulas 5-a and 5-b; and compound 5-2 and compound 5-1 were isomers for each other, that is, when the structure of compound 5-1 was the structure of formula 5-a, the structure of compound 5-2 was the structure of formula 5-b; when the structure of compound 5-1 was the structure of formula 5-b, the structure of compound 5-2 was the structure of formula 5-a.

**Example** 6 (S)-2-(4-(6-((4-cyano-2-fluorobenzyl)oxy)pyridin-2-yl)-2-fluorobenzyl)-1-(oxetan-2-ylmethyl)-1H-thieno[2,3-d]imidazole-5-carboxylic acid (Compound 6)

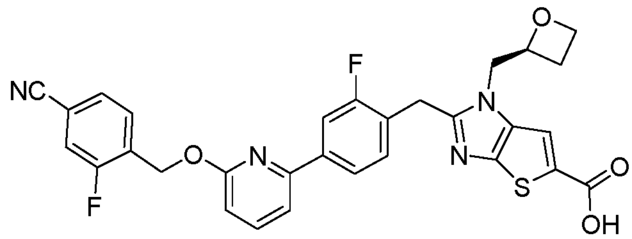

Compound 6

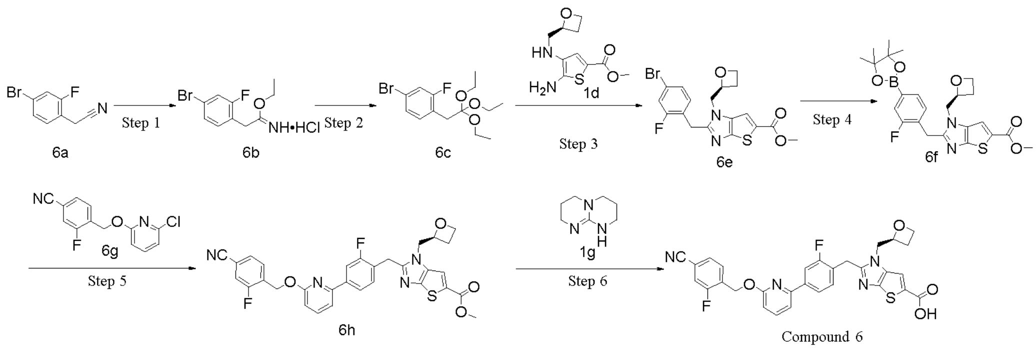


Step 1: ethyl 2-(4-bromo-2-fluorophenyl)acetimidate hydrochloride (6b)

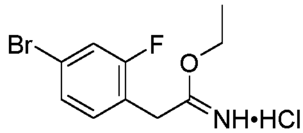

6b

6a (5.0 g, 26.04 mmol) was added to anhydrous ethanol (3.0 mL), placed at 0°C and fully stirred, and at the same time, dry hydrogen chloride gas was introduced into the reaction liquid until it was saturated, and the reaction liquid was continuously stirred at this temperature for 10 hours. Diethyl ether (40 mL) was then added to the reaction liquid, filtered to obtain a solid that was further washed with diethyl ether (100 mL × 2 ), and dried to obtain 6b (5.0 g, yield 72%).

$^{1}$H NMR (400 MHz, DMSO-$d_6$) δ 12.04 (s, 2H), 7.61 (d, 1H), 7.51 - 7.43 (m, 2H), 4.46 (q, 2H), 4.14 (s, 2H), 1.26 (t, 3H).

$^{19}$F NMR (400 MHz, DMSO-$d_6$) δ 111.30.

Step 2: 4-bromo-2-fluoro-1-(2,2,2-triethoxyethyl)benzene (6c)

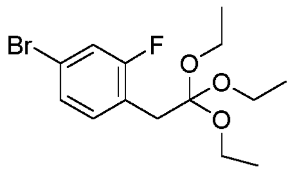

6c

6b (5.0 g, 19.30 mmol) was added into anhydrous ethanol (40 mL), and stirred at room temperature (15°C) for 48 hours. Then the reaction liquid was directly filtered to obtain a filtrate, and the filtrate was spin-dried to make a slurry with a mixed solvent of petroleum ether and ethyl acetate (100 mL) (petroleum ether/ethyl acetate (v/v) = 10:1) and filtered, and the filtrate was spin-dried to obtain 6c (4.0 g, yield 66%).

$^{1}$H NMR (400 MHz, $CDCl_3$) 7.45 (t, 1H), 7.23 - 7.14 (m, 2H), 3.50 (q, 6H), 3.07 (s, 2H), 1.16 (t, 9H).

Step 3: methyl (S)-2-(4-bromo-2-fluorobenzyl)-1-(oxetan-2-ylmethyl)-1H-thieno[2,3-d]imidazole-5-carboxylate (6e)

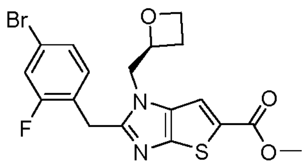

6e

6d (500 mg, 2.06 mmol) and 6c (1.0 g, 3.19 mmol) were added to glacial acetic acid (20 mL), and stirred at 60°C for 10 hours. The reaction system was cooled to room temperature, then was slowly added to an aqueous saturated sodium bicarbonate solution (200 mL) to quench the reaction, and then extracted with ethyl acetate (50 mL × 3). The organic phase was combined, dried over anhydrous sodium sulfate, and filtered. The filtrate was spin-dried, and the residue was separated and purified by silica gel column chromatography to obtain 6e (430 mg, yield 47%).

LCMS m/z = 439.0 [M+1]$^+$.

Step 4: methyl (S)-2-(2-fluoro-4-(4,4,5,5-tetramethyl-1,3,2-dioxaborolan-2-yl)benzyl)-1-(oxetan-2-ylmethyl)-1H-thieno[2,3-d]imidazole-5-carboxylate (6f)

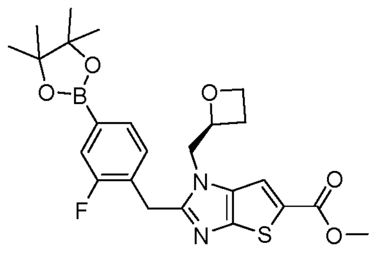

6f

6e (400 mg, 0.98 mmol) and pinacol biborate (262 mg, 1.0 mmol), Pd(dppf)$Cl_2$●DCM (81 mg, 0.1 mmol), and potassium acetate (196 mg, 2.0 mmol) were added to 1,4-dioxane (20 mL), and the reaction flask was subjected to nitrogen replacement for protection, and the reaction liquid in the reaction flask was stirred at 100°C for 5 hours. The reaction liquid was cooled to room temperature, then water (100 mL) was added and extracted with ethyl acetate (50 mL × 3). The organic phase was combined, dried over anhydrous sodium sulfate and filtered, the filtrate was spin-dried, and the residue was separated and purified by silica gel column chromatography to obtain 6f (340 mg, yield 71%).

LCMS m/z = 487.2 $[M+1]^+$.

Step 5: methyl (S)-2-(4-(6-((4-cyano-2-fluorobenzyl)oxy)pyridin-2-yl)-2-fluorobenzyl)-1-(oxetan-2-ylmethyl)-1H-thieno[2,3-d]imidazole-5-carboxylate (6h)

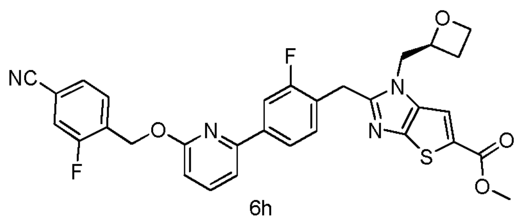

6h

6f (340 mg, 0.70 mmol) and 6g (220 mg, 0.84 mmol), Pd(dppf)$Cl_2$●DCM (83 mg, 0.1 mmol) and potassium carbonate (290 mg, 2.1 mmol) were added to a mixed solution of 1,4-dioxane (10 mL) and water (2 mL), and the reaction flask was subjected to nitrogen replacement for protection, and the reaction liquid in the reaction flask was stirred at 100°C for 5 hours. The reaction liquid was cooled to room temperature, then water (50 mL) was added, and extracted with ethyl acetate (30 mL × 3). The organic phase was combined, dried over anhydrous sodium sulfate, and filtered. The filtrate was spin-dried, and the residue was separated and purified by silica gel column chromatography to obtain 6h (200 mg, yield 48%).

LCMS m/z = 587.1 $[M+1]^+$.

Step 6: (S)-2-(4-(6-((4-cyano-2-fluorobenzyl)oxy)pyridin-2-yl)-2-fluorobenzyl)-1-(oxetan-2-ylmethyl)-1H-thieno[2,3-d]imidazole-5-carboxylic acid (Compound 6)

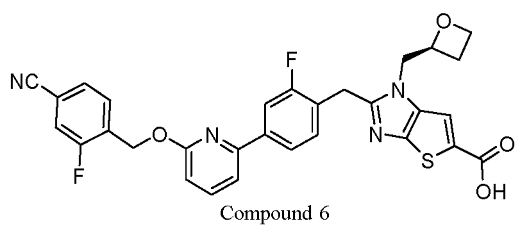

Compound 6

6h (100 mg, 0.17 mmol) and 1 g (120 mg, 0.85 mmol) were added to a mixed solution of acetonitrile (5 mL) and water (1 mL), and stirred at room temperature (12°C) for 10 hours. Then the reaction liquid was adjusted to pH=6 with 1M hydrochloric acid, continuously stirred until a white solid precipitated, and filtered and the solid was washed with

water. The solid was dissolved in dichloromethane, dried over anhydrous sodium sulfate and filtered. The organic phase was spin-dried. The residue was separated by preparative HPLC to give (S)-2-(4-(6-((4-cyano-2-fluorobenzyl)oxy)pyridin-2-yl)-2-fluorobenzyl)-1-(oxoalkane-2-ylmethyl)-1H-thieno[2,3-d]imidazole-5-carboxylic acid (compound 6) trifluoroacetate salt, which was separated and purified by silica gel column chromatography to obtain compound 6 (40 mg, yield 41%).

LCMS m/z = 573.1 $[M+1]^+$.

$^1H$ NMR (400 MHz, $CD_3OD$) δ 12.90 (br, 1H), 7.91 (d, 1H), 7.88 - 7.80 (m, 4H), 7.79 - 7.68 (m, 2H) 7.64 (d, 1H), 7.39 (t, 1H), 6.92 (d, 1H), 5.61 (s, 2H), 5.06 - 4.98 (m, 1H), 4.60(dd, 1H), 4.54 - 4.44 (m, 2H), 4.43 - 4.28 (m, 3H), 2.73 - 2.61 (m, 1H), 2.40- 2.27 (m, 1H).

**Example 7:** (S)-2-(4-(6-((4-cyano-2-fluorobenzyl)oxy)pyridin-2-yl)benzyl)-1-(oxetan-2-ylmethyl)-1H-thieno[2,3-d]imidazole-5-carboxylic acid (Compound 7)

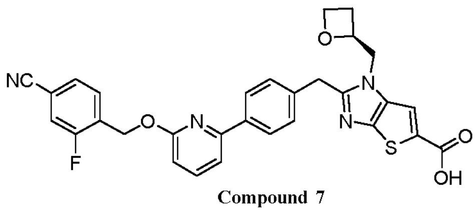

Compound 7

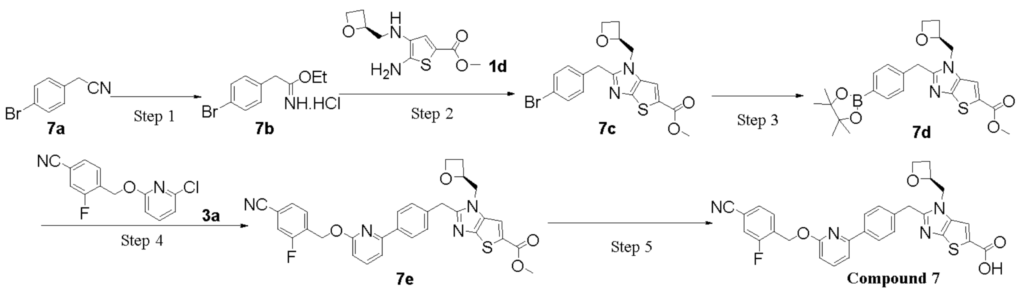


Step 1: ethyl 2-(4-bromophenyl)acetimidate hydrochloride (7b)

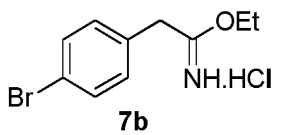

7b

Under the condition of ice bath, the substrate 7a (19.6 g, 100.0 mmol) was dissolved in a solution of 33% hydrochloric acid in ethanol (45 mL). After the addition, the temperature was naturally raised to room temperature for overnight reaction. At room temperature, anhydrous diethyl ether (100 mL) was added to the reaction system, stirred for 5 minutes and filtered, and the filter cake was washed with diethyl ether (20 mL × 2), collected and dried to obtain the product 7b of interest (22.0 g , yield 79.4%) as a white solid.

LCMS m/z = 242.1 $[M+1]^+$.

Step 2: methyl (S)-2-(4-bromobenzyl)-1-(oxetan-2-ylmethyl)-1H-thieno[2,3-d]imidazole-5-carboxylate (7c)

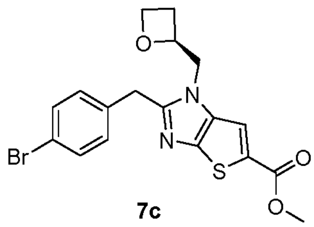

7c

At room temperature, the substrate 7b (1.4 g, 5.0 mmol) was dissolved in glacial acetic acid (50 mL), and 1d (1.2 g, 5.0 mmol) was added. After the addition, the reaction system was heated to 45°C for overnight reaction under nitrogen protection. The reaction solvent was distilled off under reduced pressure to obtain a crude product, which was separated and purified by silica gel column chromatography to obtain 7c (740.0 mg, yield 35.2%) as a white solid.
LCMS m/z = 421.0 $[M+1]^+$.

Step 3: methyl (S)-1-(oxetan-2-ylmethyl)-2-(4-(4,4,5,5-tetramethyl-1,3,2-dioxaborolan-2-yl) benzyl)-1H-thieno[2,3-d]imidazole-5-carboxylate (7d)

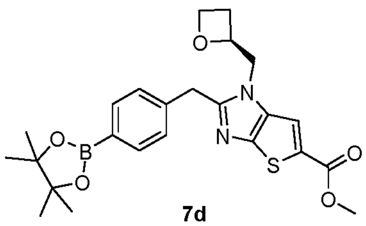

7d

At room temperature, 7c (740.0 mg, 1.8 mmol) was dissolved in 1,4-dioxane (30 mL), and then pinacol biborate (820.0 mg, 2.7 mmol), $Pd(dppf)Cl_2$ (147.0 mg, 0.2 mmol) and potassium acetate (519.0 mg, 5.3 mmol) were added successively. After the addition, the reaction system was heated to 100°C for overnight reaction under nitrogen protection. The reaction liquid was cooled to room temperature and filtered. The filter cake was washed with dichloromethane (10 mL×2). The filtrate was collected and concentrated to obtain a crude product, which was purified by silica gel column chromatography to obtain 7d (680.0 mg, yield 82.4%) as a yellow solid.
LCMS m/z = 469.2 $[M+1]^+$.

Step 4: methyl (S)-2-(4-(6-((4-cyano-2-fluorobenzyl)oxy)pyridin-2-yl)benzyl)-1-(oxetan-2-ylmethyl)-1H-thieno[2,3-d]imidazole-5-carboxylate (7e)

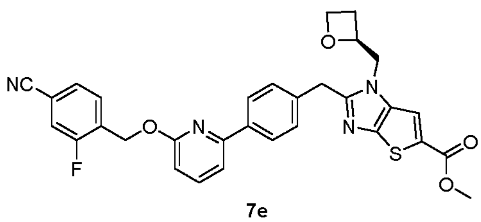

7e

At room temperature, 3a (130.0 mg, 0.5 mmol) was dissolved in a mixed solvent of 1,4-dioxane (10 mL) and water (1 mL), and then 7d (193.0 mg, 0.4 mmol) and $Pd(dppf)Cl_2$ (34.0 mg, 0.04 mmol) and cesium carbonate (261.0 mg, 0.8 mmol) were added successively. After the addition, the reaction system was heated to 100°C for overnight reaction under nitrogen protection. The reaction liquid was cooled to room temperature and filtered. The filter cake was washed with dichloromethane (10 mL × 2). The filtrate was collected and concentrated to obtain a crude product, which was separated and purified by silica gel column chromatography to obtain 7e (109.0 mg, yield 46.6% ) as a white solid.
LCMS m/z = 569.3 $[M+1]^+$.

Step 5: (S)-2-(4-(6-((4-cyano-2-fluorobenzyl)oxy)pyridin-2-yl)benzyl)-1-(oxetan-2-ylmethyl)-1H-thieno[2,3-d]imidazole-5-carboxylic acid (Compound 7)

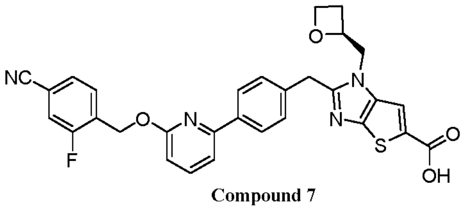

Compound 7

At room temperature, 7e (109.0 mg, 0.2 mmol) was dissolved in a mixed solvent of acetonitrile (5 mL) and water (1 mL), and then 1 g (CAS: 5807-14-7) (80.0 mg, 0.6 mmol) was added. After the addition, the reaction was carried out at room temperature for 24 hours. Acetonitrile was distilled off under reduced pressure. The crude product was dissolved in water (5 mL), adjusted to pH=6 with 1N hydrochloric acid. The aqueous phase was extracted with ethyl acetate (15 mL × 3). The organic phase was combined, dried over anhydrous sodium sulfate, filtered and concentrated to obtain a crude product, which was separated and purified by silica gel column chromatography to obtain compound 7 (26.0 mg, yield 24.5%) as a white solid.

Compound 7:

LCMS m/z = 555.2 $[M+1]^+$.

$^1$H NMR (400 MHz, DMSO-*d6*) δ 7.98 (d, 2H), 7.93 - 7.88 (m, 1H), 7.82 (t, 1H), 7.78 - 7.68 (m, 3H), 7.57 (d, 1H), 7.38 (d, 2H), 6.87 (d, 1H), 5.60 (s, 2H), 4.94 - 4.86 (m, 1H), 4.54 - 4.30 (m, 6H), 2.65 - 2.56 (m, 1H), 2.33 - 2.24 (m, 1H).

**Example 8** (S)-2-(4-(6-((4-cyano-2-fluorobenzyl)oxy)pyridin-2-yl)-2-fluoro-5-methylbenzyl)-1-(oxetan-2-ylmethyl)-1H-thieno[2,3-d]imidazole-5-carboxylic acid (Compound 8)

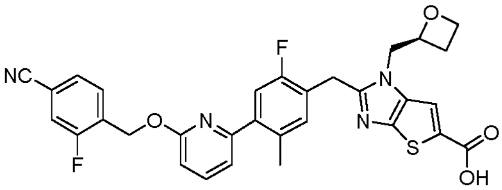

Compound 8

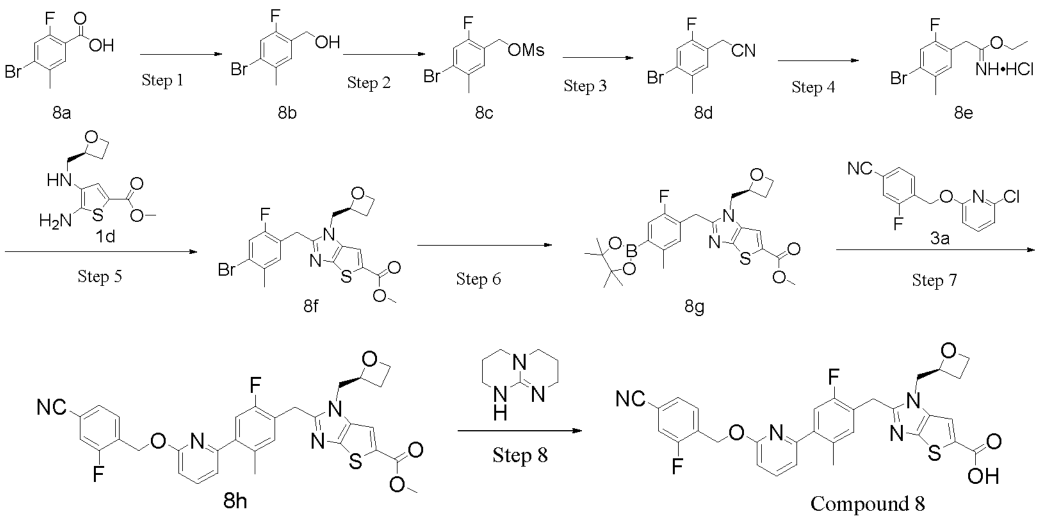

Step 1: (4-bromo-2-fluoro-5-methylphenyl)methanol (8b)

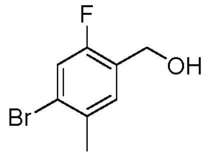

8b

8a (10.0 g, 43 mmol) and a 2M solution of borane in tetrahydrofuran (26 mL, 51.6 mmol) were added to tetrahydrofuran (40 mL) and stirred at room temperature for 30 hours. Then the reaction liquid was slowly added to 0.5 N hydrochloric acid (100 mL) to quench the reaction, and then extracted with ethyl acetate (80 mL × 3). The organic phase was dried over anhydrous sodium sulfate and filtered, and the liquid was concentrated to dryness. The residue was separated and purified by silica gel column chromatography to obtain 8b (9.4 g, yield 99%).

LCMS m/z = 201.0[M-17]$^+$.

Step 2: 4-bromo-2-fluoro-5-methylbenzyl methanesulfonate (8c)

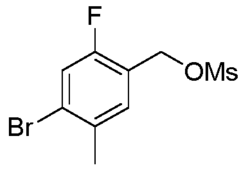

8c

8b (2.18 g, 10 mmol) and triethylamine (2.0 g, 20 mmol) were added to dry dichloromethane (20 mL) and stirred at 0°C for 10 minutes, and then methyl sulfonyl chloride (1.38 g, 12 mmol), was slowly added to the reaction system and the reaction was stirred at 0°C for further 30 minutes. Water (20 mL) was added to the reaction liquid, extracted with dichloromethane (50 mL × 3). The organic phase was dried over anhydrous sodium sulfate, filtered, and the filtrate was directly concentrated under reduced pressure to obtain 8c (3.0 g, yield 99%), which was directly used in the next reaction without further purification.

Step 3: 2-(4-bromo-2-fluoro-5-methylphenyl)acetonitrile (8d)

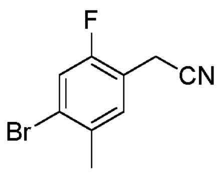

8d

8c (3.0 g, 10 mmol) and sodium cyanide (440 mg, 9 mmol) were added to DMF (15 mL), and stirred at room temperature for 40 hours. Ethyl acetate (300 mL) was added to the reaction system, washed with water (50 mL × 3). The aqueous phase was added to an aqueous sodium hypochlorite solution (50 mL) and stirred for 5 hours. The organic phase was combined, dried over anhydrous sodium sulfate and filtered. The filtrate was concentrated to dryness and purified by silica gel column chromatography to give 8d (1.5 g, yield 66%).

Step 4: ethyl 2-(4-bromo-2-fluoro-5-methylphenyl)acetimidate hydrochloride (8e)

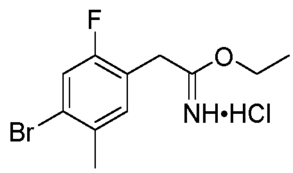

8e

8d (1.5g, 6.6 mmol) was added into ethanol (5 mL) and stirred at room temperature. Then, dry hydrogen chloride gas was introduced into the reaction liquid for 1 hour, and then continually stirred for 10 hours. Diethyl ether (10 mL) was added to the reaction liquid, stirred until a solid precipitated, and filtered. The filter cake was washed with diethyl ether and dried to obtain 8e (1.5 g, yield 73%).

Step 5: methyl (S)-2-(4-bromo-2-fluoro-5-methylbenzyl)-1-(oxetan-2-ylmethyl)-1H-thieno[2,3-d]imidazole-5-carboxylate (8f)

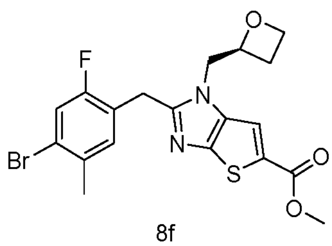

8f

8e (300 mg, 0.96 mmol) and 1d (250 mg, 1.0 mmol) were added to glacial acetic acid (10 mL), subjected to nitrogen replacement for protection and then stirred at 40°C for 10 hours. The reaction liquid was added to a aqueous saturated sodium bicarbonate solution to quench the reaction, and extracted with ethyl acetate (30 mL × 3). The organic phase was dried over anhydrous sodium sulfate and filtered. The liquid was concentrated to dryness, and the residue was separated and purified by silica gel column chromatography to obtain 8f (100 mg, yield 25%).

LCMS m/z = 453.0[M+1]$^+$.

Step 6: methyl (S)-2-(2-fluoro-5-methyl-4-(4,4,5,5-tetramethyl-1,3,2-dioxaborolan-2-yl) benzyl)-1-(oxetan-2-ylmethyl)-1H-thieno[2,3-d]imidazole-5-carboxylate (8g)

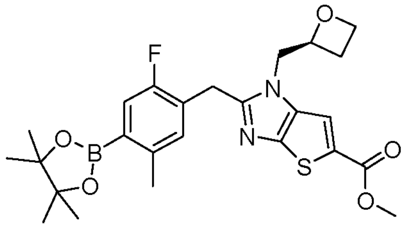

8g

8f (100 mg, 0.22 mmol), pinacol biborate (69 mg, 0.26 mmol), Pd(dppf)$Cl_2$ (18 mg, 0.022 mmol) and potassium acetate (45 mg, 0.44 mmol) were added to 1,4-dioxane (8 mL), subjected to nitrogen replacement for protection and then stirred at 100°C for 12 hours. Then the reaction liquid was cooled to room temperature, concentrated to dryness, and separated and purified by silica gel column chromatography to obtain 8g (70 mg, yield 63%).

LCMS m/z = 501.2[M+1]$^+$.

Step 7: methyl (S)-2-(4-(6-((4-cyano-2-fluorobenzyl)oxy)pyridin-2-yl)-2-fluoro-5-methylbenzyl)-1-(oxetan-2-ylmethyl)-1H-thieno[2,3-d]imidazole-5-carboxylate (8h)

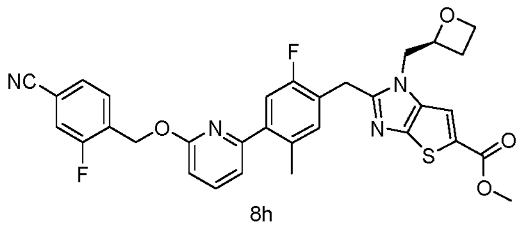

8h

8g (70 mg, 0.14 mmol), 3a (50 mg, 0.2 mmol), Pd(dppf)$Cl_2$ dichloromethane complex (18 mg, 0.02mmol) and potassium carbonate (55 mg , 0.4 mmol) were added to a mixed solvent of 1,4-dioxane (5 mL) and water (1 mL), subjected to nitrogen replacement for protection and then stirred at 100°C for 6 hours. The reaction liquid was cooled to room temperature, quenched with an aqueous saturated ammonium chloride solution (20 mL), and extracted with ethyl acetate (20 mL × 3). The organic phase was dried over anhydrous sodium sulfate, filtered, spin-dried, and separated and purified by silica gel column chromatography to obtain 8h (60 mg, yield 71%).
LCMS m/z = 601.3 [M+1]$^+$.

Step 8: (S)-2-(4-(6-((4-cyano-2-fluorobenzyl)oxy)pyridin-2-yl)-2-fluoro-5-methylbenzyl)-1-(oxetan-2-ylmethyl)-1H-thieno[2,3-d]imidazole-5-carboxylic acid (Compound 8)

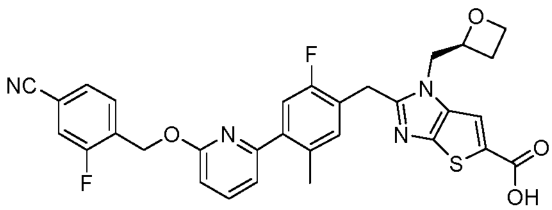

Compound 8

8h (60 mg, 0.1 mmol) and 1g (CAS: 5807-14-7) (70 mg, 0.5 mmol) were added into a mixed solvent of acetonitrile (5 mL) and water (1.0 mL), and stirred at 50°C for 5 hours. The reaction liquid was cooled to room temperature, adjusted to pH=6 with 1N hydrochloric acid, then extracted with ethyl acetate (20 mL × 3). The organic phase was combined, dried over anhydrous sodium sulfate and filtered and the liquid was concentrated to dryness under reduced pressure. The residue was separated and purified by silica gel plate chromatography to obtain compound 8 (10 mg, yield 17%).
LCMS m/z = 587.3 [M+1]$^+$.
$^1$H NMR (400 MHz, DMSO-*d6*) δ 12.89(s, 1H), 7.95 - 7.80 (m, 3H), 7.77-7.66 (m, 2H), 7.26 - 7.16 (m, 3H), 6.94(d, 1H), 6.51 (s, 2H), 5.06- 4.97 (m, 1H), 4.61 (m, 1H), 4.54 - 4.45 (m, 2H), 4.39 - 4.22 (m, 3H), 2.74 - 2.65 (m, 1H), 2.38- 2.30 (m, 1H), 2.22(s, 3H).

**Example 9:**

(S)-2-((4-(6-((4-cyano-2-fluorobenzyl)oxy)pyridin-2-yl)-6-oxopyridazin-1 (6H)-yl)methyl)-1-(oxetan-2-ylmethyl)-1H-thieno[2,3-d]imidazole-5-carboxylic acid (Compound 9)

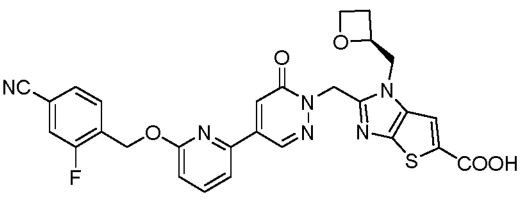

Compound 9

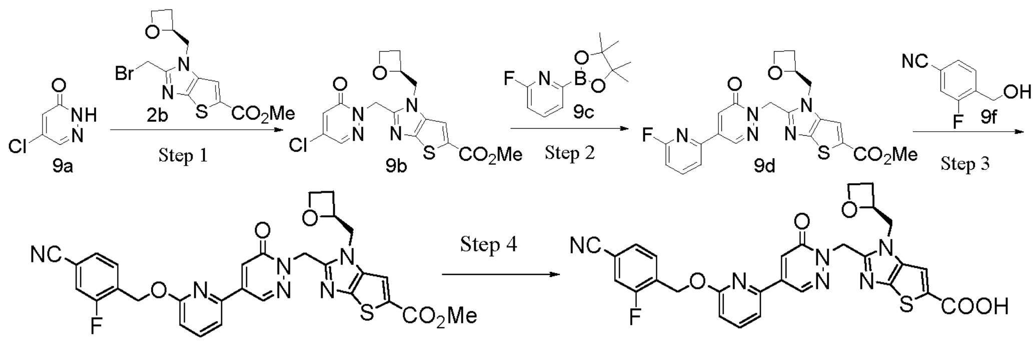


9a 2b 9b 9c 9d 9f 9h Compound 9

Step 1: methyl (S)-2-((4-chloro-6-oxopyridazin-1(6H)-yl)methyl)-1-(oxetan-2-ylmethyl)-1H-thieno[2,3-d]imidazole-5-carboxylate (9b)

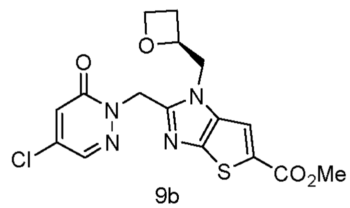

9b

The raw materials 9a (120 mg, 0.87 mmol) and 2b (300 mg, 0.87 mmol) were added into a reaction flask and dissolved in acetonitrile (10mL). Anhydrous potassium carbonate (250 mg, 1.80 mmol) was added, reacted at 40°C for 4h and cooled to room temperature. The solid was filtered, the filtrate was concentrated under reduced pressure, and the residue was purified by silica gel column chromatography to obtain product 9b (247 mg, yield 71.8%) as a yellow solid.

LCMS m/z = 395.0 $[M+H]^+$.

Step 2: methyl (S)-2-((4-(6-fluoropyridin-2-yl)-6-oxopyridazin-1(6H)-yl)methyl)-1-(oxetan-2-ylmethyl)-1H-thieno[2,3-d]imidazole-5-carboxylate (9d)

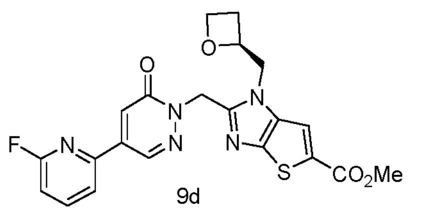

9d

9b (40 mg, 0.10 mmol), 9c (30 mg, 0.20 mmol), anhydrous potassium carbonate (40 mg, 0.20 mmol), and Pd(dppf)$Cl_2$ (15 mg, 0.01 mmol) were added to a reaction flask successively, dissolved in a mixed solution of 1,4-dioxane (2 mL)/water (0.4 mL), subjected to nitrogen replacement, stirred at 90°C for 5 h, cooled to room temperature and filtered. The filtrate was concentrated under reduced pressure, and the residue was purified by silica gel column chromatography

to obtain product 9d (22 mg, yield 48.3%) as a yellow solid.

LCMS m/z = 456.1 $[M+H]^+$.

Step 3: methyl (S)-2-((4-(6-((4-cyano-2-fluorobenzyl)oxy)pyridin-2-yl)-6-oxopyridazin-1(6H)-yl) methyl)-1-(oxetan-2-ylmethyl)-1H-thieno[2,3-d]imidazole-5-carboxylate (9h)

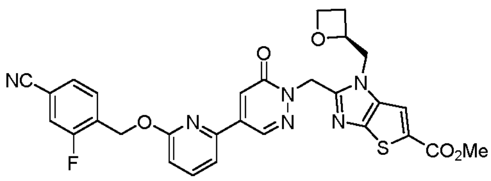

9h

9d (22 mg, 0.05 mmol) and 9f (15 mg, 0.09 mmol) were added into a reaction flask and dissolved in tetrahydrofuran solution (1 mL). Potassium tert-butoxide (10 mg, 0.07 mmol) was added under nitrogen, and stirred at room temperature for 1 h. The reaction was quenched with aqueous ammonium chloride solution (1 mL) and extracted with ethyl acetate 2 mL × 3. The organic phase was combined, dried over anhydrous sodium sulfate, filtered and concentrated, and the residue was separated and purified by silica gel column chromatography to obtain 9h (20 mg, yield 71.1 %) as a yellow solid.

LCMS m/z = 587.1 $[M+H]^+$.

Step 4: (S)-2-((4-(6-((4-cyano-2-fluorobenzyl)oxy)pyridin-2-yl)-6-oxopyridazin-1(6H)-yl)methyl)-1-(oxetan-2-ylmethyl)-1H-thieno[2,3-d]imidazole-5-carboxylic acid (Compound 9)

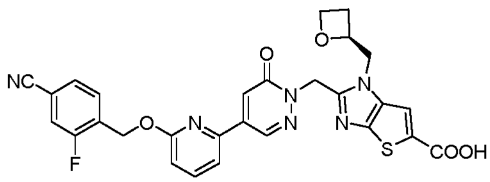

Compound 9

At room temperature, 9h (20 mg, 0.03 mmol) was dissolved in a solution of acetonitrile (2 mL) and water (0.5 mL), then 1 g (CAS: 5807-14-7) (40 mg, 0.28 mmol) was added, and the reaction was continued for 24 h. The reaction liquid was adjusted with 1N hydrochloric acid to pH=6, and then extracted with dichloromethane:methanol = 10:1 (2 mL × 3). The organic phase was combined, dried over anhydrous sodium sulfate, filtered and concentrated, and the residue was separated and purified by silica gel plate chromatography to obtain compound 9 (5 mg, yield 25.7%).

LCMS m/z = 573.1 $[M+H]^+$.

$^1H$ NMR (400 MHz, DMSO-*d6*) δ 13.14 - 12.78 (m, 1H), 8.62 (d, 1H), 7.98 - 7.68 (m, 6H), 7.57 (d, 1H), 7.08 (d, 1H), 5.68 - 5.50 (m, 4H), 5.04 (d, 1H), 4.70 (dd, 1H), 4.58 (d, 1H), 4.48 (dd, 1H), 4.33 (dd, 1H), 2.74-2.63 (m, 1H), 2.33 (dd, 1H).

**Example 10:** 2-(4-(2-(4-chloro-2-fluorophenyl)-2-methylbenzo[d][1,3]dioxol-4-yl)-2-fluorobenzyl)-1-(((S)-oxetan-2-yl)methyl)-1H-thieno[2,3-d]imidazole-5-carboxylic acid (Compound 10)

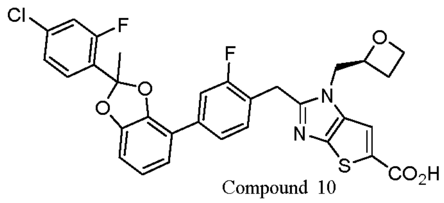

Compound 10

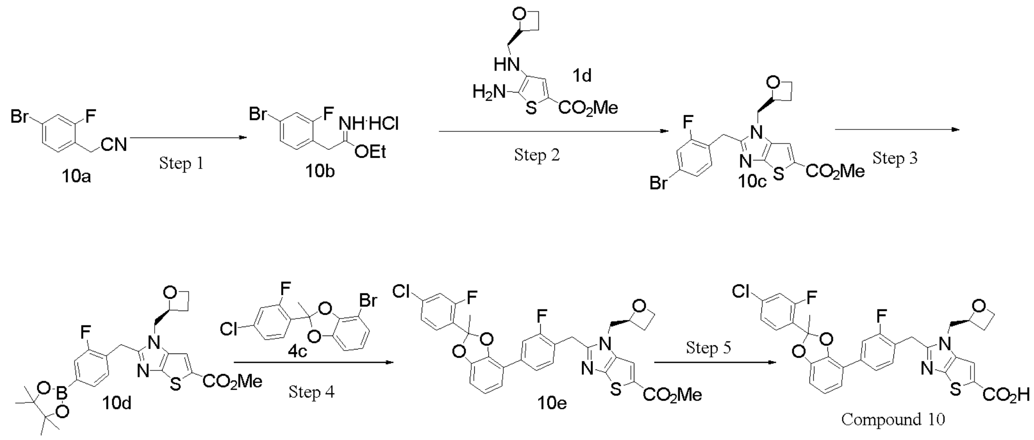


Step 1: ethyl 2-(4-bromo-2-fluorophenyl)acetimidate hydrochloride (10b)

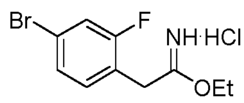

10b

10a (10.0 g, 52.1 mmol) was added into a reaction flask, and 33% hydrogen chloride-ethanol solution was introduced under ice bath, and then the temperature was gradually raised to room temperature for overnight reaction, and a large number of white solids were precipitated. Diethyl ether was added and stirred for 0.5h, the solid was filtered, washed with diethyl ether, and dried to obtain 10b (9.3 g, yield 60.6%) as a white solid.

Step 2: methyl (R)-2-(4-bromo-2-fluorobenzyl)-1-(oxetan-2-ylmethyl)-1H-thieno[2,3-d]imidazole-5-carboxylate (10c)

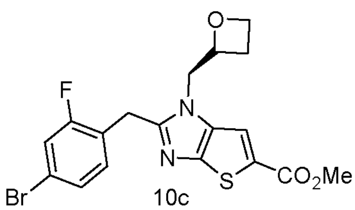

10c

10b (500 mg, 2.06 mmol) and 1d (620 mg, 2.10 mmol) were dissolved in glacial acetic acid (20 mL), subjected to nitrogen replacement, stirred at 45°C for 12 h, and cooled to room temperature. The reaction liquid was concentrated under reduced pressure, and the residue was separated and purified by silica gel column chromatography to obtain the product 10c of interest (350 mg, yield 39.9%) as a yellow solid.

LCMS m/z = 439.0 $[M+H]^+$.

Step 3: methyl 2-(2-fluoro-4-(4,4,5-trimethyl-1,3,2-dioxaborolan-2-yl)benzyl)-1-(((S)-oxetan-2-yl) methyl)-1H-thieno[2,3-d]imidazole-5-carboxylate (10d)

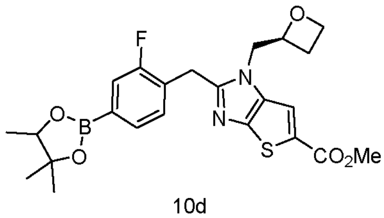

10d

10c (350 mg, 0.79 mmol) and pinacol diboronate (305 mg, 1.20 mmol), potassium acetate (235 mg, 2.40 mmol) and Pd(dppf)$Cl_2$ (65 mg, 0.08 mmol) were added to a reaction flask successively, dissolved in 1,4-dioxane solution (10 mL), subjected to nitrogen replacement, stirred at 90°C for 5 h, cooled to room temperature and filtered. The filtrate was concentrated under reduced pressure to obtain crude product 10d as a yellow oil, which was directly used in the next reaction without further purification.

Step 4: methyl 2-(4-(2-(4-chloro-2-fluorophenyl)-2-methylbenzo[d][1,3]dioxol-4-yl)-2-fluorobenzyl)-1-(((S)-oxetan-2-yl)methyl)-1H-thieno[2,3-d]imidazole-5-carboxylate (10e)

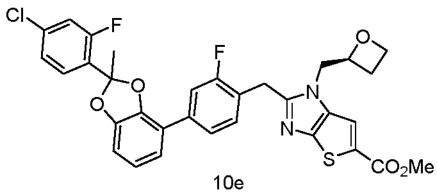

10e

The crude products 10d and 4c (344 mg, 1.01 mmol), anhydrous potassium carbonate (230 mg, 1.60 mmol) and Pd(dppf)$Cl_2$ (65 mg, 0.08 mmol) were added to a reaction flask successively, dissolved in a mixed solution of 1,4-dioxane (10 mL)/water (2 mL), subjected to nitrogen replacement, stirred at 90°C for 5 h and cooled to room temperature. the solid was filtered, the filtrate was concentrated under reduced pressure, and purified on a silica gel column to obtain the product 10e (350 mg, yield 71.2% over two steps) as a yellow solid.

LCMS m/z = 623.1 $[M+H]^+$.

Step 5: 2-(4-(2-(4-chloro-2-fluorophenyl)-2-methylbenzo[d][1,3]dioxol-4-yl)-2-fluorobenzyl)-1-(((S)-oxetan-2-yl)methyl)-1H-thieno[2,3-d]imidazole-5-carboxylic acid (Compound 10)

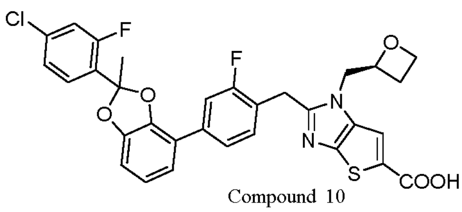

Compound 10

At room temperature, 10e (350 mg, 0.70 mmol) was dissolved in acetonitrile (5 mL) and water (1 mL), then lithium hydroxide (126.0 mg, 3.01 mmol) was added and the reaction was continued for 24 h. The reaction liquid was adjusted with 1N hydrochloric acid to pH=6, and then extracted with a mixed solvent of dichloromethane:methanol=10:1 (20 mL × 3). The organic phase was combined, dried over anhydrous sodium sulfate, concentrated by filtration, and concentrated under reduced pressure, and the residue was separated and purified by silica gel column chromatography to obtain compound 10 (100.0 mg, yield 29.3%) as a yellow solid.

LCMS m/z = 609.2 $[M+H]^+$.

$^1H$ NMR (400 MHz, DMSO-*d6*) δ 7.73 (br, 1H), 7.62-7.54 (m, 4H), 7.42-7.33 (m, 2H), 7.22-7.18 (m, 2H), 7.01-6.94 (m, 2H), 5.04-4.96 (m, 1H), 4.62-4.53 (m, 1H), 4.52-4.42 (m, 2H), 4.40-4.27 (m, 3H), 2.73-2.61 (m, 1H), 2.39-2.27 (m, 1H), 2.09 (s, 3H).

**Example 11:** 2-(4-(2-(4-chloro-2-fluorophenyl)-2-methylbenzo[d][1,3]dioxol-4-yl)benzyl)-1-(((S)-oxetan-2-yl)methyl)-1H-thieno[2,3-d]imidazole-5-carboxylic acid (Compound 11)

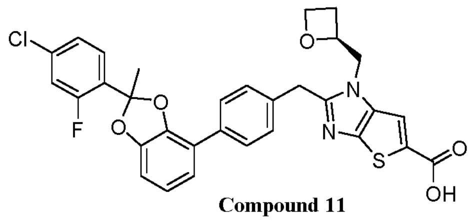

Compound 11

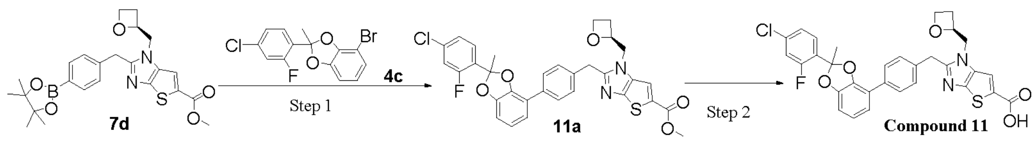


Step 1: methyl 2-(4-(2-(4-chloro-2-fluorophenyl)-2-methylbenzo[d][1,3]dioxol-4-yl)benzyl)-1-(((S)-oxetan-2-yl)methyl)-1H-thieno[2,3-d]imidazole-5-carboxylate (11a)

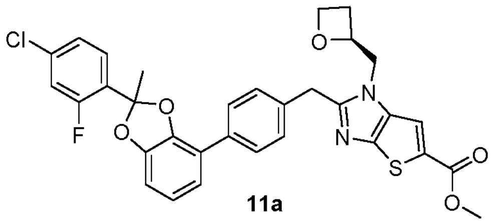

11a

At room temperature, substrate 4c (91.0 mg, 0.26 mmol) was dissolved in a mixed solvent of 1,4-dioxane (5 mL) and water (0.5 mL), and then 7d (103.0 mg, 0.22 mmol) and Pd(dppf)$Cl_2$ (18.0 mg, 0.02 mmol) and cesium carbonate (215.0 mg, 0.66 mmol) were added successively. After the addition, the reaction system was heated to 100°C for overnight reaction under nitrogen protection. The reaction liquid was cooled to room temperature and filtered. The filter cake was washed with dichloromethane (10 mL × 2). The filtrate was collected and concentrated to obtain a crude product, which was separated and purified by silica gel column chromatography to obtain product 11a of interest (110.0 mg, yield 82.7% ) as a white solid.

LCMS m/z = 605.3 $[M+1]^+$.

Step 2: 2-(4-(2-(4-chloro-2-fluorophenyl)-2-methylbenzo[d][1,3]dioxol-4-yl)benzyl)-1-(((S)-oxetan-2-yl)methyl)-1H-thieno[2,3-d]imidazole-5-carboxylic acid (Compound 11)

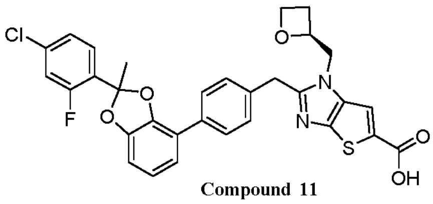

Compound 11

At room temperature, substrate 11a (110.0 mg, 0.18 mmol) was dissolved in a mixed solvent of acetonitrile (5 mL) and water (1 mL), and then 1 g (CAS: 5807-14-7) (4.4.0 ) Dec-5-ene (23.0 mg, 0.54 mmol) was added. After the addition, the reaction was carried out at room temperature for 24 hours. Acetonitrile was distilled off under reduced pressure. The crude product was dissolved in water (5 mL), adjusted to pH=6 with 1N hydrochloric acid. The aqueous phase was extracted with ethyl acetate (15 mL × 3). The organic phase was combined, dried over anhydrous sodium sulfate, filtered and concentrated to obtain a crude product, which was separated and purified by silica gel column chromatography to obtain compound 11 (25.0 mg, yield 23.4%) as a white solid.

LCMS m/z = 591.2 $[M+1]^+$.

$^1H$ NMR (400 MHz, DMSO-*d6*) δ 12.90 (brs, 1H), 7.82 (s, 1H), 7.74 - 7.67 (m, 2H), 7.62 - 7.52 (m, 2H), 7.42 - 7.37 (m, 2H), 7.36 - 7.32 (dd, 1H), 7.16 - 7.10 (m, 1H), 6.95 (d, 2H), 4.97 - 4.87 (m, 1H), 4.60 - 4.52 (m, 1H), 4.51 - 4.40 (m, 2H), 4.38 - 4.28 (m, 3H), 2.68 - 2.56 (m, 1H), 2.35 - 2.23 (m, 1H), 2.06 (s, 3H).

**Example 12** (S)-2-((4-(6-((4-cyano-2-fluorobenzyl)oxy)pyridin-2-yl)piperidin-1-yl)methyl)-1-((tetrahydrofuran-2-yl)methyl)-1H-thieno[2,3-d]imidazole-5-carboxylic acid (Compound 12)

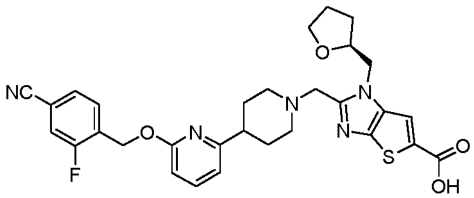

Compound 12

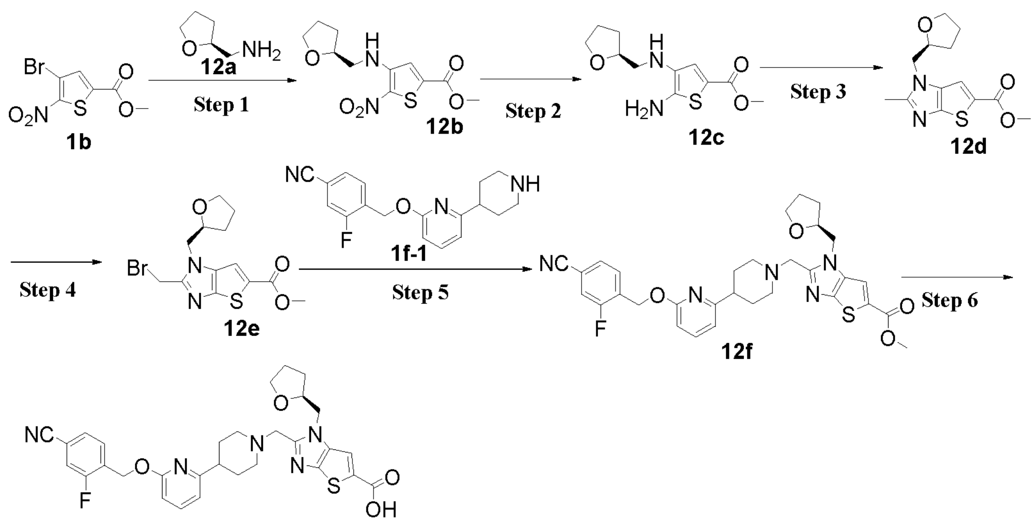


Compound 12

Step 1: methyl (S)-5-nitro-4-(((tetrahydrofuran-2-yl)methyl)amino)thiophene-2-carboxylate (12b)

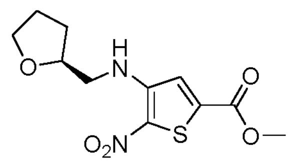

12b

1b (2 g, 7.55 mmol), 12a (838 mg, 8.31 mmol), $Pd_2(dba)_3$ (346 mg, 0.42 mmol), t-BuBrettphos (366 mg, 0.76 mmol) and cesium carbonate (4.92 g, 15.1 mmol) were added into a round bottom flask, and 1,4-dioxane (10 mL) was added. The reaction liquid was stirred at a constant temperature of 100°C for 18 hours after the reaction flask was subjected to nitrogen replacement for protection. After the reaction was completed, the reaction liquid was cooled to room temperature. 20 mL of water was added, then extracted with ethyl acetate (20 mL × 3). The organic phase was washed with aqueous saturated sodium chloride solution, then dried over anhydrous sodium sulfate, filtered and spin-dried. The residue was separated and purified by silica gel column chromatography to obtain 12b (1.6 g, yield 74%) as a light yellow solid.
LCMS m/z = 287.0 $[M+1]^+$.

Step 2: methyl (S)-5-amino-4-(((tetrahydrofuran-2-yl)methyl)amino)thiophene-2-carboxylate (12c)

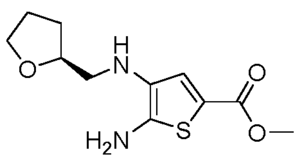

12c

Raney nickel (60 mg) was added to MeOH (2 mL) and THF (2 mL) containing 12b (200 mg, 0.70 mmol). The reaction liquid was stirred at room temperature for 1 h after the reaction flask was subjected to hydrogen replacement. After the reaction was completed, the reaction liquid was filtered over celite and spin-dried, and the obtained crude product 12c was directly used in the next step.

Step 3: methyl (S)-2-methyl-1-((tetrahydrofuran-2-yl)methyl)-1H-thieno[2,3-d]imidazole-5-carboxylate (12d)

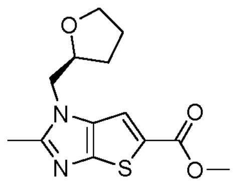

12d

At room temperature, 12c was added to acetonitrile (5 mL), and ytterbium trifluoromethanesulfonate (11 mg, 0.02 mmol) and trimethyl orthoacetate (63 mg, 0.52 mmol) were added to the reaction liquid successively, and the temperature was raised to 70°C for reaction for 2 hours. The reaction liquid was cooled to room temperature. Glacial acetic acid was distilled off under reduced pressure, and the residue was separated and purified by silica gel column chromatography to obtain 12d (50 mg, yield 26% over two step).
LCMS m/z = 281.1 $[M+1]^+$.

Step 4: methyl (S)-2-(bromomethyl)-1-((tetrahydrofuran-2-yl)methyl)-1H-thieno[2,3-d]imidazole-5-carboxylate (12e)

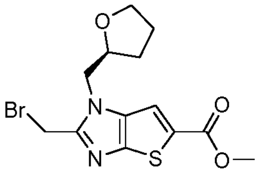

12e

At room temperature, 12d (50 mg, 0.18 mmol) was dissolved in 1,2-dichloroethane (2 mL) solution, then AIBN (3.2 mg, 0.02 mmol) was added, and NBS(80.1 g, 0.45 mmol) was added in portions in the process of slowly raising the temperature to 50°C. After the addition, the reaction liquid was heated to 70°C and continued to react for 8 h. The reaction liquid was cooled to room temperature, saturated sodium thiosulfate solution (5 mL) was added to quench the reaction and extracted with dichloromethane (5 mL × 3). The organic phase was combined, dried over anhydrous sodium sulfate, filtered and concentrated to obtain a crude product. The residue was separated and purified by silica gel column chromatography to obtain 12e (26 mg, yield 40%) as a dark yellow solid.
LCMS m/z = 359.0 $[M+1]^+$.

Step 5: methyl (S)-2-((4-(6-((4-cyano-2-fluorobenzyl)oxy)pyridin-2-yl)piperidin-1-yl)methyl)-1-((tetrahydrofuran-2-yl)methyl)-1H-thieno[2,3-d]imidazole-5-carboxylate (12f)

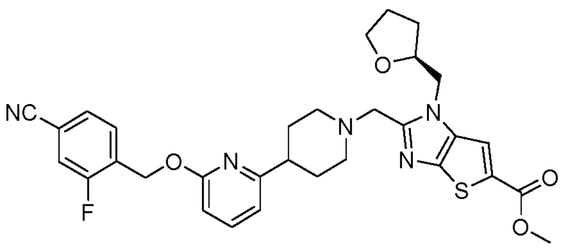

12f

At room temperature, 12e (26 mg, 0.07 mmol) was dissolved in acetonitrile (1 mL) solution, then potassium carbonate (29.0 mg, .21 mmol) and 1f-1 (24.8 mg, 0.08 mmol) were added, stirred at room temperature for 18 h. The reaction liquid was filtered to remove the inorganic base, and the filtrate was collected and concentrated to obtain a crude product, which was separated and purified by silica gel column chromatography to obtain 12f (20 mg, yield 49%) as a yellow solid.
LCMS m/z = 590.3 $[M+1]^+$.

Step 6: (S)-2-((4-(6-((4-cyano-2-fluorobenzyl)oxy)pyridin-2-yl)piperidin-1-yl)methyl)-1-((tetrahydrofuran-2-yl)methyl)-1H-thieno[2,3-d]imidazole-5-carboxylic acid (Compound 12)

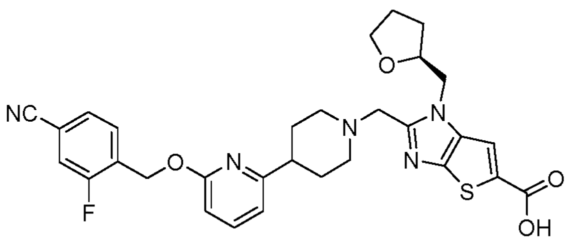

Compound 12

At room temperature, 12f (20mg, 0.03 mmol) was dissolved in a solution of acetonitrile (1 mL) and water (0.2 mL), then 1 g (CAS: 5807-14-7) (20.9 mg, 0.15 mmol) was added, and the reaction was continued for 24 h. The reaction liquid was adjusted with 1N hydrochloric acid to pH=6, and then extracted with a mixed solvent of dichloromethane:methanol=10:1 (20 mL × 3). The organic phase was combined, dried over anhydrous sodium sulfate, filtered and concentrated.

The obtained crude product was subjected to prep-HPLC to obtain compound 12 (5.0 mg, yield 29 %) as a white solid.

Preparation conditions:

instrument and preparative column: waters 2767 (preparative liquid phase chromatographic instrument) was used; the preparative column model was SunFire@Prep C18 (19 mm × 250 mm).

Preparation method: The crude product was dissolved in DMF and filtered with a 0.45 μm filter membrane to prepare into a sample liquid.

Mobile phase system: acetonitrile/water (containing 1% TFA). Gradient elution: acetonitrile content 20-65%, elution flow rate: 12 mL/min, elution time 18 min.

LCMS m/z = 576.3 $[M+1]^+$.

$^1H$ NMR (400 MHz, $CD_3OD$) δ 7.87 (s, 1H), 7.68 (t, 2H), 7.57 (t, 2H), 6.93 (d, 1H), 6.78 (d, 1H), 5.54 (s, 2H), 4.76-4.62 (m, 2H), 4.62-4.56 (m, 1H), 4.40-4.33 (m, 1H), 4.24-4.15 (m, 1H), 3.95-3.73 (m, 4H), 3.42-3.36 (m, 1H), 3.09-2.98 (m, 1H), 2.27-2.09 (m, 5H), 2.03-1.81 (m, 3H), 1.69-1.58 (m, 1H).

**Example** 13 (S)-2-((4-(6-((4-cyano-2-fluorobenzyl)oxy)pyridin-2-yl)piperazin-1-yl)methyl)-1-(oxetan-2-ylmethyl)-1H-thieno[2,3-d]imidazole-5-carboxylic acid

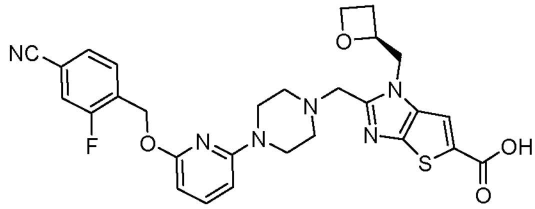

Compound 13

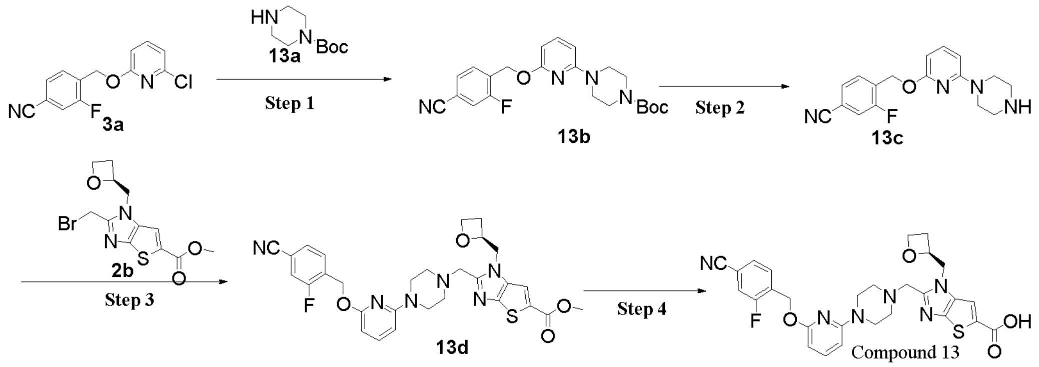


Step 1: tert-butyl 4-(6-((4-cyano-2-fluorobenzyl)oxy)pyridin-2-yl)piperazine-1-carboxylate (13b)

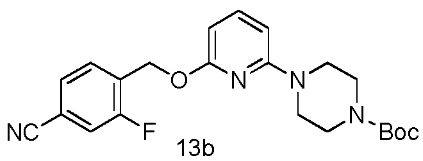

13b

3a (1 g, 3.82 mmol), 13a (781 mg, 4.20 mmol), $Pd_2(dba)_3$ (174 mg, 0.19 mmol), RuPhos (178 mg, 0.38 mmol) and cesium carbonate (2.48 g, 7.64 mmol) were added into a round bottom flask, and 1,4-dioxane (10 mL) was added. The reaction liquid was stirred at a constant temperature of 100°C for 18 hours after the reaction flask was subjected to nitrogen replacement for protection. After the reaction was completed, the reaction liquid was cooled to room temperature. 20 mL of water was added, then extracted with ethyl acetate (20 mL × 3). The organic phase was washed with aqueous saturated NaCl solution, then dried over anhydrous sodium sulfate, filtered and spin-dried. The obtained crude product was separated and purified by silica gel column chromatography to obtain 13b (700 mg, yield 44%) as a light yellow solid.

LCMS m/z = 357.1 $[M-55]^+$.

Step 2: 3-fluoro-4-(((6-(piperazin-1-yl)pyridin-2-yl)oxy)methyl)benzonitrile (13c)

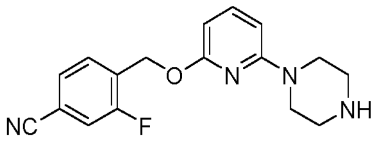

13c

13b (100 mg, 0.24 mmol) was added into a round bottom flask, then trifluoroacetic acid (0.5 mL) and dichloromethane (1 mL) were added and stirred at room temperature for 5 h. After the reaction was completed, the reaction liquid was directly concentrated under reduced pressure to obtain 13c (90 mg, yield 92%) as a colorless oil. It was directly used in the next step without further purification.

LCMS m/z = 313.1 $[M+1]^+$.

Step 3: methyl (S)-2-((4-(6-((4-cyano-2-fluorobenzyl)oxy)pyridin-2-yl)piperazin-1-yl)methyl)-1-(oxetan-2-ylmethyl)-1H-thieno[2,3-d]imidazole-5-carboxylate (13d)

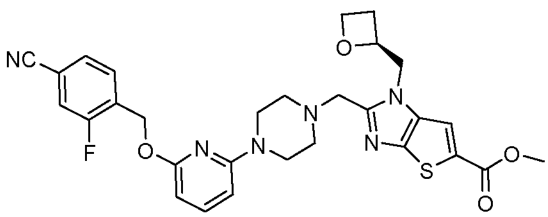

13d

At room temperature, 13c (50 mg, 0.16 mmol) was dissolved in acetonitrile (2 mL) solution, then potassium carbonate (66.3 mg, 0.48 mmol) and 1d-1 (55.0 mg, 0.16 mmol) were added, stirred at room temperature for 2 h. The reaction liquid was filtered to remove the inorganic base, and the filtrate was collected and concentrated to obtain a crude product, which was separated and purified by silica gel column chromatography to obtain 13d (50 mg, yield 54%) as a yellow solid.

LCMS m/z = 577.2 $[M+1]^+$.

Step 4: (S)-2-((4-(6-((4-cyano-2-fluorobenzyl)oxy)pyridin-2-yl)piperazin-1-yl)methyl)-1-(oxetan-2-ylmethyl)-1H-thieno[2,3-d]imidazole-5-carboxylic acid (Compound 13)

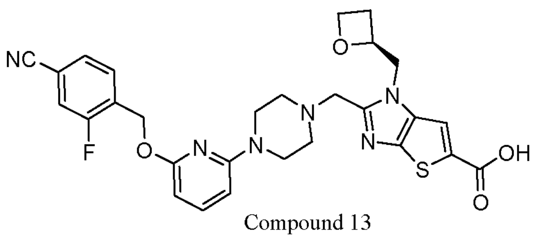

Compound 13

At room temperature, 13d (50 mg, 0.09 mmol) was dissolved in a solution of acetonitrile (1 mL) and water (0.2 mL), then 1 g (CAS: 5807-14-7) (62.6 mg, 0.45 mmol) was added, and the reaction was continued for 24 h. The reaction liquid was adjusted with 1N hydrochloric acid to pH=6, and then extracted with dichloromethane:methanol=10:1 (20 mL × 3). The organic phase was combined, dried over anhydrous sodium sulfate, filtered and concentrated. The obtained crude product was subjected to prep-HPLC to obtain compound 13 (30 mg, yield 59 %) as a white solid.

Preparation conditions:

instrument and preparative column: waters 2767 (preparative liquid phase chromatographic instrument) was used; the preparative column model was SunFire@Prep C18 (19 mm × 250 mm).

Preparation method: The crude product was dissolved in DMF and filtered with a 0.45 μm filter membrane to prepare into a sample liquid.

Mobile phase system: acetonitrile/water (containing 1% TFA). Gradient elution: acetonitrile content 20-65%, elution flow rate: 12 mL/min, elution time 18 min.

LCMS m/z = 563.2 $[M+1]^+$.

$^1H$ NMR (400 MHz, DMSO-*d6*) δ 13.17 (brs, 1H), 7.96 (s, 1H), 7.88 (d, 1H), 7.74-7.65 (m, 2H), 7.56 (t, 1H), 6.45 (d, 1H), 6.23 (d, 1H), 5.42 (s, 2H), 5.07-4.97 (m, 1H), 4.75-4.66 (m, 1H), 4.63-4.46 (m, 4H), 4.38-4.31 (m, 1H), 4.06-3.52 (m, 4H), 3.43-3.15 (m, 4H), 2.79-2.64 (m, 1H), 2.37-2.26 (m, 1H).

**Example 14** (S)-2-((4-(6-((4-cyano-2-fluorobenzyl)oxy)pyridin-2-yl)-1,4-diazepan-1-yl)methyl)-1-(oxetan-2-ylmethyl)-1H-thieno[2,3-d]imidazole-5-carboxylic acid (Compound 14)

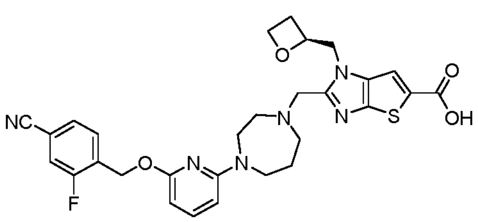

Compound 14

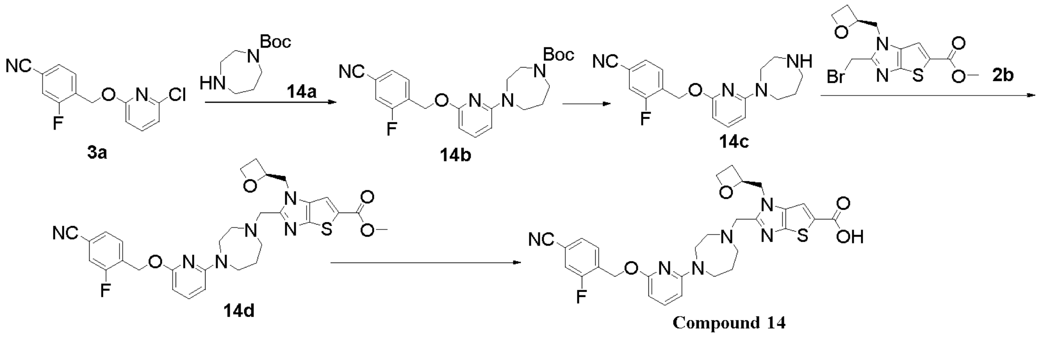


Step 1: tert-butyl 4-(6-((4-cyano-2-fluorobenzyl)oxy)pyridin-2-yl)-1,4-diazepane-1-carboxylate (14b)

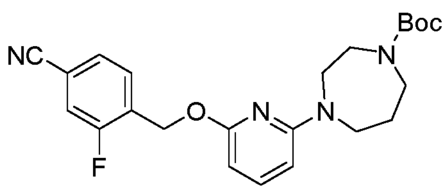

14b

At room temperature, substrate 3a (393.0 mg, 1.5 mmol) was dissolved in 1,4-dioxane (25 mL), and then 14a (301.0 mg, 1.5 mmol), $Pd_2(dba)_3$ (138.0 mg, 0.15 mmol), Ru-Phos (140.0 mg, 0.3 mmol) and cesium carbonate (1.5 g, 4.5 mmol) were added successively. After addition, the reaction system was heated to 100°C under the protection of nitrogen for overnight reaction. The reaction liquid was cooled to room temperature, filtered to remove inorganic salts, and concentrated to obtain a crude product, which was separated and purified by silica gel column chromatography to obtain the compound 14b of interest (462.0 mg, yield 72.3%).

LCMS m/z = 427.2 $[M+1]^+$.

Step 2: 4-(((6-(1,4-Diazepan-1-yl)pyridin-2-yl)oxy)methyl)-3-fluorobenzonitrile (14c)

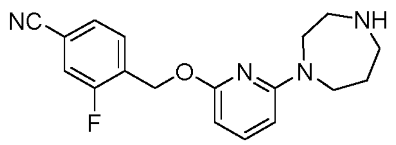

14c

At room temperature, the substrate 14b (462.0 mg, 1.1 mmol) was dissolved in dichloromethane (20 mL), and then trifluoroacetic acid (4 mL) was added dropwise to the reaction system. After the dropwise addition, the reaction was carried out at room temperature for 2 hours. The reaction liquid was concentrated to obtain the crude product 14c as a brown viscous liquid, which was directly used in the next reaction without any purification.

LCMS m/z = 327.1 $[M+1]^+$.

Step 3: methyl (S)-2-((4-(6-((4-cyano-2-fluorobenzyl)oxy)pyridin-2-yl)-1,4-diazepan-1-yl)methyl)-1-(oxetan-2-ylmethyl)-1H-thieno[2,3-d]imidazole-5-carboxylate (14d)

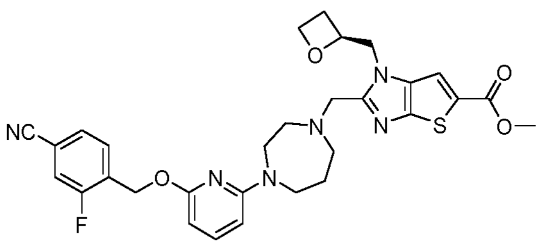

**14d**

At room temperature, the substrate 14c (115.0 mg, 0.35 mmol) was dissolved in acetonitrile (5 mL), then 2b (121.0 mg, 0.35 mmol) and potassium carbonate (244.0 mg, 1.8 mmol) were added successively. After the addition, the reaction system was reacted at room temperature for 4 hours. The reaction liquid was filtered, and the filter cake was washed with dichloromethane (10 mL × 2). The filtrate was collected and concentrated to obtain a crude product, which was separated and purified by silica gel column chromatography to obtain 3d (138.0 mg, yield 66.5%) as a white solid.

LCMS m/z = 591.3 $[M+1]^+$.

Step 4: (S)-2-((4-(6-((4-cyano-2-fluorobenzyl)oxy)pyridin-2-yl)-1,4-diazepan-1-yl)methyl)-1-(oxetan-2-ylmethyl)-1H-thieno[2,3-d]imidazole-5-carboxylic acid (Compound 14)

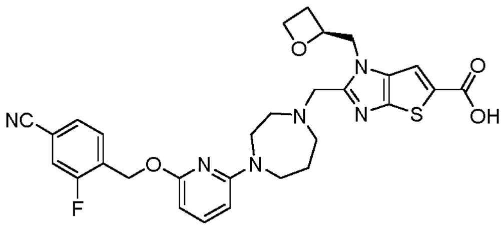

**Compound 14**

At room temperature, substrate 14d (138.0 mg, 0.2 mmol) was dissolved in a mixed solvent of acetonitrile (5 mL) and water (1 mL), and then 1 g (CAS: 5807-14-7) (98.0 mg, 0.7 mmol) was added. After the addition, the reaction was carried out at room temperature for 24 hours. Acetonitrile was distilled off under reduced pressure. The crude product was dissolved in water (5 mL), adjusted to pH=6 with 1N hydrochloric acid. The aqueous phase was extracted

with ethyl acetate (15 mL × 3). The organic phase was combined, dried over anhydrous sodium sulfate, filtered and concentrated to obtain a crude product, which was separated and purified by silica gel column chromatography to obtain compound 14 (66.0 mg, yield 48.9%) as a white solid.

LCMS m/z = 577.2 [M+1]$^+$.

$^1$H NMR (400 MHz, DMSO-*d6*) δ 12.94 (brs, 1H), 7.87 - 7.80 (m, 1H), 7.77 (s, 1H), 7.68 - 7.63 (m, 1H), 7.62 - 7.56 (m, 1H), 7.43 (t, 1H), 6.16 (d, 1H), 6.05 (d, 1H), 5.36 (s, 2H), 4.99 - 4.92 (m, 1H), 4.53 - 4.40 (m, 2H), 4.38 - 4.28 (m, 2H), 3.87-3.74 (m, 2H), 3.61 (t, 2H), 3.51 (t, 2H), 2.70 - 2.62 (m, 2H), 2.60 - 2.52 (m, 3H), 2.34 - 2.20 (m, 1H), 1.75 - 1.66 (m, 2H).

**Example 15** (S)-2-((4-(2-((4-cyano-2-fluorobenzyl)oxy)pyridin-3-yl)piperidin-1-yl)methyl)-1-(oxetan-2-ylmethyl)-1H-thieno[2,3-d]imidazole-5-carboxylic acid (Compound 15)

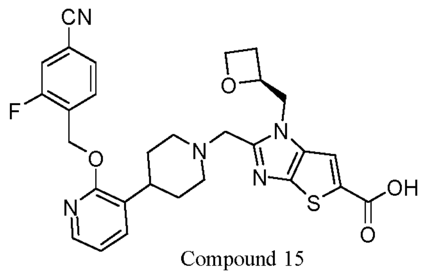

Compound 15

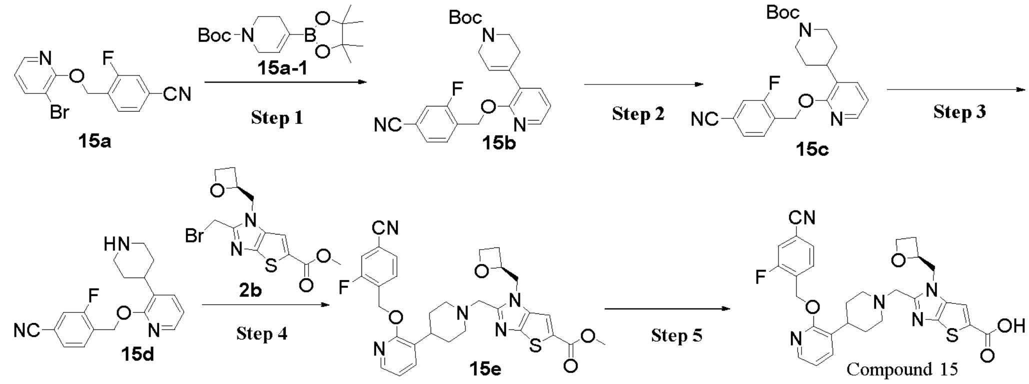


Step 1: tert-butyl 2-((4-cyano-2-fluorobenzyl)oxy)-3’,6’-dihydro-[3,4’-bipyridine]-1’(2’H)-carboxylate (15b)

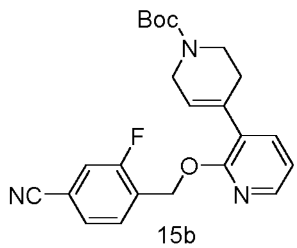

15b

15a-1 (504.9 mg, 1.63 mmol), 15a (500 mg, 1.63 mmol), Pd(dppf)$Cl_2$ (133 mg, 0.16 mmol) and cesium carbonate (1.06 g, 3.26 mmol) were added into a round bottom flask, and 1,4-dioxane (10 mL) was added. The reaction liquid was stirred at a constant temperature of 100°C for 18 hours after the reaction flask was subjected to nitrogen replacement for protection. After the reaction was completed, the reaction liquid was cooled to room temperature. 10 mL of water was added, then extracted with ethyl acetate (6 mL × 3). The organic phase was washed with aqueous saturated NaCl solution, then dried over anhydrous sodium sulfate and filtered. The filtrate was concentrated to dryness. The obtained crude product was separated and purified by silica gel column chromatography to obtain 15b (600 mg, yield 90%) as a

light yellow solid.

LCMS m/z = 410.2 $[M+1]^+$.

Step 2: tert-butyl 4-(2-((4-cyano-2-fluorobenzyl)oxy)pyridin-3-yl)piperidine-1-carboxylate (15c)

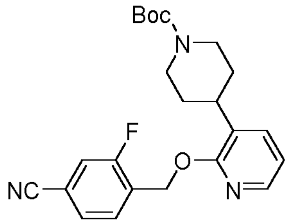

15c

Palladium on carbon (60 mg) was added to tetrahydrofuran (10 mL) containing 15b (600 mg, 1.47 mmol). The reaction liquid was stirred at room temperature for 18 h after the reaction flask was subjected to hydrogen replacement. After the reaction was completed, the reaction liquid was filtered over celite, and the liquid was concentrated to dryness. The crude product was separated and purified by silica gel column chromatography to obtain 15c (450 mg, yield 74%) as a light yellow solid.

LCMS m/z = 412.1 $[M+1]^+$.

Step 3: 3-fluoro-4-(((3-(piperidin-4-yl)pyridin-2-yl)oxy)methyl)benzonitrile (15d)

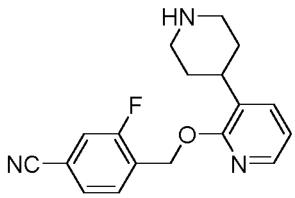

15d

15c (100 mg, 0.24 mmol) was added into a round bottom flask, then trifluoroacetic acid (0.5 mL) and dichloromethane (1 mL) were added and stirred at room temperature for 5 h. After the reaction was completed, the reaction liquid was directly concentrated under reduced pressure to obtain 15d (90 mg, yield 92%) as a colorless oil. It was directly used in the next step without further purification.

LCMS m/z = 312.1 $[M+1]^+$.

Step 4: methyl (S)-2-((4-(2-((4-cyano-2-fluorobenzyl)oxy)pyridin-3-yl)piperidin-1-yl)methyl)-1-(oxetan-2-ylmethyl)-1H-thieno[2,3-d]imidazole-5-carboxylate (15e)

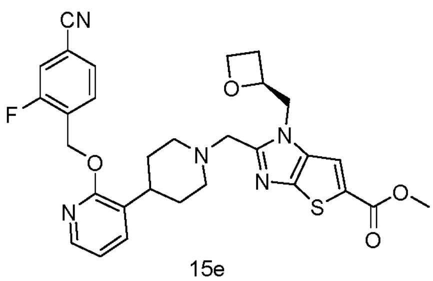

15e

At room temperature, 15d (25 mg, 0.08 mmol) was dissolved in acetonitrile (1 mL) solution, then potassium carbonate (33.2 mg, 0.24 mmol) and 2b (27.5 mg, 0.08 mmol) were added, stirred at room temperature for 5 h. The reaction liquid was filtered to remove the inorganic base, and the filtrate was collected and concentrated to obtain a crude product, which was separated and purified by silica gel column chromatography to obtain 15e (20 mg, yield 43%) as a brown solid.
LCMS m/z = 576.2 $[M+1]^+$.

Step 5: (S)-2-((4-(2-((4-cyano-2-fluorobenzyl)oxy)pyridin-3-yl)piperidin-1-yl)methyl)-1-(oxetan-2-ylmethyl)-1H-thieno[2,3-d]imidazole-5-carboxylic acid (Compound 15)

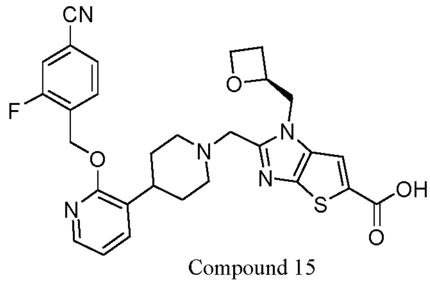

Compound 15

At room temperature, 15e (20mg, 0.03 mmol) was dissolved in a solution of acetonitrile (1 mL) and water (0.2 mL), then 1 g (CAS: 5807-14-7) (20.9 mg, 0.15 mmol) was added, and the reaction was continued for 24 h. The reaction liquid was adjusted with 1N hydrochloric acid to pH=6, and then extracted with a mixed solvent of dichloromethane:methanol=10:1 (20 mL × 3). The organic phase was combined, dried over anhydrous sodium sulfate, filtered and concentrated. The obtained crude product was subjected to prep-HPLC to obtain compound 15 (6.0 mg, yield 32%) as a white solid.
Preparation conditions:
instrument and preparative column: waters 2767 (preparative liquid phase chromatographic instrument) was used; the preparative column model was SunFire@Prep C18 (19 mm × 250 mm).
Preparation method: The crude product was dissolved in DMF and filtered with a 0.45 μm filter membrane to prepare into a sample liquid.
Mobile phase system: acetonitrile/water (containing 1% TFA). Gradient elution: acetonitrile content 20-65%, elution flow rate: 12 mL/min, elution time 18 min.

Compound 15:

LCMS m/z = 562.2 $[M+1]^+$.
$^1$H NMR (400 MHz, DMSO-*d6*) δ 13.22 (brs, 1H), 8.07-8.04 (m, 1H), 7.98 (s, 1H), 7.91 (d, 1H), 7.74-7.67 (m, 2H), 7.63-7.56 (m, 1H), 7.08-7.02 (m, 1H), 5.53 (s, 2H), 5.06-4.98 (m, 1H), 4.77-4.63 (m, 3H), 4.63-4.56 (m, 1H), 4.54-4.47 (m, 1H), 4.38-4.31 (m, 1H), 3.74-3.64 (m, 2H), 3.36-3.23 (m, 2H), 3.15-3.04 (m, 1H), 2.77-2.66 (m, 1H), 2.37-2.26 (m, 1H), 2.09-1.99 (m, 2H), 1.96-1.84 (m, 2H).

**Example** 16 (S)-2-((4-(6-((4-cyano-2-fluorobenzyl)oxy)pyridin-2-yl)piperidin-1-yl)methyl)-3-(oxetan-2-ylmethyl)-3H-thieno[2,3-d]imidazole-5-carboxylic acid

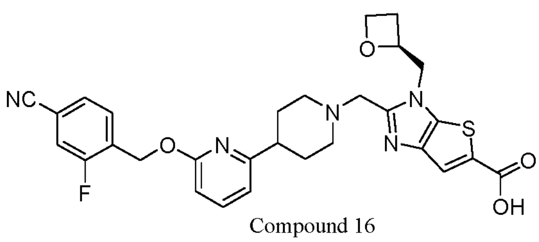

Compound 16

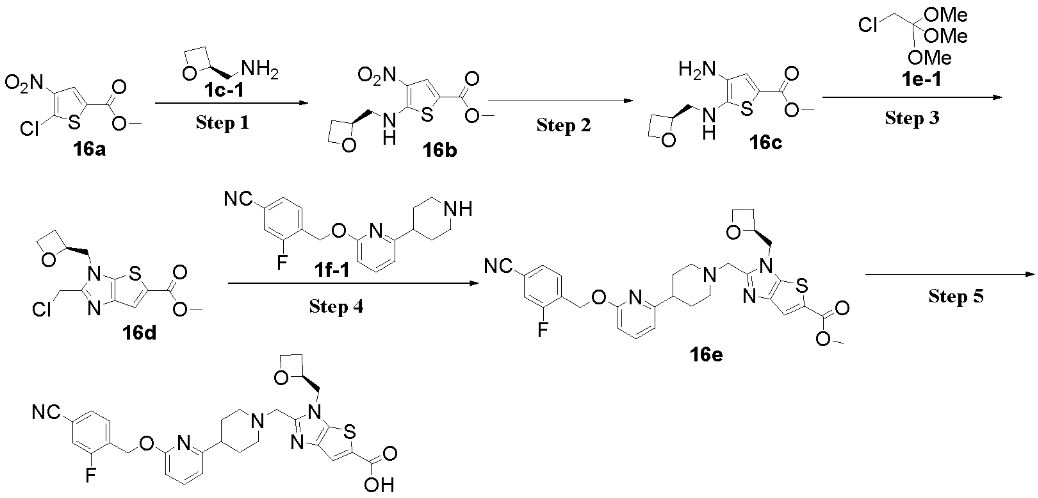

Compound 16

Step 1: methyl (S)-4-nitro-5-((oxetan-2-ylmethyl)amino)thiophene-2-carboxylate (16b)

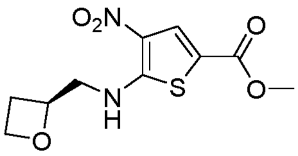
16b

At room temperature, 16a (2 g, 9.0 mmol) was dissolved in acetonitrile (50 mL) solution, then potassium carbonate (3.73 g, 27.0 mmol) and 1c-1 (783 mg, 9.0 mmol) were added, and the temperature was raised to 70°C for 18 h. The reaction liquid was filtered to remove solids, and the filtrate was collected and concentrated to obtain a crude product, which was separated and purified by silica gel column chromatography to obtain 16b (2 g, yield 83%).
LCMS m/z = 273.1 $[M+1]^+$.

Step 2: methyl (S)-4-amino-5-((oxetan-2-ylmethyl)amino)thiophene-2-carboxylate (16c)

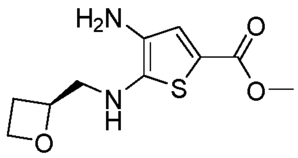
16c

Zinc powder (1.2 g, 18.4 mmol) was added to acetic acid (20 mL) containing 16b (1.0 g, 3.68 mmol), and the reaction liquid was stirred at room temperature for 1 h. After the reaction was completed, the reaction liquid was filtered over celite, and the filtrate was concentrated to dryness. The residue was separated and purified by silica gel column chromatography to obtain 16c (700 mg, yield 79%) as a light yellow solid.
LCMS m/z = 243.1 $[M+1]^+$.

Step 3: methyl (S)-2-(chloromethyl)-3-(oxetan-2-ylmethyl)-3H-thieno[2,3-d]imidazole-5-carboxylate (16d)

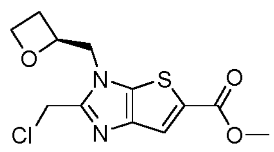

16d

At room temperature, 16c (700 mg, 2.89 mmol) was added to acetonitrile (20 mL), and 1e-1 (668.2 mg, 4.34 mmol) and p-toluenesulfonic acid (24.9 mg, 0.14 mmol) were added to the reaction liquid successively, heated to 60°C for 5 hours. Acetonitrile was distilled off under reduced pressure, and the obtained crude product was purified by silica gel column chromatography to obtain 16d (45 mg, yield 5.2%) as a yellow solid.
LCMS m/z = 301.1 $[M+1]^+$.

Step 4: methyl (S)-2-((4-(6-((4-cyano-2-fluorobenzyl)oxy)pyridin-2-yl)piperidin-1-yl)methyl)-3-(oxetan-2-ylmethyl)-3H-thieno[2,3-d]imidazole-5-carboxylate (16e)

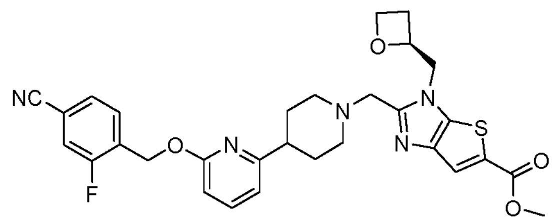

16e

At room temperature, 16d (45 mg, 0.15 mmol) was dissolved in acetonitrile (2 mL) solution, then potassium carbonate (62.1 mg, 0.45 mmol) and 1f-1 (46.5 mg, 0.15 mmol) were added, stirred at room temperature for 18 h. The reaction liquid was filtered to remove solids, and the filtrate was collected and concentrated to obtain a crude product, which was separated and purified by silica gel column chromatography to obtain 16e (50 mg, yield 58%) as a yellow solid.
LCMS m/z = 576.2 $[M+1]^+$.

Step 5: (S)-2-((4-(6-((4-cyano-2-fluorobenzyl)oxy)pyridin-2-yl)piperidin-1-yl)methyl)-3-(oxetan-2-ylmethyl)-3H-thieno[2,3-d]imidazole-5-carboxylic acid (Compound 16)

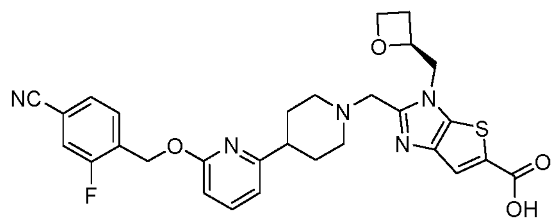

Compound 16

At room temperature, 16e (40 mg, 0.07 mmol) was dissolved in a solution of acetonitrile (1 mL) and water (0.2 mL), then 1 g (CAS: 5807-14-7) (48.7 mg, 0.35 mmol) was added, and the reaction was continued for 24 h. The reaction liquid was adjusted with 1N hydrochloric acid to pH=6, and then extracted with a mixed solvent of dichloromethane:methanol=10:1 (20 mL × 3). The organic phase was combined, dried over anhydrous sodium sulfate, filtered and concentrated. The obtained crude product was subjected to prep-HPLC to obtain compound 16 (20 mg, yield 51 %) as a white solid.
Preparation conditions:
instrument and preparative column: waters 2767 (preparative liquid phase chromatographic instrument) was used; the preparative column model was SunFire@Prep C18 (19 mm × 250 mm).
Preparation method: The crude product was dissolved in DMF and filtered with a 0.45 μm filter membrane to prepare into a sample liquid.

Mobile phase system: acetonitrile/water (containing 1% TFA). Gradient elution: acetonitrile content 20-65%, elution flow rate: 12 mL/min, elution time 18 min

LCMS m/z = 562.3 $[M+1]^+$.

$^1H$ NMR (400 MHz, DMSO-*d6*) δ 13.15 (brs, 1H), 7.89 (d, 1H), 7.86 (s, 1H), 7.76-7.69 (m, 3H), 6.98-6.91 (m, 1H), 6.79 (d, 1H), 5.49 (s, 2H), 5.14-5.06 (m, 1H), 4.69

(s, 2H), 4.66-4.60 (m, 1H), 4.57-4.49 (m, 2H), 4.40-4.33 (m, 1H), 3.80-3.65 (m, 2H), 3.32-3.19 (m, 2H), 2.96-2.86 (m, 1H), 2.80-2.70 (m, 1H), 2.42-2.31 (m, 1H), 2.12-1.93 (m, 4H).

**Example 17** (S)-2-((4-(6-((4-cyano-2-fluorobenzyl)oxy)-5-fluoropyridin-2-yl)piperidin-1-yl)methyl)-1-(oxetan-2-ylmethyl)-1H-thieno[2,3-d]imidazole-5-carboxylic acid **(Compound 17)**

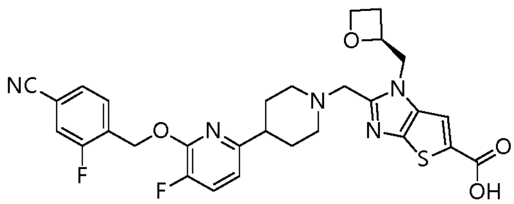

Compound 17

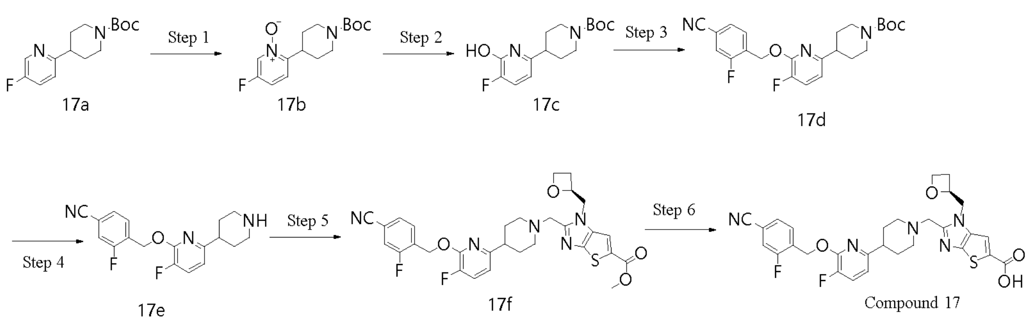


17a 17b 17c 17d

17e 17f Compound 17

Step 1: 2-(1-(tert-butoxycarbonyl)piperidin-4-yl)-5-fluoropyridine1-oxide **(17b)**

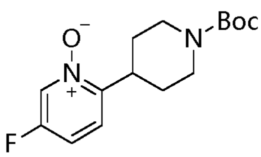

17b

At room temperature, m-chloroperoxybenzoic acid (3.08 g, 17.82 mmol) was added to a solution of 17a (2.00 g, 7.13 mmol) in dichloromethane (20 ml), and then stirred and reacted at room temperature for 14 hours. The insolubles were removed by filtration, the liquid was washed with an equal volume of water, dried over anhydrous sodium sulfate and concentrated to dryness, and the residue was separated and purified by silica gel column chromatography to obtain the product **17b** of interest (1.20 g, yield: 56.8%).

Step 2: tert-butyl4-(5-fluoro-6-hydroxypyridin-2-yl)piperidine-1-carboxylate **(17c)**

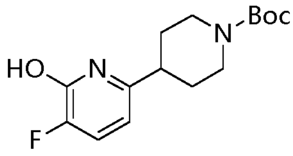

17c

Under ice-water bath, triethylamine (0.82 g, 8.10 mmol) was added to a solution of **17b** (1.20 g, 4.05 mmol) and trifluoroacetic anhydride (8.51 g, 40.5 mmol) in tetrahydrofuran (20 ml). After one hour, the temperature was raised to room temperature to react for 14 hours. The reaction liquid was concentrated to dryness under reduced pressure, and the residue was separated and purified by column chromatography to obtain**17c** (0.8 g, yield: 66.6%).
LCMS m/z=241.1 [M-55] $^+$.

Step 3: tert-butyl 4-(6-((4-cyano-2-fluorobenzyl)oxy)-5-fluoropyridin-2-yl)piperidine-1-carboxylate **(17d)**

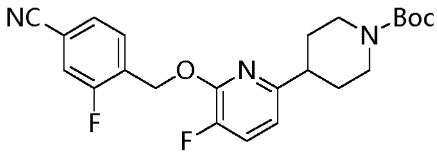

17d

At room temperature, tri-n-butylphosphine (513 mg, 2.54 mmol), diisopropyl azodicarboxylate (513 mg, 2.54 mmol) were added to a solution of **17c** (0.5 g, 1.69 mmol) and 3-Fluoro-4-(hydroxymethyl)benzonitrile (383 mg, 2.54 mmol) in toluene (5 ml). The temperature was raised to 80°C to react for 14 hours. The reaction liquid was concentrated under reduced pressure. The residue was separated and purified by silica gel column chromatography to obtain **17d** (0.2 g, yield: 27.6%).
LCMS m/z=374.1 [M-55] $^+$.

Step 4: 3-fluoro-4-(((3-fluoro-6-(piperidin-4-yl)pyridin-2-yl)oxy)methyl)benzonitrile **(17e)**

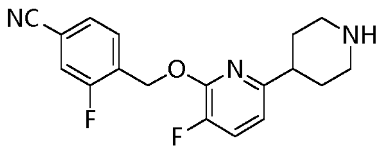

17e

At room temperature, trifluoroacetic acid (2 ml) was added to a solution of **17d** (0.2 g, 0.47 mmol) in dichloromethane (2 ml), and then reacted at room temperature for 3 hours. The reaction liquid was concentrated to dryness under reduced pressure, and the residue was separated and purified by column chromatography to obtain **17e** (0.1 g, yield: 64.60%).

Step 5: methyl (S)-2-((4-(6-((4-cyano-2-fluorobenzyl)oxy)-5-fluoropyridin-2-yl)piperidin-1-yl)methyl)-1-(oxetan-2-ylmethyl)-1H-thieno[2,3-d]imidazole-5-carboxylate **(17f)**

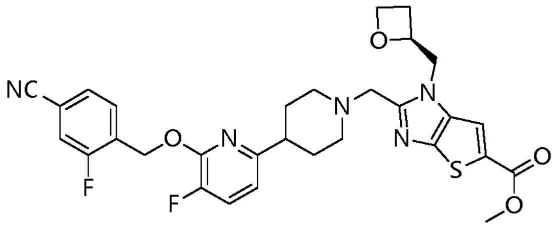

17f

At room temperature, potassium carbonate (82.93 mg, 0.60 mmol) was added to a solution of **17e** (0.1 g, 0.30 mmol) and **2b** (103.56 mg, 0.30 mmol) in acetonitrile (10 ml), and then reacted at room temperature for 4 hours. The solid was removed by filtration, and the liquid was concentrated to dryness to obtain **17f.** The crude product yield was calculated as 100% and the next step was directly carried out.

LCMS m/z=594.2(M+1) $^+$.

Step 6: (S)-2-((4-(6-((4-cyano-2-fluorobenzyl)oxy)-5-fluoropyridin-2-yl)piperidin-1-yl)methyl)-1-(oxetan-2-ylmethyl)-1H-thieno[2,3-d]imidazole-5-carboxylic acid **(Compound 17)**

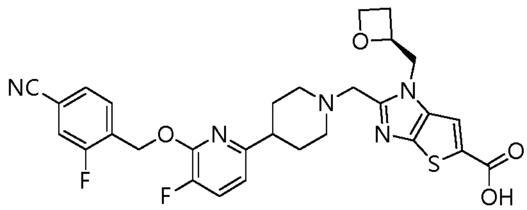

Compound 17

At room temperature, 1g (CAS: 5807-14-7) (139.2 mg, 1.00 mmol) was added to a mixed solution of **17f** (0.15 g, 0.25 mmol) in acetonitrile (5 ml) and water (5 ml) and then reacted at room temperature for 14 hours. The reaction liquid was concentrated to obtain a crude product. The crude product was purified by Pre-HPLC (instrument and preparative column: Waters 2767 (preparative liquid phase chromatographic instrument) was used, and the preparative column model was Sunfire@PrepC18, 5 μm, inner diameter × length = 19 mm × 250 mm). Preparation method: The crude product was dissolved in DMF and filtered with a 0.45 μm filter membrane to prepare into a sample liquid. Mobile phase system: acetonitrile/water (containing 0.5% ammonium bicarbonate). Gradient elution method: gradient elution with acetonitrile from 5% to 50% (flow rate: 15 mL/min; elution time 15 min), **compound 17** (0.05 g, yield: 34.51%) was obtained.

LCMS m/z = 580.2[M+1] $^+$.

$^1$H NMR (400 MHz,$CD_3OD$) δ 7.69 (t, 1H), 7.65 (s, 1H), 7.63-7.53 (m, 2H), 7.45-7.38 (m, 1H), 6.87-6.83(m, 1H), 5.59 (s, 2H), 5.26-5.18 (m, 1H), 4.72-4.54(m, 3H), 4.47-4.39 (m, 1H), 4.12-4.00 (m, 2H), 3.28-3.16 (m, 2H), 2.82-2.69 (m, 2H), 2.59-2.44 (m, 3H), 1.95-1.82 (m, 4H).

**Example 18** (S)-2-(4-(6-((4-cyano-2-fluorobenzyl)oxy)-5-fluoropyridin-2-yl)-2-fluorobenzyl)-1-(oxetan-2-ylmethyl)-1H-thieno[2,3-d]imidazole-5-carboxylic acid **(Compound 18)**

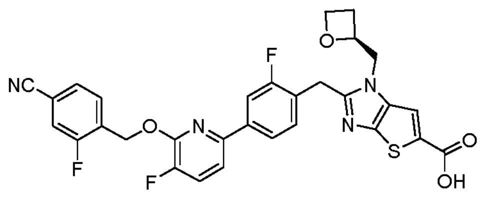

Compound 18

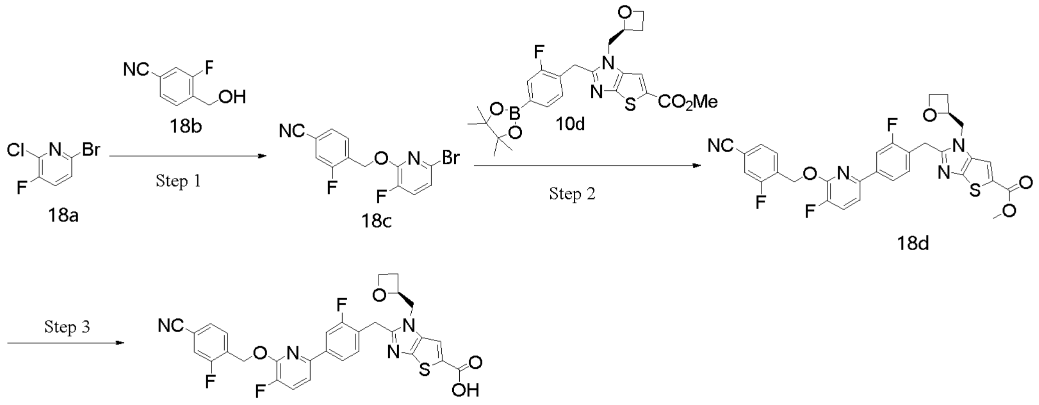


Compound 18

Step 1: 4-(((6-bromo-3-fluoropyridin-2-yl)oxy)methyl)-3-fluorobenzonitrile **(18c)**

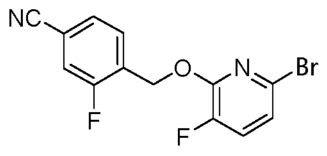

18c

Under ice bath, 18a (278 mg, 1.32 mmol) was added to a solution of 18b (200 mg, 1.32 mmol) and potassium tert-butoxide (221.8 mg, 1.98 mmol) in tetrahydrofuran (6 ml) and reacted at room temperature for 3 hours. The insolubles were removed by filtration, the liquid was washed once with aqueous saturated ammonium chloride solution (6 ml), dried over anhydrous sodium sulfate, concentrated to dryness, and the residue was separated and purified by silica gel column chromatography to obtain the product **18c** of interest (250 mg, yield: 58.5%).

Step 2: methyl (S)-2-(4-(6-((4-cyano-2-fluorobenzyl)oxy)-5-fluoropyridin-2-yl)-2-fluorobenzyl)-1-(oxetan-2-ylmethyl)-1H-thieno[2,3-d]imidazole-5-carboxylate **(18d)**

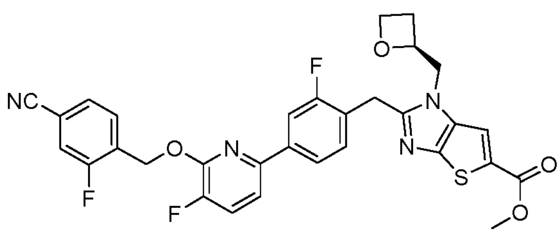

18d

The crude products 10d and 18c (178 mg, 0.55 mmol), anhydrous potassium carbonate (115 mg, 0.80 mmol) and Pd(dppf)$Cl_2$ (32.5 mg, 0.04 mmol) were added to a reaction flask successively, dissolved in a mixed solution of 1,4-dioxane (5mL)/water (1mL), subjected to nitrogen replacement, stirred at 90°C for 5 h and filtered to remove solids. The filtrate was concentrated under reduced pressure, and the residue was separated and purified by silica gel column chromatography to obtain the product 18b (140 mg, yield 42.1% over two steps) as a yellow solid.

LCMS m/z = 605.2 $[M+H]^+$.

Step 3: (S)-2-(4-(6-((4-cyano-2-fluorobenzyl)oxy)-5-fluoropyridin-2-yl)-2-fluorobenzyl)-1-(oxetan-2-ylmethyl)-1H-thieno[2,3-d]imidazole-5-carboxylic acid **(Compound 18)**

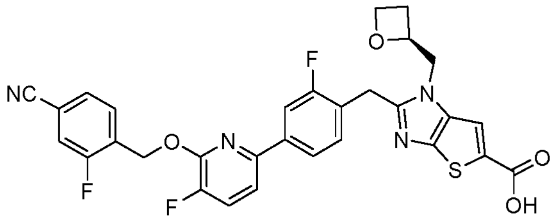

Compound 18

At room temperature, 18b (80 mg, 0.13 mmol) was dissolved in a solution of acetonitrile (3 mL) and water (0.6 mL), then 1 g (CAS: 5807-14-7) (54.2 mg, 0.39 mmol) was added, and the reaction was continued for 24 h. The reaction liquid was adjusted with 1N hydrochloric acid to pH=6, and then extracted with a mixed solvent of dichloromethane:methanol=10:1 (20 mL × 3). The organic phase was combined, dried over anhydrous sodium sulfate, concentrated by filtration, and concentrated under reduced pressure, and the residue was separated and purified by silica gel column chromatography to obtain compound 18 (7 mg, yield 9.1%) as a white solid.

LCMS m/z = 591.1 $[M+H]^+$.

$^1H$ NMR (400 MHz, DMSO-$d_6$) δ 7.96-7.91 (m, 1H), 7.86-7.72 (m, 6H), 7.70-7.65 (m, 1H), 7.29 (t, 1H), 5.70 (s, 2H), 5.05-4.97 (m, 1H), 4.64-4.56 (m, 1H), 4.52-4.45 (m, 2H), 4.41-4.28 (m, 3H), 2.70-2.64 (m, 1H), 2.35-2.29 (m, 1H).

**Example 19** (S)-2-(4-(6-((4-cyanobenzyl)oxy)-5-fluoropyridin-2-yl)-2-fluorobenzyl)-1-(oxetan-2-ylmethyl)-1H-thieno[2,3-d]imidazole-5-carboxylic acid **(Compound 19)**

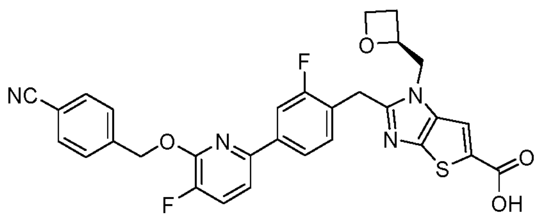

Compound 19

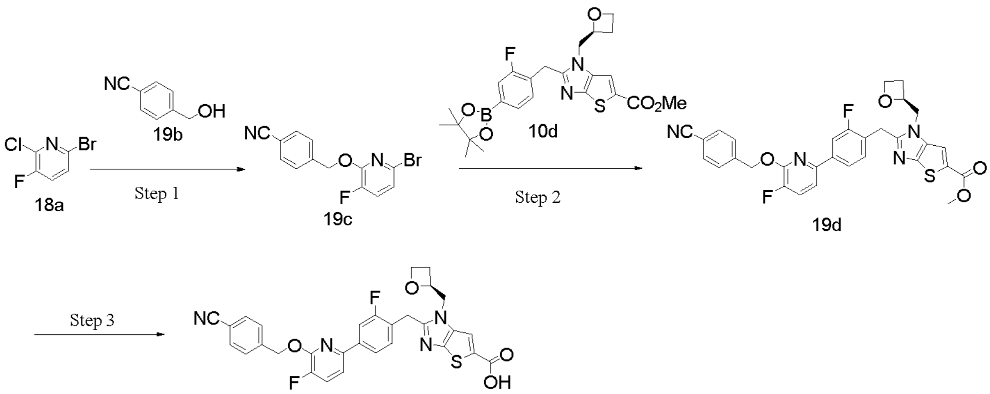


Compound 19

Step 1: 4-(((6-bromo-3-fluoropyridin-2-yl)oxy)methyl)benzonitrile **(19c)**

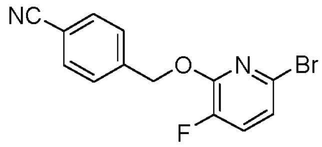

19c

Under ice bath, 18a (300 mg, 1.43 mmol) was added to a solution of 19b (285.7 mg, 1.43 mmol) and potassium tert-butoxide (240 mg, 2.15 mmol) in tetrahydrofuran (10 ml) and stirred and reacted at room temperature for 3 hours. The insolubles were removed by filtration, the liquid was washed with an equal volume of water, dried over anhydrous sodium sulfate and concentrated to dryness, and the residue was separated and purified by silica gel column chromatography to obtain the product **19c** of interest (250 mg, yield: 57.1%).

Step 2: methyl(S)-2-(4-(6-((4-cyanobenzyl)oxy)-5-fluoropyridin-2-yl)-2-fluorobenzyl)-1-(oxetan-2-ylmethyl)-1H-thieno[2,3-d]imidazole-5-carboxylate **(19d)**

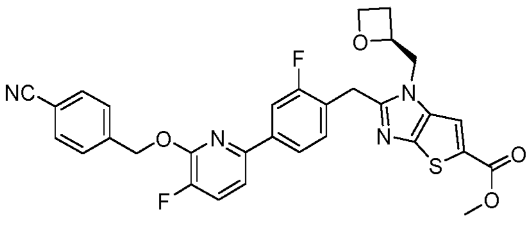

19d

The crude products 10d and 19c (168.3 mg, 0.55 mmol), anhydrous potassium carbonate (115 mg, 0.80 mmol) and Pd(dppf)$Cl_2$ (32.5 mg, 0.04 mmol) were added to a reaction flask successively, dissolved in a mixed solution of 1,4-dioxane (5 mL)/water (1 mL), subjected to nitrogen replacement, stirred at 90°C for 5 h and cooled to room temperature. the solid was filtered, the filtrate was concentrated under reduced pressure, and purified on a silica gel column to obtain the product 19d (180 mg, yield 67 % over two steps) as a yellow solid.
LCMS m/z = 587.1 $[M+H]^+$.

Step 3: (S)-2-(4-(6-((4-cyanobenzyl)oxy)-5-fluoropyridin-2-yl)-2-fluorobenzyl)-1-(oxetan-2-ylmethyl)-1H-thieno[2,3-d]imidazole-5-carboxylic acid **(Compound 19)**

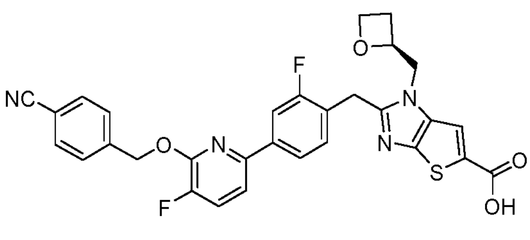

Compound 19

At room temperature, 19d (80 mg, 0.14 mmol) was dissolved in a solution of acetonitrile (3 mL) and water (0.6 mL), then 1 g (CAS: 5807-14-7) (56.9 mg, 0.41 mmol) was added, and the reaction was continued for 24 h. The reaction liquid was adjusted with 1N hydrochloric acid to pH=6, and then extracted with a mixed solvent of dichloromethane:methanol=10:1 (20 mL × 3). The organic phase was combined, dried over anhydrous sodium sulfate, concentrated by filtration, and concentrated under reduced pressure, and the residue was separated and purified by silica gel column chromatography to obtain compound 19 (40 mg, yield 50%) as a white solid.
LCMS m/z = 573.1 $[M+H]^+$.
$^1H$ NMR (400 MHz, DMSO-$d_6$) δ 7.90-7.77 (m, 6H), 7.71 (d, 2H), 7.68-7.64 (m, 1H), 7.38 (t, 1H), 5.67 (s, 2H),

5.04-4.96 (m, 1H), 4.63-4.55 (m, 1H), 4.53-4.44 (m, 2H), 4.42-4.28 (m, 3H), 2.72-2.62 (m, 1H), 2.38-2.27 (m, 1H).

**Example 20** (S)-2-(4-(6-((4-cyanobenzyl)oxy)pyridin-2-yl)-2-fluorobenzyl)-1-(oxetan-2-ylmethyl)-1H-thieno[2,3-d]imidazole-5-carboxylic acid **(Compound 20)**

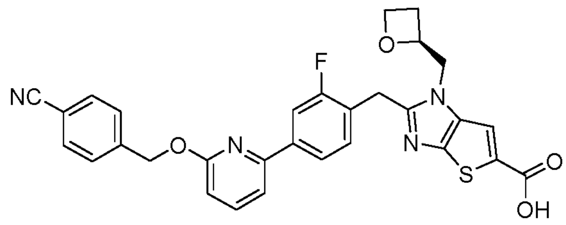

Compound 20

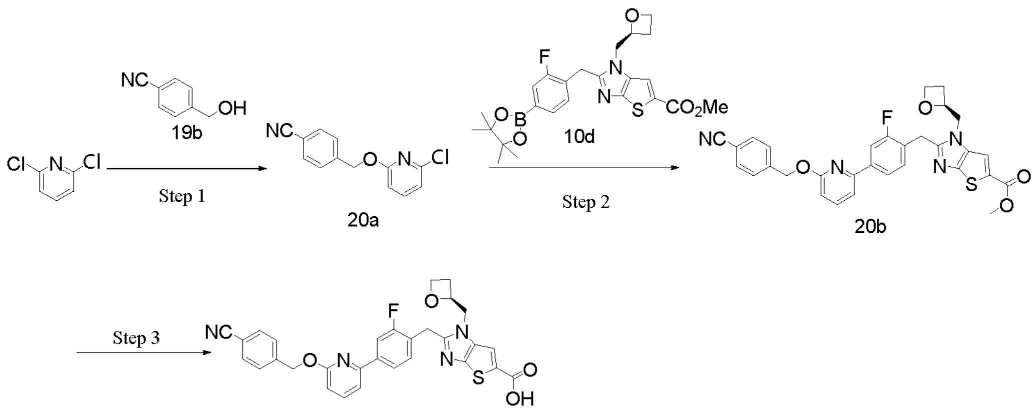


Compound 20

Step 1: 4-(((6-chloropyridin-2-yl)oxy)methyl)benzonitrile **(20a)**

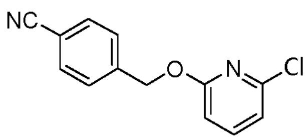

20a

Under ice bath, 2,6-dichloropyridine (5 g, 33.8 mmol) was added to a solution of 19b (4.4 g, 33.8 mmol) and potassium tert-butoxide (7.6 g, 67.6 mmol) in tetrahydrofuran (100 ml) and stirred and reacted at room temperature for 3 hours. The insolubles were removed by filtration, the liquid was washed with an equal volume of water, dried over anhydrous sodium sulfate and concentrated to dryness, and the residue was separated and purified by silica gel column chromatography to obtain the product **20a** of interest (4.7 g, yield: 57.0%).

Step 2: methyl(S)-2-(4-(6-((4-cyanobenzyl)oxy)pyridin-2-yl)-2-fluorobenzyl)-1-(oxetan-2-ylmethyl)-1H-thieno[2,3-d]imidazole-5-carboxylate **(20b)**

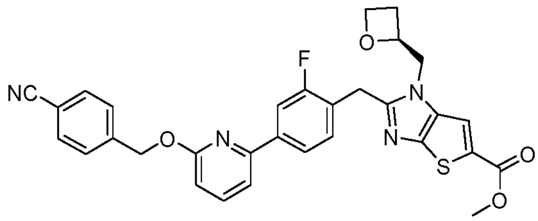

20b

The crude products 10d and 20a (134.2 mg, 0.55 mmol), anhydrous potassium carbonate (115 mg, 0.80 mmol) and Pd(dppf)$Cl_2$ (32.5 mg, 0.04 mmol) were added to a reaction flask successively, dissolved in a mixed solution of 1,4-dioxane (5 mL)/water (1 mL), subjected to nitrogen replacement, stirred at 90°C for 5 h and cooled to room temperature. the solid was filtered, the filtrate was concentrated under reduced pressure, and purified on a silica gel column to obtain the product 20b (160 mg, yield 61.5% over two steps) as a yellow solid.

LCMS m/z = 569.1 $[M+H]^+$.

Step 3: (S)-2-(4-(6-((4-cyanobenzyl)oxy)pyridin-2-yl)-2-fluorobenzyl)-1-(oxetan-2-ylmethyl)-1H-thieno[2,3-d]imidazole-5-carboxylic acid **(Compound 20)**

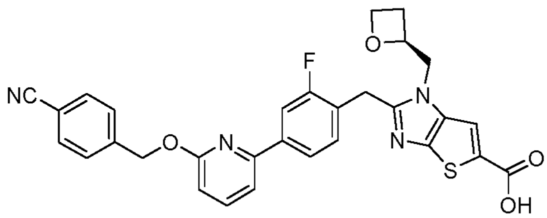

Compound 20

At room temperature, 19d (80 mg, 0.14 mmol) was dissolved in a solution of acetonitrile (3 mL) and water (0.6 mL), then 1 g (CAS: 5807-14-7) (56.9 mg, 0.41 mmol) was added, and the reaction was continued for 24 h. The reaction liquid was adjusted with 1N hydrochloric acid to pH=6, and then extracted with a mixed solvent of dichloromethane:methanol=10:1 (20 mL × 3). The organic phase was combined, dried over anhydrous sodium sulfate, concentrated by filtration, and concentrated under reduced pressure. The residue was purified by Pre-HPLC (instrument and preparative column: Waters 2767 (preparative liquid phase chromatographic instrument) was used, and the preparative column model was Sunfire@PrepC18, 5 μm, inner diameter × length = 19 mm × 250 mm). Preparation method: The crude product was dissolved in DMF and filtered with a 0.45 μm filter membrane to prepare into a sample liquid. Mobile phase system: acetonitrile/water (containing 0.5% ammonium bicarbonate). Gradient elution method: acetonitrile content 5-50% (flow rate: 15 mL/min; elution time 15 min), **compound 20** (20 mg, yield: 25.8%) was obtained.

LCMS m/z=555.1 $[M+1]^+$.

$^1H$ NMR (400 MHz, DMSO-$d_6$) δ 7.88-7.80 (m, 6H), 7.68 (d, 2H), 7.63 (d, 1H), 7.38 (t, 1H), 6.93 (d, 1H), 5.69 (s, 2H), 5.05-4.97 (m, 1H), 4.63-4.56(m, 1H), 4.52-4.44 (m, 2H), 4.42-4.29 (m, 3H), 2.72-2.62 (m, 1H), 2.37-2.28 (m, 1H).

**Example 21** (S)-2-(4-(6-((4-cyano-2-fluorobenzyl)oxy)pyridin-2-yl)-2,5-difluorobenzyl)-1-(oxetan-2-ylmethyl)-1H-thieno[2,3-d]imidazole-5-carboxylic acid (Compound 21)

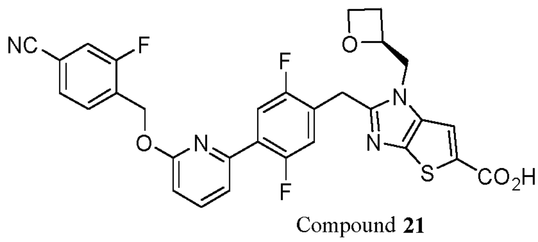

Compound **21**

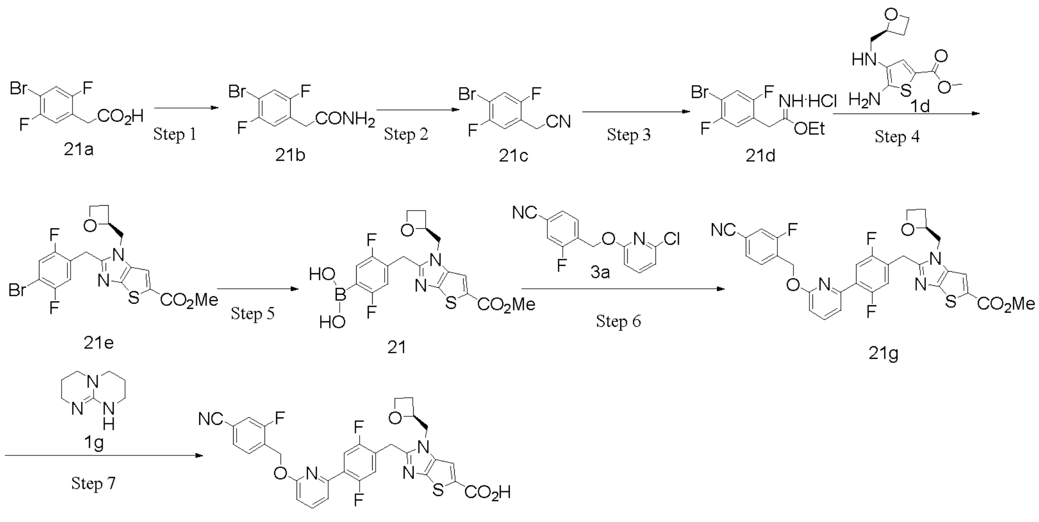

Compound 21

Step 1: 2-(4-bromo-2,5-difluorophenyl)acetic acid (21b)

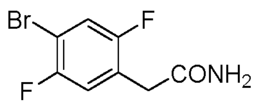

21b

21a (7.5 g, 29.88 mmol) was added into thionyl chloride (20.0 mL), and stirred at 70°C for 2 hours. Then the reaction liquid was cooled to room temperature and concentrated to dryness and the concentrated residue was added to dichloromethane (30 mL). The system was placed at 0°C and ammonia water (15 mL) was added, and a large amount of white solid was precipitated and filtered. The obtained solid was further washed with water (20 mL × 2), and the solid was dried to give 21b (7.3 g, yield 97%).
LCMS m/z = 250.1 $[M+1]^+$.

Step 2: 2-(4-bromo-2,5-difluorophenyl)acetonitrile (21c)

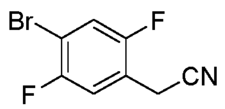

21c

21b (7.3 g, 29.20 mmol) was added into phosphorus oxychloride (30.0 mL), and stirred at 100°C for 2 hours. Then the reaction liquid was cooled to room temperature, the system was poured into ice water (100 mL), and then extracted with ethyl acetate (100 mL × 3). The organic phase was combined, dried over anhydrous sodium sulfate and filtered, the filtrate was concentrated to dryness, and the residue was separated and purified by silica gel column chromatography to obtain 21c (6.0 g, yield 88%).
LCMS m/z = 232.1 $[M+1]^+$.

Step 3: ethyl 2-(4-bromo-2,5-difluorophenyl)acetimidate hydrochloride (21d)

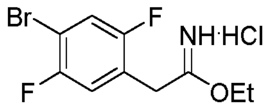

21d

21c (6.0 g, 25.86 mmol) was added to anhydrous ethanol (3.0 mL) and fully stirred at 0°C. At the same time, 33% hydrochloric acid-ethanol solution (25 mL) was added. The temperature was gradually raised to room temperature and the reaction liquid was stirred for 10 hours. Diethyl ether (40 mL) was then added, filtered to obtain a solid that was further washed with diethyl ether (100 mL × 2 ), and dried to obtain 21d (5.8 g, yield 71%).
LCMS m/z = 278.1 $[M-Cl]^+$.

Step 4: methyl (S)-2-(4-bromo-2,5-difluorobenzyl)-1-(oxetan-2-ylmethyl)-1H-thieno[2,3-d]imidazole-5-carboxylate (21e)

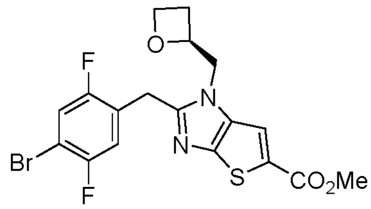

21e

21d (3.0 g, 9.58 mmol) and 1d (2.5 g, 10.3 mmol) were added to glacial acetic acid (50 mL), and stirred at 60°C for 10 hours. The reaction system was cooled to room temperature, then was slowly added to an aqueous saturated sodium bicarbonate solution (200 mL) to quench the reaction, and then extracted with ethyl acetate (50 mL × 3). The organic phase was combined, dried over anhydrous sodium sulfate, and filtered. The filtrate was spin-dried, and the residue was separated and purified by silica gel column chromatography to obtain 21e (1.34 g, yield 30%).
LCMS m/z = 457.1 $[M+1]^+$.

Step 5: (S)-(2,5-difluoro-4-((5-(methoxycarbonyl)-1-(oxetan-2-ylmethyl)-1H-thieno[2,3-d]imidazol-2-yl)methyl)phenyl)boronic acid (21f)

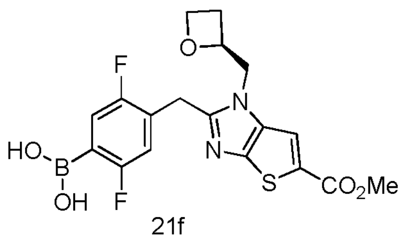

21f

21e (600 mg, 1.3 mmol) and pinacol biborate (519 mg, 1.9 mmol), Pd(dppf)$Cl_2$•DCM (110 mg, 0.1 mmol), and potassium acetate (400 mg, 4.0 mmol) were added to 1,4-dioxane (10 mL), and the reaction flask was subjected to nitrogen replacement for protection, and the reaction liquid in the reaction flask was stirred at 100°C for 5 hours. The reaction liquid was cooled to room temperature, then water (50 mL) was added, and extracted with ethyl acetate (50 mL × 3). The organic phase was combined, dried over anhydrous sodium sulfate, and filtered. The filtrate was concentrated to dryness to obtain crude compound 21f (438 mg, yield 80%).
LCMS m/z = 423.1 $[M+1]^+$.

Step 6: methyl (S)-2-(4-(6-((4-cyano-2-fluorobenzyl)oxy)pyridin-2-yl)-2,5-difluorobenzyl)-1-(oxetan-2-ylmethyl)-1H-thieno[2,3-d]imidazole-5-carboxylate (21g)

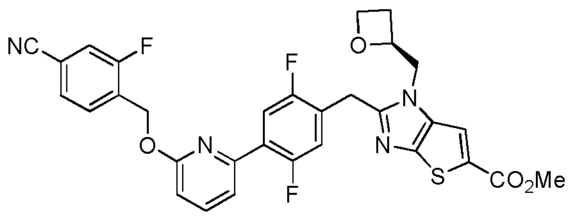

21g

21f (60 mg, 0.14 mmol) and 3a (34 mg, 0.13 mmol), Pd(dppf)$Cl_2$•DCM (10 mg, 0.01 mmol) and potassium carbonate (36 mg, 0.26 mmol) were added to a mixed solution of 1,4-dioxane (2 mL) and water (0.5 mL), and the reaction flask was subjected to nitrogen replacement for protection, and the reaction liquid in the reaction flask was stirred at 100°C for 5 hours. The reaction liquid was cooled to room temperature, then water (2 mL) was added, and extracted with ethyl acetate (10 mL × 3). The organic phase was combined, dried over anhydrous sodium sulfate, and filtered. The filtrate was concentrated to dryness, and the residue was separated and purified by silica gel column chromatography to obtain 21g (46 mg, yield 54%).

LCMS m/z = 605.2 $[M+1]^+$.

Step 7: (S)-2-(4-(6-((4-cyano-2-fluorobenzyl)oxy)pyridin-2-yl)-2,5-difluorobenzyl)-1-(oxetan-2-ylmethyl)-1H-thieno[2,3-d]imidazole-5-carboxylic acid (Compound 21)

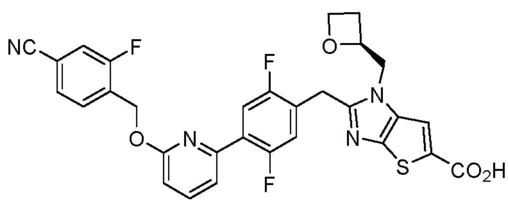

Compound **21**

21g (46 mg, 0.076 mmol) and 1 g (32 mg, 0.228 mmol) were added to a mixed solution of acetonitrile (3 mL) and water (0.5 mL), and stirred at room temperature for 16 hours. Then the reaction liquid was adjusted to pH=6 with 1N hydrochloric acid, continuously stirred until a white solid precipitated, and filtered and the solid was washed with water. The solid was dissolved in dichloromethane, dried over anhydrous sodium sulfate and filtered. The organic phase was concentrated to dryness, the residue was separated and purified by silica gel column chromatography to obtain compound 21 (10 mg, yield 22%).

LCMS m/z = 591.0 $[M+1]^+$.

$^1H$ NMR (400 MHz, DMSO-*d6*) δ 12.91 (br, 1H), 7.93-7.83 (m, 3H), 7.78-7.67 (m, 3H), 7.53-7.48 (m, 1H), 7.37-7.30 (m, 1H), 7.01-6.95 (m, 1H), 5.59 (s, 2H), 5.08-4.99 (m, 1H), 4.67-4.58 (m, 1H), 4.55-4.45 (m, 2H), 4.43-4.29 (m, 3H), 2.74-2.64 (m, 1H), 2.40-2.29 (m, 1H).

**Example 22** (S)-2-(4-(6-((4-cyano-2-fluorobenzyl)oxy)-5-fluoropyridin-2-yl)-2,5-difluorobenzyl)-1-(oxetan-2-ylmethyl)-1H-thieno[2,3-d]imidazole-5-carboxylic acid (Compound 22)

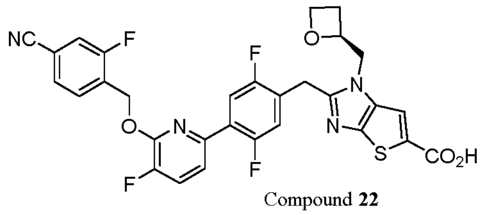

Compound **22**

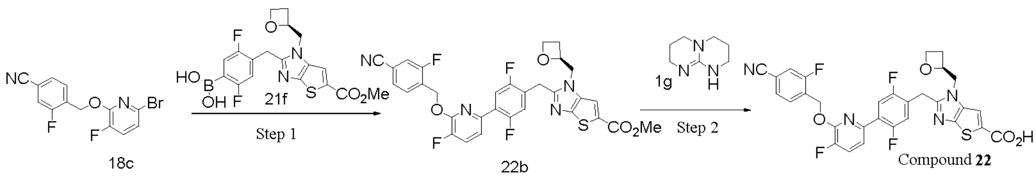


Step 1: methyl (S)-2-(4-(6-((4-cyano-2-fluorobenzyl)oxy)-5-fluoropyridin-2-yl)-2,5-difluorobenzyl)-1-(oxetan-2-ylmethyl)-1H-thieno[2,3-d]imidazole-5-carboxylate (22b)

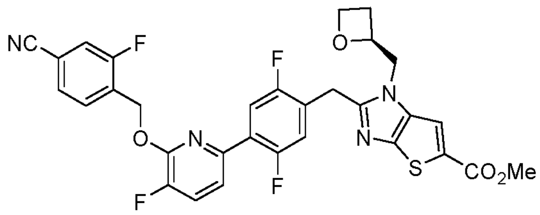

22b

21f (143 mg, 0.34mmol) and 18c (110 mg, 0.34mmol), Pd(dppf)$Cl_2$•DCM (40 mg, 0.04 mmol) and potassium carbonate (140 mg, 1.01 mmol) were added to a mixed solution of 1,4-dioxane (5 mL) and water (1 mL), and the reaction flask was subjected to nitrogen replacement for protection, and the reaction liquid in the reaction flask was stirred at 100°C for 5 hours. The reaction liquid was cooled to room temperature, then water (5 mL) was added, and extracted with ethyl acetate (10 mL × 3). The organic phase was combined, dried over anhydrous sodium sulfate, and filtered. The filtrate was concentrated to dryness, and the residue was separated and purified by silica gel column chromatography to obtain 22b (170 mg, yield 80%).

LCMS m/z = 623.1 $[M+1]^+$.

Step 2: (S)-2-(4-(6-((4-cyano-2-fluorobenzyl)oxy)-5-fluoropyridin-2-yl)-2,5-difluorobenzyl)-1-(oxetan-2-ylmethyl)-1H-thieno[2,3-d]imidazole-5-carboxylic acid (Compound 22)

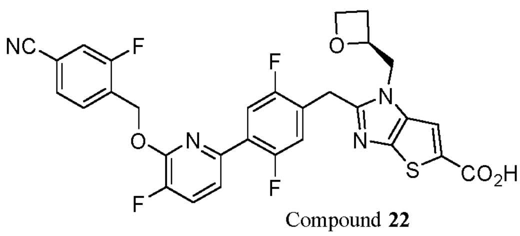

Compound **22**

22b (50 mg, 0.08 mmol) and 1 g (34 mg, 0.24 mmol) were added to a mixed solution of acetonitrile (5 mL) and water (1 mL), and stirred at room temperature for 16 hours. Then the reaction liquid was adjusted to pH=6 with 1N hydrochloric acid, continuously stirred until a white solid precipitated, and filtered and the solid was washed with water. The solid was dissolved in dichloromethane, dried over anhydrous sodium sulfate and filtered. The organic phase was concentrated to dryness, the residue was separated by preparative HPLC to obtain compound 22 (10 mg, yield 20%).

LCMS m/z = 609.1 $[M+1]^+$.

$^1H$ NMR (400 MHz, DMSO-*d6*) δ 7.95-7.90 (m, 1H), 7.88-7.82 (m, 2H), 7.80-7.67 (m, 3H), 7.55-7.50(m, 1H), 7.37-7.31 (m, 1H), 5.68 (s, 2H), 5.06-5.00 (m, 1H), 4.66-4.59 (m, 1H), 4.54-4.45 (m, 2H), 4.40-4.29 (m, 3H), 2.69-2.64 (m, 1H), 2.37-2.30 (m, 1H).

**Example 23** (S)-2-(4-(6-((4-cyanobenzyl)oxy)pyridin-2-yl)-2,5-difluorobenzyl)-1-(oxetan-2-ylmethyl)-1H-thieno[2,3-d]imidazole-5-carboxylic acid (Compound 23)

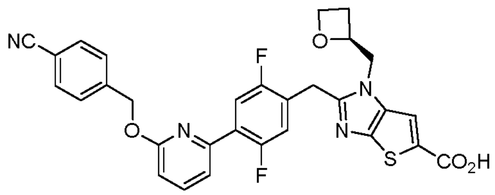

Compound **23**

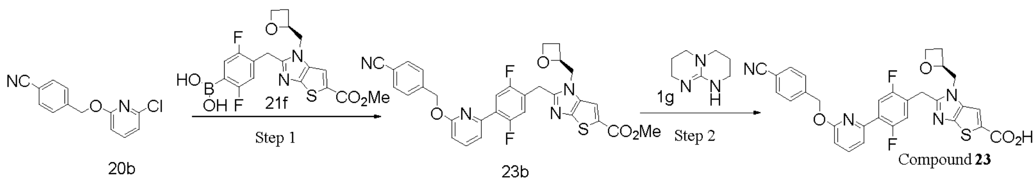


20b

23b

Compound **23**

Step 1: methyl (S)-2-(4-(6-((4-cyano-2-fluorobenzyl)oxy)-5-fluoropyridin-2-yl)-2,5-difluorobenzyl)-1-(oxetan-2-ylmethyl)-1H-thieno[2,3-d]imidazole-5-carboxylate (23b)

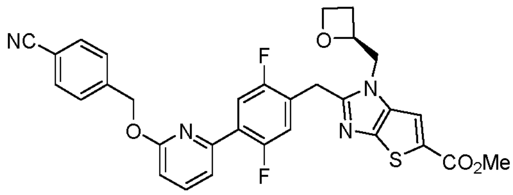

23b

21f (200 mg, 0.49mmol) and 20b (120 mg, 0.49mmol), Pd(dppf)$Cl_2$•DCM (40 mg, 0.04 mmol) and potassium carbonate (140 mg, 1.01 mmol) were added to a mixed solution of 1,4-dioxane (5 mL) and water (1 mL), and the reaction flask was subjected to nitrogen replacement for protection, and the reaction liquid in the reaction flask was stirred at 100°C for 5 hours. The reaction liquid was cooled to room temperature, then water (5 mL) was added, and extracted with ethyl acetate (10 mL × 3). The organic phase was combined, dried over anhydrous sodium sulfate, and filtered. The filtrate was concentrated to dryness, and the residue was separated and purified by silica gel column chromatography to obtain 23b (143 mg, yield 50%).

LCMS m/z = 587.0 $[M+1]^+$.

Step 2: (S)-2-(4-(6-((4-cyanobenzyl)oxy)pyridin-2-yl)-2,5-difluorobenzyl)-1-(oxetan-2-ylmethyl)-1H-thieno[2,3-d]imidazole-5-carboxylic acid (Compound 23)

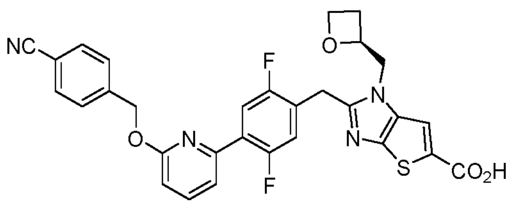

Compound **23**

23b (50 mg, 0.08 mmol) and 1 g (34 mg, 0.24 mmol) were added to a mixed solution of acetonitrile (5 mL) and water (1 mL), and stirred at room temperature for 16 hours. Then the reaction liquid was adjusted to pH=6 with 1N hydrochloric acid, continuously stirred until a white solid precipitated, and filtered and the solid was washed with water. The solid was dissolved in dichloromethane, dried over anhydrous sodium sulfate and filtered. The organic phase was concentrated to dryness, the residue was separated and purified by silica gel column chromatography to obtain compound 23 (15 mg, yield 30%).

LCMS m/z = 573.1 $[M+1]^+$.

$^1H$ NMR (400 MHz, DMSO-*d6*) δ 7.92-7.83 (m, 4H), 7.73-7.64 (m, 3H), 7.53-7.47 (m, 1H), 7.37-7.29 (m, 1H), 7.02-6.96 (m, 1H), 5.57 (s, 2H), 5.08-5.00 (m, 1H), 4.66-4.59 (m, 1H), 4.54-4.45 (m, 2H), 4.39-4.29 (m, 3H), 2.75-2.64 (m, 1H), 2.39-2.29 (m, 1H).

**Example 24:** 2-((4-(2-(5-chloropyridin-2-yl)-2-methylbenzo[d][1,3]dioxol-4-yl)piperidin-1-yl)methyl)-1-(((S)-oxetan-2-yl)methyl)-1H-thieno[2,3-d]imidazole-5-carboxylic acid

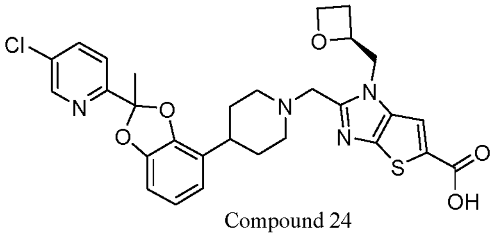

Compound 24

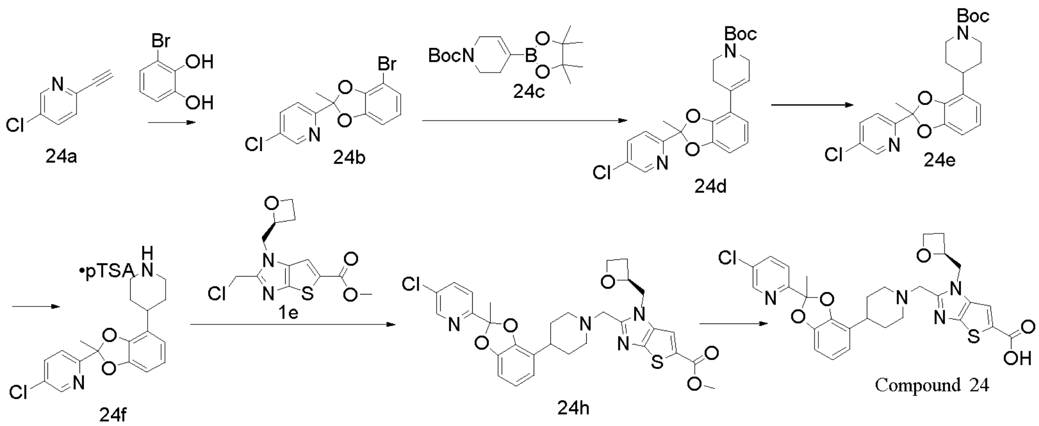

Step 1: 2-(4-bromo-2-methylbenzo[d][1,3]dioxol-2-yl)-5-chloropyridine (24b)

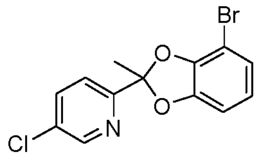

24b

15a (276 mg, 2.0 mmol) and 3-bromocatechol (378 mg, 2.0 mmol) were dissolved in tetrahydrofuran (6 mL), and $Ru_3(CO)_{12}$ (128 mg, 0.2 mmol) was added to the system. The reaction flask was subjected to nitrogen replacement for protection, and the reaction liquid in the reaction flask was stirred at 110°C for 10 hours. The reaction liquid was cooled to room temperature, then concentrated under reduced pressure and purified by silica gel column chromatography to obtain 1b as a gray oily liquid (190 mg, yield 30%).

LCMS m/z = 326.0 $[M+1]^+$.

Step 2: tert-butyl 4-(2-(5-chloropyridin-2-yl)-2-methylbenzo[d][1,3]dioxol-4-yl)-3,6-dihydropyridine-1(2H)-carboxylate (24d)

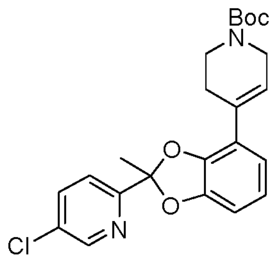

1b (190 mg, 0.58 mmol), 1c (180 mg, 0.0.58 mmol), cesium carbonate (945 mg, 2.9 mmol) and $Pd(dppf)Cl_2$•DCM (47 mg, 0.058 mmol) were dissolved in a mixed solvent of 1,4-dioxane (15 mL) and water (3 mL), subjected to nitrogen replacement and then heated to 90°C for 3 hours. The reaction liquid was cooled to room temperature. Water (30 mL) was added, extracted with ethyl acetate (30 mL × 3). The organic phase was combined and washed with saturated brine (30 mL × 1), and the obtained organic phase was dried over anhydrous sodium sulfate and filtered. The filtrate was concentrated under reduced pressure, and purified by silica gel column chromatography to obtain 1d (180 mg, yield 72%) as a colorless oily liquid.

LCMS m/z = 373.1 $[M-55]^+$.

Step 3: tert-butyl 4-(2-(5-chloropyridin-2-yl)-2-methylbenzo[d][1,3]dioxol-4-yl) piperidine-1-carboxylate (24e)

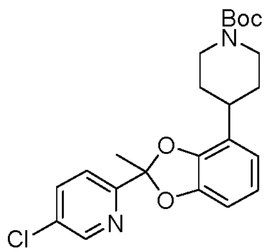

1d (170 mg, 0.4 mmol) was dissolved in a mixed solvent of tetrahydrofuran (3 mL) and methanol (3 mL), and Raney-Ni (20 mg) was added. The reaction was carried out at room temperature for 10 h after hydrogen replacement for protection. The reaction liquid was filtered, the filter cake (20 mL × 3) was washed with tetrahydrofuran, the filtrate was concentrated under reduced pressure, and purified by silica gel column chromatography to obtain 24e as a colorless oily liquid (120 mg, yield 70%).

LCMS m/z = 431.1 $[M+1]^+$.

Step 4: 5-chloro-2-(2-methyl-4-(piperidin-4-yl)benzo[d][1,3]dioxol-2-yl)pyridine p-toluenesulfonate (24f)

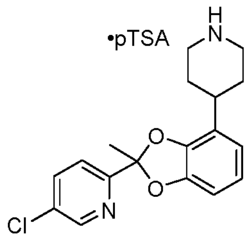

24e (120 mg, 0.28 mmol) was dissolved in ethyl acetate (5 mL), and p-toluenesulfonic acid monohydrate (80 mg, 0.42 mmol) was added. The reaction was carried out at 50°C for 10 hours after hydrogen replacement for protection. The reaction liquid was cooled to room temperature and then concentrated under reduced pressure to obtain the p-toluenesulfonate salt of crude product 24f, which was directly used in the next reaction without further purification.
LCMS m/z = 331.2 $[M+1]^+$.

Step 5: methyl 2-((4-(2-(5-chloropyridin-2-yl)-2-methylbenzo[d][1,3]dioxol-4-yl)piperidin-1-yl)methyl)-1-(((S)-oxetan-2-yl)methyl)-1H-thieno[2,3-d]imidazole-5-carboxylate (24h)

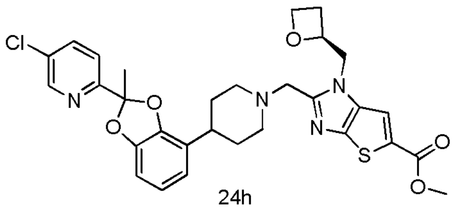

24h

The crude product 24f obtained in the previous step was dissolved in acetonitrile (3 mL), then potassium carbonate (60 mg, 0.42 mmol) and intermediate 1e (42 mg, 0.14 mmol) were added. The reaction was carried out at 60°C for 6 hours after the reaction flask was subjected to nitrogen replacement for protection. The reaction liquid was cooled to room temperature, then concentrated under reduced pressure and purified by silica gel column chromatography to obtain 24h (80 mg, yield 96%) as a light yellow solid.
LCMS m/z = 595.2 $[M+1]^+$.

Step 6: 2-((4-(2-(5-chloropyridin-2-yl)-2-methylbenzo[d][1,3]dioxol-4-yl)piperidin-1-yl)methyl)-1-(((S)-oxetan-2-yl)methyl)-1H-thieno[2,3-d]imidazole-5-carboxylic acid (Compound 24)

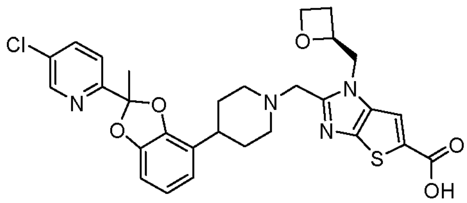

Compound 24

Compound 24h (80 mg, 0.134 mmol) and lithium hydroxide (56 mg, 1.34 mmol) were added to a mixed solvent of acetonitrile (3 mL) and water (0.6 mL), and stirred at 50°C for 6 hours. 1N hydrochloric acid was used to adjust pH = 6, then water (15 mL) was added, extracted with ethyl acetate (20 mL × 3), washed with saturated brine (20 mL × 1). The organic phase was dried over anhydrous sodium sulfate and filtered. The solvent was removed from the filtrate under reduced pressure, and the residue was purified by column chromatography (dichloromethane/methanol=15:1) to

obtain the compound 24 of interest (53 mg, yield 70%).

LCMS m/z = 581.2 $[M+H]^+$.

$^1H$ NMR (400 MHz, $CD_3OD$) δ 8.59 (s, 1H), 7.88 (dd, 1H), 7.73 - 7.60 (m, 2H), 6.84 - 6.76 (m, 1H), 6.72 (d, 2H), 5.24 - 5.15 (m, 1H), 4.71 - 4.60 (m, 2H), 4.60 - 4.51 (m, 1H), 4.47 - 4.38 (m, 1H), 4.21 (s, 2H), 3.45 - 3.32 (m, 2H), 2.90 - 2.66 (m, 4H), 2.55 - 2.42 (m, 1H), 2.01 (s, 3H), 1.99 - 1.89 (m, 4H).

**Biological test examples**

1. HEK293/CRE-luc/GLP-1R cell viability

Cell: HEK293/CRE-luc/GLP-1R
Cell culture medium: DMEM + 10% FBS + 400 μg/ml G418 + 100 μg/ml Hygromycin B
Frozen stock solution: 90% FBS, 10% (V/V) DMSO
Detection buffer: DMEM + 1% FBS

Experimental procedure

Cells were cultured in DMEM medium + 10% FBS + 400 μg/ml G418 + 100 μg/ml Hygromycin B in a CO2 incubator at 37°C, and passaged once every 3-4 days.

Cell plating: Trypsinization adjusted the cell density to $1.67\times10^5$ cells/mL; 60 μL of cells per well (10000 cells/well) was inoculated in a 384-well plate containing compounds; A NC well (negative control) and a background well (no cells) were set. Incubation was performed for about 18 ± 2 h in the incubator.

Compounds were serially diluted with the detection buffer, and the detection concentration was 0.01 nM-1000 nM.

The cell culture plate was removed and all supernatant was aspirated from the cells. The cells were washed gently 2 times with 1X PBS.

The diluted compound was added to a 384-well plate (10 μL/well), and three replicate wells were set for each concentration. 10 μL of the detection buffer was added to the NC well, sealed and incubated at 37°C for 6 hours.

The plate was removed, the cells were allowed to equilibrate to room temperature (at least 15 min), and then all supernatant was aspirated from the cells.

Steady-Glo® Reagent was added to the sample well at 10 μL/well and incubated at room temperature for 5 min to lyse the cells.

The test results were read with a BMG microplate reader.

Data processing

The mean background value was calculated.

The induction factor (Fold of induction, FI) = (induction well value - background value) / (negative control well value - background value) was calculated.

The $EC_{50}$ value of the sample was calculated using Graphpad Prim 8.0 software using four-parameter fitting analysis.

Statistic analysis

Mean and standard error (Mean ± SEM) were calculated for all results and Graphpad Prism software was used for statistical analysis. Specific data were presented in the form of graphs. $P<0.05$ was considered to be statistically different.

Biological test results:

| Compounds | $EC_{50}$ (nM) |
|---|---|
| Trifluoroacetate salt of compound 1 | A |

(continued)

| Compounds | $EC_{50}$ (nM) |
|---|---|
| Trifluoroacetate salt of compound 2 | A |
| Trifluoroacetate salt of compound 3 | A |
| Compound 4 | A |
| Compound 5 | A |
| Compound 5-2 | A |
| Compound 6 | A |
| Compound 7 | A |
| Compound 8 | A |
| Compound 9 | A |
| Compound 10 | A |
| Compound 11 | A |
| Compound 12 | A |
| Compound 13 | A |
| Compound 15 | A |
| Compound 16 | A |
| Compound 17 | A |
| Compound 18 | A |
| Compound 20 | A |
| Compound 21 | A |
| Compound 22 | A |
| Compound 23 | A |
| Compound 24 | A |
| A < 10 nM, 10 nM ≤ B < 50 nM, 50nM ≤ C < 100 nM | |

Conclusion: The compound of the present invention has good agonistic effect on GLP-1 receptor, and has a $EC_{50}$ value less than 100 nM, for example the $EC_{50}$ values of the trifluoroacetate salt of compound 1, the trifluoroacetate salt of compound 2, the trifluoroacetate salt of compound 3, Compound 4, Compound 5, Compound 5-2, Compound 6, Compound 7, Compound 8, Compound 9, Compound 10, Compound 11, Compound 12, Compound 13, Compound 15, Compound 16, Compound 17, Compound 18, Compound 20, Compound 21, Compound 22, Compound 23, and Compound 24 were less than 10 nM.

**2.** Effects of the compounds on hERG potassium channels

Experimental platform: electrophysiological manual patch-clamp system
Cell line: Chinese hamster ovary (CHO) cells stably expressing hERG potassium ion channel
Experimental method: For CHO (Chinese Hamster Ovary) cells stably expressing hERG potassium channel, whole cell patch-clamp technique was used to recorded hERG potassium channel current at room temperature in this experiment. The glass microelectrode is made of a glass electrode blank (BF150-86-10, Sutter) by a puller. The tip resistance after filling the liquid in the electrode is about 2-5 MΩ. The glass microelectrode can be connected to the patch-clamp amplifier by inserting the glass microelectrode into an amplifier probe. The clamping voltage and data recording are controlled and recorded by the pClamp 10 software through a computer. The sampling frequency is 10 kHz, and the filtering frequency is 2 kHz. After the whole cell records were obtained, the cells were clamped at

-80 mV, and the step voltage that induced the hERG potassium current ($I_{hERG}$) was depolarized from -80 mV to +20 mV for 2 s, then repolarized to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells (n≥2) were tested at each concentration.

Data processing:

Data analysis processing was carried out by using pClamp 10, GraphPad Prism 5 and Excel software. The inhibition degree of hERG potassium current (peak value of hERG tail current induced at -50 mV) at different compound concentrations was calculated by the following formula: Inhibition % = [1 - ($I/Io$)]×100%.

Among the formula, Inhibition % represents the percentage of inhibition of hERG potassium current by the compound, and $I$ and $Io$ represent the amplitude of hERG potassium current after and before dosing, respectively.

Compound $IC_{50}$ was calculated using GraphPad Prism 5 software by fitting according to the following equation:

$$Y = Bottom + (Top-Bottom)/(1+10^{\wedge}((LogIC_{50}-X) \times HillSlope))$$

Among the equation, X represents the Log value of the tested concentration of the test sample, Y represents the inhibition percentage at the corresponding concentration, and Bottom and Top represent the minimum and maximum inhibition percentage, respectively.

Biological test results

| Compounds | Structure | $IC_{50}$ (μM) |
|---|---|---|
| Compound 1 | 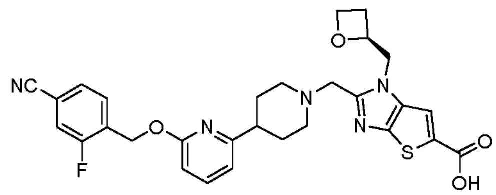 | 33.6 |
| Compound 2 | 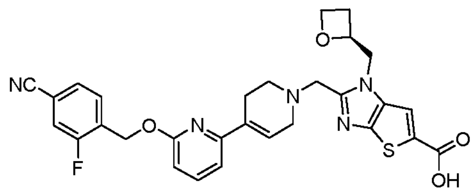 | 52.2 |
| Compound 5-2 | 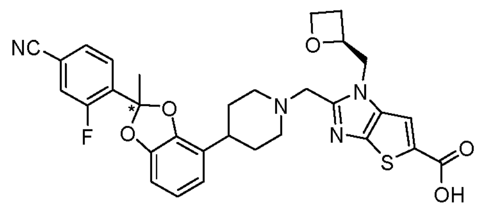 | > 40 |
| Compound 6 | 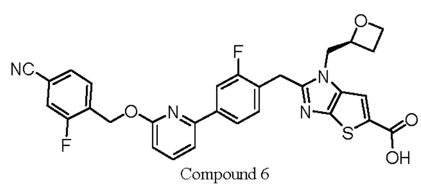 Compound 6 | > 40 |

Conclusion: The compounds of the present invention have less hERG inhibitory effect.

3. Stability test in mouse liver microsomes

At 37°C, 1 μM of the test compound was incubated with mouse liver microsomes (0.5 mg/mL) supplemented with NADPH regeneration system for 5, 10, 20, 30 and 60 minutes, and then LC-MS/MS method was used to detect the concentration of the test compound in the generated sample. The half-life ($T_{1/2}$) and inherent clearance rate ($CL_{int(mic)}$) of the compound in mouse liver microsome solution were obtained by calculating at the corresponding time points and the remaining percentage of the compound.

Biological test results:

| Compounds | Structure | $T_{1/2}$ (min) | $CL_{int(mic)}$ (μL/min/mg) |
|---|---|---|---|
| Compound 2 | 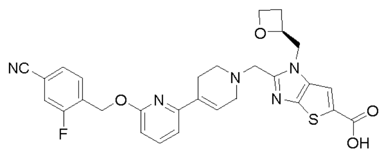 | 1419 | 0.977 |
| Compound 5-2 | 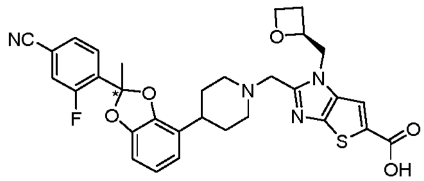 | 5588 | 0.252 |
| Compound 6 | 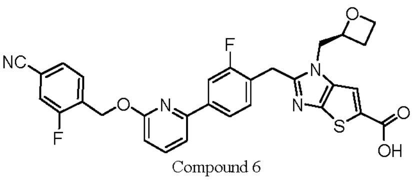 Compound 6 | 296 | 4.69 |
| Compound DZ-6 (See WO 2020207474 for Compound 84) | 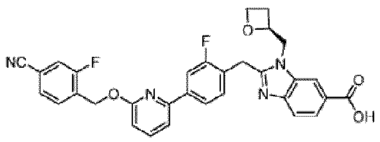 | 130 | 10.6 |

Conclusion: The compound of the present invention has good stability in mouse liver microsomes. For example, compared with the control compound, the clearance rate of compound 6 in mouse liver microsomes was significantly slowed down, and the half-life was significantly prolonged. The $T_{1/2}$ ratio of compound 6 to compound DZ-6 was greater than 2.

4. Stability test in monkey liver microsomes

At 37°C, 1 μM of the test compound was incubated with monkey liver microsomes (0.5 mg/mL) supplemented with NADPH regeneration system for 5, 15, 30, 45 and 60 minutes, and then LC-MS/MS method was used to detect the concentration of the test compound in the generated sample. The half-life ($T_{1/2}$) and inherent clearance rate ($CL_{int(mic)}$) of the compound in monkey liver microsome solution were obtained by calculating at the corresponding time points and the remaining percentage of the compound.

Biological test results:

| Compounds | Structure | $T_{1/2}$ (min) | $CL_{int(mic)}$ (μL/min/mg) |
|---|---|---|---|
| Compound 2 | 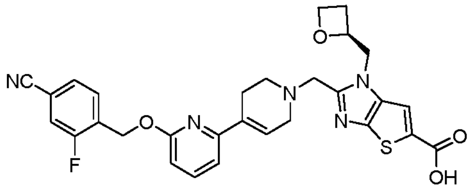 | 842 | 1.60 |

Conclusion: The compound of the present invention has good stability in monkey liver microsomes.

6. Stability test in canine liver microsomes

At 37°C, 1 μM of the test compound was incubated with canine liver microsomes (0.5 mg/mL) supplemented with NADPH regeneration system for 5, 15, 30, 45 and 60 minutes, and then LC-MS/MS method was used to detect the concentration of the test compound in the generated sample. The half-life ($T_{1/2}$) and inherent clearance rate ($CL_{int(mic)}$) of the compound in canine liver microsome solution were obtained by calculating at the corresponding time points and the remaining percentage of the compound.

Biological test results:

| Compounds | Structure | $T_{1/2}$ (min) | $CL_{int(mic)}$ (μL/min/mg) |
|---|---|---|---|
| Compound 1 | 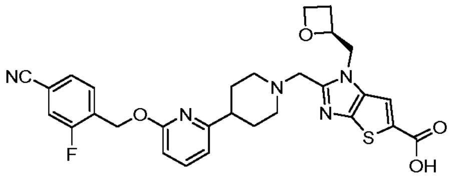 | 6116 | 0.23 |

Conclusion: The compound of the present invention has good stability in canine liver microsomes.

7. Stability test in human liver microsomes

At 37°C, 1 μM of the test compound was incubated with human liver microsomes (0.5 mg/mL) supplemented with NADPH regeneration system for 5, 10, 20, 30 and 60 minutes, and then LC-MS/MS method was used to detect the concentration of the test compound in the generated sample. The half-life ($T_{1/2}$) and inherent clearance rate ($CL_{int(mic)}$) of the compound in human liver microsome solution were obtained by calculating at the corresponding time points and the remaining percentage of the compound.

Biological test results:

| Compounds | Structure | $T_{1/2}$ (min) | $CL_{int(mic)}$ (μL/min/mg) |
|---|---|---|---|
| Compound 6 | 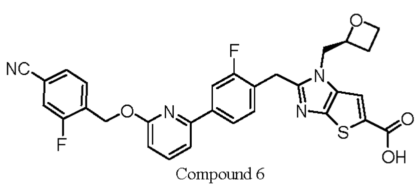 Compound 6 | 554 | 2.50 |
| Compound DZ-6 (See WO 2020207474 for Compound 84) | 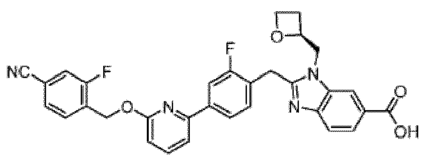 | 173 | 8.01 |

Conclusion: The compound of the present invention has good stability in canine liver microsomes. For example,

compared with the control compound, the clearance rate of compound 6 in monkey liver microsomes was significantly slowed down, and the half-life was significantly prolonged. The $T_{1/2}$ ratio of compound 6 to compound DZ-6 was greater than 3.

8. Activity of the compounds on PDE10A1

In this experiment, the effect of the compounds on the inhibition of PDE10A1 activity was detected by the method of fluorescence polarization. PDE10A1 kit was used for detection (Biomol, BPS-60385). According to the kit instruction, FAM-Cyclic-3',5'-AMP (200 nM) dilution was added to wells other than the blank control well(Blank) at 12.5 μl/well; PDE test buffer was added to the blank control well (Blank) at 22.5 μl/well; PDE test buffer was added to the Substrate control well at 10 μl/well; a compound working solution was added to the corresponding well of the test plate at 2.5 μl/well; PDE test buffer (10% DMSO) was added to the positive control well (Positive control), the Substrate control well, and the blank control (Blank) well at 2.5 μl/well, respectively; PDE10A1 enzyme was diluted to 1 pg/μl with PDE test buffer, and the enzyme solution was added to the Substrate control well and the positive control well (Positive control) at 10 μl/well; the final concentration of the test compound was 300 μM-15.24 nM, and the final concentration of the positive reference compound TAK063 was 100 nM-0.00508 nM; incubation was performed at room temperature for 1 hour; the Binding Agent was diluted in a ratio of 1: 100 with Binding Agent Diluent (cAMP) for later use; after the incubation, the Binding Agent dilution was added to the test plate at 50 μl/well, and incubated at room temperature for 20 minutes with slow shaking; after the incubation, Envision was used for FP detection (Excitation 480 nm, Emission 535 nm); Data were normalized to % inhibition and $IC_{50}$ values were calculated using a Graphpad 4 parameter logistic equation.
Conclusion: The compounds of the present invention have a small inhibitory effect on PDE10A1, for example, for the inhibitory effect of Compound 2 on PDE10A1, $IC_{50}$ (μM) > 500.

8. Pharmacokinetic test

**Evaluation of pharmacokinetics in mice**

Male C57 mice (purchased from Hunan SJA Laboratory Animal Co., Ltd, license number: SCXK (Xiang) 2019-0004), 20-25g, were fasted overnight. On the day of the experiment, 18 male C57 mice were divided into 2 groups, with 9 mice in each group. The test compound was administered intravenously and intragastrically at doses of 5 mg/kg and 20 mg/kg respectively. Before administration and 5 min, 15 min, 30 min, 1 h, 2 h, 4 h, 6 h, 8 h and 24 h after administration, 0.1 ml of blood was collected from the orbital venous plexus, placed in an EDTAK2 centrifuge tube, and centrifuged at 6000 rpm at 4°C for 5 min, and then the plasma was collected. The plasma sample was added to an acetonitrile solution containing an internal standard (dexamethasone, 500 ng/mL), vortexed and centrifuged at 10,000 rpm for 10 min, and the supernatant was taken for LC-MS/MS analysis. Pharmacokinetic parameters were calculated using the non-compartmental model in WinNonlin Version 8.0 (Pharsight, Mountain View, CA) pharmacokinetic software.
Conclusion: The compounds of the present invention have good oral performance, for example, the bioavailability of compound 6 in C57 mice is >25%.

**Evaluation of pharmacokinetics in cynomolgus monkeys**

Male cynomolgus monkeys (raised in Suzhou LEOLAB Bio Tech. Co.,Ltd.), weighing 3-5 kg, were fasted overnight (14-18 h), 3-5 kg, were fasted overnight. On the day of the experiment, 6 male cynomolgus monkeys were divided into 2 groups, with 3 mice in each group. The test compound was administered intravenously and intragastrically at doses of 2 mg/kg and 10 mg/kg respectively. Before administration and 5 min, 15 min, 30 min, 1 h, 2 h, 4 h, 6 h, 8 h 10 h, 12 h and 24 h after administration, 1 ml of blood was collected via the limb vein, placed in an EDTAK2 centrifuge tube, and centrifuged at 6000 rpm at 4°C for 5 min, and then the plasma was collected. The plasma sample was added to an acetonitrile solution containing an internal standard (dexamethasone, 500 ng/mL), vortexed and centrifuged at 10,000 rpm for 10 min, and the supernatant was taken for LC-MS/MS analysis. Pharmacokinetic parameters were calculated using the non-compartmental model in WinNonlin Version 8.0 (Pharsight, Mountain View, CA) pharmacokinetic software. Conclusion: The compounds of the present invention have good metabolic properties. For example, compound 6 has a low clearance rate and a long half-life in monkeys, which has the advantages of longer drug action time and lower frequency of administration.

9. **Cytochrome P450 isoenzyme inhibition test**

Experimental objective: The inhibitory effect of the test compound on the activity of human liver microsomal

cytochrome P450 isoenzyme (CYP1A2, CYP2C19, CYP2D6 and CYP3A4) was determined.

Experimental operation: The test compound at an incubation concentration of 0, 0.03, 0.1, 0.3, 1, 3, 10, 30 and 100 μM was pre-incubated with the human liver microsome solutions containing 5 enzyme mixed substrates (liver microsome concentration: 0.1 mg/ mL) in a water bath at 37°C for 10 min, respectively, and then a coenzyme factor (NADPH) was added to start the reaction. After incubation in a water bath at 37°C for 10 min, the reaction was terminated by adding glacial acetonitrile solution containing an internal standard, shaken for 1 min, centrifuged at 4°C and 10,000 rpm for 10 min, and the supernatant was taken for LC/MS/MS analysis. The amount of metabolites produced by each CYP enzyme substrate was detected.

The activity of each CYP isoenzyme was reflected by the production rate of each metabolite. The activity of each isoenzyme in the solvent control incubation system without test substances was set as 100%, and the ratio of the production rate of metabolites in the case of different concentrations of test substances to the production rate of metabolites in the case of a solvent control sample was taken as the percentage of residual activity of each isoenzyme. With the residual activity percentage as the ordinate and the inhibitor concentration as the abscissa, Graphpad prism 5.0 was used to plot the data and calculate the $IC_{50}$ value of the test substance for each CYP isoenzyme.

Conclusion: The compounds of the present invention have weak inhibitory activity on cytochrome P450 isoenzymes, for example, for the inhibitory activity of compound 6 on CYP2C19, CYP1A2, CYP2D6 and CYP3A4-M, IC50 > 50 μM.

## Claims

1. A compound or a stereoisomer, tautomer, deuterated substance, solvate, prodrug, metabolite, pharmaceutically acceptable salt or co-crystal thereof, which compound is selected from a compound represented by general formula (I), wherein,

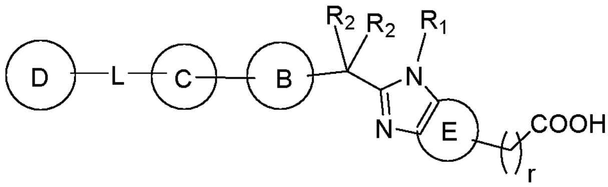

(I)

ring B is selected from a 4- to 12-membered heterocyclic ring, $C_{5-12}$ carbocyclic ring, $C_{6-10}$ aromatic ring or a 5- to 12-membered heteroaromatic ring; the carbocyclic ring, heterocyclic ring, aromatic ring or heteroaromatic ring is optionally further substituted with 0 to 4 $R_B$; the heterocyclyl or heteroaromatic ring contains 1 to 3 heteroatoms selected from O, S and N;

ring C is selected from $C_{6-10}$ carbocyclic ring, a 5- to 10-membered heterocyclic ring, $C_{6-10}$ aromatic ring or a 5- to 10-membered heteroaromatic ring; the carbocyclic ring, heterocyclic ring, aromatic ring or heteroaromatic ring is optionally further substituted with 0 to 4 Rc; the heterocyclic ring or heteroaromatic ring contains 1 to 5 heteroatoms selected from O, S and N;

L is selected from a bond, O, S, $-NR_L-$, $-C(R_L)_2-$, $-C(R_L)_2-C(R_L)_2-$, $-Y-C(R_L)_2-$, $-C(R_L)_2-Y-$, $-Y-C(R_L)_2-C(R_L)_2-$, $-C(R_L)_2-C(R_L)_2-Y-$ or $-C(R_L)_2-Y-C(R_L)_2-$;

Y is selected from O, S or $-NR_L-$;

ring D is selected from a 6- to 10-membered aromatic ring or a 5- to 12-membered heteroaromatic ring; the aromatic ring or heteroaromatic ring is optionally further substituted with 0 to 5 $R_D$; the heteroaromatic ring contains 1 to 5 heteroatoms selected from O, S and N;

ring E is selected from a 5-membered heterocyclic ring, and the heterocyclic ring is optionally further substituted with 0 or 1 substituent selected from H, halogen, CN, OH, $NH_2$, $C_{1-6}$ alkyl, $C_{1-6}$ alkoxy or $C_{3-6}$ cycloalkyl; the heterocyclic ring contains 1 to 2 heteroatoms selected from O, S and N;

$R_1$ is selected from H, halogen, OH, -SH, $CF_3$, CN, $C_{1-6}$ alkyl, $C_{1-6}$ alkoxy, $-C_{1-3}$ alkylene-Z-$C_{0-3}$ alkylene-$R_{1a}$, $C_{0-4}$ alkylene-$R_{1a}$; the alkyl, alkoxy, and alkylene are optionally further substituted with 0 to 4 substituents selected from H, halogen, =O, CN, OH, $-N(R_{1b})_2$, $C_{1-6}$ alkyl, halogen-substituted $C_{1-6}$ alkyl, hydroxy-substituted $C_{1-6}$ alkyl, cyano-substituted $C_{1-6}$ alkyl, $C_{1-6}$ alkoxy, $C_{3-6}$ cycloalkyl, a 3- to 6 -membered heterocycloalkyl, a 5- to 10-membered heteroaryl; the heterocycloalkyl or heteroaryl contains 1 to 3 heteroatoms selected from O, S and N;

Z is selected from a bond, $N(R_{1b})$, O or S;

$R_{1a}$ is selected from $C_{1-6}$ alkyl, $C_{1-6}$ alkoxy, $C_{3-8}$ cycloalkyl, a 3- to 8-membered heterocycloalkyl, a 6- to 10-membered aryl or a 5- to 12-membered heteroaryl; the cycloalkyl, heterocycloalkyl, aryl or heteroaryl is optionally further substituted with 0 to 4 substituents selected from H, halogen, =O, OH, CN, $C_{1-6}$ alkyl, $C_{1-6}$ alkoxy or -$N(R_{1b})_2$; the alkyl or alkoxy is optionally further substituted with 0 to 4 substituents selected from H, halogen, =O, CN, OH, -$N(R_{1b})_2$, $C_{1-6}$ alkyl, $C_{1-6}$ alkoxy or $C_{3-6}$ cycloalkyl; the heterocycloalkyl or heteroaryl contains 1 to 3 heteroatoms selected from O, S and N;

each $R_{1b}$ is independently selected from H or $C_{1-6}$ alkyl; the alkyl is optionally further substituted with 0 to 3 substituents selected from H, halogen, =O, CN, OH, $NH_2$, $C_{1-6}$ alkyl, $C_{1-6}$ alkoxy or $C_{3-6}$ cycloalkyl;

$R_2$ is selected from H, halogen or $C_{1-6}$alkyl;

each $R_L$ is independently selected from H, halogen, -SH, CN, OH, $CF_3$, $NH_2$, $C_{1-6}$ alkyl, $C_{1-6}$ alkoxy or $C_{3-6}$ cycloalkyl; the alkyl, cycloalkyl or alkoxy is optionally further substituted with 0 to 4 substituents selected from H, halogen, =O, OH, CN, $NH_2$, $C_{1-6}$ alkyl, $C_{3-6}$ cycloalkyl or $C_{1-6}$ alkoxy;

each $R_B$, $R_C$ or $R_D$ is independently selected from H, halogen, =O, CN, $CF_3$, OH, -SH, $NH_2$, $C_{1-6}$ alkyl, $C_{3-6}$ cycloalkyl or $C_{1-6}$ alkoxy; the alkyl, cycloalkyl or alkoxy is optionally further substituted with 0 to 4 substituents selected from H, halogen, =O, OH, CN, $NH_2$, $C_{1-6}$ alkyl, $C_{3-6}$ cycloalkyl or $C_{1-6}$ alkoxy;

alternatively, two $R_B$, two $R_C$, two $R_D$, $R_C$ and $R_L$, $R_D$ and $R_L$ or $R_B$ and $R_2$ each independently form a $C_{3-10}$carbocyclic ring or a 3- to 10-membered heterocyclic ring together with the atoms attached thereto; the carbocyclic ring or heterocyclic ring is optionally further substituted with 0 to 4 substituents selected from H, halogen, =O, OH, CN, $NH_2$, $C_{1-6}$ alkyl, $C_{3-6}$ cycloalkyl or $C_{1-6}$ alkoxy; the alkyl, cycloalkyl or alkoxy is optionally further substituted with 0 to 4 substituents selected from H, halogen, =O, OH, CN, $NH_2$, $C_{1-6}$ alkyl, $C_{3-6}$ cycloalkyl or $C_{1-6}$ alkoxy; the heterocyclic ring contains 1 to 3 heteroatoms selected from O, S and N;

and r is selected from 0 or 1.

**2.** The compound or the stereoisomer, tautomer, deuterated substance, solvate, prodrug, metabolite, pharmaceutically acceptable salt or co-crystal thereof according to claim 1, wherein,

$R_1$ is selected from H, halogen, OH, -SH, $CF_3$, CN, $C_{1-4}$ alkyl, $C_{1-4}$ alkoxy, -$C_{1-2}$ alkylene-Z-$C_{0-2}$ alkylene-$R_{1a}$ or -$C_{0-4}$ alkylene-$R_{1a}$; the alkyl, alkoxy or alkylene is optionally further substituted with 0 to 4 substituents selected from H, halogen, =O, CN, OH, -$N(R_{1b})_2$, $C_{1-4}$ alkyl, halogen-substituted $C_{1-4}$ alkyl, hydroxy-substituted $C_{1-4}$ alkyl, cyano-substituted $C_{1-4}$ alkyl, $C_{1-4}$ alkoxy, $C_{3-6}$ cycloalkyl, a 3- to 6 -membered heterocycloalkyl or a 5- to 6-membered heteroaryl; the heterocycloalkyl or heteroaryl contains 1 to 3 heteroatoms selected from O, S and N;

$R_{1a}$ is selected from $C_{1-4}$ alkyl, $C_{1-4}$ alkoxy, $C_{3-6}$ cycloalkyl, a 3- to 6-membered heterocycloalkyl, phenyl or a 5- to 6-membered heteroaryl; the cycloalkyl, heterocycloalkyl, phenyl or heteroaryl is optionally further substituted with 0 to 4 substituents selected from H, halogen, =O, OH, CN, $C_{1-4}$ alkyl, $C_{1-4}$ alkoxy or -$N(R_{1b})_2$; the alkyl or alkoxy is optionally further substituted with 0 to 4 substituents selected from H, halogen, =O, CN, OH, -$N(R_{1b})_2$, $C_{1-4}$ alkyl, $C_{1-4}$ alkoxy or $C_{3-6}$ cycloalkyl; the heterocycloalkyl or heteroaryl contains 1 to 3 heteroatoms selected from O, S and N;

each $R_{1b}$ is independently selected from H or $C_{1-4}$ alkyl; the alkyl is optionally further substituted with 0 to 3 substituents selected from H, halogen, =O, CN, OH, $NH_2$, $C_{1-4}$ alkyl, $C_{1-4}$ alkoxy or $C_{3-6}$ cycloalkyl;

each $R_L$ is independently selected from H, halogen, CN, OH, -SH, $CF_3$, $NH_2$, $C_{1-4}$ alkyl, $C_{1-4}$ alkoxy or $C_{3-6}$ cycloalkyl; the alkyl, cycloalkyl or alkoxy is optionally further substituted with 0 to 4 substituents selected from H, halogen, =O, OH, CN, $NH_2$, $C_{1-4}$ alkyl, $C_{3-6}$ cycloalkyl or $C_{1-4}$ alkoxy;

each $R_B$, $R_C$ or $R_D$ is independently selected from H, halogen, =O, CN, $CF_3$, OH, -SH, $NH_2$, $C_{1-4}$ alkyl, $C_{3-6}$ cycloalkyl or $C_{1-4}$ alkoxy; the alkyl, cycloalkyl or alkoxy is optionally further substituted with 0 to 4 substituents selected from H, halogen, =O, OH, CN, $NH_2$, $C_{1-4}$ alkyl, $C_{3-6}$ cycloalkyl or $C_{1-4}$ alkoxy;

alternatively, two $R_B$, two $R_C$, two $R_D$, $R_C$ and $R_L$ or $R_B$ and $R_2$ each independently form a $C_{3-7}$ carbocyclic ring or a 4- to 7-membered heterocyclic ring together with the atoms attached thereto; the carbocyclic ring or heterocyclic ring is optionally further substituted with 0 to 4 substituents selected from H, halogen, =O, OH, CN, $NH_2$, $C_{1-4}$ alkyl, $C_{3-6}$ cycloalkyl or $C_{1-4}$ alkoxy; the alkyl, cycloalkyl or alkoxy is optionally further substituted with 0 to 4 substituents selected from H, halogen, =O, OH, CN, $NH_2$, $C_{1-4}$ alkyl, $C_{3-6}$ cycloalkyl or $C_{1-4}$ alkoxy; the heterocyclic ring contains 1 to 3 heteroatoms selected from O, S and N.

**3.** The compound or the stereoisomer, tautomer, deuterated substance, solvate, prodrug, metabolite, pharmaceutically acceptable salt or co-crystal thereof according to claim 2, wherein,

ring B is selected from a benzene ring, a 4- to 8-membered heterocyclyl, $C_{5-8}$ carbocyclyl or a 5- to 6-membered heteroaromatic ring; the benzene ring, carbocyclyl, heterocyclyl or heteroaromatic ring is optionally further substituted with 0 to 4 $R_B$, the heterocyclic ring contains 1 to 3 heteroatoms selected from O, S and N;

ring C is selected from a benzene ring, a 5- to 6-membered monocyclic heteroaromatic ring, a 5-membered

fused 5-membered heteroaromatic ring, a 5-membered fused 6 membered heteroaromatic ring or a 6-membered fused 6-membered heteroaromatic ring; the benzene ring or heteroaromatic ring is optionally further substituted with 0 to 4 Rc, the heteroaromatic ring contains 1 to 5 heteroatoms selected from O, S and N;

ring D is selected from a benzene ring, a naphthalene ring, a 5- to 6-membered monocyclic heteroaromatic ring, a 5-membered fused 5-membered heteroaromatic ring, a 5-membered fused 6-membered heteroaromatic ring or a 6-membered fused 6-membered heteroaromatic ring; the benzene ring, naphthalene ring or heteroaromatic ring is optionally further substituted with 0 to 4 $R_D$, the heteroaromatic ring contains 1 to 5 heteroatoms selected from O, S and N;

ring E is selected from a 5-membered non-aromatic heterocyclic ring or a 5-membered heteroaromatic ring, and the heterocyclic ring or heteroaromatic ring is optionally further substituted with 0 or 1 substituent selected from H, halogen, CN, OH, $NH_2$, $C_{1-4}$ alkyl, $C_{1-4}$ alkoxy or $C_{3-6}$ cycloalkyl; the heterocyclic ring or heteroaromatic ring contains 1 to 2 heteroatoms selected from O, S and N;

$R_1$ is selected from H, F, Cl, OH, CN, $CF_3$, methyl, ethyl, propyl, methoxy, ethoxy, propoxy, isopropoxy, -ethylene-Z-methylene-$R_{1a}$, -ethylene-Z-ethylene-$R_{1a}$, -ethylene-Z-$R_{1a}$, -$R_{1a}$, -methylene-$R_{1a}$, -ethylene-$R_{1a}$; the methyl, ethyl, propyl, methoxy, ethoxy, methylene, ethylene are optionally further substituted with 0 to 3 substituents selected from H, F, Cl, =O, CN, OH, -$N(R_{1b})_2$, methyl, ethyl, propyl, $CF_3$, -$CH_2F$, -$CHF_2$, 1 to 3 F-substituted ethyl, hydroxymethyl, hydroxyethyl, cyano-substituted methyl, cyano-substituted ethyl, methoxy, ethoxy, isopropoxy, cyclopropyl, cyclobutyl, cyclopentyl, cyclohexyl, oxiranyl, aziridinyl, oxetanyl, azetidinyl, tetrahydrofuranyl, tetrahydro-2*H*-pyranyl, dioxolanyl, dioxanyl, pyrrolidinyl, piperidinyl, imidazolidinyl, oxazolidinyl, oxazinanyl, morpholinyl, hexahydropyrimidinyl, piperazinyl, pyrrolyl, pyridyl, pyrazolyl, triazolyl, tetrazolyl, imidazolyl, thiazolyl, thienyl, furyl, oxazolyl, isoxazolyl, oxadiazolyl, isothiazolyl, pyrimidinyl, pyrazinyl or pyridazinyl;

Z is selected from O, S or $N(R_{1b})$;

$R_{1a}$ is selected from methyl, ethyl, isopropyl, propyl, methoxy, ethoxy, propoxy, isopropoxy, cyclopropyl, cyclobutyl, cyclopentyl, cyclohexyl, oxiranyl, aziridinyl, oxetanyl, azetidinyl, tetrahydrofuranyl, tetrahydro-2*H*-pyranyl, dioxolanyl, dioxanyl, dioxanyl, pyrrolidinyl, piperidinyl, imidazolidinyl, oxazolidinyl, oxazinanyl, morpholinyl, hexahydropyrimidinyl, piperazinyl, pyrrolyl, pyridyl, pyrazolyl, triazolyl, tetrazolyl, imidazolyl, thiazolyl, thienyl, furyl, oxazolyl, isoxazolyl, oxadiazolyl, isothiazolyl, pyrimidinyl, pyrazinyl or phenyl, the $R_{1a}$ is optionally further substituted with 0 to 3 substituents selected from H, F, =O, OH, CN, methyl, ethyl, propyl, $CF_3$, -$CH_2F$, -$CHF_2$, 1 to 3 F-substituted ethyl, hydroxymethyl, hydroxyethyl, cyano-substituted methyl, cyano-substituted ethyl, methoxy, ethoxy or -$N(R_{1b})_2$;

each $R_{1b}$ is independently selected from H, methyl or ethyl;

$R_2$ is selected from H, F, methyl or ethyl;

each $R_L$ is independently selected from H, F, Cl, CN, $CF_3$, OH, $NH_2$, methyl, ethyl, propyl, $CF_3$, -$CH_2F$, -$CHF_2$, 1 to 3 F-substituted ethyl, hydroxymethyl, hydroxyethyl, cyano-substituted methyl, cyano-substituted ethyl, methoxy, ethoxy, cyclopropyl, cyclobutyl or cyclopentyl;

each $R_B$, $R_C$ or $R_D$ is independently selected from H, F, Cl, =O, CN, $CF_3$, OH, $NH_2$, methyl, ethyl, propyl, -$CH_2F$, -$CHF_2$, 1 to 3 F-substituted ethyl, hydroxymethyl, hydroxyethyl, cyano-substituted methyl, cyano-substituted ethyl, methoxy, ethoxy, propoxy, isopropoxy, tert-butyloxy, trifluoromethoxy, cyclopropyl, cyclobutyl or cyclopentyl;

alternatively, $R_B$ and $R_2$ together with the atoms attached thereto form a $C_{3-6}$ carbocyclic ring or a 4- to 7-membered heterocyclic ring; the carbocyclic ring or heterocyclic ring is further substituted with 0 to 4 substituents selected from H, halogen, =O, OH, CN, $NH_2$, $C_{1-4}$ alkyl, $C_{3-6}$ cycloalkyl or $C_{1-4}$ alkoxy; the alkyl, cycloalkyl or alkoxy is optionally further substituted with 0 to 4 substituents selected from H, halogen, =O, OH, CN, $NH_2$, $C_{1-4}$ alkyl, $C_{3-6}$ cycloalkyl or $C_{1-4}$ alkoxy; the heterocyclic ring contains 1 to 3 heteroatoms selected from O, S and N;

alternatively, $R_C$ and $R_L$ together with the atoms attached thereto form a $C_{3-6}$ carbocyclic ring or a 4- to 7-membered heterocyclic ring; the carbocyclic ring or heterocyclic ring is further substituted with 0 to 4 substituents selected from H, halogen, =O, OH, CN, $NH_2$, $C_{1-4}$ alkyl, $C_{3-6}$ cycloalkyl or $C_{1-4}$ alkoxy; the alkyl, cycloalkyl or alkoxy is optionally further substituted with 0 to 4 substituents selected from H, halogen, =O, OH, CN, $NH_2$, $C_{1-4}$ alkyl, $C_{3-6}$ cycloalkyl or $C_{1-4}$ alkoxy; the heterocyclic ring contains 1 to 3 heteroatoms selected from O, S and N.

**4.** The compound or the stereoisomer, tautomer, deuterated substance, solvate, prodrug, metabolite, pharmaceutically acceptable salt or co-crystal thereof according to claim 3, wherein,

L is selected from a bond, O, S, -$NR_L$-, -$CHR_L$-, -$CHR_L$-$CHR_L$-, -Y-$CHR_L$-, -$CHR_L$-Y-, -Y-$CHR_L$-$CHR_L$-, -$CHR_L$-$CHR_L$-Y- or -$CHR_L$-Y-$CHR_L$-;

each $R_L$ is independently selected from H, F, methyl, ethyl or propyl;

Y is selected from O, S or -$NR_L$-;

ring B is selected from one of the following substituted or unsubstituted groups: cyclohexyl, cyclohexenyl,

azacyclohexenyl, piperidinyl, phenyl, pyrazolyl, pyridyl, pyridazinyl, pyrimidinyl, pyrazinyl or triazinyl, which, when substituted, is optionally further substituted with 0 to 4 $R_B$;

or ring B is selected from one of the following substituted or unsubstituted groups: piperazinyl, tetrahydropyrrolyl or 1,4-diazepanyl, which, when substituted, is optionally further substituted with 0 to 4 $R_B$;

ring C is selected from one of the following substituted or unsubstituted groups: a benzene ring, a pyrrole ring, a pyrazole ring, a pyridine ring, a furan ring, a thiophene ring, an imidazole ring, a thiazole ring, a oxazole ring, an isothiazole ring, an isoxazole ring, a triazole ring, a tetrazole ring, a oxadiazole ring, a thiadiazole ring, a pyridazine ring, a pyrimidine ring, a pyrazine ring or a triazine ring, which, when substituted, is optionally further substituted with 0 to 4 $R_C$;

ring D is selected from one of the following substituted or unsubstituted groups: a benzene ring, a naphthalene ring, a pyrrole ring, a pyrazole ring, a pyridine ring, a furan ring, a thiophene ring, an imidazole ring, a thiazole ring, a oxazole ring, an isothiazole ring, an isoxazole ring, a triazole ring, a oxadiazole ring, a thiadiazole ring, a pyridazine ring, a pyrimidine ring, a pyrazine ring or a triazine ring, which, when substituted, is optionally further substituted with 0 to 4 $R_D$;

when r=1,

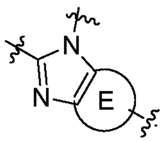

is selected from

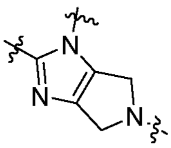;

when r=0,

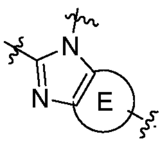

is selected from

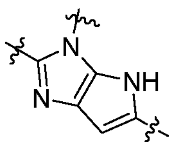, 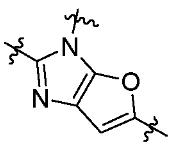, 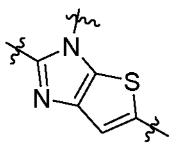,

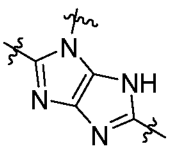, 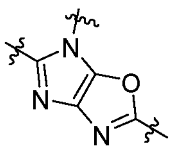, 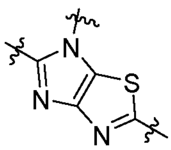, 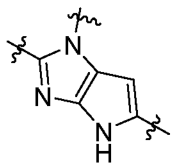, 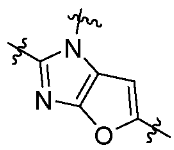, 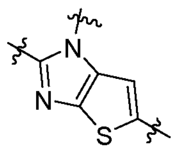,

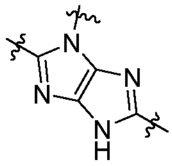, 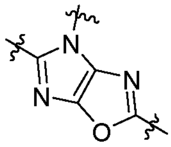 or 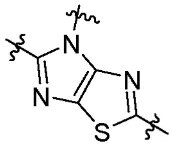.

5. The compound or the stereoisomer, tautomer, deuterated substance, solvate, prodrug, metabolite, pharmaceutically acceptable salt or co-crystal thereof according to claim 4, wherein,

L is selected from -Y-$CHR_L$-, -$CHR_L$-Y-, -$CHR_L$-$CHR_L$-, -$CHR_L$-$CHR_L$-Y- or -Y-$CHR_L$-$CHR_L$-;
Y is selected from O, S or -$NR_L$-;
each $R_L$ is independently selected from H or methyl;
ring B is selected from one of the following substituted or unsubstituted groups:

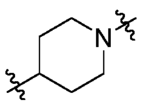, 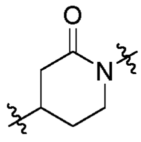, 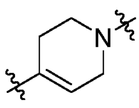, 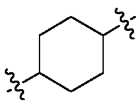, 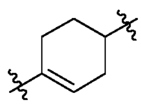, 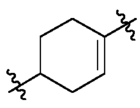 or 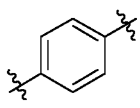,

the left side of which is directly connected to ring C, and which, when substituted, is optionally further substituted with 1, 2 or 3 $R_B$;
or ring B is selected from one of the following substituted or unsubstituted groups:

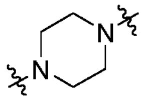, 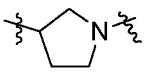 or 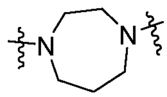,

the left side of which is directly connected to ring C, and which, when substituted, is optionally further substituted with 1, 2 or 3 $R_B$;
or ring B is selected from one of the following substituted or unsubstituted groups:

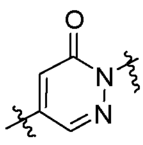, 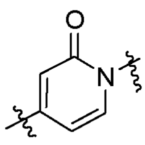, 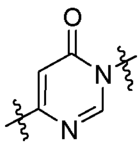, 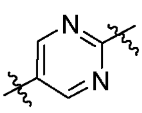, 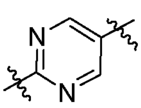, 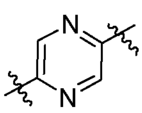, 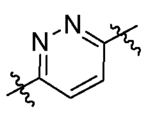,

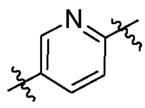 or 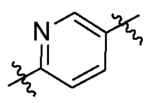,

the left side of which is directly connected to ring C, and which, when substituted, is optionally further substituted with 1, 2 or 3 $R_B$;
or

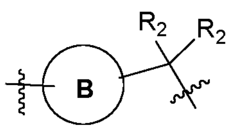

is selected from

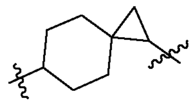 or 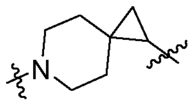 ,

the left side of which is directly connected to ring C, and which, when the 6-membered ring in the spiro ring is substituted, is optionally further substituted with 1, 2 or 3 $R_B$;

ring C is selected from one of the following substituted or unsubstituted groups: a benzene ring,

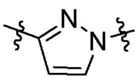 , 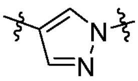 , 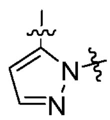 , 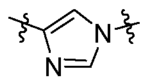 , 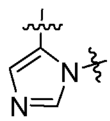 , 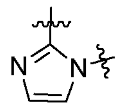 ,

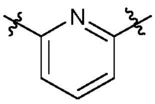 , 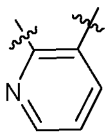 , 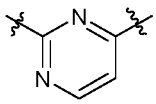 , 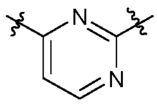 , 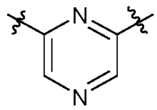 , 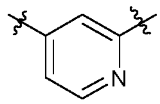 , 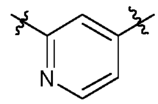

or

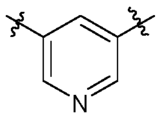 ,

which, when substituted, is optionally further substituted with 1, 2 or 3 Rc, and the left side of which is connected to L;

ring D is selected from a benzene ring or a pyridine ring, and the benzene ring or pyridine ring is optionally further substituted with 1, 2 or 3 $R_D$;

each $R_B$ is independently selected from H, F, =O, OH, CN, $CF_3$, methyl, ethyl, propyl, cyclopropyl, cyclobutyl, cyclopentyl, methoxy or ethoxy;

each $R_C$ is independently selected from H, F, Cl, OH, CN, $NH_2$, $CF_3$, methyl, ethyl, propyl, cyclopropyl, cyclobutyl, cyclopentyl, methoxy or ethoxy;

each $R_D$ is independently selected from H, F, Cl, CN, $CF_3$, $CHF_2$, methyl, ethyl, methoxy, ethoxy, tert-butyloxy or trifluoromethoxy;

alternatively, $R_C$ and $R_L$ together with the atoms attached thereto form a 4- to 7-membered heterocyclic ring; the heterocyclic ring is further substituted with 0, 1, 2, 3 or 4 substituents selected from H, F, methyl or ethyl; the heterocyclic ring contains 1 to 2 heteroatoms selected from O, S and N.

**6.** The compound or the stereoisomer, tautomer, deuterated substance, solvate, prodrug, metabolite, pharmaceutically acceptable salt or co-crystal thereof according to claim 5, wherein,

L is selected from $-CH_2O-$, and the right side of L is connected to ring C;

$R_1$ is selected from H, CN, $CF_3$, $CHF_2$, $CH_2F$, $-CH_2OH$, $-CH(OH)CH_3$, methyl, ethyl, methoxy, ethoxy, propoxy, isopropoxy, methoxymethyl, methoxyethyl, ethoxymethyl, isopropoxymethyl, -ethylene-Z-methylene-$R_{1a}$, -ethylene-Z-ethylene-$R_{1a}$, -ethylene-Z-$R_{1a}$, -$R_{1a}$ or -methylene-$R_{1a}$;

Z is selected from NH, $N(CH_3)$ or O;

$R_{1a}$ is selected from methyl, ethyl, isopropyl, propyl, methoxy, ethoxy, propoxy, isopropoxy, cyclopropyl, cyclobutyl, cyclopentyl, cyclohexyl, oxiranyl, oxetanyl, azetidinyl, tetrahydrofuranyl, tetrahydro-2*H*-pyranyl, pyrrolidinyl, piperidinyl, pyrazolyl, 1,2,3-triazolyl, 1,2,4-triazolyl, imidazolyl, thiazolyl, thienyl, furyl, oxazolyl, isoxazolyl, 1,2,4-oxadiazolyl, isothiazolyl, pyrimidyl, pyridine or phenyl, the $R_{1a}$ is optionally further substituted with 0, 1, 2 or 3 substituents selected from H, F, CN, methyl, ethyl, propyl, $CF_3$, methoxy or ethoxy;

ring C is selected from one of the following substituted or unsubstituted groups:

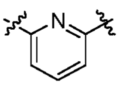 or 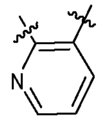,

which, when substituted, is optionally further substituted with 1, 2 or 3 $R_C$, and the left side of which is connected to L;

or

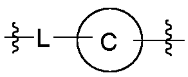

is selected from

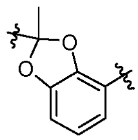,

the right side of which is connected to ring B, and in which the benzene ring is optionally further substituted with 1, 2 or 3 Rc;

ring D is selected from a benzene ring or pyridine, and the benzene ring or pyridine is optionally further substituted with 1, 2 or 3 $R_D$;

each $R_B$ is independently selected from H, =O, F, CN, $CF_3$, methyl, ethyl or cyclopropyl;

each $R_C$ is independently selected from H, F, Cl, CN, $CF_3$, methyl, ethyl, methoxy or ethoxy;

each $R_D$ is independently selected from H, F, Cl, CN, $CF_3$, $CHF_2$, methyl or ethyl.

**7.** The compound or the stereoisomer, tautomer, deuterated substance, solvate, prodrug, metabolite, pharmaceutically acceptable salt or co-crystal thereof according to claim 6, wherein,

$R_1$ is selected from

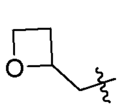, 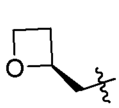, 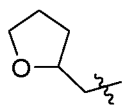 or 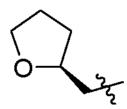;

$R_2$ is selected from H;

ring B is selected from one of the following substituted or unsubstituted groups:

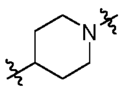, 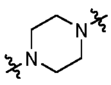, 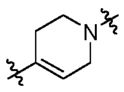, 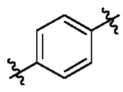 or 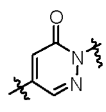,

the left side of which is directly connected to ring C, and which, when substituted, is optionally further substituted with 1, 2 or 3 $R_B$;

or

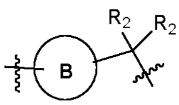

is selected from

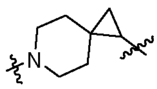,

the left side of which is directly connected to ring C, and which, when the 6-membered ring in the spiro ring is substituted, is optionally further substituted with 1, 2 or 3 $R_B$;
r=0,

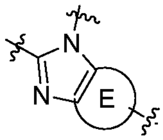

is selected from

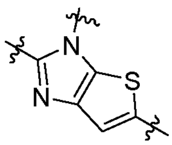, 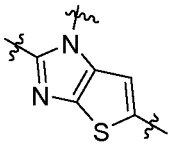 or 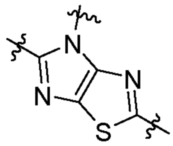.

**8.** The compound or the stereoisomer, tautomer, deuterated substance, solvate, prodrug, metabolite, pharmaceutically acceptable salt or co-crystal thereof according to claim 1, wherein the compound is selected from one of the following structures:

| | |
|---|---|
| 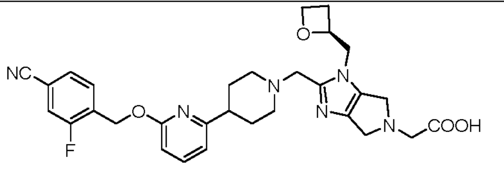 | 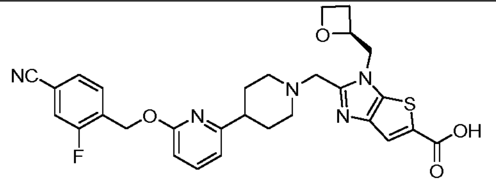 |
| 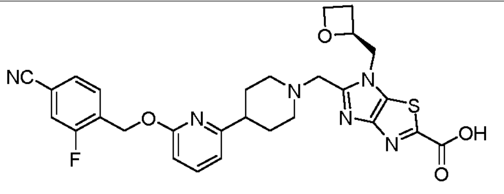 | 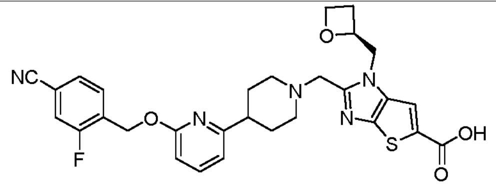 |
| 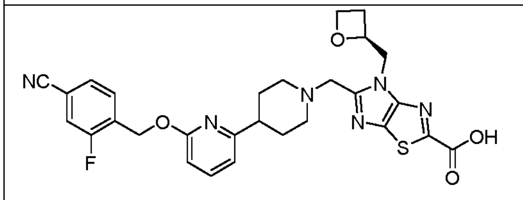 | 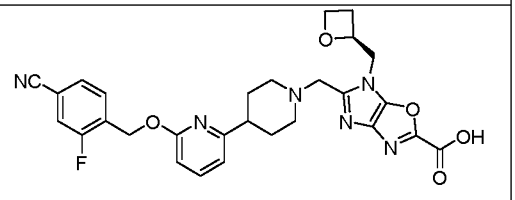 |
| 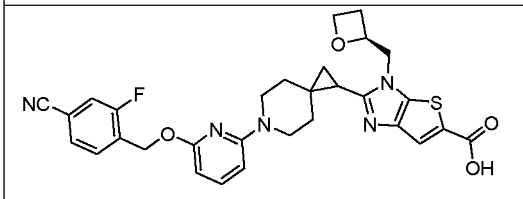 | 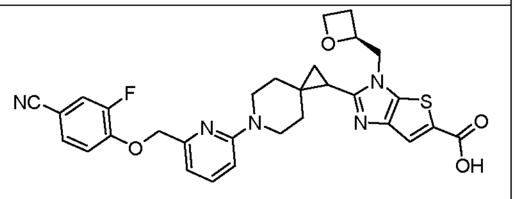 |
| 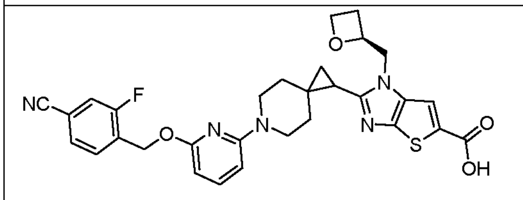 | 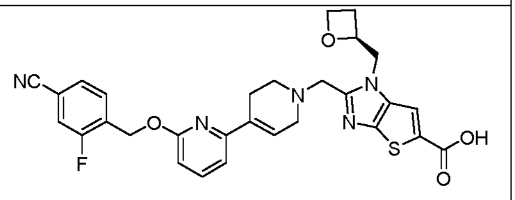 |
| 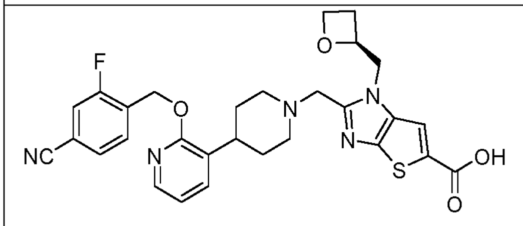 | 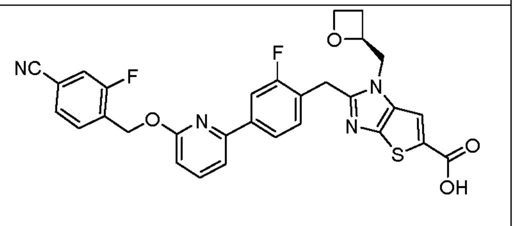 |
| 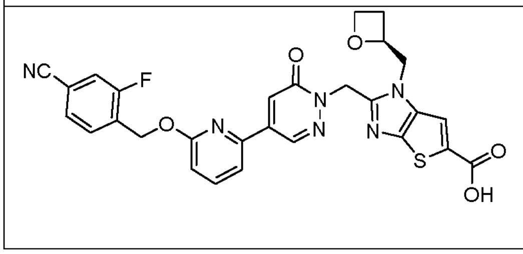 | 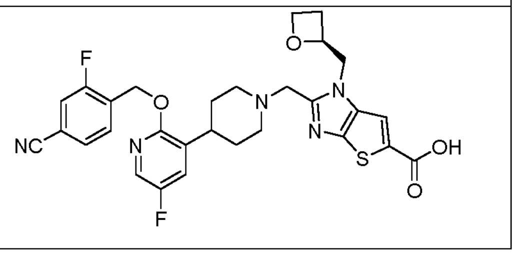 |

| | |
|---|---|
| 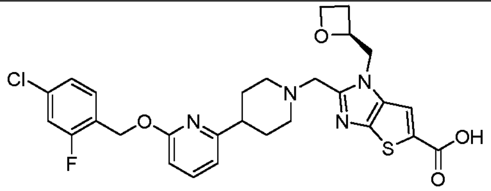 | 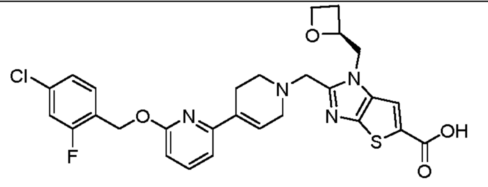 |
| 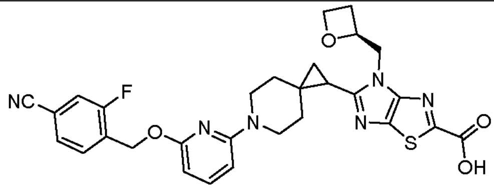 | 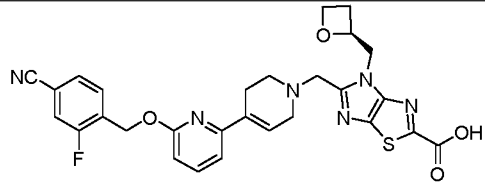 |
| 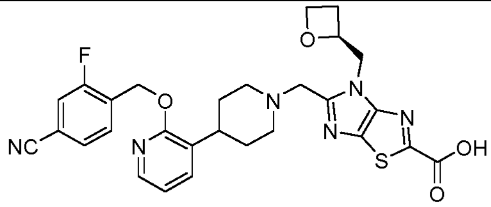 | 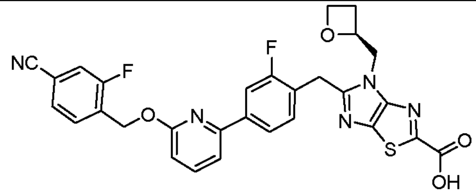 |
| 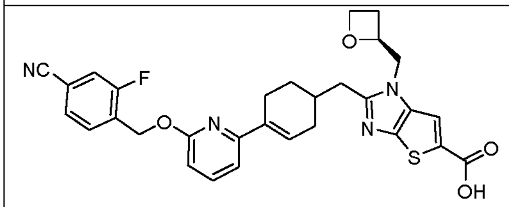 | 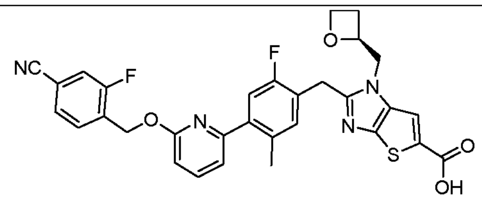 |
| 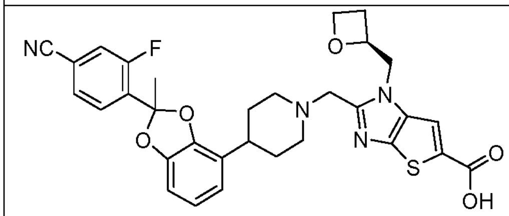 | 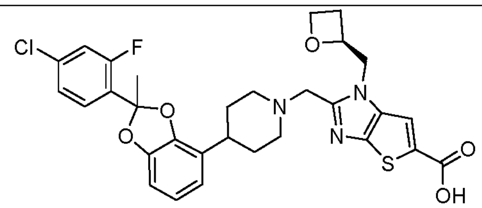 |
| 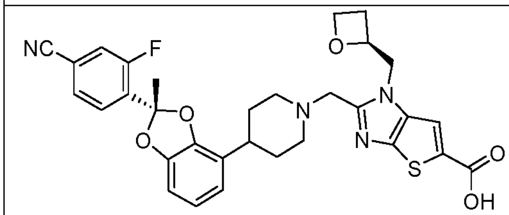 | 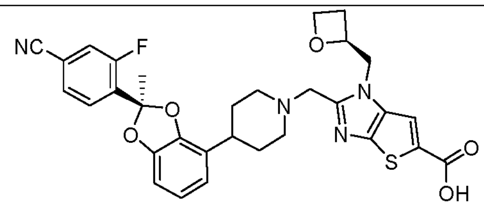 |
| 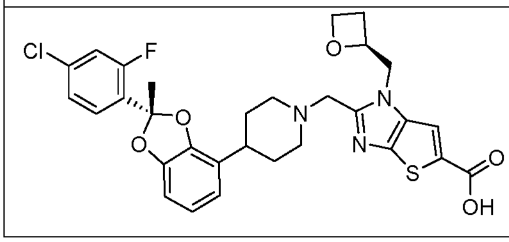 | 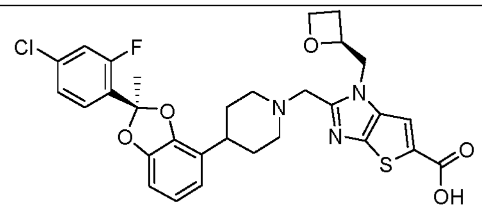 |

| | |
|---|---|
| 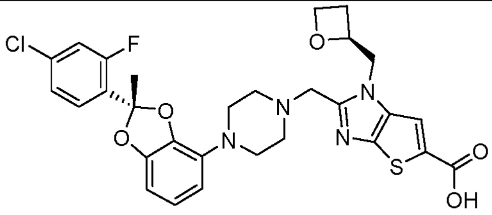 | 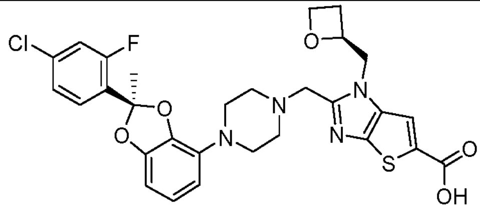 |
| 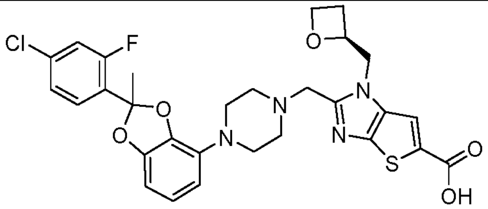 | 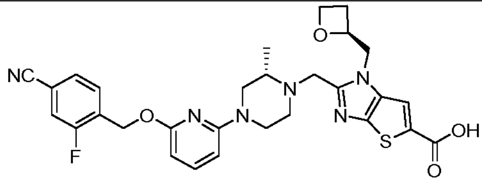 |
| 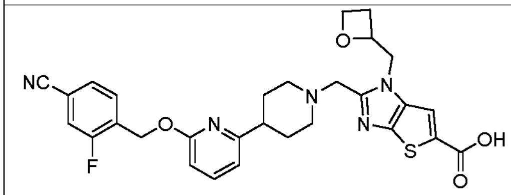 | 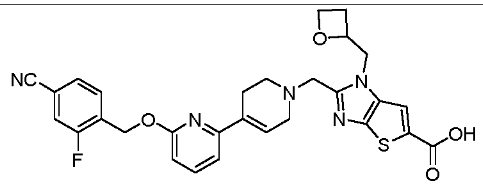 |
| 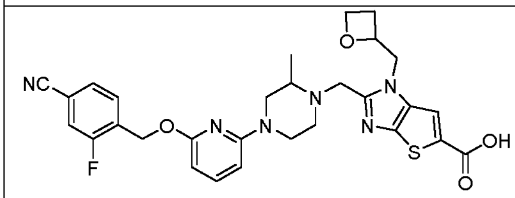 | 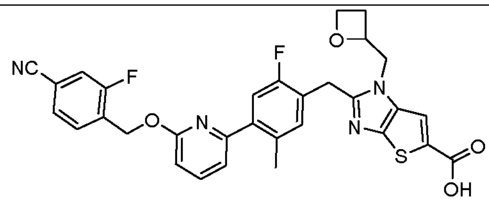 |
| 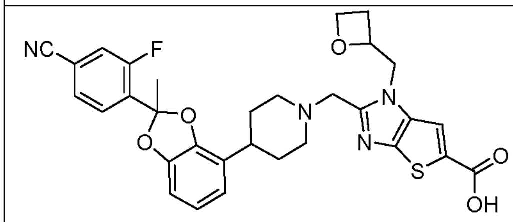 | 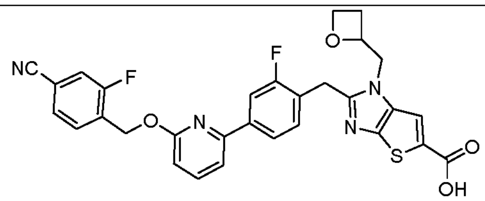 |
| 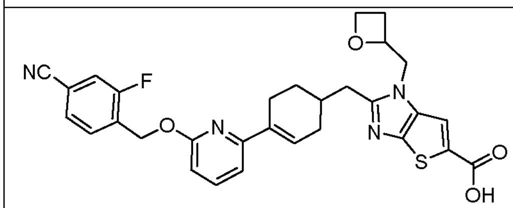 | 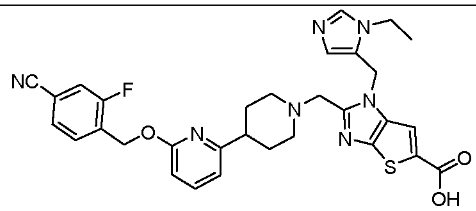 |
| 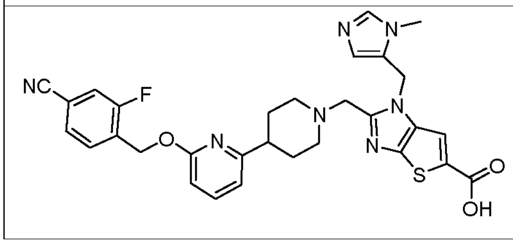 | 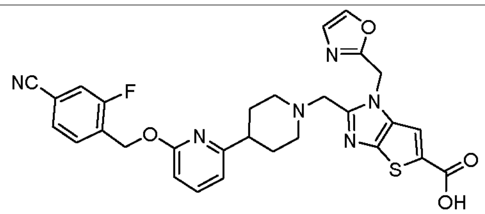 |

| | |
|---|---|
| 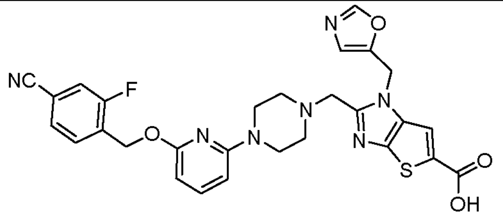 | 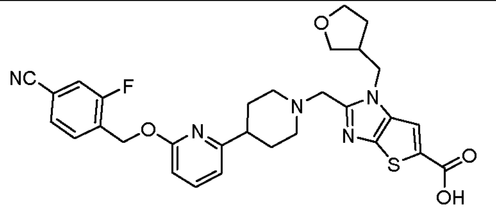 |
| 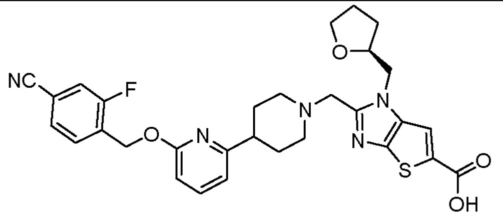 | 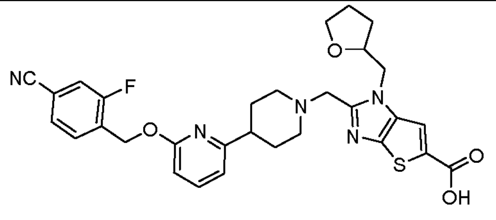 |
| 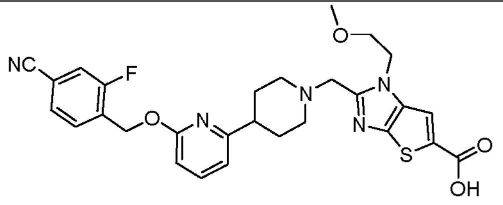 | 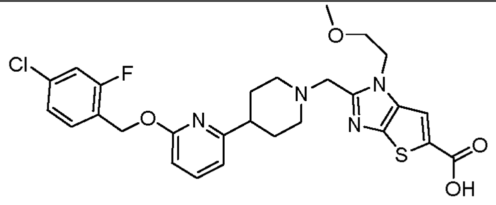 |
| 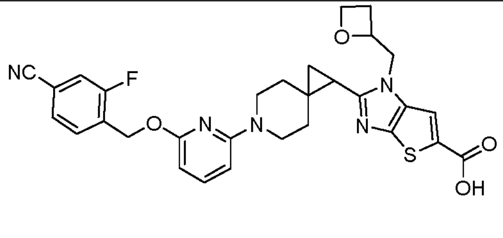 | 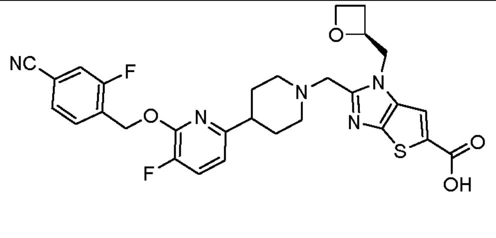 |
| 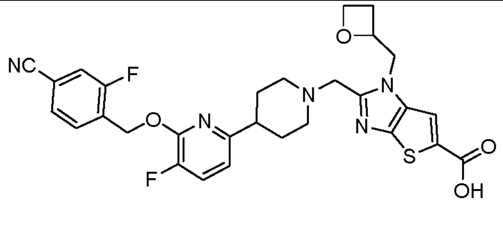 | 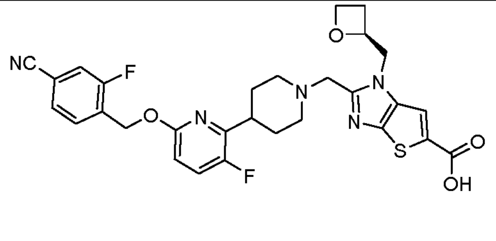 |
| 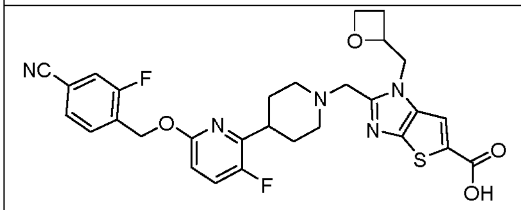 | 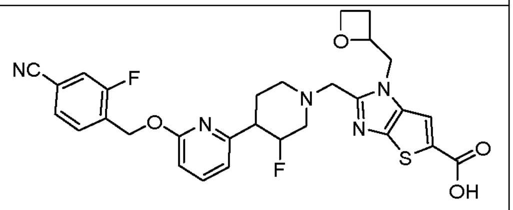 |
| 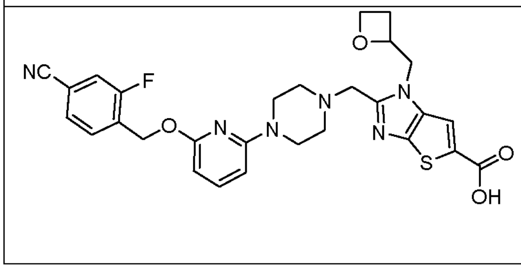 | 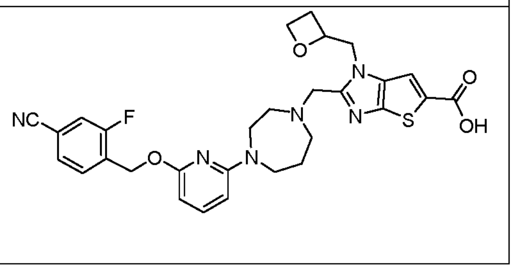 |

| | |
|---|---|
| 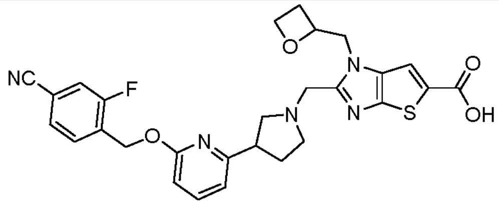 | 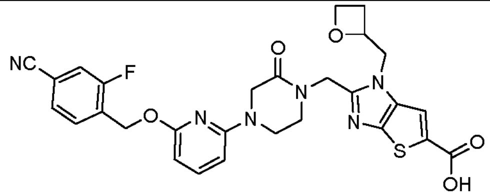 |
| 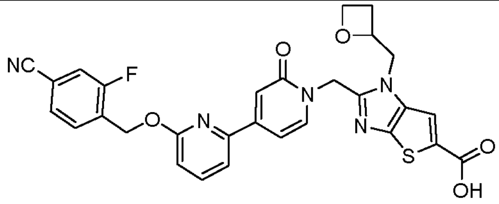 | 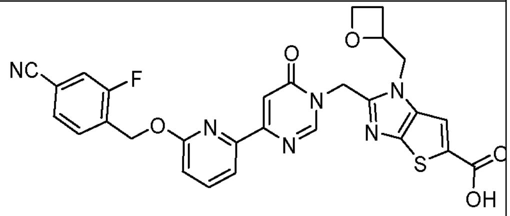 |
| 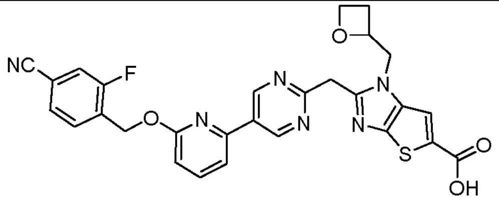 | 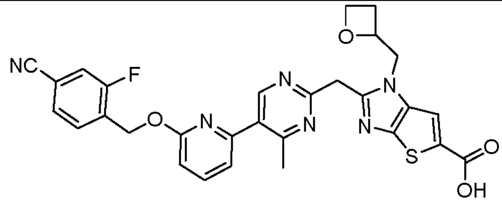 |
| 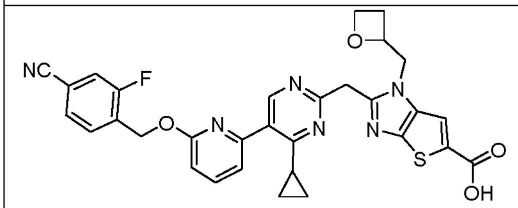 | 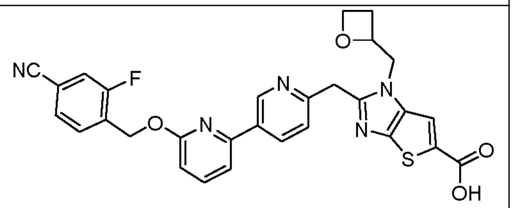 |
| 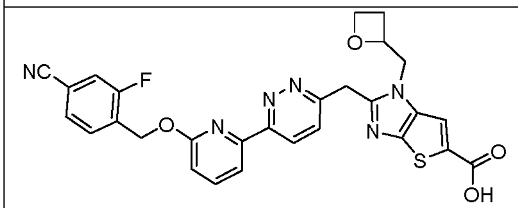 | 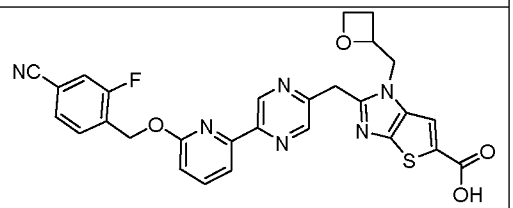 |
| 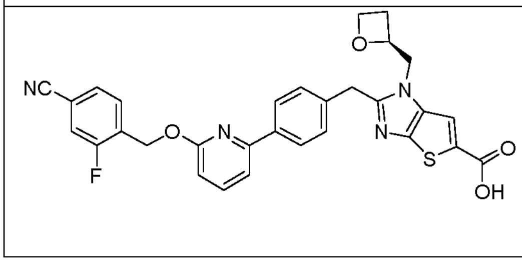 | 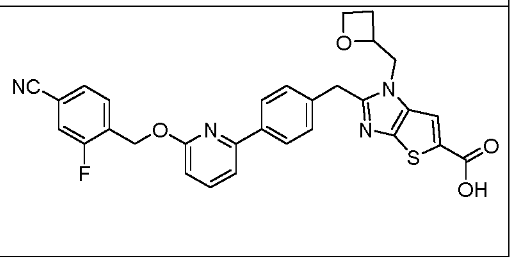 |

| | |
|---|---|
| 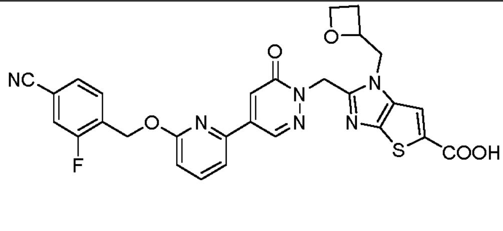 | 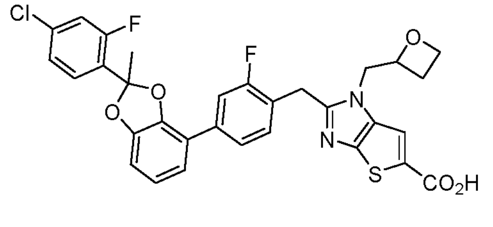 |
| 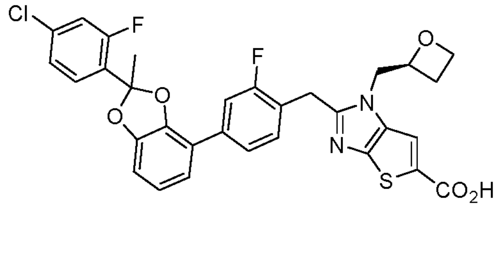 | 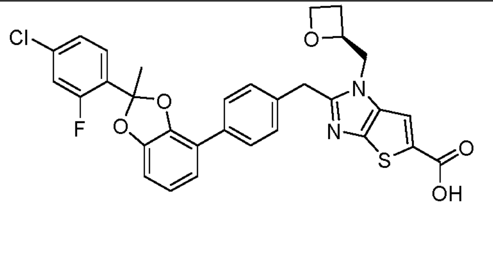 |
| 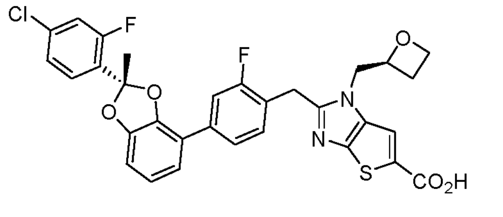 | 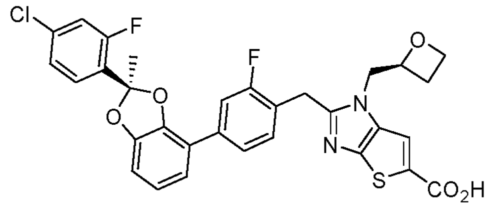 |
| 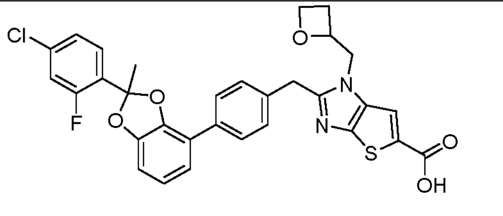 | 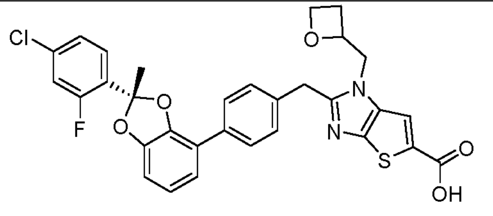 |
| 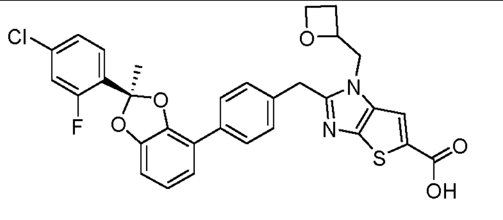 | 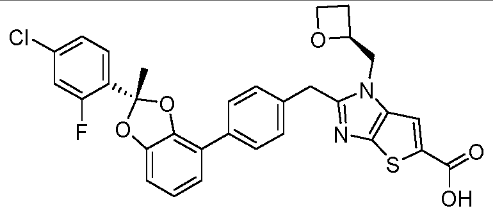 |
| 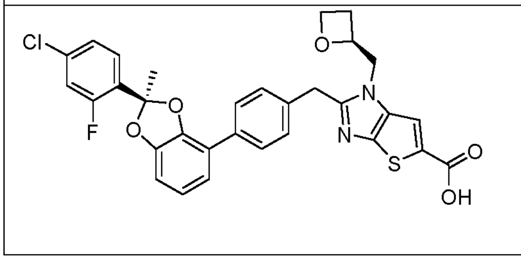 | 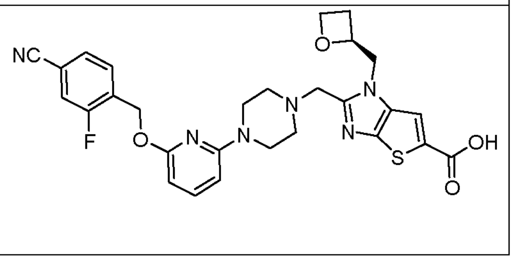 |

| | |
|---|---|
| 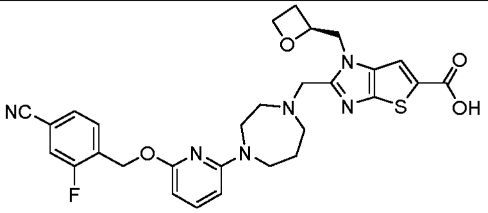 | 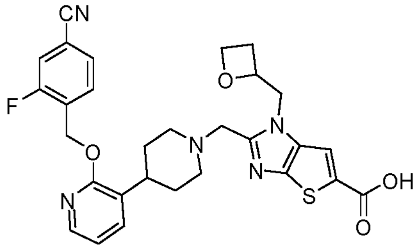 |
| 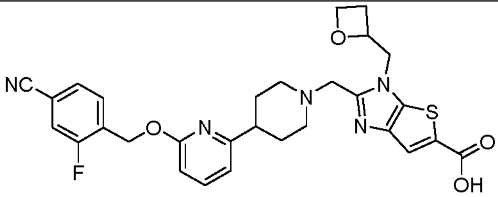 | 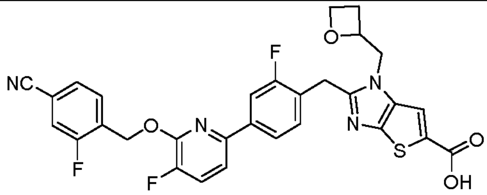 |
| 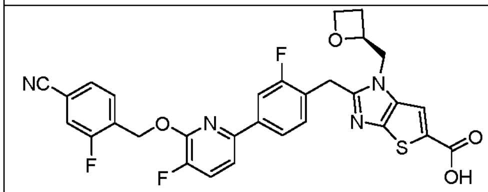 | 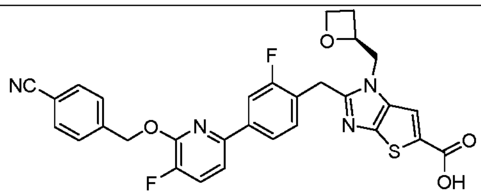 |
| 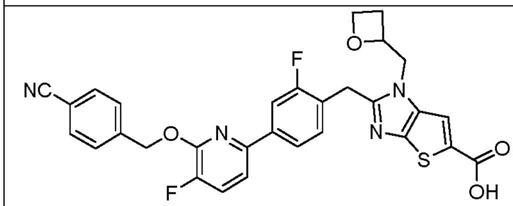 | 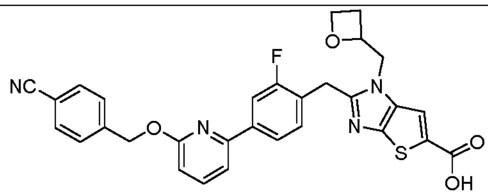 |
| 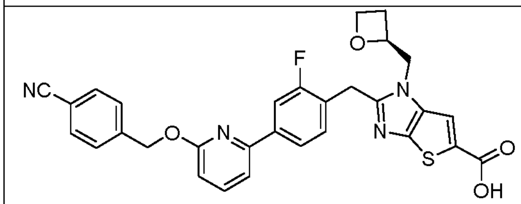 | 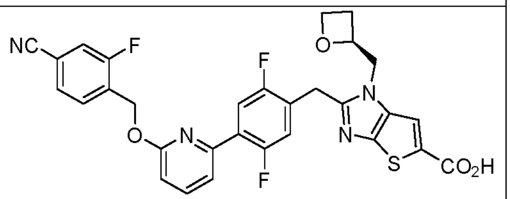 |
| 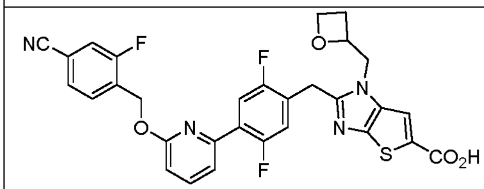 | 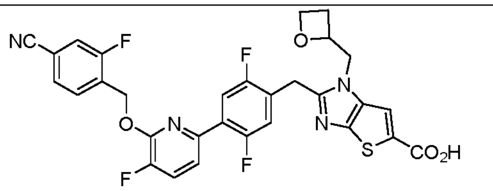 |
| 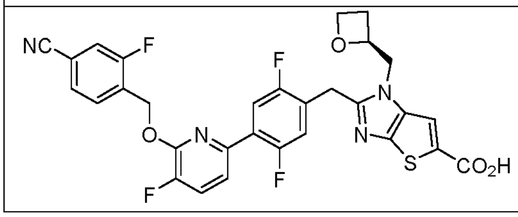 | 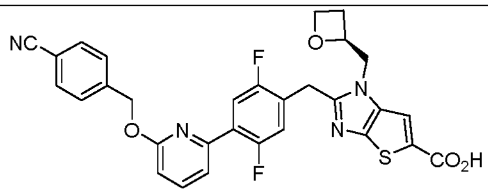 |

| | |
|---|---|
| 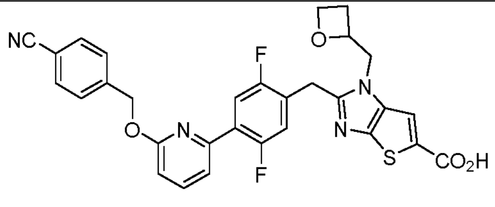 | 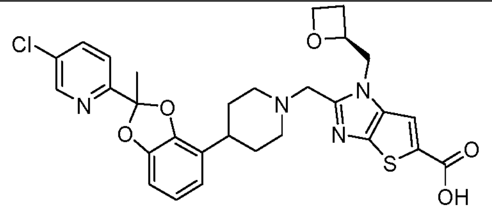 |
| 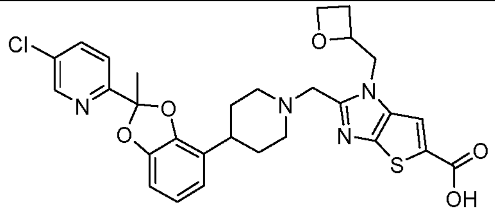 | 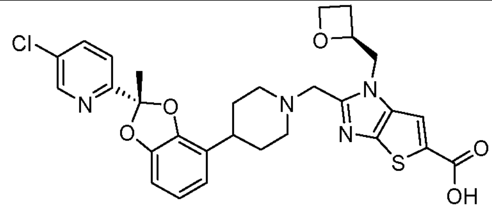 |
| 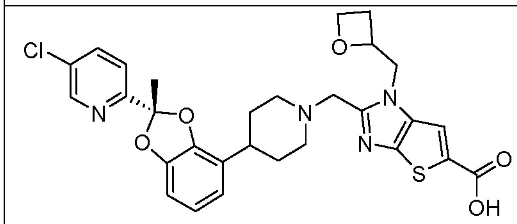 | 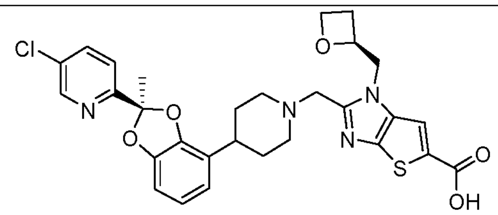 |
| 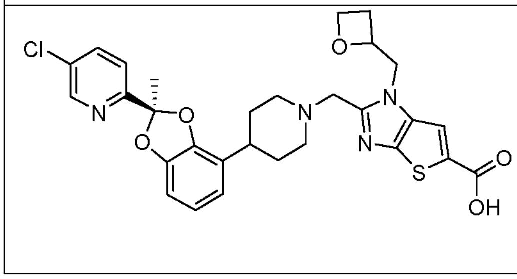 | 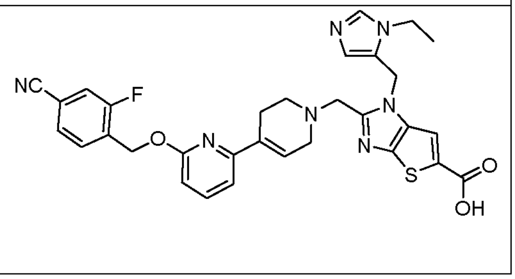 |

.

**9.** A pharmaceutical composition, comprising the compound or the stereoisomer, tautomer, deuterated substance, solvate, prodrug, metabolite, pharmaceutically acceptable salt or co-crystal thereof according to any one of claims 1 to 8, and a pharmaceutically acceptable carrier.

**10.** Use of the compound or the stereoisomer, tautomer, deuterated substance, solvate, prodrug, metabolite, pharmaceutically acceptable salt or co-crystal thereof according to any one of claims 1 to 8 or the pharmaceutical composition according to claim 9 in the preparation of a drug for treating diabetes.

**11.** A compound, comprising the following structure:

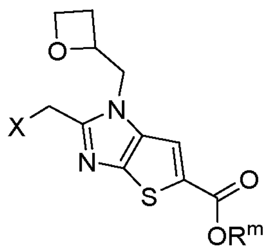

wherein X is selected from H or Br;
$R^m$ is selected from H or $C_{1-4}$ alkyl; the alkyl is optionally further substituted with 0 to 4 substituents selected from halogen, $C_{1-4}$ alkyl, $C_{1-4}$ alkoxy or $C_{6-10}$ carbocyclic ring.

## INTERNATIONAL SEARCH REPORT

International application No.
**PCT/CN2021/141243**

**A. CLASSIFICATION OF SUBJECT MATTER**

C07D 401/04(2006.01)i; C07D 513/04(2006.01)i; C07D 413/14(2006.01)i; C07D 495/04(2006.01)i; C07D 405/14(2006.01)i; A61K 31/497(2006.01)i; A61P 3/10(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNKI, CNABS, CNTXT, WPI, EPODOC, CAPLUS, REGISTRY, CASREACT, MARPAT: 咪唑, 噻吩, 稠环, 稠合, GLP-1, 胰岛素, imidazolyl, thienyl, fused, structure search, 海思科制药有限公司

**C. DOCUMENTS CONSIDERED TO BE RELEVANT**

| Category* | Citation of document, with indication, where appropriate, of the relevant passages | Relevant to claim No. |
|---|---|---|
| X | WO 2020207474 A1 (QILU REGOR THERAPEUTICS INC.) 15 October 2020 (2020-10-15) description page 7 line 1 to line 15, description page 26 line 12 to line 19, embodiment 4 compound 74 | 1-10 |
| A | WO 2020207474 A1 (QILU REGOR THERAPEUTICS INC.) 15 October 2020 (2020-10-15) description page 7 line 1 to line 15, description page 26 line 12 to line 19, embodiment 4 compound 74 | 11 |
| PX | WO 2021249492 A1 (MEDSHINE DISCOVERY INC.) 16 December 2021 (2021-12-16) description, page 2, line 1 to page 12, line 5, page 19, last paragraph, paragraphs 8-5 | 1-11 |
| PX | WO 2021244645 A1 (SCIWIND BIOSCIENCES CO., LTD. et al.) 09 December 2021 (2021-12-09) description page 2 paragraph 4 to page 5 paragraph 2, embodiments 1-11 | 1-11 |
| A | CN 110325530 A (PFIZER INC.) 11 October 2019 (2019-10-11) entire document | 1-11 |
| A | WO 2020103815 A1 (QILU REGOR THERAPEUTICS INC.) 28 May 2020 (2020-05-28) entire document | 1-11 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 March 2022** | **22 March 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/141243**

**C. DOCUMENTS CONSIDERED TO BE RELEVANT**

| Category* | Citation of document, with indication, where appropriate, of the relevant passages | Relevant to claim No. |
|---|---|---|
| A | CN 1747936 A (TRANSTECH PHARMA INC.) 15 March 2006 (2006-03-15)<br>entire document | 1-11 |

Form PCT/ISA/210 (second sheet) (January 2015)

**INTERNATIONAL SEARCH REPORT**
**Information on patent family members**

International application No.
**PCT/CN2021/141243**

| Patent document cited in search report | Publication date (day/month/year) | Patent family member(s) | Publication date (day/month/year) |
|---|---|---|---|
| WO 2020207474 A1 | 15 October 2020 | AU 2020256647 A1 | 18 November 2021 |
| | | EP 3953344 A1 | 16 February 2022 |
| | | PE 20220143 A1 | 27 January 2022 |
| | | CO 2021015060 A2 | 19 November 2021 |
| | | IL 287128 D0 | 01 December 2021 |
| | | DO P2021000208 A | 21 November 2021 |
| | | SG 11202110825 W A | 28 October 2021 |
| | | KR 20210152513 A | 15 December 2021 |
| | | CA 3136375 A1 | 15 October 2020 |
| WO 2021249492 A1 | 16 December 2021 | None | |
| WO 2021244645 A1 | 09 December 2021 | None | |
| CN 110325530 A | 11 October 2019 | UA 122035 C2 | 25 August 2020 |
| | | US 2019119255 A1 | 25 April 2019 |
| | | US 10669259 B2 | 02 June 2020 |
| | | EC SP19041071 A | 30 June 2019 |
| | | UY 37514 A | 31 July 2018 |
| | | MX 2019007077 A | 01 August 2019 |
| | | IL 267288 D0 | 29 August 2019 |
| | | IL 267288 A | 30 June 2021 |
| | | PH 12019501360 A1 | 10 February 2020 |
| | | AR 110387 A1 | 27 March 2019 |
| | | CL 2019001651 A1 | 04 October 2019 |
| | | TW 201835066 A | 01 October 2018 |
| | | TW I713809 B | 21 December 2020 |
| | | US 2020255406 A1 | 13 August 2020 |
| | | US 10851081 B2 | 01 December 2020 |
| | | US 2018170908 A1 | 21 June 2018 |
| | | US 10208019 B2 | 19 February 2019 |
| | | AU 2017374860 A1 | 20 June 2019 |
| | | AU 2017374860 B2 | 02 September 2021 |
| | | EP 3555064 A1 | 23 October 2019 |
| | | KR 20190094433 A | 13 August 2019 |
| | | KR 102314286 B1 | 21 October 2021 |
| | | JP 6637641 B1 | 29 January 2020 |
| | | JP 2020511411 A | 16 April 2020 |
| | | JP 2020063287 A | 23 April 2020 |
| | | JP 6982054 B2 | 17 December 2021 |
| | | US 2021047298 A1 | 18 February 2021 |
| | | CO 2019006046 A2 | 19 June 2019 |
| | | PE 20191501 A1 | 22 October 2019 |
| | | ZA 201904616 B | 26 May 2021 |
| | | BR 112019012211 A2 | 12 November 2019 |
| | | KR 20210127782 A | 22 October 2021 |
| | | DO P2019000166 A | 31 July 2019 |
| | | CR 20190289 A | 21 August 2019 |
| | | RU 2740135 C1 | 11 January 2021 |
| | | WO 2018109607 A1 | 21 June 2018 |
| | | CA 2988721 A1 | 16 June 2018 |
| | | EA 201991193 A1 | 15 January 2020 |
| | | EA 037318 B1 | 11 March 2021 |

Form PCT/ISA/210 (patent family annex) (January 2015)

**INTERNATIONAL SEARCH REPORT**
**Information on patent family members**

International application No.
**PCT/CN2021/141243**

| Patent document cited in search report | Publication date (day/month/year) | Patent family member(s) | Publication date (day/month/year) |
|---|---|---|---|
| | | CU 20190056 A7 | 03 January 2020 |
| | | GE P20217265 B | 25 June 2021 |
| WO 2020103815 A1 | 28 May 2020 | SG 11202105241 Y A | 29 June 2021 |
| | | TW 202039458 A | 01 November 2020 |
| | | AU 2019382642 A1 | 10 June 2021 |
| | | BR 112021009877 A2 | 17 August 2021 |
| | | JP 2022508203 A | 19 January 2022 |
| | | CN 113227068 A | 06 August 2021 |
| | | CR 20210341 A | 25 November 2021 |
| | | KR 20210106447 A | 30 August 2021 |
| | | US 2022024901 A1 | 27 January 2022 |
| | | US 2020283424 A1 | 10 September 2020 |
| | | US 10844049 B2 | 24 November 2020 |
| | | PH 12021551165 A1 | 25 October 2021 |
| | | IL 283322 D0 | 29 July 2021 |
| | | EP 3883928 A1 | 29 September 2021 |
| | | PE 20211871 A1 | 21 September 2021 |
| | | CA 3120499 A1 | 28 May 2020 |
| | | EA 202191432 A1 | 14 October 2021 |
| | | CO 2021008089 A2 | 09 September 2021 |
| CN 1747936 A | 15 March 2006 | JP 2006518738 A | 17 August 2006 |
| | | AU 2004210711 A1 | 26 August 2004 |
| | | AU 2004210711 B2 | 08 July 2010 |
| | | CA 2514363 A1 | 26 August 2004 |
| | | WO 2004071447 A2 | 26 August 2004 |
| | | WO 2004071447 A3 | 23 December 2004 |
| | | WO 2004071447 B1 | 10 March 2005 |
| | | WO 2004071447 A9 | 13 October 2005 |
| | | EP 1594847 A2 | 16 November 2005 |
| | | US 2011077399 A1 | 31 March 2011 |
| | | US 2004192743 A1 | 30 September 2004 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2020207474 A

### Non-patent literature cited in the description

- *CHEMICAL ABSTRACTS,* 5807-14-7